(19) Europäisches Patentamt
European Patent Office
Office européen des brevets

(11) EP 4 740 948 A1

(12) **EUROPEAN PATENT APPLICATION**
published in accordance with Art. 153(4) EPC

(43) Date of publication:
**13.05.2026 Bulletin 2026/20**

(21) Application number: **24835527.3**

(22) Date of filing: **04.07.2024**

(51) International Patent Classification (IPC):
***A61K 31/5377*** *(2006.01)* ***A61K 31/444*** *(2006.01)*
***A61K 31/506*** *(2006.01)* ***A61P 35/00*** *(2006.01)*

(52) Cooperative Patent Classification (CPC):
**A61K 31/443; A61K 31/4436; A61K 31/4439; A61K 31/444; A61K 31/4545; A61K 31/506; A61K 31/5377; A61K 31/541; A61P 35/00**

(86) International application number:
**PCT/IB2024/056539**

(87) International publication number:
**WO 2025/008783 (09.01.2025 Gazette 2025/02)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **06.07.2023 KR 20230087953**

(71) Applicants:
- **Dong Wha Pharm. Co., Ltd.**
  **Seoul 04516 (KR)**
- **Oncocross Co., Ltd.**
  **Seoul 05836 (KR)**

(72) Inventors:
- **PARK, Whui Jung**
  **Suwon-si Gyeonggi-do 16509 (KR)**
- **SHIN, Dong Hyuk**
  **Suwon-si Gyeonggi-do 16685 (KR)**
- **JEONG, Seong Su**
  **Pyeongtaek-si Gyeonggi-do 18030 (KR)**
- **JEONG, Seo Hee**
  **Suwon-si Gyeonggi-do 16698 (KR)**
- **LEE, Sang Ho**
  **Yongin-si Gyeonggi-do 17166 (KR)**
- **WOO, Ji Young**
  **Hwaseong-si Gyeonggi-do 18442 (KR)**
- **HEO, Woon**
  **Yongin-si Gyeonggi-do 17095 (KR)**
- **JEONG, Doc Gyun**
  **Yongin-si Gyeonggi-do 16918 (KR)**
- **OH, Yeon Kyung**
  **Seongnam-si Gyeonggi-do 13331 (KR)**
- **LIM, Jae Kyung**
  **Suwon-si Gyeonggi-do 16325 (KR)**
- **KIM, Seung Hwan**
  **Suwon-si Gyeonggi-do 16513 (KR)**
- **HWANG, Yun Ha**
  **Gunpo-si Gyeonggi-do 15822 (KR)**
- **KIM, Yi Rang**
  **Seoul 05836 (KR)**

(74) Representative: **D Young & Co LLP**
**3 Noble Street**
**London EC2V 7BQ (GB)**

(54) **PHARMACEUTICAL COMPOSITION COMPRISING 1,3-BENZODIOXOLE DERIVATIVE COMPOUND**

(57) The present invention relates to a pharmaceutical composition comprising a compound represented by chemical formula I, a stereoisomer thereof, a pharmaceutically acceptable salt thereof, a hydrate or solvate thereof.

EP 4 740 948 A1

## Description

### Technical Field

The present invention relates to a pharmaceutical composition including a compound represented by chemical formula I, a stereoisomer thereof, a pharmaceutically acceptable salt thereof, a hydrate or solvate thereof.

### Background

The chromosome changes its higher-order structure by methylation of its constituent DNA or various modifications (acetylation, methylation, phosphorylation, ubiquitination, etc.) of histones (histones H2A, H2B, H3, and H4), thereby dynamically controlling the replication or transcription of genes.

Generally, trimethylation (H3K4me3) of the fourth lysine from the N-terminus of histone H3 functions in a direction of activating transcription, and trimethylation (H3K27me3) of the 27th lysine functions in a direction of inhibiting transcription, in which the former is modified by a trithorax complex, and the latter is modified by a polycomb repressive complex 2 (PRC2).

A polycomb genogroup was identified as a gene which controls the embryonic development of fruit flies, which is also conserved in vertebrates. In fruit flies, the enhancer of zeste protein is a catalytic subunit responsible for H3K27 methylation variation of PRC2. In addition, both enhancer of zeste homolog 1 (EZH1, drosophila) and enhancer of zeste homolog 2 (EZH2, drosophila) are mammalian homologs of drosophila enhancer of zeste. The enzyme active (SET) domains of EZH1 and EZH2 have high homology, and two types of PRC2 (PRC2-EZH1, PRC2-EZH2) having EZH1 or EZH2 as a catalytic subunit are present in humans or mice.

In embryonic stem (ES) cells, EZH1 and EZH2 function cooperatively or complementarily to participate in the maintenance of the ES cells. EZH1 and EZH2 have been reported to cooperatively play a role in the formation and maintenance of hair follicles and differentiation of Merkel cells, and to play an important role in the maintenance of hematopoietic stem cells.

So far, there have been reports about hyperactivity of EZH2 expression in many cancers including prostate cancer, breast cancer, stomach cancer, colorectal cancer, lung cancer, ovarian cancer, pancreatic cancer, kidney cancer, head and neck cancer, and adrenal cancer, and among these cancers, a correlation between hyperactivity of EZH2 expression and poor prognosis has been reported.

It has been reported that knockdown of EZH2 in such cancer-derived cell lines inhibits cell proliferation. In addition, when EZH2 is overexpressed in an epithelial non-cancer cell line, a characteristic phenotype of cancer, such as invasion, cell proliferation hyperactivity in soft agar medium, etc., appears. This suggests that knockdown of EZH2 is useful in the treatment of cancers in which the expression of EZH2 has been enhanced.

SWI/SNF complex is a protein complex which has an opposite action of PRC2 in its chromatin control function, and has been reported to have many mutations in several carcinomas. Double removal or inhibition of EZH1/EZH2 may affect an SWI/SNF complex-linked chromatin rearrangement through simultaneous inhibition of EZH1/EZH2, such as observation that ARID1A, SMARCA4, and SMARCB1, which are constituents of the SWI/SNF complex, are rapidly introduced into chromatin formation. Thus, it means that there is a need for simultaneous inhibition of EZH1/EZH2 for solid cancers with mutations such as knockdown, knockout or the like in the SWI/SNF complex.

### Detailed Description of the Invention

### Technical Problem

The present invention provides a 1,3-benzodioxole derivative compound represented by chemical formula I, a stereoisomer thereof, a pharmaceutically acceptable salt thereof, a hydrate or solvate thereof.

The present invention provides a pharmaceutical composition for preventing or treating EZH1 or EZH2 activity-associated diseases, including a compound represented by chemical formula I, a stereoisomer thereof, a pharmaceutically acceptable salt thereof, a hydrate or solvate thereof.

The present invention provides a method for preventing or treating EZH1 or EZH2 activity-associated diseases, including administering a compound represented by chemical formula I, a stereoisomer thereof, a pharmaceutically acceptable salt thereof, a hydrate or solvate thereof into an individual.

The present invention provides a use of a compound represented by chemical formula I, a stereoisomer thereof, a pharmaceutically acceptable salt thereof, a hydrate or solvate thereof for preventing or treating EZH1 or EZH2 activity-associated diseases.

The present invention provides a use of a compound represented by chemical formula I, a stereoisomer thereof, a pharmaceutically acceptable salt thereof, a hydrate or solvate thereof in the preparation of a medicament for preventing or

treating EZH1 or EZH2 activity-associated diseases.

**Technical Solution**

Hereinafter, the present invention will be described in more detail. All the combinations of various elements disclosed in the present invention may fall within the scope of the present invention. In addition, it cannot be seen that the scope of the present invention is limited to the specific description below.

This work was supported by the Technology Innovation Program (or Industrial Strategic Technology Development Program-The Mid-sized Enterprise Co-Innovation Program) (R&D, P0020651, Non-clinical and indication expansion research to develop EZH1/2 inhibitory anticancer drugs) funded By the Ministry of Trade, Industry & Energy (MOTIE, Korea) and Korea Institute for Advancement of Technology (KIAT). The grant number may be used in combination as "1415189559."

**1,3-benzodioxole derivative compound, stereoisomer thereof, pharmaceutically acceptable salt thereof, hydrate or solvate thereof**

The present invention provides a compound according to any one of (1) to (5) below, a stereoisomer thereof, a pharmaceutically acceptable salt thereof, a hydrate or solvate thereof.

(1) A compound represented by chemical formula I below, a stereoisomer thereof, a pharmaceutically acceptable salt thereof, a hydrate or solvate thereof:

<Chemical Formula I>

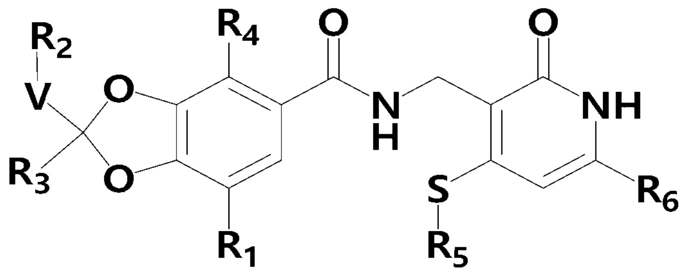

in the chemical formula I, it may be provided that

$R_1$ is H; $C_1$-$C_6$ alkyl; $C_2$-$C_6$ alkenyl; $C_2$-$C_6$ alkynyl; $C_3$-$C_8$ cycloalkyl; $C_3$-$C_8$ cycloalkenyl; $C_1$-$C_6$ alkoxy; 3 to 12-membered heterocycloalkyl or heterocycloalkenyl including in a ring one to three heteroatoms independently selected from the group consisting of N, O, and S; 6 to 14-membered aryl; 5 to 12-membered heteroaryl including in a ring one to three heteroatoms independently selected from the group consisting of N, O and S; -(C=O)-($C_1$-$C_6$ alkyl); -CN; benzodioxolyl or halogen,

in the $R_1$, at least one H of $C_1$-$C_6$ alkyl; $C_3$-$C_8$ cycloalkyl; 3 to 12-membered heterocycloalkyl or heterocycloalkenyl including in a ring one to three heteroatoms independently selected from the group consisting of N, O, and S; 6 to 14-membered aryl; 5 to 12-membered heteroaryl including in a ring one to three heteroatoms independently selected from the group consisting of N, O and S or benzodioxolyl may be substituted or unsubstituted with Ra,

the Ra is $C_1$-$C_6$ alkyl; $C_1$-$C_6$ alkoxy; 3 to 12-membered heterocycloalkyl including in a ring one to three heteroatoms independently selected from the group consisting of N, O, and S; -NRxRy; or halogen,

at least one H of the Ra may be substituted or unsubstituted with Rb,

the Rb is $C_1$-$C_6$ alkyl; 3 to 12-membered heterocycloalkyl including in a ring one to three heteroatoms independently selected from the group consisting of N, O, and S; or halogen,

$R_2$ is H; $C_1$-$C_6$ alkyl; $C_3$-$C_8$ cycloalkyl; 3 to 12-membered heterocycloalkyl or heterocycloalkenyl including in a ring one to three heteroatoms independently selected from the group consisting of N, O, and S; 6 to 14-membered aryl; or 5 to 12-membered heteroaryl including in a ring one to three heteroatoms independently selected from the group consisting of N, O and S,

at least one H of the $R_2$ may be substituted or unsubstituted with Rc,

the Rc is $C_1$-$C_6$ alkyl; $C_3$-$C_8$ cycloalkyl; 3 to 12-membered heterocycloalkyl including in a ring one to three heteroatoms independently selected from the group consisting of N, O, and S; -NRpRq; -(HC=O); -(C=O)-($C_1$-$C_6$ alkyl); -S(=O)$_2$-($C_1$-$C_6$ alkyl); or halogen,

at least one H of the Rc may be substituted or unsubstituted with Rd,
the Rd is $C_1$-$C_6$ alkoxy which may be substituted or unsubstituted with halogen; 3 to 12-membered heterocycloalkyl including in a ring one to three heteroatoms independently selected from the group consisting of N, O, and S; 6 to 14-membered aryl; halogen; or -OH,
$R_3$ is H; or $C_1$-$C_6$ alkyl,
$R_4$ is H; $C_1$-$C_6$ alkyl; or halogen,
$R_5$ and $R_6$ are each independently H; or $C_1$-$C_6$ alkyl,
Rx and Ry are each independently H; or $C_1$-$C_6$ alkyl,
Rp and Rq are each independently H; $C_1$-$C_6$ alkyl, $C_3$-$C_8$ cycloalkyl or -(C=O)-($C_1$-$C_6$ alkyl), and
V is a single bond; or -($C_1$-$C_6$ alkylene)-.

(2) With regard to above (1), in the chemical formula I, it may be provided that

$R_1$ is 6 to 14-membered aryl; 5 to 12-membered heteroaryl including in a ring one to three heteroatoms independently selected from the group consisting of N, O and S or benzodioxolyl,
at least one H of the $R_1$ may be substituted or unsubstituted with Ra,
the Ra is $C_1$-$C_6$ alkyl; $C_1$-$C_6$ alkoxy; 3 to 12-membered heterocycloalkyl including in a ring one to three heteroatoms independently selected from the group consisting of N, O, and S; -NRxRy; or halogen,
at least one H of the Ra may be substituted or unsubstituted with Rb,
the Rb is $C_1$-$C_6$ alkyl; 3 to 12-membered heterocycloalkyl including in a ring one to three heteroatoms independently selected from the group consisting of N, O, and S; or halogen,
$R_2$ is $C_3$-$C_8$ cycloalkyl; or 3 to 12-membered heterocycloalkyl including in a ring one to three heteroatoms independently selected from the group consisting of N, O, and S,
at least one H of the $R_2$ may be substituted or unsubstituted with Rc,
the Rc is $C_1$-$C_6$ alkyl; $C_3$-$C_8$ cycloalkyl; 3 to 12-membered heterocycloalkyl including in a ring one to three heteroatoms independently selected from the group consisting of N, O, and S; -NRpRq or -(C=O)-($C_1$-$C_6$ alkyl),
at least one H of the Rc may be substituted or unsubstituted with Rd,
the Rd is $C_1$-$C_6$ alkoxy which may be substituted or unsubstituted with halogen; 3 to 12-membered heterocycloalkyl including in a ring one to three heteroatoms independently selected from the group consisting of N, O, and S; 6 to 14-membered aryl; halogen; or -OH,
$R_3$ to $R_6$ are each independently $C_1$-$C_6$ alkyl,
Rx and Ry are each independently $C_1$-$C_6$ alkyl,
Rp and Rq are each independently H; $C_1$-$C_6$ alkyl, $C_3$-$C_8$ cycloalkyl or -(C=O)-($C_1$-$C_6$ alkyl), and
V is a single bond.

**(3)** With regard to above **(1)** or **(2),** in the chemical formula I, it may be provided that

$R_1$ is phenyl, pyridinyl, pyrimidinyl, indolyl, thiophenyl, isoxazolyl, furanyl, benzofuranyl or benzodioxolyl,
at least one H of the $R_1$ may be substituted or unsubstituted with Ra,
the Ra is methyl, methoxy, $-N(CH_3)_2$, $-N(CH_3)(CH_2CH_3)$, - $N(CH_3)(CH_2CH_2CH_2CH_3)$, morpholinyl, thiomorpholinyl, pyrrolidinyl, piperidinyl, piperazinyl,

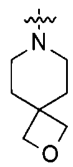

or halogen,
at least one H of the Ra may be substituted or unsubstituted with Rb,
the Rb is methyl, ethyl, morpholinyl or halogen,
$R_2$ is cyclohexyl or piperidinyl,
at least one H of the $R_2$ may be substituted or unsubstituted with Rc,
the Rc is methyl, ethyl, propyl, isopropyl, butyl, cyclohexyl, azetidinyl, $-NH_2$, - $N(CH_3)_2$, $-N(CH_3)(CH_2CH_3)$, $-N(CH_3)(CH_2CH_2CH_3)$, $-N(CH_3)(CH)(CH_3)_2$, - $N(CH_3)(CH_2CH_2CH_2CH_3)$, $-N(CH_3)(C_6H_{11})$, $-N(CH_3)(C=O)CH_3$ or $-(C=O)CH_3$,
at least one H of the Rc may be substituted or unsubstituted with Rd,
the Rd is methoxy, ethoxy which may be substituted or unsubstituted with halogen, propoxy, butoxy which may be

substituted or unsubstituted with halogen, isopropoxy, dioxolanyl, phenyl, halogen or -OH, and $R_3$ to $R_6$ are methyl.

In the present invention, "Cm-Cn" (in which m and n are each independently an integer of 1 or more) means the number of carbons, for example, "$C_1$-$C_5$ alkyl" may represent an alkyl having one to five carbon atoms.

In the present invention, the term "alkyl" means a linear or branched saturated hydrocarbon group represented by $C_nH_{2n+1}$, unless otherwise stated. Examples of alkyl may include methyl, ethyl, n-propyl, isopropyl, n-butyl, sec-butyl, tert-butyl, isobutyl, n-pentyl, sec-pentyl, tert-pentyl, isopentyl, sec-isopentyl, neo-pentyl, n-hexyl, etc., but are not limited thereto.

In the present invention, the term "alkenyl" means an unsaturated hydrocarbon group including at least one carbon-carbon double bond in the alkyl, unless otherwise stated.

In the present invention, the term "alkynyl" means an unsaturated hydrocarbon group including at least one carbon-carbon triple bond in the alkyl, unless otherwise stated.

In the present invention, the term "alkoxy" means a monovalent group derived from a linear or branched saturated hydrocarbon moiety represented by $OC_nH_{2n+1}$, unless otherwise stated. Examples of alkoxy may include methoxy, ethoxy, propoxy, isopropoxy, n-butoxy, sec-butoxy, tert-butoxy, pentoxy, hexoxy, etc., but are not limited thereto.

In the present invention, the term "cycloalkyl" means a saturated hydrocarbon cyclic group having three or more carbon atoms, and a saturated hydrocarbon ring may include both monocyclic and polycyclic structures, unless otherwise stated. Examples of cycloalkyl may include cyclopropyl, cyclobutyl, cyclopentyl, cyclohexyl, cycloheptyl, cyclooctyl, etc., but are not limited thereto.

In the present invention, the term "cycloalkenyl" means a cyclic group including at least one carbon-carbon double bond in the cycloalkyl, unless otherwise stated.

In the present invention, the term "heterocycloalkyl" means a cyclic group in which at least one carbon atom constituting a ring in the cycloalkyl is independently substituted with a heteroatom or functional group selected from the group consisting of N, O, S, SO, and $SO_2$, unless otherwise stated. Examples of heterocycloalkyl may include oxiranyl, oxetanyl, tetrahydrofuranyl, tetrahydropyranyl, tetrahydrothiopyranyl, tetrahydrothiophenyl, oxepanyl, piperidinyl, piperazinyl, morpholinyl, thiomorpholinyl, pyrrolidinyl, azetidinyl, aziridinyl, azepanyl, etc., but are not limited thereto.

In the present invention, the term "heterocycloalkenyl" means a cyclic group including at least one carbon-carbon double bond in the heterocycloalkyl, unless otherwise stated.

In the present invention, the term "aryl" means a monocyclic or polycyclic cyclic group having at least one fused or non-fused aromatic ring, unless otherwise stated. Examples of aryl may include phenyl, naphthyl, tetrahydronaphthyl, indenyl, anthracenyl, etc., but are not limited thereto.

In the present invention, the term "heteroaryl" means a monocyclic or polycyclic cyclic group including one to three heteroatoms selected from the group consisting of N, O, and S and having at least one fused or non-fused aromatic ring, unless otherwise stated. Examples of heteroaryl may include thiazolyl, oxazolyl, thiophenyl, furanyl, pyrrolyl, imidazolyl, isoxazolyl, pyrazolyl, triazolyl, thiadiazolyl, tetrazolyl, oxadiazolyl, pyridinyl, pyridazinyl, pyrimidinyl, pyrazinyl, indolyl, benzothiophenyl, benzofuranyl, benzimidazolyl, benzoxazolyl, benzisoxazolyl, benzthiazolyl, benzthiadiazolyl, benztriazolyl, quinolinyl, isoquinolinyl, purinyl, furopyridinyl, etc., but are not limited thereto.

In the present invention, the term "halogen" may be F, Cl, Br or I.

**(4)** With regard to any one of above **(1)** to **(3),** it may be provided that the compound represented by the chemical formula I is a compound described in a following table.

| Structure | Structure |
|---|---|
| 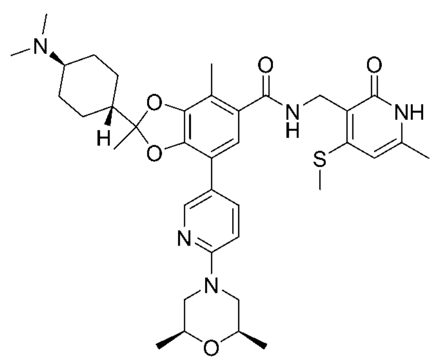 | 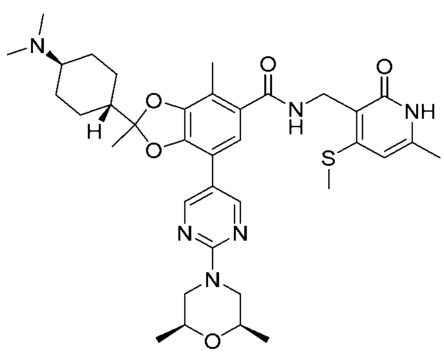 |

(continued)

| Structure | Structure |
|---|---|
| 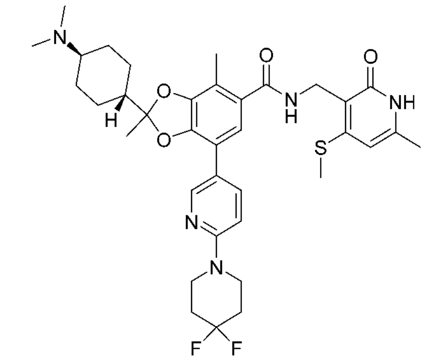 | 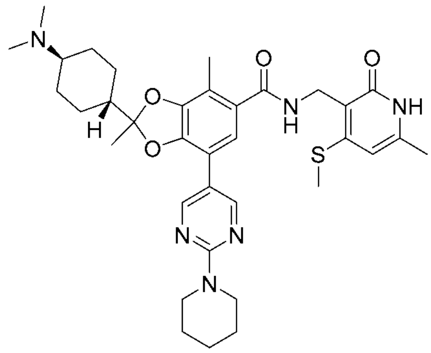 |
| 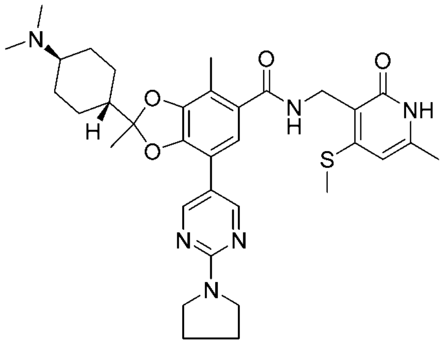 | 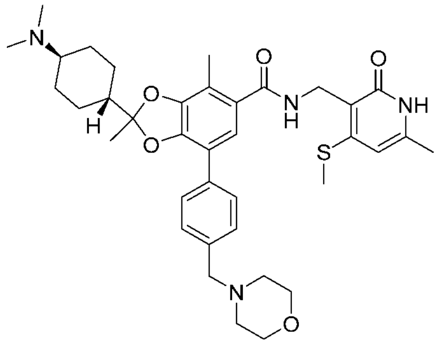 |
| 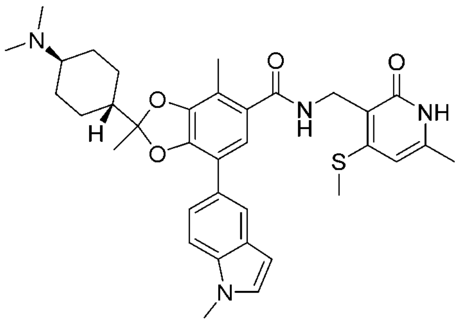 | 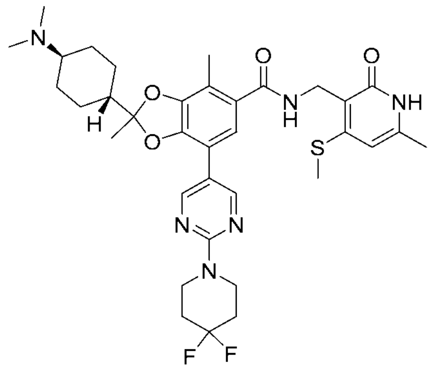 |
| 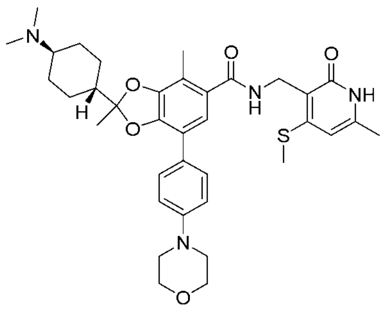 | 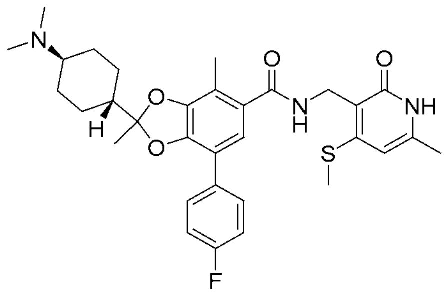 |

(continued)

| Structure | Structure |
|---|---|
| 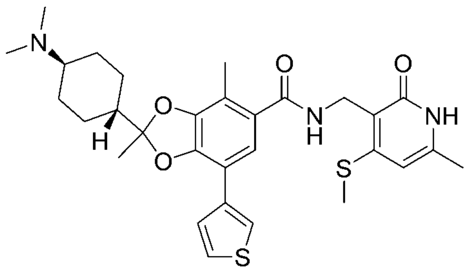 | 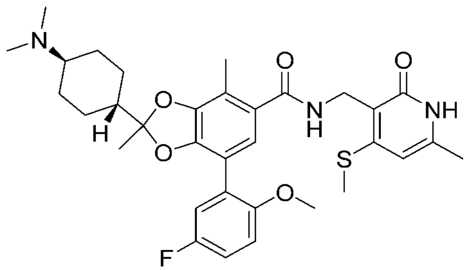 |
| 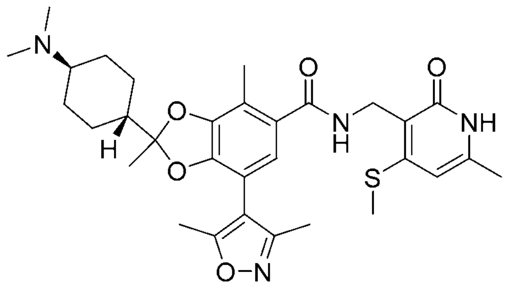 | 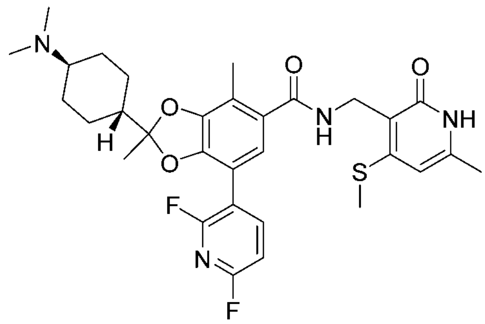 |
| 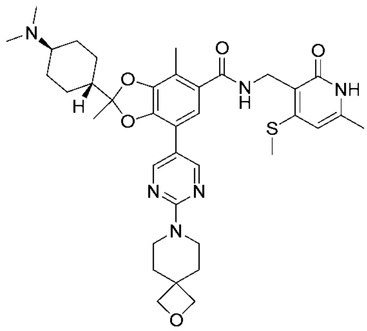 | 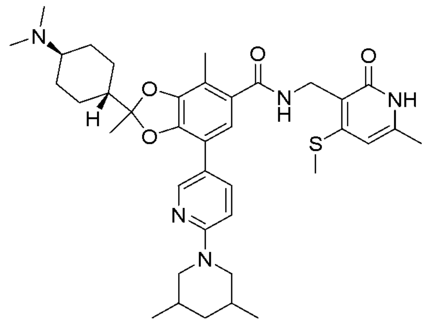 |
| 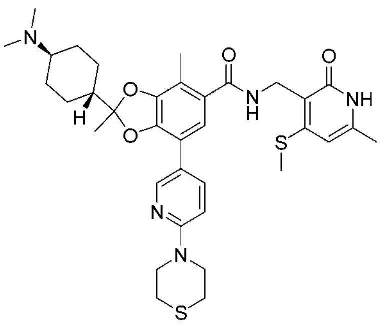 | 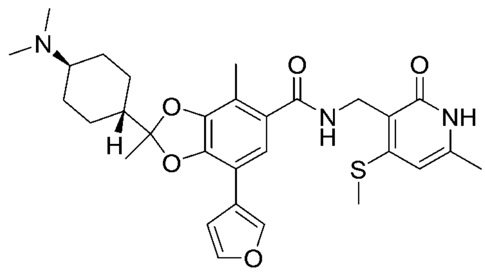 |
| 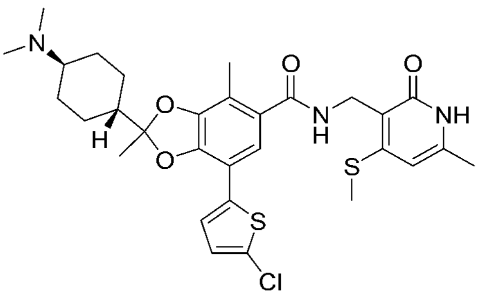 | 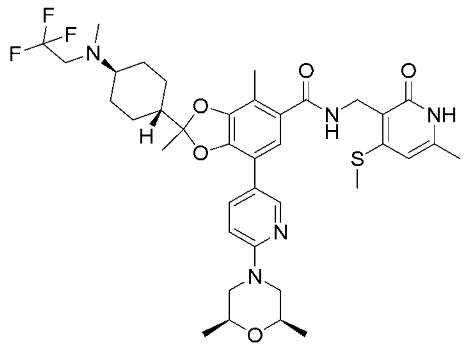 |

(continued)

| Structure | Structure |
|---|---|
| 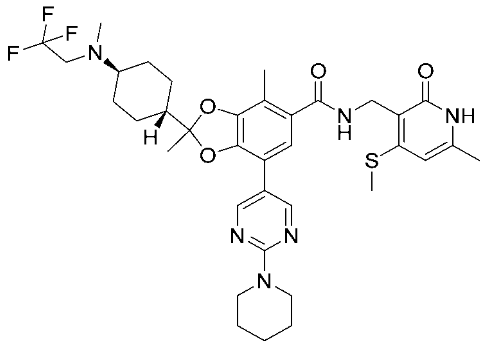 | 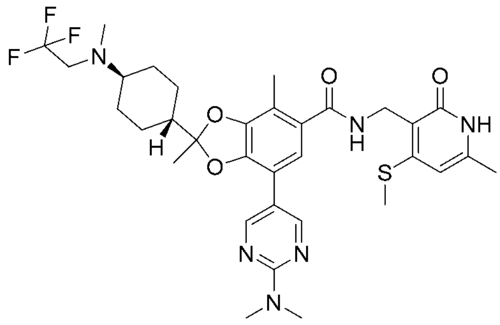 |
| 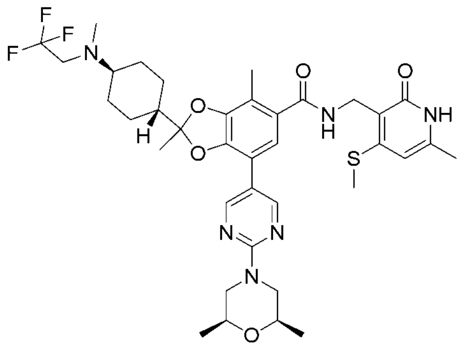 | 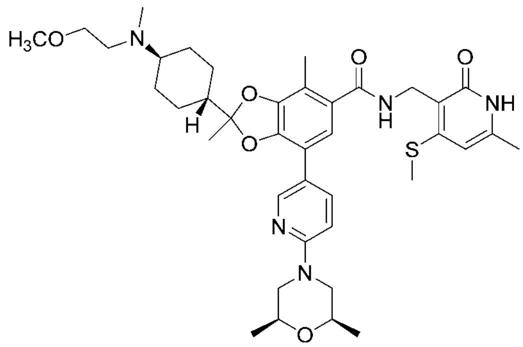 |
| 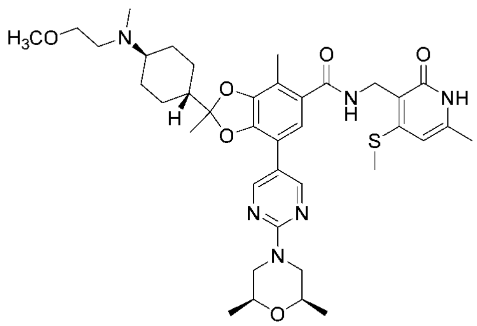 | 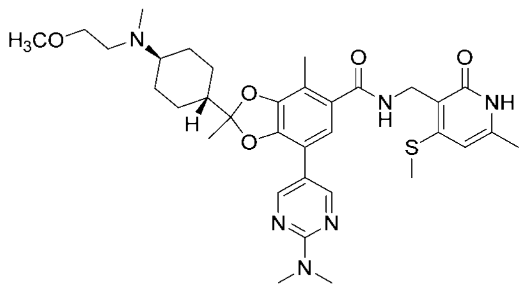 |
| 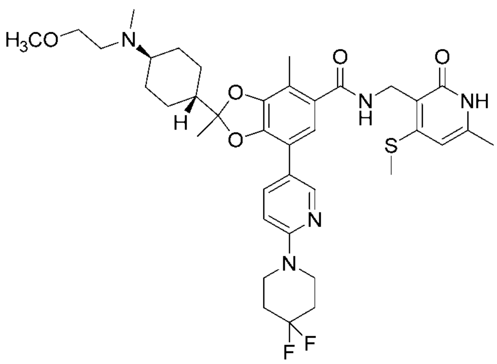 | 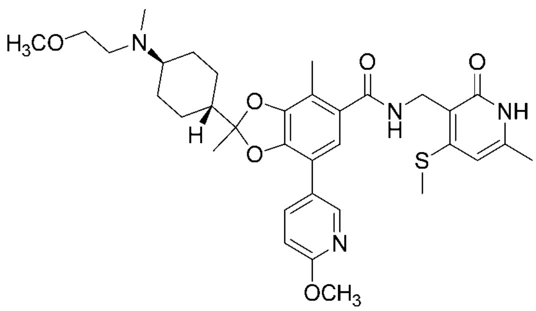 |

(continued)

| Structure | Structure |
|---|---|
| 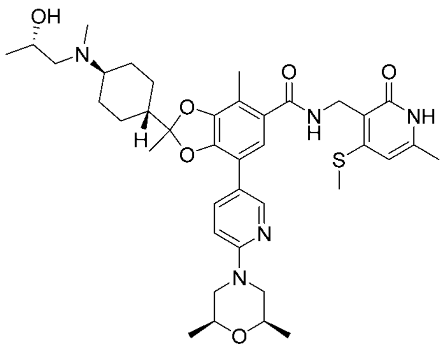 | 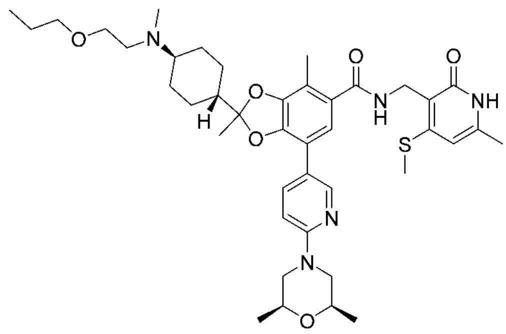 |
| 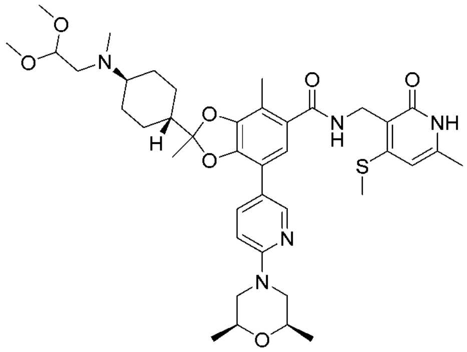 | 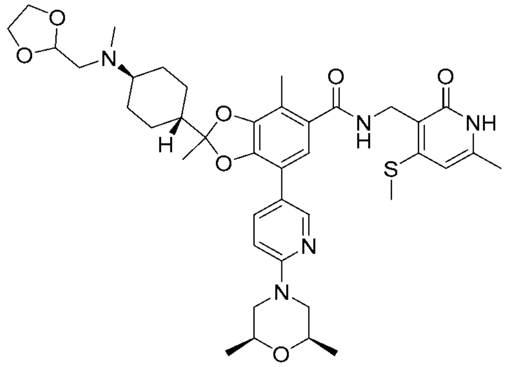 |
| 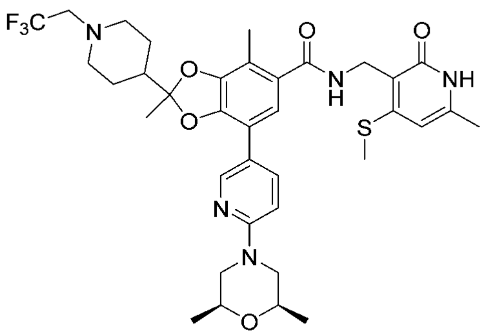 | 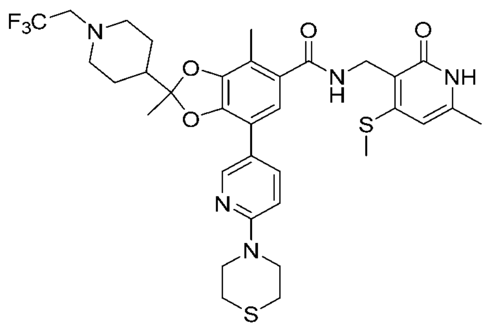 |
| 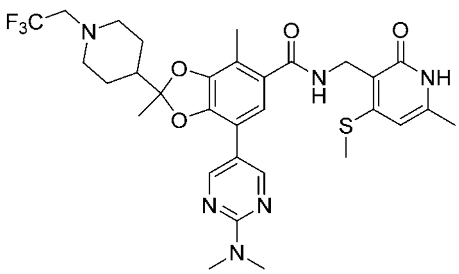 | 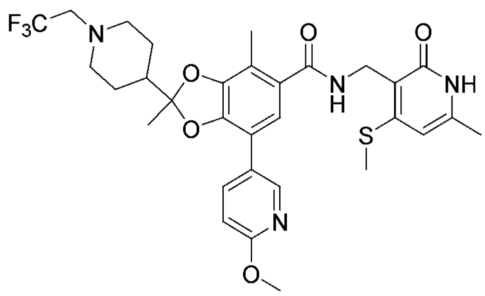 |

(continued)

| Structure | Structure |
|---|---|
| 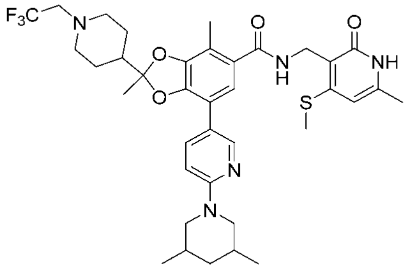 | 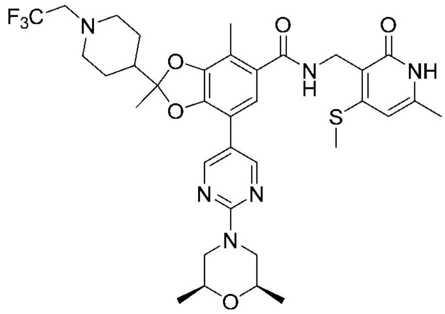 |
| 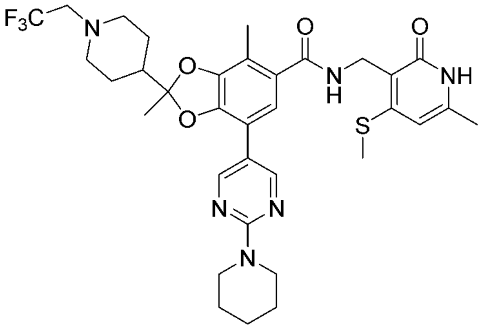 | 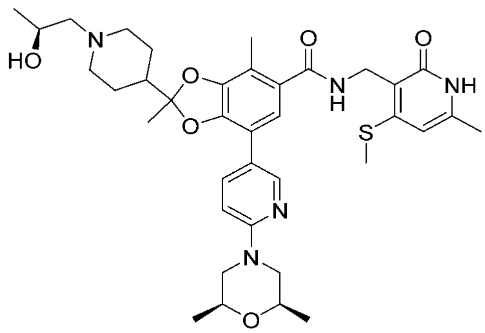 |
| 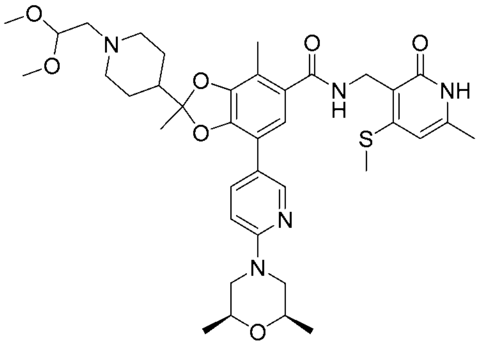 | 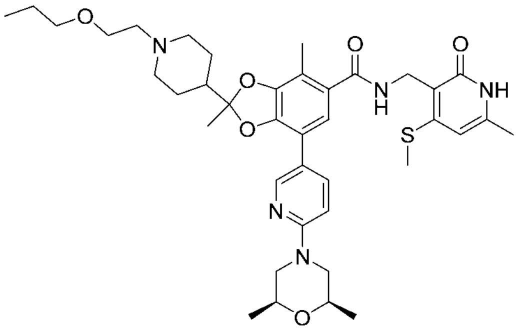 |
| 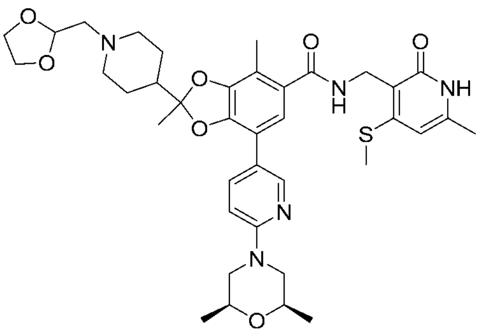 | 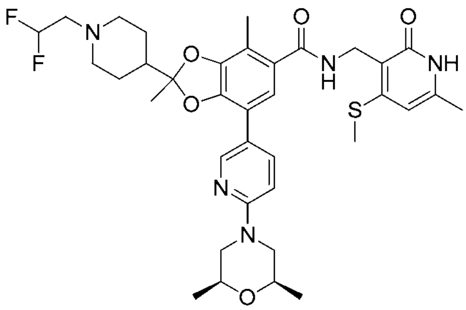 |

(continued)

| Structure | Structure |
|---|---|
| 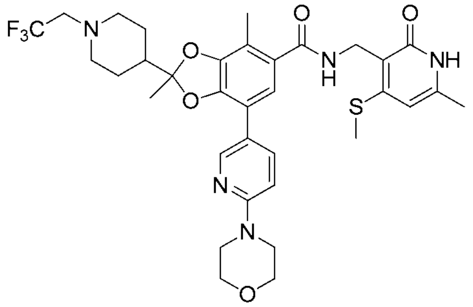 | 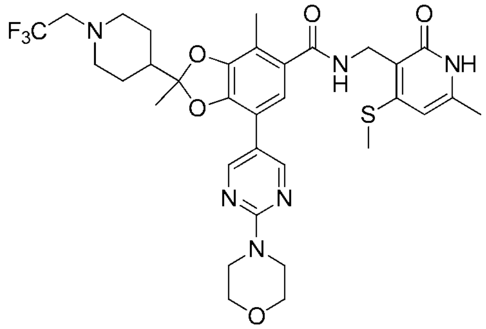 |
| 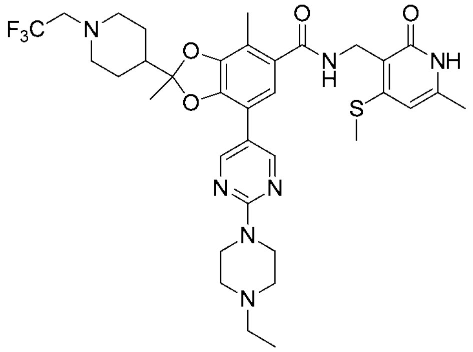 | 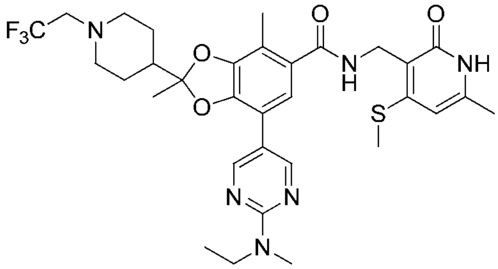 |
| 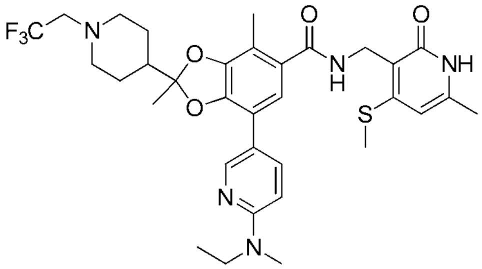 | 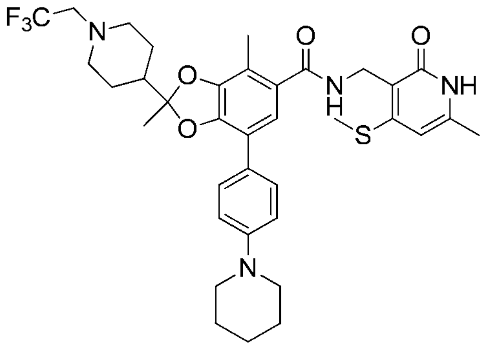 |
| 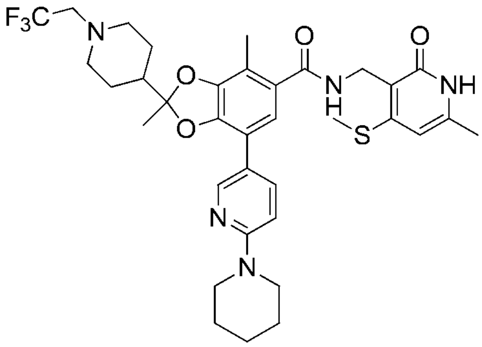 | 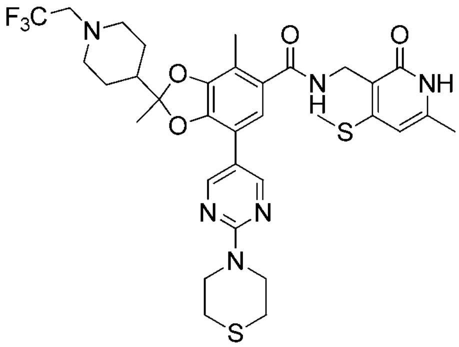 |

(continued)

| Structure | Structure |
|---|---|
| 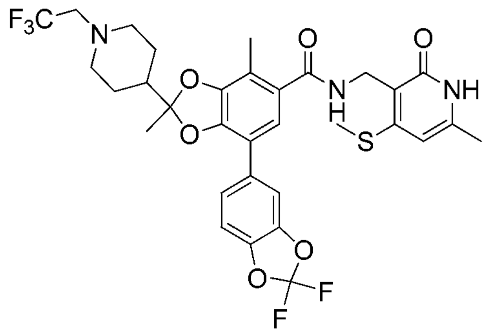 | 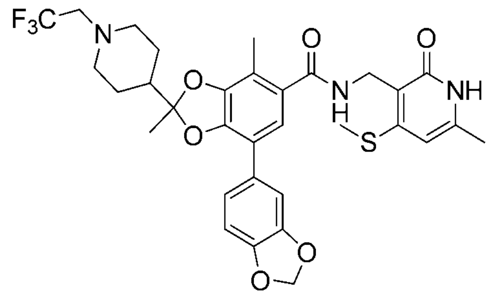 |
| 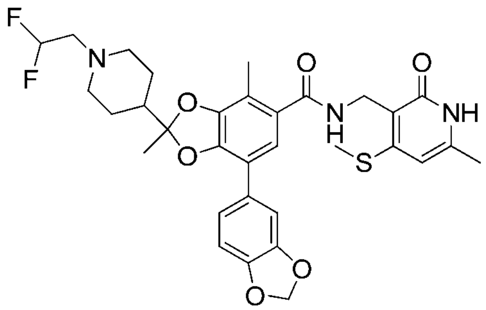 | 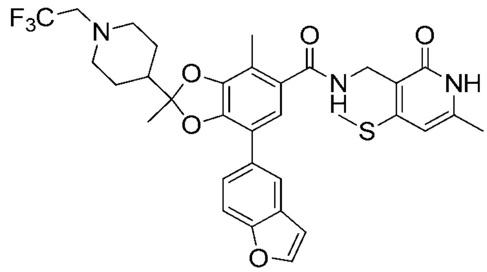 |
| 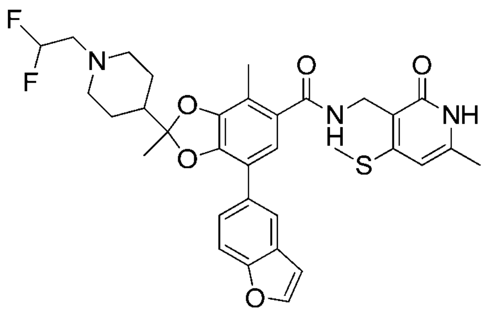 | 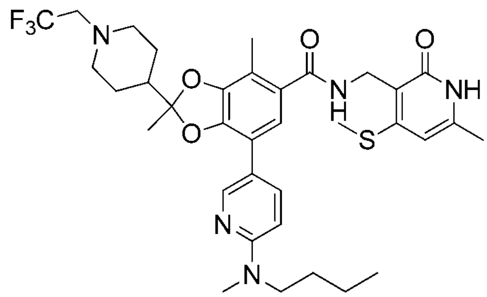 |
| 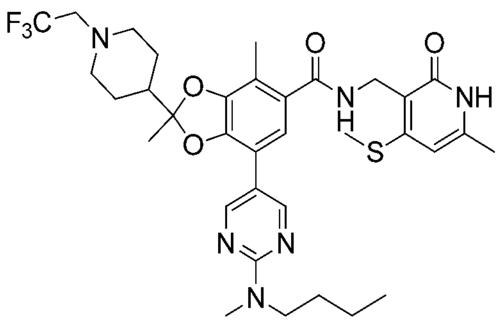 | 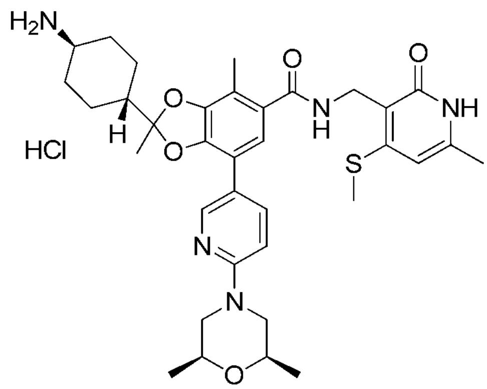 HCl |

(continued)

| Structure | Structure |
|---|---|
| 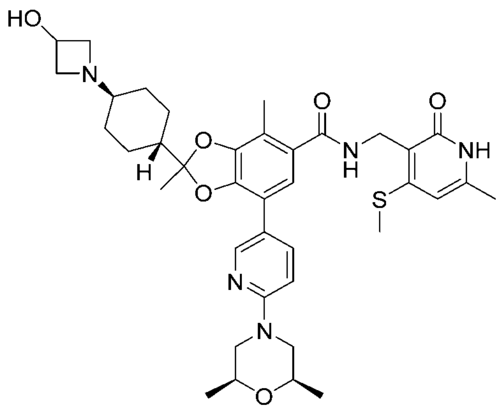 | 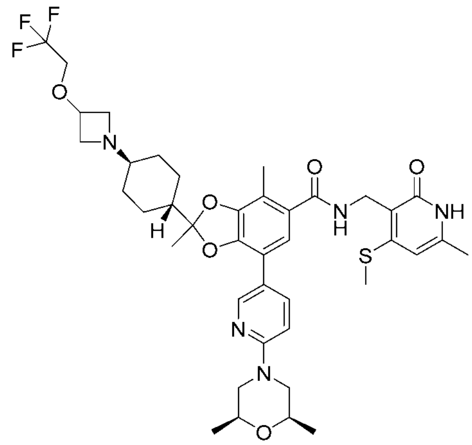 |
| 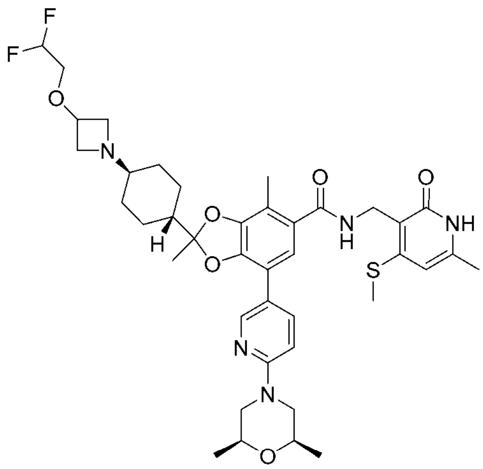 | 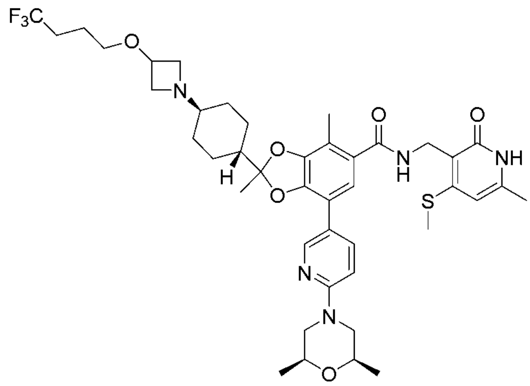 |
| 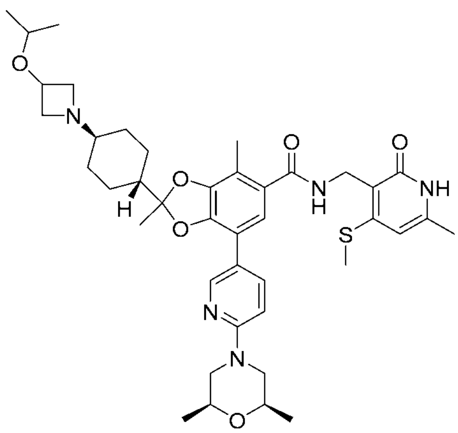 | 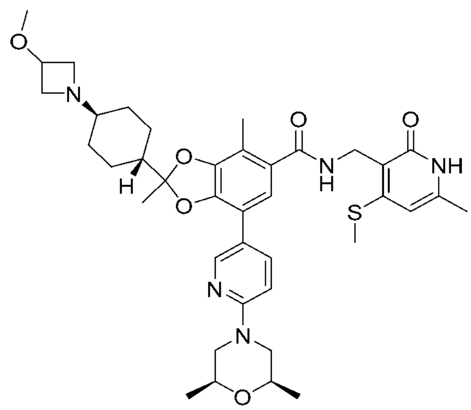 |

(continued)

| Structure | Structure |
|---|---|
| 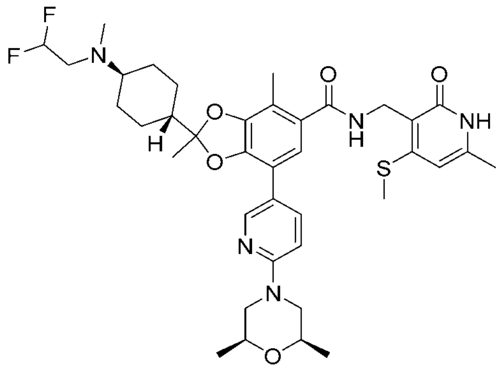 | 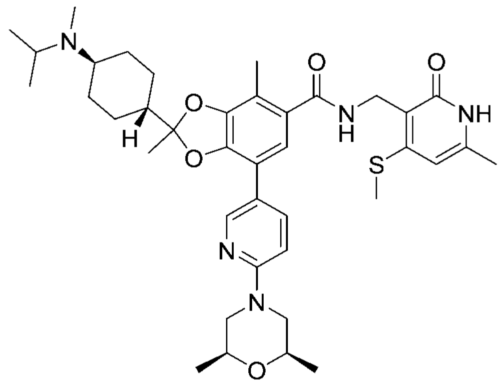 |
| 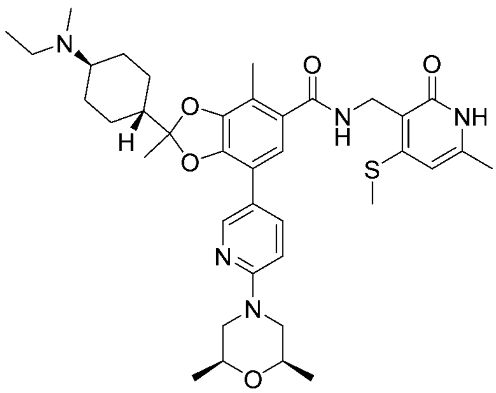 | 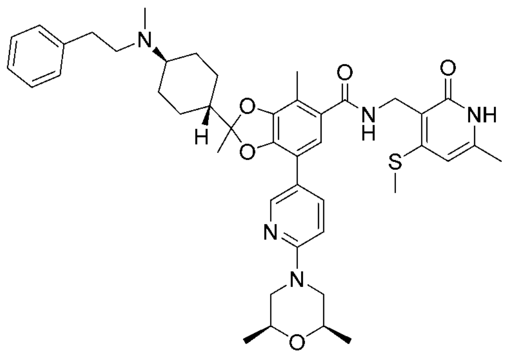 |
| 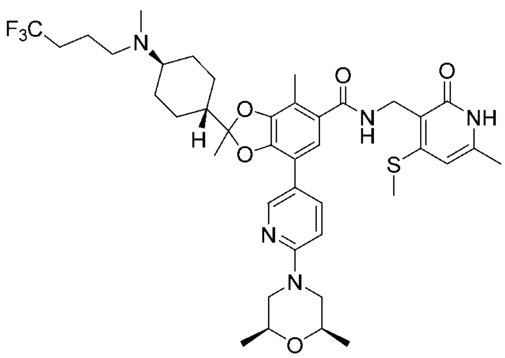 | 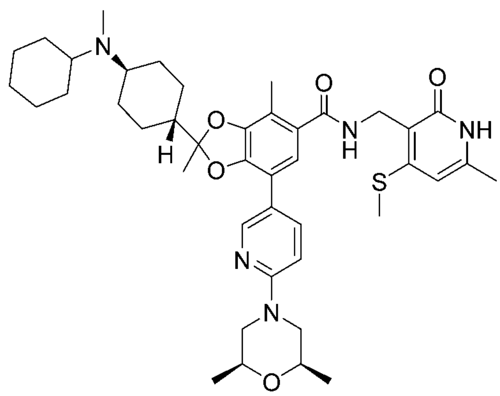 |
| 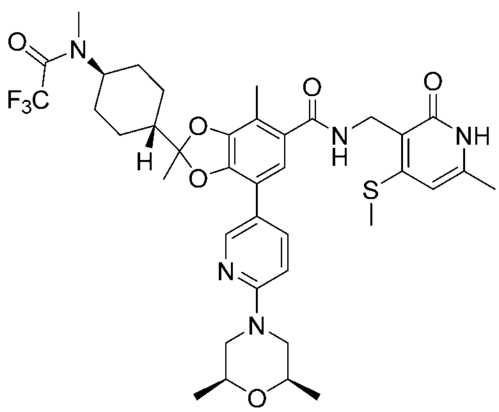 | 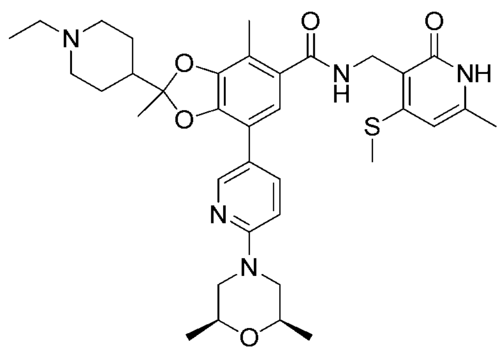 |

(continued)

| Structure | Structure |
|---|---|
| 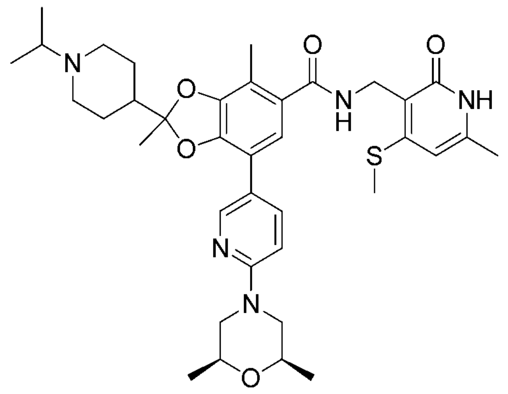 | 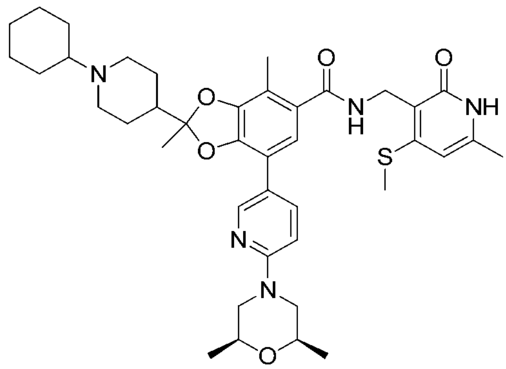 |
| 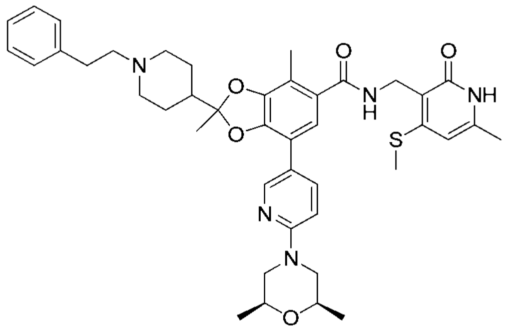 | 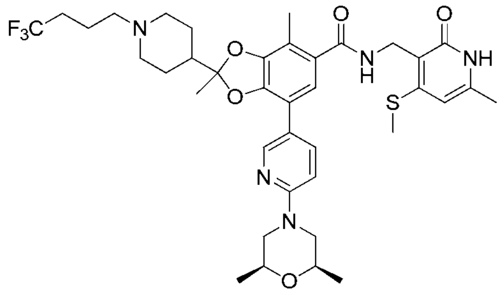 |
| 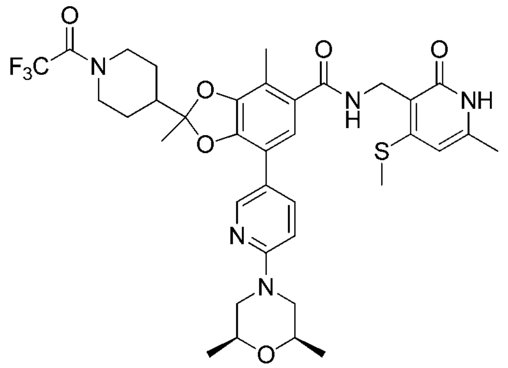 | 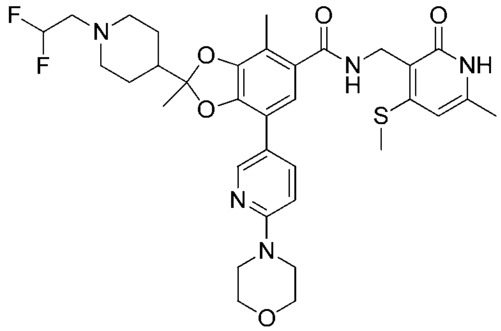 |
| 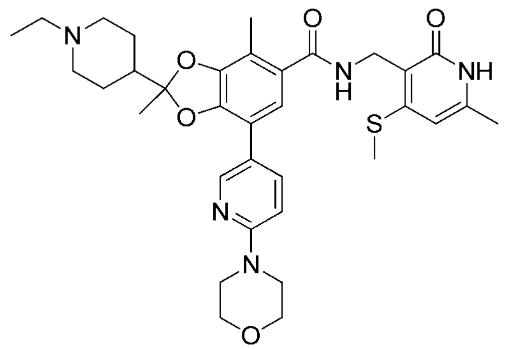 | 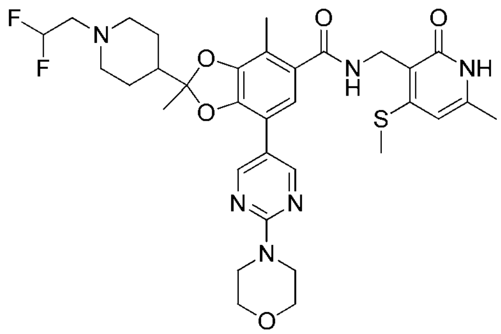 |

(continued)

| Structure | Structure |
|---|---|
| 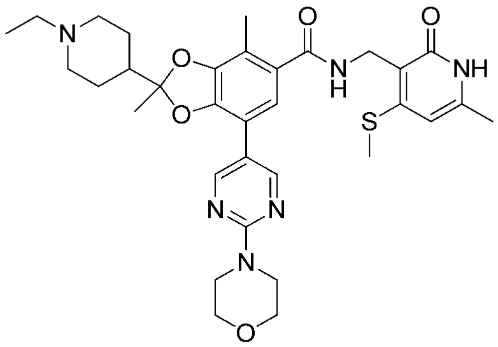 | 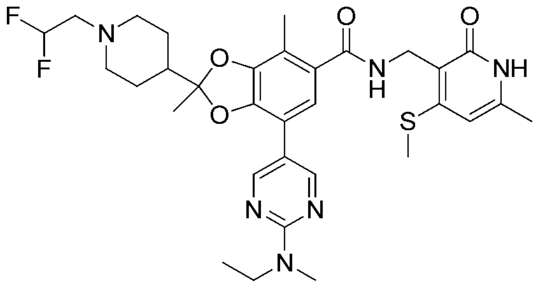 |
| 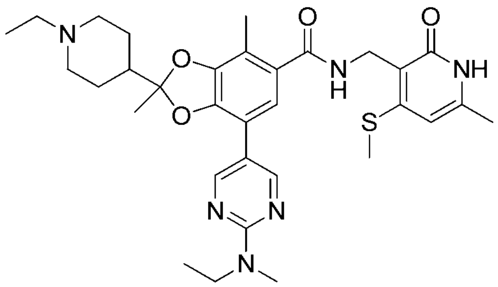 | 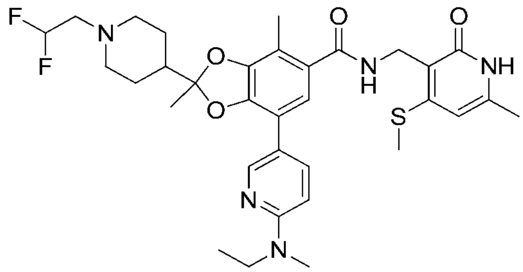 |
| 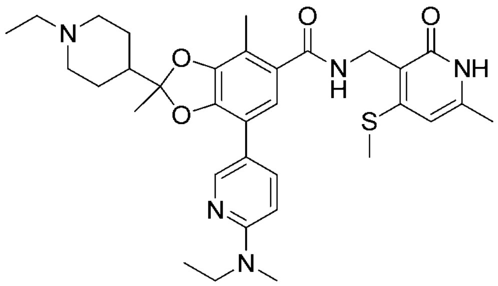 | 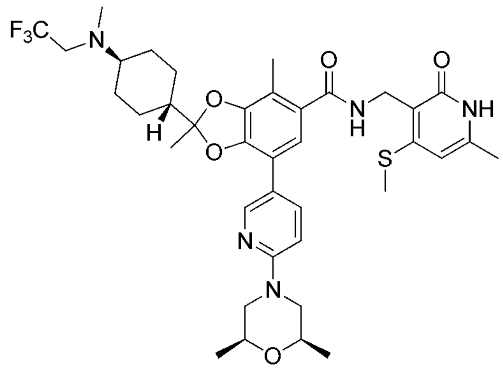 |

**(5)** A following compound, a stereoisomer thereof, a pharmaceutically acceptable salt thereof, a hydrate or solvate thereof:

1) 2-(trans-4-(dimethylamino)cyclohexyl)-7-(6-((2S,6R)-2,6-dimethylmorpholino)pyridin-3-yl)-2,4-dimethyl-N-((6-methyl-4-(methylthio)-2-oxo-1,2-dihydropyridin-3-yl)methyl)benzo[d][1,3]dioxole-5-carboxamide
2) 2-(trans-4-(dimethylamino)cyclohexyl)-7-(2-((2S,6R)-2,6-dimethylaminomorpholino)pyrimidin-5-yl)-2,4-dimethyl-N-((6-methyl-4-(methylthio)-2-oxo-1,2-dihydropyridin-3-yl)methyl)benzo[d][1,3]dioxole-5-carboxamide
3) 7-(6-(4,4-difluoropiperidin-1-yl)pyridin-3-yl)-2-(trans-4-(dimethylamino)cyclohexyl)-2,4-dimethyl-N-((6-methyl-4-(methylthio)-2-oxo-1,2-dihydropyridin-3-yl)methyl)benzo[d][1,3]dioxole-5-carboxamide
4) 2-(trans-4-(dimethylamino)cyclohexyl)-2,4-dimethyl-N-((6-methyl-4-(methylthio)-2-oxo-1,2-dihydropyridin-3-yl)methyl)-7-(2-(piperidin-1-yl)pyrimidin-5-yl)benzo[d][1,3]dioxole-5-carboxamide
5) 2-(trans-4-(dimethylamino)cyclohexyl)-2,4-dimethyl-N-((6-methyl-4-(methylthio)-2-oxo-1,2-dihydropyridin-3-yl)methyl)-7-(2-(pyrrolidin-1-yl)pyrimidin-5-yl)benzo[d][1,3]dioxole-5-carboxamide
6) 2-(trans-4-(dimethylamino)cyclohexyl)-2,4-dimethyl-N-((6-methyl-4-(methylthio)-2-oxo-1,2-dihydropyridin-3-yl)methyl)-7-(4-(morpholinomethyl)phenyl)benzo[d][1,3]dioxole-5-carboxamide
7) 2-(trans-4-(dimethylamino)cyclohexyl)-2,4-dimethyl-7-(1-methyl-1H-indol-5-yl)-N-((6-methyl-4-(methylthio)-2-oxo-1,2-dihydropyridin-3-yl)methyl)benzo[d][1,3]dioxole-5-carboxamide
8) 7-(2-(4,4-difluoropiperidin-1-yl)pyrimidin-5-yl)-2-(trans-4-(dimethylamino)cyclohexyl)-2,4-dimethyl-N-((6-methyl-4-(methylthio)-2-oxo-1,2-dihydropyridin-3-yl)methyl)benzo[d][1,3]dioxole-5-carboxamide
9) 2-(trans-4-(dimethylamino)cyclohexyl)-2,4-dimethyl-N-((6-methyl-4-(methylthio)-2-oxo-1,2-dihydropyridin-3-yl)methyl)-7-(4-morpholinophenyl)benzo[d][1,3]dioxole-5-carboxamide
10) 2-(trans-4-(dimethylamino)cyclohexyl)-7-(4-fluorophenyl)-2,4-dimethyl-N-((6-methyl-4-(methylthio)-2-oxo-1,2-dihydropyridin-3-yl)methyl)benzo[d][1,3]dioxole-5-carboxamide
11) 2-(trans-4-(dimethylamino)cyclohexyl)-2,4-dimethyl-N-((6-methyl-4-(methylthio)-2-oxo-1,2-dihydropyridin-3-yl)methyl)-7-(thiophen-3-yl)benzo[d][1,3]dioxole-5-carboxamide
12) 2-(trans-4-(dimethylamino)cyclohexyl)-7-(5-fluoro-2-methoxyphenyl)-2,4-dimethyl-N-((6-methyl-4-(methylthio)-2-oxo-1,2-dihydropyridin-3-yl)methyl)benzo[d][1,3]dioxole-5-carboxamide
13) 2-(trans-4-(dimethylamino)cyclohexyl)-7-(3,5-dimethylisoxazol-4-yl)-2,4-dimethyl-N-((6-methyl-4-(methylthio)-2-oxo-1,2-dihydropyridin-3-yl)methyl)benzo[d][1,3]dioxole-5-carboxamide
14) 7-(2,6-difluoropyridin-3-yl)-2-(trans-4-(dimethylamino)cyclohexyl)-2,4-dimethyl-N-((6-methyl-4-(methylthio)-2-oxo-1,2-dihydropyridin-3-yl)methyl)benzo[d][1,3]dioxole-5-carboxamide
*15)* 7-(2-(2-oxa-7-azaspiro[3.5]nonan-7-yl)pyrimidin-5-yl)-2-(trans-4-(dimethylamino)cyclohexyl)-2,4-dimethyl-N-((6-methyl-4-(methylthio)-2-oxo-1,2-dihydropyridin-3-yl)methyl)benzo[d][1,3]dioxole-5-carboxamide
16) 2-(trans-4-(dimethylamino)cyclohexyl)-7-(6-(3,5-dimethylpiperidin-1-yl)pyridin-3-yl)-2,4-dimethyl-N-((6-methyl-4-(methylthio)-2-oxo-1,2-dihydropyridin-3-yl)methyl)benzo[d][1,3]dioxole-5-carboxamide
17) 2-(trans-4-(dimethylamino)cyclohexyl)-2,4-dimethyl-N-((6-methyl-4-(methylthio)-2-oxo-1,2-dihydropyridin-3-yl)methyl)-7-(6-thiomorpholinopyridin-3-yl)benzo[d][1,3]dioxole-5-carboxamide
18) 2-(trans-4-(dimethylamino)cyclohexyl)-7-(furan-3-yl)-2,4-dimethyl-N-((6-methyl-4-(methylthio)-2-oxo-1,2-dihydropyridin-3-yl)methyl)benzo[d][1,3]dioxole-5-carboxamide
19) 7-(5-chlorothiophen-2-yl)-2-(trans-4-(dimethylamino)cyclohexyl)-2,4-dimethyl-N-((6-methyl-4-(methylthio)-2-oxo-1,2-dihydropyridin-3-yl)methyl)benzo[d][1,3]dioxole-5-carboxamide
20) 7-(6-((2S,6R)-2,6-dimethylmorpholino)pyridin-3-yl)-2,4-dimethyl-2-(trans-4-(methyl(2,2,2-trifluoroethyl)amino)cyclohexyl)-N-((6-methyl-4-(methylthio)-2-oxo-1,2-dihydropyridin-3-yl)methyl)benzo[d][1,3]dioxole-5-carboxamide
21) 2,4-dimethyl-2-(trans-4-(methyl(2,2,2-trifluoroethyl)amino)cyclohexyl)-N-((6-methyl-4-(methylthio)-2-oxo-1,2-dihydropyridin-3-yl)methyl)-7-(2-(piperidin-1-yl)pyrimidin-5-yl)benzo[d][1,3]dioxole-5-carboxamide
22) 7-(2-(dimethylamino)pyrimidin-5-yl)-2,4-dimethyl-2-(trans-4-(methyl(2,2,2-trifluoroethyl)amino)cyclohexyl)-N-((6-methyl-4-(methylthio)-2-oxo-1,2-dihydropyridin-3-yl)methyl)benzo[d][1,3]dioxole-5-carboxamide
23) 7-(2-((2S,6R)-2,6-dimethylmorpholino)pyrimidin-5-yl)-2,4-dimethyl-2-(trans-4-(methyl(2,2,2-trifluoroethyl)amino)cyclohexyl)-N-((6-methyl-4-(methylthio)-2-oxo-1,2-dihydropyridin-3-yl)methyl)benzo[d][1,3]dioxole-5-carboxamide
24) 7-(6-((2S,6R)-2,6-dimethylmorpholino)pyridin-3-yl)-2-(trans-4-((2-methoxyethyl)(methyl)amino)cyclohexyl)-2,4-dimethyl-N-((6-methyl-4-(methylthio)-2-oxo-1,2-dihydropyridin-3-yl)methyl)benzo[d][1,3]dioxole-5-carboxamide
25) 7-(2-((2S,6R)-2,6-dimethylmorpholino)pyrimidin-5-yl)-2-(trans-4-((2-methoxyethyl)(methyl)amino)cyclohexyl)-2,4-dimethyl-N-((6-methyl-4-(methylthio)-2-oxo-1,2-dihydropyridin-3-yl)methyl)benzo[d][1,3]dioxole-5-carboxamide
26) 7-(2-(dimethylamino)pyrimidin-5-yl)-2-(trans-4-((2-methoxyethyl)(methyl)amino)cyclohexyl)-2,4-dimethyl-N-((6-methyl-4-(methylthio)-2-oxo-1,2-dihydropyridin-3-yl)methyl)benzo[d][1,3]dioxole-5-carboxamide
27) 7-(6-(4,4-difluoropiperidin-1-yl)pyridin-3-yl)-2-(trans-4-((2-methoxyethyl)(methyl)amino)cyclohexyl)-2,4-dimethyl-N-((6-methyl-4-(methylthio)-2-oxo-1,2-dihydropyridin-3-yl)methyl)benzo[d][1,3]dioxole-5-carboxamide
28) 2-(trans-4-((2-methoxyethyl)(methyl)amino)cyclohexyl)-7-(6-methoxypyridin-3-yl)-2,4-dimethyl-N-((6-methyl-4-(methylthio)-2-oxo-1,2-dihydropyridin-3-yl)methyl)benzo[d][1,3]dioxole-5-carboxamide
29) 7-(6-((2S,6R)-2,6-dimethylmorpholino)pyridin-3-yl)-2-(trans-4-(((S)-2-hydroxypropyl)(methyl)amino)cyclohexyl)-2,4-dimethyl-N-((6-methyl-4-(methylthio)-2-oxo-1,2-dihydropyridin-3-yl)methyl)benzo[d][1,3]dioxole-5-carboxamide
30) 7-(6-((2S,6R)-2,6-dimethylmorpholino)pyridin-3-yl)-2,4-dimethyl-2-(trans-4-(methyl(2-propoxyethyl)amino)cyclohexyl)-N-((6-methyl-4-(methylthio)-2-oxo-1,2-dihydropyridin-3-yl)methyl)benzo[d][1,3]dioxole-5-carboxamide
31) 2-(trans-4-((2,2-dimethoxyethyl)(methyl)amino)cyclohexyl)-7-(6-((2S,6R)-2,6-dimethylmorpholino)pyridin-3-yl)-2,4-dimethyl-N-((6-methyl-4-(methylthio)-2-oxo-1,2-dihydropyridin-3-yl)methyl)benzo[d][1,3]dioxole-5-carboxamide
32) 2-(trans-4-(((1,3-dioxolan-2-yl)methyl)(methyl)amino)cyclohexyl)-7-(6-((2S,6R)-2,6-dimethylmorpholino)pyridin-3-yl)-2,4-dimethyl-N-((6-methyl-4-(methylthio)-2-oxo-1,2-dihydropyridin-3-yl)methyl)benzo[d][1,3]dioxole-5-carboxamide
33) 7-(6-((2S,6R)-2,6-dimethylmorpholino)pyridin-3-yl)-2,4-dimethyl-N-((6-methyl-4-(methylthio)-2-oxo-1,2-dihydropyridin-3-yl)methyl)-2-(1-(2,2,2-trifluoroethyl)piperidin-4-yl)benzo[d][1,3]dioxole-5-carboxamide
34) 2,4-dimethyl-N-((6-methyl-4-(methylthio)-2-oxo-1,2-dihydropyridin-3-yl)methyl)-7-(6-thiomorpholinopyridin-3-yl)-2-(1-(2,2,2-trifluoroethyl)piperidin-4-yl)benzo[d][1,3]dioxole-5-carboxamide
35) 7-(2-(dimethylamino)pyrimidin-5-yl)-2,4-dimethyl-N-((6-methyl-4-(methylthio)-2-oxo-1,2-dihydropyridin-3-yl)methyl)-2-(1-(2,2,2-trifluoroethyl)piperidin-4-yl)benzo[d][1,3]dioxole-5-carboxamide

36) 7-(6-methoxypyridin-3-yl)-2,4-dimethyl-N-((6-methyl-4-(methylthio)-2-oxo-1,2-dihydropyridin-3-yl)methyl)-2-(1-(2,2,2-trifluoroethyl)piperidin-4-yl)benzo[d][1,3]dioxole-5-carboxamide

37) 7-(6-(3,5-dimethylpiperidin-1-yl)pyridin-3-yl)-2,4-dimethyl-N-((6-methyl-4-(methylthio)-2-oxo-1,2-dihydropyridin-3-yl)methyl)-2-(1-(2,2,2-trifluoroethyl)piperidin-4-yl)benzo[d][1,3]dioxole-5-carboxamide

38) 7-(2-((2S,6R)-2,6-dimethylmorpholino)pyrimidin-5-yl)-2,4-dimethyl-N-((6-methyl-4-(methylthio)-2-oxo-1,2-dihydropyridin-3-yl)methyl)-2-(1-(2,2,2-trifluoroethyl)piperidin-4-yl)benzo[d][1,3]dioxole-5-carboxamide

39) 2,4-dimethyl-N-((6-methyl-4-(methylthio)-2-oxo-1,2-dihydropyridin-3-yl)methyl)-7-(2-(piperidin-1-yl)pyrimidin-5-yl)-2-(1-(2,2,2-trifluoroethyl)piperidin-4-yl)benzo[d][1,3]dioxole-5-carboxamide

40) 7-(6-((2S,6R)-2,6-dimethylmorpholino)pyridin-3-yl)-2-(1-((S)-2-hydroxypropyl)piperidin-4-yl)-2,4-dimethyl-N-((6-methyl-4-(methylthio)-2-oxo-1,2-dihydropyridin-3-yl)methyl)benzo[d][1,3]dioxole-5-carboxamide

41) 2-(1-(2,2-dimethoxyethyl)piperidin-4-yl)-7-(6-((2S,6R)-2,6-dimethylmorpholino)pyridin-3-yl)-2,4-dimethyl-N-((6-methyl-4-(methylthio)-2-oxo-1,2-dihydropyridin-3-yl)methyl)benzo[d][1,3]dioxole-5-carboxamide

42) 2-(4-(dimethylamino)cyclohexyl)-2,4-dimethyl-N-((6-methyl-4-(methylthio)-2-oxo-1,2-dihydropyridin-3-yl)methyl)-7-(thiophen-3-yl)benzo[d][1,3]dioxole-5-carboxamide

d 2-(4-(dimethylamino)cyclohexyl)-7-(5-fluoro-2-methoxyphenyl)-2,4-dimethyl-N-((6-methyl-4-(methylthio)-2-oxo-1,2-dihydropyridin-3-yl)methyl)benzo[d][1,3]dioxole-5-carboxamide

44) Stereoisomer B of 7-(6-((2S,6R)-2,6-dimethylmorpholino)pyridin-3-yl)-2,4-dimethyl-N-((6-methyl-4-(methylthio)-2-oxo-1,2-dihydropyridin-3-yl)methyl)-2-(1-(2,2,2-trifluoroethyl)piperidin-4-yl)benzo[d][1,3]dioxole-5-carboxamide

45) Stereoisomer A of 7-(6-((2S,6R)-2,6-dimethylmorpholino)pyridin-3-yl)-2,4-dimethyl-N-((6-methyl-4-(methylthio)-2-oxo-1,2-dihydropyridin-3-yl)methyl)-2-(1-(2,2,2-trifluoroethyl)piperidin-4-yl)benzo[d][1,3]dioxole-5-carboxamide

46) 2-(1-(2,2-difluoroethyl)piperidin-4-yl)-7-(6-((2S,6R)-2,6-dimethylmorpholino)pyridin-3-yl)-2,4-dimethyl-N-((6-methyl-4-(methylthio)-2-oxo-1,2-dihydropyridin-3-yl)methyl)benzo[d][1,3]dioxole-5-carboxamide

47) Stereoisomer B of 2-(1-(2,2-difluoroethyl)piperidin-4-yl)-7-(6-((2S,6R)-2,6-dimethylmorpholino)pyridin-3-yl)-2,4-dimethyl-N-((6-methyl-4-(methylthio)-2-oxo-1,2-dihydropyridin-3-yl)methyl)benzo[d][1,3]dioxole-5-carboxamide

48) Stereoisomer A of 2-(1-(2,2-difluoroethyl)piperidin-4-yl)-7-(6-((2S,6R)-2,6-dimethylmorpholino)pyridin-3-yl)-2,4-dimethyl-N-((6-methyl-4-(methylthio)-2-oxo-1,2-dihydropyridin-3-yl)methyl)benzo[d][1,3]dioxole-5-carboxamide

49) Stereoisomer B of 7-(6-((2S,6R)-2,6-dimethylmorpholino)pyridin-3-yl)-2-(1-ethylpiperidin-4-yl)-2,4-dimethyl-N-((6-methyl-4-(methylthio)-2-oxo-1,2-dihydropyridin-3-yl)methyl)benzo[d][1,3]dioxole-5-carboxamide

50) Stereoisomer A of 7-(6-((2S,6R)-2,6-dimethylmorpholino)pyridin-3-yl)-2-(1-ethylpiperidin-4-yl)-2,4-dimethyl-N-((6-methyl-4-(methylthio)-2-oxo-1,2-dihydropyridin-3-yl)methyl)benzo[d][1,3]dioxole-5-carboxamide

51) Stereoisomer B of 7-(6-((2S,6R)-2,6-dimethylmorpholino)pyridin-3-yl)-2-(1-isopropylpiperidin-4-yl)-2,4-dimethyl-N-((6-methyl-4-(methylthio)-2-oxo-1,2-dihydropyridin-3-yl)methyl)benzo[d][1,3]dioxole-5-carboxamide

52) Stereoisomer A of 7-(6-((2S,6R)-2,6-dimethylmorpholino)pyridin-3-yl)-2-(1-isopropylpiperidin-4-yl)-2,4-dimethyl-N-((6-methyl-4-(methylthio)-2-oxo-1,2-dihydropyridin-3-yl)methyl)benzo[d][1,3]dioxole-5-carboxamide

53) Stereoisomer B of 2-(1-cyclohexylpiperidin-4-yl)-7-(6-((2S,6R)-2,6-dimethylmorpholino)pyridin-3-yl)-2,4-dimethyl-N-((6-methyl-4-(methylthio)-2-oxo-1,2-dihydropyridin-3-yl)methyl)benzo[d][1,3]dioxole-5-carboxamide

54) Stereoisomer A of 2-(1-cyclohexylpiperidin-4-yl)-7-(6-((2S,6R)-2,6-dimethylmorpholino)pyridin-3-yl)-2,4-dimethyl-N-((6-methyl-4-(methylthio)-2-oxo-1,2-dihydropyridin-3-yl)methyl)benzo[d][1,3]dioxole-5-carboxamide

55) Stereoisomer B of 7-(6-((2S,6R)-2,6-dimethylmorpholino)pyridin-3-yl)-2,4-dimethyl-N-((6-methyl-4-(methylthio)-2-oxo-1,2-dihydropyridin-3-yl)methyl)-2-(1-phenethylpiperidin-4-yl)benzo[d][1,3]dioxole-5-carboxamide

56) Stereoisomer A of 7-(6-((2S,6R)-2,6-dimethylmorpholino)pyridin-3-yl)-2,4-dimethyl-N-((6-methyl-4-(methylthio)-2-oxo-1,2-dihydropyridin-3-yl)methyl)-2-(1-phenethylpiperidin-4-yl)benzo[d][1,3]dioxole-5-carboxamide

57) Stereoisomer B of 7-(6-((2S,6R)-2,6-dimethylmorpholino)pyridin-3-yl)-2,4-dimethyl-N-((6-methyl-4-(methylthio)-2-oxo-1,2-dihydropyridin-3-yl)methyl)-2-(1-(4,4,4-trifluorobutyl)piperidin-4-yl)benzo[d][1,3]dioxole-5-carboxamide

58) Stereoisomer A of 7-(6-((2S,6R)-2,6-dimethylmorpholino)pyridin-3-yl)-2,4-dimethyl-N-((6-methyl-4-(methylthio)-2-oxo-1,2-dihydropyridin-3-yl)methyl)-2-(1-(4,4,4-trifluorobutyl)piperidin-4-yl)benzo[d][1,3]dioxole-5-carboxamide

59) Stereoisomer B of 7-(6-((2S,6R)-2,6-dimethylmorpholino)pyridin-3-yl)-2,4-dimethyl-N-((6-methyl-4-(methylthio)-2-oxo-1,2-dihydropyridin-3-yl)methyl)-2-(1-(2,2,2-trifluoroacetyl)piperidin-4-yl)benzo[d][1,3]dioxole-5-carboxamide

60) Stereoisomer A of 7-(6-((2S,6R)-2,6-dimethylmorpholino)pyridin-3-yl)-2,4-dimethyl-N-((6-methyl-4-(methylthio)-2-oxo-1,2-dihydropyridin-3-yl)methyl)-2-(1-(2,2,2-trifluoroacetyl)piperidin-4-yl)benzo[d][1,3]dioxole-5-carboxamide

61) 2,4-dimethyl-N-((6-methyl-4-(methylthio)-2-oxo-1,2-dihydropyridin-3-yl)methyl)-7-(6-morpholinopyridin-3-yl)-2-(1-(2,2,2-trifluoro)piperidin-4-yl)benzo[d][1,3]dioxole-5-carboxamide

62) Stereoisomer B of 2,4-dimethyl-N-((6-methyl-4-(methylthio)-2-oxo-1,2-dihydropyridin-3-yl)methyl)-7-(6-morpholinopyridin-3-yl)-2-(1-(2,2,2-trifluoro)piperidin-4-yl)benzo[d][1,3]dioxole-5-carboxamide

63) Stereoisomer A of 2,4-dimethyl-N-((6-methyl-4-(methylthio)-2-oxo-1,2-dihydropyridin-3-yl)methyl)-7-(6-morpholinopyridin-3-yl)-2-(1-(2,2,2-trifluoro)piperidin-4-yl)benzo[d][1,3]dioxole-5-carboxamide

64) Stereoisomer B of 2-(1-(2,2-difluoroethyl)piperidin-4-yl)-2,4-dimethyl-N-((6-methyl-4-(methylthio)-2-oxo-1,2-dihydropyridin-3-yl)methyl)-7-(6-morpholinopyridin-3-yl)benzo[d][1,3]dioxole-5-carboxamide

65) Stereoisomer A of 2-(1-(2,2-difluoroethyl)piperidin-4-yl)-2,4-dimethyl-N-((6-methyl-4-(methylthio)-2-oxo-1,2-dihydropyridin-3-yl)methyl)-7-(6-morpholinopyridin-3-yl)benzo[d][1,3]dioxole-5-carboxamide

66) Stereoisomer B of 2-(1-ethylpiperidin-4-yl)-2,4-dimethyl-N-((6-methyl-4-(methylthio)-2-oxo-1,2-dihydropyridin-3-yl)methyl)-7-(6-morpholinopyridin-3-yl)benzo[d][1,3]dioxole-5-carboxamide

67) Stereoisomer A of 2-(1-ethylpiperidin-4-yl)-2,4-dimethyl-N-((6-methyl-4-(methylthio)-2-oxo-1,2-dihydropyridin-3-yl)methyl)-7-(6-morpholinopyridin-3-yl)benzo[d][1,3]dioxole-5-carboxamide

68) 2,4-dimethyl-N-((6-methyl-4-(methylthio)-2-oxo-1,2-dihydropyridin-3-yl)methyl)-7-(2-morpholinopyrimidin-5-yl)-2-(1-(2,2,2-trifluoro)piperidin-4-yl)benzo[d][1,3]dioxole-5-carboxamide

69) Stereoisomer B of 2,4-dimethyl-N-((6-methyl-4-(methylthio)-2-oxo-1,2-dihydropyridin-3-yl)methyl)-7-(2-morpholinopyrimidin-5-yl)-2-(1-(2,2,2-trifluoro)piperidin-4-yl)benzo[d][1,3]dioxole-5-carboxamide

70) Stereoisomer A of 2,4-dimethyl-N-((6-methyl-4-(methylthio)-2-oxo-1,2-dihydropyridin-3-yl)methyl)-7-(2-morpholinopyrimidin-5-yl)-2-(1-(2,2,2-trifluoro)piperidin-4-yl)benzo[d][1,3]dioxole-5-carboxamide

71) Stereoisomer B of 2-(1-(2,2-difluoroethyl)piperidin-4-yl)-2,4-dimethyl-N-((6-methyl-4-(methylthio)-2-oxo-1,2-dihydropyridin-3-yl)methyl)-7-(2-morpholinopyrimidin-5-yl)benzo[d][1,3]dioxole-5-carboxamide

72) Stereoisomer A of 2-(1-(2,2-difluoroethyl)piperidin-4-yl)-2,4-dimethyl-N-((6-methyl-4-(methylthio)-2-oxo-1,2-dihydropyridin-3-yl)methyl)-7-(2-morpholinopyrimidin-5-yl)benzo[d][1,3]dioxole-5-carboxamide

73) Stereoisomer B of 2-(1-ethylpiperidin-4-yl)-2,4-dimethyl-N-((6-methyl-4-(methylthio)-2-oxo-1,2-dihydropyridin-3-yl)methyl)-7-(2-morpholinopyrimidin-5-yl)benzo[d][1,3]dioxole-5-carboxamide

74) Stereoisomer A of 2-(1-ethylpiperidin-4-yl)-2,4-dimethyl-N-((6-methyl-4-(methylthio)-2-oxo-1,2-dihydropyridin-3-yl)methyl)-7-(2-morpholinopyrimidin-5-yl)benzo[d][1,3]dioxole-5-carboxamide

75) 7-(2-(4-ethylpiperazin-1-yl)pyrimidin-5-yl)-2,4-dimethyl-N-((6-methyl-4-(methylthio)-2-oxo-1,2-dihydropyridin-3-yl)methyl)-2-(1-(2,2,2-trifluoroethyl)piperidin-4-yl)benzo[d][1,3]dioxole-5-carboxamide

76) Stereoisomer B of 7-(2-(4-ethylpiperazin-1-yl)pyrimidin-5-yl)-2,4-dimethyl-N-((6-methyl-4-(methylthio)-2-oxo-1,2-dihydropyridin-3-yl)methyl)-2-(1-(2,2,2-trifluoroethyl)piperidin-4-yl)benzo[d][1,3]dioxole-5-carboxamide

77) Stereoisomer A of 7-(2-(4-ethylpiperazin-1-yl)pyrimidin-5-yl)-2,4-dimethyl-N-((6-methyl-4-(methylthio)-2-oxo-1,2-dihydropyridin-3-yl)methyl)-2-(1-(2,2,2-trifluoroethyl)piperidin-4-yl)benzo[d][1,3]dioxole-5-carboxamide

78) 7-(2-(ethyl(methyl)amino)pyridin-5-yl)-2,4-dimethyl-N-((6-methyl-4-(methylthio)-2-oxo-1,2-dihydropyridin-3-yl)methyl)-2-(1-(2,2,2-trifluoroethyl)piperidin-4-yl)benzo[d][1,3]dioxole-5-carboxamide

79) Stereoisomer B of 7-(2-(ethyl(methyl)amino)pyridin-5-yl)-2,4-dimethyl-N-((6-methyl-4-(methylthio)-2-oxo-1,2-dihydropyridin-3-yl)methyl)-2-(1-(2,2,2-trifluoroethyl)piperidin-4-yl)benzo[d][1,3]dioxole-5-carboxamide

80) Stereoisomer A of 7-(2-(ethyl(methyl)amino)pyridin-5-yl)-2,4-dimethyl-N-((6-methyl-4-(methylthio)-2-oxo-1,2-dihydropyridin-3-yl)methyl)-2-(1-(2,2,2-trifluoroethyl)piperidin-4-yl)benzo[d][1,3]dioxole-5-carboxamide

81) Stereoisomer B of 2-(1-(2,2-difluoroethyl)piperidin-4-yl)-7-(2-(ethyl(methyl)amino)pyrimidin-5-yl)-2,4-dimethyl-N-((6-methyl-4-(methylthio)-2-oxo-1,2-dihydropyridin-3-yl)methyl)benzo[d][1,3]dioxole-5-carboxamide

82) Stereoisomer A of 2-(1-(2,2-difluoroethyl)piperidin-4-yl)-7-(2-(ethyl(methyl)amino)pyrimidin-5-yl)-2,4-dimethyl-N-((6-methyl-4-(methylthio)-2-oxo-1,2-dihydropyridin-3-yl)methyl)benzo[d][1,3]dioxole-5-carboxamide

83) Stereoisomer B of 7-(2-(ethyl(methyl)amino)pyrimidin-5-yl)-2-(1-ethylpiperidin-4-yl)-2,4-dimethyl-N-((6-methyl-4-(methylthio)-2-oxo-1,2-dihydropyridin-3-yl)methyl)benzo[d][1,3]dioxole-5-carboxamide

84) Stereoisomer A of 7-(2-(ethyl(methyl)amino)pyrimidin-5-yl)-2-(1-ethylpiperidin-4-yl)-2,4-dimethyl-N-((6-methyl-4-(methylthio)-2-oxo-1,2-dihydropyridin-3-yl)methyl)benzo[d][1,3]dioxole-5-carboxamide

85) 7-(6-(ethyl(methyl)amino)pyridin-3-yl)-2,4-dimethyl-N-((6-methyl-4-(methylthio)-2-oxo-1,2-dihydropyridin-3-yl)methyl)-2-(1-(2,2,2-trifluoro)piperidin-4-yl)benzo[d][1,3]dioxole-5-carboxamide

86) Stereoisomer B of 7-(6-(ethyl(methyl)amino)pyridin-3-yl)-2,4-dimethyl-N-((6-methyl-4-(methylthio)-2-oxo-1,2-dihydropyridin-3-yl)methyl)-2-(1-(2,2,2-trifluoroethyl)piperidin-4-yl)benzo[d][1,3]dioxole-5-carboxamide

87) Stereoisomer A of 7-(6-(ethyl(methyl)amino)pyridin-3-yl)-2,4-dimethyl-N-((6-methyl-4-(methylthio)-2-oxo-1,2-dihydropyridin-3-yl)methyl)-2-(1-(2,2,2-trifluoroethyl)piperidin-4-yl)benzo[d][1,3]dioxole-5-carboxamide

88) Stereoisomer B of 2-(1-(2,2-difluoroethyl)piperidin-4-yl)-7-(6-(ethyl(methyl)amino)pyridin-3-yl)-2,4-dimethyl-N-((6-methyl-4-(methylthio)-2-oxo-1,2-dihydropyridin-3-yl)methyl)benzo[d][1,3]dioxole-5-carboxamide

89) Stereoisomer B of 7-(6-(ethyl(methyl)amino)pyridin-3-yl)-2-(1-ethylpiperidin-4-yl)-2,4-dimethyl-N-((6-methyl-4-(methylthio)-2-oxo-1,2-dihydropyridin-3-yl)methyl)benzo[d][1,3]dioxole-5-carboxamide
90) 2,4-dimethyl-N-((6-methyl-4-(methylthio)-2-oxo-1,2-dihydropyridin-3-yl)methyl)-7-(4-(piperidin-1-yl)phenyl)-2-(1-(2,2,2-trifluoroethyl)piperidin-4-yl)benzo[d][1,3]dioxole-5-carboxamide
91) 2,4-dimethyl-N-((6-methyl-4-(methylthio)-2-oxo-1,2-dihydropyridin-3-yl)methyl)-7-(6-(piperidin-1-yl)pyridin-3-yl)-2-(1-(2,2,2-trifluoroethyl)piperidin-4-yl)benzo[d][1,3]dioxole-5-carboxamide
92) 2,4-dimethyl-N-((6-methyl-4-(methylthio)-2-oxo-1,2-dihydropyridin-3-yl)methyl)-7-(2-thiomorpholinopyrimidin-5-yl)-2-(1-(2,2,2-trifluoroethyl)piperidin-4-yl)benzo[d][1,3]dioxole-5-carboxamide
93) 2',2'-difluoro-2,7-dimethyl-N-((6-methyl-4-(methylthio)-2-oxo-1,2-dihydropyridin-3-yl)methyl)-2-(1-(2,2,2-trifluoroethyl)piperidin-4-yl)-[4,5'-bibenzo[d][1,3]dioxole]-6-carboxamide
94) 2,7-dimethyl-N-((6-methyl-4-(methylthio)-2-oxo-1,2-dihydropyridin-3-yl)methyl)-2-(1-(2,2,2-trifluoroethyl)piperidin-4-yl)-[4,5'-bibenzo[d][1,3]dioxole]-6-carboxamide
95) 2-(1-(2,2-difluoroethyl)piperidin-4-yl)-2,7-dimethyl-N-((6-methyl-4-(methylthio)-2-oxo-1,2-dihydropyridin-3-yl)methyl)-[4,5'-bibenzo[d][1,3]dioxole]-6-carboxamide
96) 7-(benzofuran-5-yl)-2,4-dimethyl-N-((6-methyl-4-(methylthio)-2-oxo-1,2-dihydropyridin-3-yl)methyl)-2-(1-(2,2,2-trifluoroethyl)piperidin-4-yl)benzo[d][1,3]dioxole-5-carboxamide
97) 7-(benzofuran-5-yl)-2-(1-(2,2-difluoroethyl)piperidin-4-yl)-2,4-dimethyl-N-((6-methyl-4-(methylthio)-2-oxo-1,2-dihydropyridin-3-yl)methyl)benzo[d][1,3]dioxole-5-carboxamide
98) 7-(6-(butyl(methyl)amino)pyridin-3-yl)-2,4-dimethyl-N-((6-methyl-4-(methylthio)-2-oxo-1,2-dihydropyridin-3-yl)methyl)-2-(1-(2,2,2-trifluoroethyl)piperidin-4-yl)benzo[d][1,3]dioxole-5-carboxamide
99) 7-(6-(butyl(methyl)amino)pyridin-3-yl)-2,4-dimethyl-N-((6-methyl-4-(methylthio)-2-oxo-1,2-dihydropyridin-3-yl)methyl)-2-(1-(2,2,2-trifluoroethyl)piperidin-4-yl)benzo[d][1,3]dioxole-5-carboxamide
100) 2-(trans-4-aminocyclohexyl)-7-(6-((2S,6R)-2,6-dimethylmorpholino)pyridin-3-yl)-2,4-dimethyl-N-((6-methyl-4-(methylthio)-2-oxo-1,2-dihydropyridin-3-yl)methyl)benzo[d][1,3]dioxole-5-carboxamide hydrochloride
101) 7-(6-((2S,6R)-2,6-dimethylmorpholino)pyridin-3-yl)-2-(trans-4-(3-hydroxyazetidin-1-yl)cyclohexyl)-2,4-dimethyl-N-((6-methyl-4-(methylthio)-2-oxo-1,2-dihydropyridin-3-yl)methyl)benzo[d][1,3]dioxole-5-carboxamide
102) 7-(6-((2S,6R)-2,6-dimethylmorpholino)pyridin-3-yl)-2,4-dimethyl-N-((6-methyl-4-(methylthio)-2-oxo-1,2-dihydropyridin-3-yl)methyl)-2-(trans-4-(3-(2,2,2-trifluoroethyl)azetidin-1-yl)cyclohexyl)benzo[d][1,3]dioxole-5-carboxamide
103) 2-(trans-4-(3-(2,2-difluoroethoxy)azetidin-1-yl)cyclohexyl)-7-(6-((2S,6R)-2,6-dimethylmorpholino)pyridin-3-yl)-2,4-dimethyl-N-((6-methyl-4-(methylthio)-2-oxo-1,2-dihydropyridin-3-yl)methyl)benzo[d][1,3]dioxole-5-carboxamide
104) 2-(trans-4-(3-(2,2-difluoroethoxy)azetidin-1-yl)cyclohexyl)-7-(6-((2S,6R)-2,6-dimethylmorpholino)pyridin-3-yl)-2,4-dimethyl-N-((6-methyl-4-(methylthio)-2-oxo-1,2-dihydropyridin-3-yl)methyl)benzo[d][1,3]dioxole-5-carboxamide
105) 7-(6-((2S,6R)-2,6-dimethylmorpholino)pyridin-3-yl)-2-(trans-4-(3-isopropoxyazetidin-1-yl)cyclohexyl)-2,4-dimethyl-N-((6-methyl-4-(methylthio)-2-oxo-1,2-dihydropyridin-3-yl)methyl)benzo[d][1,3]dioxole-5-carboxamide
106) 7-(6-((2S,6R)-2,6-dimethylmorpholino)pyridin-3-yl)-2-(trans-4-(3-methoxyazetidin-1-yl)cyclohexyl)-2,4-dimethyl-N-((6-methyl-4-(methylthio)-2-oxo-1,2-dihydropyridin-3-yl)methyl)benzo[d][1,3]dioxole-5-carboxamide
107) Stereoisomer B of 7-(6-((2S,6R)-2,6-dimethylmorpholino)pyridin-3-yl)-2,4-dimethyl-2-(trans-4-(methyl(2,2,2-trifluoroethyl)amino)cyclohexyl)-N-((6-methyl-4-(methylthio)-2-oxo-1,2-dihydropyridin-3-yl)methyl)benzo[d][1,3]dioxole-5-carboxamide
108) Stereoisomer A of 7-(6-((2S,6R)-2,6-dimethylmorpholino)pyridin-3-yl)-2,4-dimethyl-2-(trans-4-(methyl(2,2,2-trifluoroethyl)amino)cyclohexyl)-N-((6-methyl-4-(methylthio)-2-oxo-1,2-dihydropyridin-3-yl)methyl)benzo[d][1,3]dioxole-5-carboxamide
109) 2-(trans-4-((2,2-difluoroethyl(methyl)amino)cyclohexyl)-7-(6-((2S,6R)-2,6-dimethylmorpholino)pyridin-3-yl)-2,4-dimethyl-N-((6-methyl-4-(methylthio)-2-oxo-1,2-dihydropyridin-3-yl)methyl)benzo[d][1,3]dioxole-5-carboxamide
110) 7-(6-((2S,6R)-2,6-dimethylmorpholino)pyridin-3-yl)-2-(trans-4-(isopropyl(methyl)amino)cyclohexyl)-2,4-dimethyl-N-((6-methyl-4-(methylthio)-2-oxo-1,2-dihydropyridin-3-yl)methyl)benzo[d][1,3]dioxole-5-carboxamide
111) 7-(6-((2S,6R)-2,6-dimethylmorpholino)pyridin-3-yl)-2-(trans-4-(ethyl(methyl)amino)cyclohexyl)-2,4-dimethyl-N-((6-methyl-4-(methylthio)-2-oxo-1,2-dihydropyridin-3-yl)methyl)benzo[d][1,3]dioxole-5-carboxamide
112) 7-(6-((2S,6R)-2,6-dimethylmorpholino)pyridin-3-yl)-2,4-dimethyl-2-(trans-4-(methyl(phenethyl)amino)cyclohexyl)-N-((6-methyl-4-(methylthio)-2-oxo-1,2-dihydropyridin-3-yl)methyl)benzo[d][1,3]dioxole-5-carboxamide
113) 7-(6-((2S,6R)-2,6-dimethylmorpholino)pyridin-3-yl)-2,4-dimethyl-2-(trans-4-(methyl(phenethyl)amino)cyclohexyl)-N-((6-methyl-4-(methylthio)-2-oxo-1,2-dihydropyridin-3-yl)methyl)benzo[d][1,3]dioxole-5-carboxamide
114) 2-(trans-4-(cyclohexyl(methyl)amino)cyclohexyl)-7-(6-((2S,6R)-2,6-dimethylmorpholino)pyridin-3-yl)-2,4-dimethyl-N-((6-methyl-4-(methylthio)-2-oxo-1,2-dihydropyridin-3-yl)methyl)benzo[d][1,3]dioxole-5-carboxamide
115) 7-(6-((2S,6R)-2,6-dimethylmorpholino)pyridin-3-yl)-2,4-dimethyl-N-((6-methyl-4-(methylthio)-2-oxo-1,2-dihydropyridin-3-yl)methyl)-2-(trans-4-(2,2,2-trifluoro-N-methylacetamido)cyclohexyl)benzo[d][1,3]dioxole-5-carboxamide

In the present invention, the term "pharmaceutically acceptable" may refer to the one which is physiologically acceptable and does not conventionally cause an allergic response such as gastrointestinal disturbance and dizziness, or other responses similar thereto, when administered into an individual.

The pharmaceutically acceptable salt of the present invention may be prepared by a conventional method known to those skilled in the art.

The pharmaceutically acceptable salt of the present invention may not be particularly limited as long as it is prepared using an acid forming a non-toxic acid addition salt containing a pharmaceutically acceptable anion. For example, inorganic acid salts prepared from hydrochloric acid, sulfuric acid, nitric acid, phosphoric acid, hydrobromic acid, hydroiodic acid, etc.; organic acid salts prepared from tartaric acid, formic acid, citric acid, acetic acid, trichloroacetic acid, trifluoroacetic acid, gluconic acid, benzoic acid, lactic acid, fumaric acid, maleic acid, etc.; and sulfonic acid salts prepared from methanesulfonic acid, benzenesulfonic acid, p-toluenesulfonic acid, naphthalenesulfonic acid, etc., but are not limited thereto. In one embodiment of the present invention, the pharmaceutically acceptable salt may be hydrochloride.

In the present invention, a compound according to any one of above **(1)** to **(5)** may have at least one asymmetric carbon center, and thus may be present as an enantiomer mixture including a racemic mixture, a single enantiomer (optical isomer), a mixture of diastereomers, and a single diastereomer. Such isomer may be separated by split according to the related art, for example, column chromatography, HPLC or the like. Alternatively, the isomer may be stereospecifically synthesized by using a known array of optically pure starting materials and/or reagents. Specifically, the stereoisomer may be an optical isomer.

In the present invention, the "hydrate" may refer to the one in which a compound according to any one of above **(1)** to **(5)** or a pharmaceutically acceptable salt thereof and water are bound by a non-covalent intermolecular force, and may include a stoichiometric or non-stoichiometric amount of water.

In the present invention, the "solvate" may refer to the one in which a compound according to any one of above **(1)** to **(5)** or a pharmaceutically acceptable salt thereof and a solvent other than water are bound by a non-covalent intermolecular force, and may include a stoichiometric or non-stoichiometric amount of the solvent.

A compound according to any one of above **(1)** to **(5)** of the present invention, a stereoisomer thereof, a pharmaceutically acceptable salt thereof, a hydrate or solvate thereof may inhibit cell proliferation by suppressing the activity of EZH1 and/or EZH2, and may also be useful in preventing or treating various diseases related to the activity of EZH1 and/or EZH2 with excellent in vivo and blood absorption properties.

In other words, a compound according to any one of above **(1)** to **(5)** of the present invention, a stereoisomer thereof, a pharmaceutically acceptable salt thereof, a hydrate or solvate thereof may also be useful in preventing or treating EZH1 and/or EZH2 activity-associated diseases.

**Method for preparing 1,3-benzodioxole derivative compound**

The present invention provides a method for preparing a compound according to any one of above **(1)** to **(5),** a stereoisomer thereof or a pharmaceutically acceptable salt thereof.

A compound according to any one of above **(1)** to **(5)** of the present invention, a stereoisomer thereof or a pharmaceutically acceptable salt thereof may be prepared, for example, according to a method of Reaction Formula 1 below, but is not limited thereto.

<Reaction Formula 1>

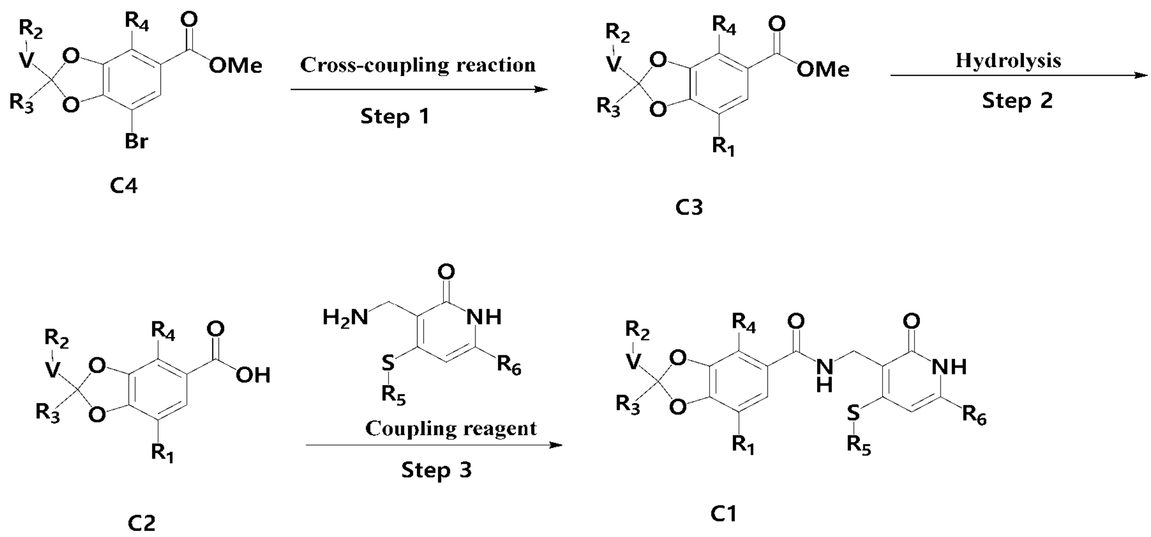


In above Reaction Formula 1, each of $R_1$ to $R_6$ and V of C1 to C4 is the same as defined in the section of the compound represented by above chemical formula I.

**[Step 1] Cross-coupling reaction**

This is a step of preparing above compound C3 in such a way that above compound C4 is stirred under heating conditions for 0.5 to 24 hours by using a palladium catalyst or a nickel catalyst; and an equivalent or excessive amount of boronic acid, boronic acid pinacol ester (for Suzuki-Miyaura coupling), an organotin reagent (for Stille coupling), or an alkene compound (for Heck reaction) in the presence of a base in a solvent inert to a reaction.

Specifically, above compound C3 is prepared by stirring at 60 to 120°C for 0.5 to 12 hours. Said solvent may not be particularly limited as long as it is inert to this reaction, but methanol, ethanol, tetrahydrofuran, 1,2-dimethoxyethane, 1,4-dioxane, water, N,N-dimethylformamide, dimethyl sulfoxide, benzene, toluene, xylene, or a mixture thereof may be used. As said palladium catalyst, tetrakis(triphenylphosphine)palladium, [1,1'-bis(diphenylphosphino)ferrocene]dichloropalladium, tris(dibenzylideneacetone)dipalladium, palladium acetate, acetylacetonpalladium, bis(triphenylphosphine) palladium dichloride, or the like may be used. As said nickel catalyst, [1,1-bis(diphenylphosphino)ferrocene]nickel dichloride, bis(triphenylphosphine)nickel dichloride, or the like may be used. As said base, an organic base such as triethylamine, diisopropylethylamine, 1,8-diazabicyclo[5.4.0]-7-undecene (DBU), 1,5-diazabicyclo[4.3.0]-5-nonene (DBN), or the like; or an inorganic base such as potassium hydrogen carbonate, sodium hydrogen carbonate, potassium carbonate, sodium carbonate, potassium hydroxide, sodium hydroxide, potassium phosphate, sodium phosphate, or the like may be used.

**[Step 2] Hydrolysis reaction**

This is a step of preparing above compound C2 in such a way that above compound C3 is stirred under cooling or heating conditions for three to 96 hours using an equivalent or excessive amount of base aqueous solution in a solvent. Specifically, above compound C2 may be prepared by stirring at room temperature (10 to 25°C) to 60°C for three to 48 hours. Said solvent may not be particularly limited, as long as it is a solvent that does not inhibit this reaction, but methanol, ethanol, tetrahydrofuran, dimethoxyethane, acetonitrile, a mixture thereof, or the like may be used. As said base, an inorganic base such as lithium hydroxide, sodium hydroxide, potassium hydroxide, or the like may be used.

**[Step 3] Amidation reaction**

This is a step of preparing above compound C1 in such a way that above compound C2 is stirred under cooling or heating conditions for one to 24 hours using an equivalent or excessive amount of corresponding amine and condensing agent in a solvent inert to a reaction. Specifically, above compound C1 may be prepared by stirring at room temperature to 120°C for one to eight hours. Said solvent may not be particularly limited, as long as it is inert to this reaction, but N,N-dimethylformamide, dimethylacetamide, dichloromethane, 1,2-dichloroethane, chloroform, tetrahydrofuran, 1,2-dimethoxyethane, acetonitrile, a mixture thereof, or the like may be used. As said condensing agent, pentafluorophenyl

trifluoroacetate, dicyclocarbodiimide (DCC), 1-ethyl-3-(3-dimethylaminopropyl)carbodiimide (EDCI), 1,1'-carbonyldiimidazole, etc. may be used. In addition, additives or bases may be further used in the reaction. As said additive, N-hydroxysuccinimide (HOSu), 1-hydroxybenzotriazole (HOBt), 1-hydroxy-7-azabenzotriazole (HOAt), etc. may be used. As said base, an organic base such as triethylamine, diisopropylethylamine, etc.; or an inorganic base such as potassium carbonate, sodium carbonate, potassium hydroxide, sodium hydroxide, or the like may be used.

**Composition including 1,3-benzodioxole derivative compound, treatment method using same and use thereof**

The present invention provides a pharmaceutical composition including a compound according to any one of above **(1)** to **(5),** a stereoisomer thereof, a pharmaceutically acceptable salt thereof, a hydrate or solvate thereof as an active ingredient.

The present invention provides a pharmaceutical composition for preventing or treating enhancer of zeste homolog 1 (EZH1) or enhancer of zeste homolog 2 (EZH2) activity-associated diseases, including a compound according to any one of above **(1)** to **(5),** a stereoisomer thereof, a pharmaceutically acceptable salt thereof, a hydrate or solvate thereof as an active component.

In the present invention, the term "prevention" means all the acts, which inhibit or delay the occurrence of diseases by administering a compound according to any one of above **(1)** to **(5)** of the present invention, a stereoisomer thereof, a pharmaceutically acceptable salt thereof, a hydrate or solvate thereof; or a pharmaceutical composition including the same.

In the present invention, the term "treatment" means all the acts, by which a symptom of an individual likely to develop or suffering from a disease gets better or takes a favorable turn by administering a compound according to any one of above **(1)** to **(5)** of the present invention, a stereoisomer thereof, a pharmaceutically acceptable salt thereof, a hydrate or solvate thereof; or a pharmaceutical composition including the same.

In the present invention, the term "EZH1 or EZH2 activity-associated diseases" means diseases which may be prevented or treated by inhibiting the activity of one or more enzymes selected from EZH1 and EZH2. The term "activity of at least one enzyme selected from EZH1 and EZH2" means the activity of an enzyme having a methyl group introduced into 27th lysine of histone H3 of EZH1 and/or EZH2.

In the present invention, the EZH1 or EZH2 activity-associated disease may be a solid cancer in which cancer cells grow to form a lump. Said solid cancer may be at least one selected from rhabdoid tumor, melanoma, pancreatic cancer, colorectal cancer, adrenal cancer, prostate cancer, bladder cancer, lung cancer, breast cancer, ovarian cancer, liver cancer, brain cancer, stomach cancer, renal cancer, thyroid cancer, epithelioid sarcoma, synovial sarcoma, and the like.

In this regard, in one specific embodiment of the present invention, the inhibitory activity of a compound according to any one of above **(1)** to **(5)** of the present invention, a stereoisomer thereof, or a pharmaceutically acceptable salt thereof on EZH1 and/or EZH2 and tumor proliferation was confirmed.

The pharmaceutical composition of the present invention may inhibit the activity of at least one enzyme selected from EZH1 and EZH2, and thus may be advantageously used in the prevention or treatment of various diseases related thereto.

The pharmaceutical composition of the present invention may further include at least one type of a pharmaceutically acceptable carrier, in addition to a compound according to any one of above **(1)** to **(5),** a stereoisomer thereof, a pharmaceutically acceptable salt thereof, a hydrate or solvate thereof. The pharmaceutically acceptable carriers may be those conventionally used in the art, specifically including, but not limited thereto, lactose, dextrose, sucrose, sorbitol, mannitol, glycine, starch, tragacanth rubber, acacia rubber, calcium phosphate, calcium chloride, sodium chloride, alginic acid, sodium alginate, gelatin, calcium silicate, microcrystalline cellulose, polyvinyl pyrrolidine, polyethylene glycol, cellulose, water, ethanol, syrup, methyl cellulose, methyl hydroxybenzoate, propyl hydroxybenzoate, talc, magnesium stearate, magnesium aluminum silicate, silica, orange essence, strawberry essence, vanilla essence, or mineral oil.

The pharmaceutical composition of the present invention may further include binders, isotonic agents, adsorbents, disintegrants, antioxidants, glidants, fillers, solubilizers, solubilizing aids, lubricants, humectants, sweetening agents, flavoring agents, emulsifiers, suspending agents, preservatives, dispersing agents, stabilizing agents, or the like, in addition to said ingredients. In addition, the pharmaceutical composition of the present invention may be formulated into an oral dosage form such as tablet, pill, powder, granule, capsule, suspension, emulsion, liquid for internal use, oiling agent, syrup, etc., as well as a form of external preparation, suppository or sterile injection by using a pharmaceutically acceptable carrier and excipient, and thus may be prepared in a unit dose form or prepared by being inserted into a multidose container. Such preparations may be prepared according to a conventional method used for formulation in the art or a method disclosed in Remington's Pharmaceutical Science ($19^{th}$ ed., 1995), and may be formulated into various preparations depending on each disease or ingredient.

The pharmaceutical composition of the present invention may be subject to oral administration or parenteral administration depending on an intended method, preferably oral administration, but is not limited thereto.

Non-limiting examples of formulations for oral administration may include tablets, pills, powders, capsules, syrups, emulsions, or the like, but are not limited thereto. If the pharmaceutical composition of the present invention is in the form of oral administration, the pharmaceutically acceptable carriers used may include cellulose, calcium silicate, corn starch, lactose, sucrose, dextrose, calcium phosphate, stearic acid, magnesium stearate, calcium stearate, gelatin, talc, etc., but are limited thereto.
Non-limiting examples of formulations for parenteral administration may include injections, etc., but are not limited thereto. If the pharmaceutical composition of the present invention is in the form of parenteral administration, the pharmaceutically acceptable carriers used may include water, saline solution, glucose aqueous solution, pseudosugar aqueous solution, alcohol, glycol, ether, oil, fatty acid, fatty acid ester, glyceride, etc., but are not limited thereto.
A daily dosage of a compound according to any one of above **(1)** to **(5)** of the present invention, a stereoisomer thereof, a pharmaceutically acceptable salt thereof, a hydrate or solvate thereof may be about 0.1 to about 2,000 mg/day for an adult who has a weight of 70 kg, but is not limited thereto, and may be administered at one time a day or several times a day by dividing the daily dosage of the compound. Said dosage may vary depending on a patient's health condition, age, weight, gender, dosage form, and disease severity.
A pharmaceutically effective dose and an effective dosage of the pharmaceutical composition of the present invention may vary depending on a method for formulating the pharmaceutical composition, an administration mode, an administration time, an administration route, and/or the like, and may be diversified according to various factors including a type and degree of reaction to be achieved by administration of the pharmaceutical composition, a type of an individual for administration, the individual's age, weight, general health condition, disease symptom or severity, gender, diet and excretion, ingredients of other drug compositions to be used for the corresponding individual at the same time or different times, etc., as well as other similar factors well known in a pharmaceutical field, and those skilled in the art may easily determine and prescribe an effective dosage for intended treatment.
The pharmaceutical composition of the present invention may be administered once a day or several times a day by dividing the daily dosage of the composition. The pharmaceutical composition of the present invention may be administered as an individual therapeutic agent or in combination with other therapeutic agents, and may be administered sequentially or simultaneously with a conventional therapeutic agent. Considering all the above factors, the pharmaceutical composition of the present invention may be administered in such an amount that a maximum effect may be achieved by a minimum amount without a side effect, and such amount may be easily determined by those skilled in the art to which the present invention pertains.
The pharmaceutical composition of the present invention may show an excellent effect even when solely used, but may be further used in combination with various methods such as hormone therapy, drug treatment, etc. in order to increase a therapeutic efficiency.
The present invention provides a method for preventing or treating EZH1 or EZH2 activity-associated diseases, including administering a compound according to any one of above **(1)** to **(5),** a stereoisomer thereof, a pharmaceutically acceptable salt thereof, a hydrate or solvate thereof, or a pharmaceutical composition including the same into an individual.
In the present invention, the term "administration" refers to introducing a predetermined substance into an individual by an appropriate method.
In the present invention, the term "individual" refers to all the animals such as rats, mice, livestock, etc., including humans, who are likely to develop or have already developed EZH1 or EZH2 activity-associated diseases, and specifically may refer to mammals including humans, but is not limited thereto.
The method for preventing and treating EZH1 or EZH2 activity-associated diseases of the present invention may include administering a therapeutically effective amount of a compound according to any one of above **(1)** to **(5),** a stereoisomer thereof, or a pharmaceutically acceptable salt thereof.
In the present invention, the term "therapeutically effective amount" refers to an amount enough to treat a disease at a reasonable risk/benefit ratio applicable to medical treatment and not to cause a side effect, and may be determined by those skilled in the art according to factors including a patient's gender, age, weight and health condition, a type of disease, severity, activity of a drug, sensitivity to a drug, an administration method, an administration time, an administration route, an excretion rate, a treatment period, a drug combined or concurrently used, as well as other factors well known in a pharmaceutical field. It is preferable to differently apply a specific therapeutically effective amount for a certain patient depending on various factors including a type and degree of reaction to be achieved therefrom, a specific composition including a presence of other preparations used in some cases, a patient's age, weight, general health condition, gender and diet, an administration time, an administration route, a secretion rate of the composition, a treatment period and a drug used together with the specific composition or simultaneously therewith, as well as other similar factors well known in a pharmaceutical field.
The method for preventing or treating EZH1 or EZH2 activity-associated diseases according to the present invention includes not only dealing with the diseases per se before expression of their symptoms, but also inhibiting or avoiding such symptoms by administering a compound according to any one of above **(1)** to **(5),** a stereoisomer thereof, a

pharmaceutically acceptable salt thereof, a hydrate or solvate thereof or a pharmaceutical composition including the same. In managing the disease, a preventive or therapeutic dose of a certain active ingredient may vary depending on properties and severity of the disease or condition, and a route in which the active ingredient is administered. A dose and a frequency thereof may vary depending on an individual patient's age, weight and reactions. A suitable dose and usage may be easily selected by those skilled in the art, naturally considering such factors. In addition, the method for preventing or treating EZH1 or EZH2 activity-associated diseases according to the present invention may further include administering a therapeutically effective amount of an additional active agent, which is helpful in preventing or treating the diseases, along with a compound according to any one of above **(1)** to **(5),** a stereoisomer thereof, a pharmaceutically acceptable salt thereof, a hydrate or solvate thereof. The additional active agent may show a synergy effect or an additive effect together with a compound according to any one of above **(1)** to **(5),** a stereoisomer thereof, a pharmaceutically acceptable salt thereof, a hydrate or solvate thereof.

The present invention provides a use of a compound according to any one of above **(1)** to **(5),** a stereoisomer thereof, a pharmaceutically acceptable salt thereof, a hydrate or solvate thereof, or a pharmaceutical composition including the same for preventing or treating EZH1 or EZH2 activity-associated diseases.

The present invention provides a use of a compound according to any one of above **(1)** to **(5),** a stereoisomer thereof, a pharmaceutically acceptable salt thereof, a hydrate or solvate thereof, or a pharmaceutical composition including the same for preparing a medication for preventing or treating EZH1 or EZH2 activity-associated diseases.

For preparing a medication, a compound according to any one of above **(1)** to **(5)** of the present invention, a stereoisomer thereof, a pharmaceutically acceptable salt thereof, a hydrate or solvate thereof may be mixed with pharmaceutically acceptable adjuvants, diluents, carriers, etc., and may be prepared into a complex preparation together with other active agents, thus providing a synergy action.

Matters mentioned in each of the items of the present invention, that is, the 1,3-benzodioxole derivative compound, the preparation method thereof, the pharmaceutical composition including the same, the treatment method using the same, and the use thereof are applied the same, if not contradictory to each other.

## Advantageous Effects

A compound represented by chemical formula I of the present invention, a stereoisomer thereof, a pharmaceutically acceptable salt thereof, a hydrate or solvate thereof, and a pharmaceutical composition including the same as an active ingredient may be advantageously used for preventing or treating EZH1 or EZH2 activity-associated diseases.

## Mode for Invention

Hereinafter, the present invention will be described in more detail through exemplary embodiments. These exemplary embodiments are provided only for the purpose of illustrating the present invention, and thus it will be apparent to those skilled in the art that the scope of the present invention is not limited thereto.

### Preparation of compound represented by chemical formula I

The compound represented by chemical formula I of the present invention may be prepared through the method described below. Unless otherwise described, a starting material may be commercially available or may be prepared by known methods. All examples provided herein, or the use of an exemplary language, are merely intended to better illustrate the invention and do not limit the scope of the claimed invention.

### <Preparation Example>

Hereinafter, in the preparation example, a specific optical rotation was measured by JASCO P-2000 Digital Polarimeter as an instrumental model.

### Preparation Example 1. Synthesis of 2-(trans-4-(dimethylamino)cyclohexyl)-7-(6-((2S,6R)-2,6-dimethylmorpholino)pyridin-3-yl)-2,4-dimethyl-N-((6-methyl-4-(methylthio)-2-oxo-1.2-dihydropyridin-3-yl)methyl)benzo[d][1,3]dioxole-5-carboxamide and hydrochloride thereof (compound 1 and hydrochloride thereof)

<Reaction Formula 2>

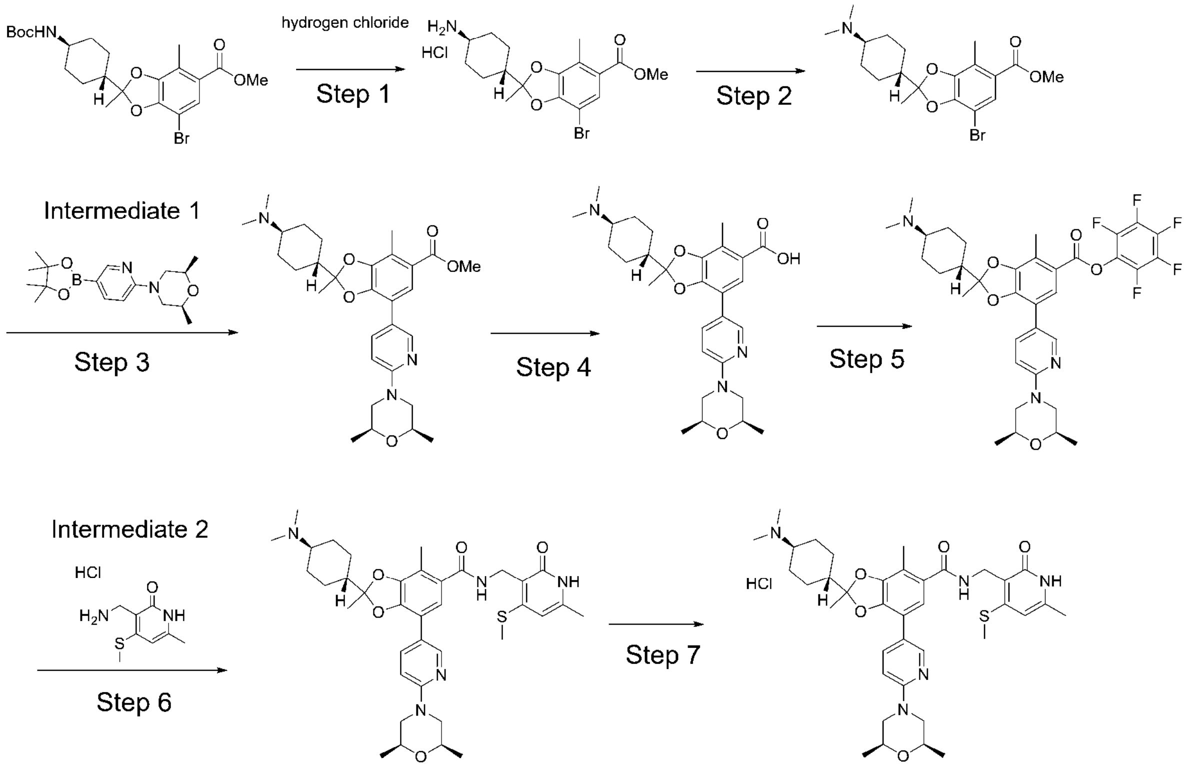


**[Step 1] Synthesis of methyl 2-(trans-4-aminocyclohexyl)-7-bromo-2,4-dimethylbenzo[d][1,3]dioxole-5-carboxylate hydrochloride**

A 1 M-hydrochloric acid aqueous solution (80 mL) was added to methyl 7-bromo-2-(trans-4-((tert-butoxycarbonyl)amino)cyclohexyl)-2,4-dimethylbenzo[d][1,3]dioxole-5-carboxylate (20 g, 41.3 mmole), and the resulting mixture was heated and stirred. When the reaction was completed, an internal temperature was cooled to room temperature, and isopropyl alcohol (80 mL) was added dropwise and stirred. The resulting crystal was filtered and dried at an internal temperature of 60°C to obtain the title compound (17.2 g).

**[Step 2] Synthesis of methyl 7-bromo-2-(trans-4-(dimethylamino)cyclohexyl)-2,4-dimethylbenzo[d][1,3]dioxole-5-carboxylate**

Methanol (173 mL) was added to the compound (17 g, 41 mmole) obtained in above [Step 1] and stirred at room temperature. Dimethylamine (23 mL) was added and stirred. A 35% formaldehyde aqueous solution (12 mL) was slowly added and stirred. After cooling, sodium triacetoxyborohydride (35 g) was slowly added thereto and stirred at room temperature. When the reaction was completed, dichloromethane (173 mL) was added thereto and a sodium bicarbonate aqueous solution (173 mL) was added thereto to perform washing. Then, magnesium sulfate was added, filtered, and concentrated to obtain the title compound (16.8 g).

**[Step 3] Synthesis of methyl 2-(trans-4-(dimethylamino)cyclohexyl)-7-(6-((2S,6R)-2,6-dimethylmorpholino)pyridin-3-yl)-2,4-dimethylbenzo[d][1,3]dioxole-5-carboxylate**

Dioxane (160 mL) and purified water (80 mL) were added to the compound (16 g, 39 mmole) obtained in above [Step 2]. Subsequently, (2S,6R)-2,6-dimethyl-4-(5-(4,4,5,5-tetramethyl-1,3,2-dioxaborolan-2-yl)pyridin-2-yl)morpholine (16 g) was added thereto, tetrakis(triphenylphosphine)palladium (4.5 g) was added thereto, sodium carbonate (12.3 g) was added thereto, and the resulting mixture was heated and stirred. When the reaction was completed, hexane was added to filter the precipitated solid using silica/celite, and the filtrate was washed with purified water (160 mL). Magnesium sulfate was added thereto, filtered, and concentrated to obtain the title compound (20 g).

**[Step 4] Synthesis of 2-(trans-4-(dimethylamino)cyclohexyl)-7-(6-((2S,6R)-2,6-dimethylmorpholino)pyridin-3-yl)-2,4-dimethylbenzo[d][1,3]dioxole-5-carboxylic acid**

Tetrahydrofuran (160 mL) and methanol (80 mL) were added to the compound (20 g, 38.2 mmole) obtained in above [Step 3]. A 2 N-sodium hydroxide aqueous solution (76 mL) was added thereto, heated, and stirred. When the reaction was completed, a 2 N-hydrochloric acid aqueous solution (57 mL) was added thereto and concentrated. The resulting crystal was filtered, and ethyl acetate (400 mL) was added thereto. Then, purified water (200 mL) was added thereto to perform washing, and magnesium sulfate was added thereto, filtered, and then concentrated to obtain the title compound (19.2 g).

**[Step 5] Synthesis of pentafluorophenyl 2-(trans-4-(dimethylamino)cyclohexyl)-7-(6-((2S,6R)-2,6-dimethylmorpholino)pyridin-3-yl)-2,4-dimethylbenzo[d][1,3]dioxole-5-carboxylate**

Dimethylformamide (95 mL) was added to the compound (19 g, 37.3 mmole) obtained in above [Step 4], and triethylamine (21 mL) was added thereto. Then, pentafluorophenyl trifluoroacetate (10 mL) was added dropwise thereto and stirred at room temperature. When the reaction was completed, the following reaction was performed.

**[Step 6] Synthesis of 2-(trans-4-(dimethylamino)cyclohexyl)-7-(6-((2S,6R)-2,6-dimethylmorpholino)pyridin-3-yl)-2,4-dimethyl-N-((6-methyl-4-(methylthio)-2-oxo-1,2-dihydropyridin-3-yl)methyl)benzo[d][1,3]dioxole-5-carboxamide (compound 1)**

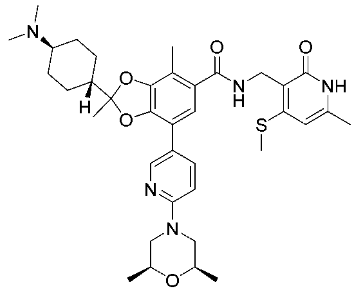

Triethylamine (16 mL) was added to the reaction solution reacted in above [Step 5]. Then, 3-(aminomethyl)-6-methyl-4-(methylthio)pyridin-2(1H)-one hydrochloride (9.2 g) was added and stirred at an internal temperature of 50°C. When the reaction was completed, the reaction product was added dropwise to purified water (1,900 mL) to filter the resulting solid. The solid was dissolved in methylene chloride (380 mL) to separate an aqueous layer. Then, magnesium sulfate was added thereto, filtered, and concentrated to obtain the title compound (25 g).

$^{1}$H NMR (Chloroform-d): 8.53 (d, 1H), 7.68 (d, 1H), 7.20 (t, 1H), 7.05 (s, 1H), 6.53 (d, 1H), 5.91 (s, 1H), 4.58 (d, 2H), 3.97 (d, 2H), 3.66 (m, 2H), 2.47 (t, 2H), 2.42 (s, 3H), 2.25 (d, 9H), 2.13 (m, 4H), 1.95 (m, 4H), 1.78 (t, 1H), 1.54 (s, 3H), 1.22 (m, 10H).

LC-MS (ESI, m/z) = 676.4 (M+H+)

**[Step 7] Synthesis of methyl 2-(trans-4-(dimethylamino)cyclohexyl)-7-(6-((2S,6R)-2,6-dimethylmorpholino)pyridin-3-yl)-2,4-dimethyl-N-((6-methyl-4-(methylthio)-2-oxo-1,2-dihydropyridin-3-yl)methyl)benzo[d] [1,3]dioxole-5-carboxamide hydrochloride (hydrochloride of compound 1)**

A 1 M-hydrochloric acid aqueous solution (130 mL) was added to the compound (25 g, 37 mmole) obtained in above [Step 6], and the resulting mixture was heated and stirred. When the reaction was completed, the resulting mixture was concentrated, dissolved in methanol (50 mL), slowly added dropwise to ethyl acetate (200 mL), and stirred. After that, the resulting mixture was filtered and dried at an internal temperature of 60°C to obtain the title compound (24 g).

**Preparation Example 2. Synthesis of 2-(trans-4-(dimethylamino)cyclohexyl)-7-(2-((2S,6R)-2,6-dimethylaminomorpholino)pyrimidin-5-yl)-2,4-dimethyl-N-((6-methyl-4-(methylthio)-2-oxo-1,2-dihydropyridin-3-yl)methyl) benzo[d][1,3]dioxole-5-carboxamide (compound 2)**

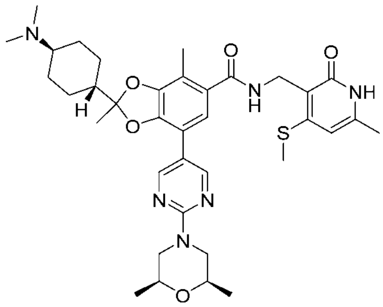

The same reaction as in Preparation Example 1 was performed except that (2S,6R)-2,6-dimethyl-4-(5-(4,4,5,5-tetramethyl-1,3,2-dioxaborolan-2-yl)pyrimidin-2-yl)morpholine was used instead of (2S,6R)-2,6-dimethyl-4-(5-(4,4,5,5-tetramethyl-1,3,2-dioxaborolan-2-yl)pyridin-2-yl)morpholine in [Step 3] of Preparation Example 1 to obtain the title compound (30 mg).

$^1$H NMR (Chloroform-d): 8.56 (s, 2H), 7.10 (s, 1H), 7.00 (s, 1H), 5.92 (s, 1H), 4.57 (d, 2H), 4.56 (d, 2H), 3.58 (s, 2H), 2.58 (t, 2H), 2.41 (s, 3H), 2.26 (s, 9H), 1.94 (s, 4H), 1.78 (m, 1H), 1.54 (s, 3H), 1.19 (s, 9H).

LC-MS (ESI, m/z) = 677.4 (M+H$^+$)

**Preparation Example 3. Synthesis of 7-(6-(4,4-difluoropiperidin-1-yl)pyridin-3-yl)-2-(trans-4-(dimethylamino)cyclohexyl)-2,4-dimethyl-N-((6-methyl-4-(methylthio)-2-oxo-1,2-dihydropyridin-3-yl)methyl)benzo[d][1,3]dioxole-5-carboxamide (compound 3)**

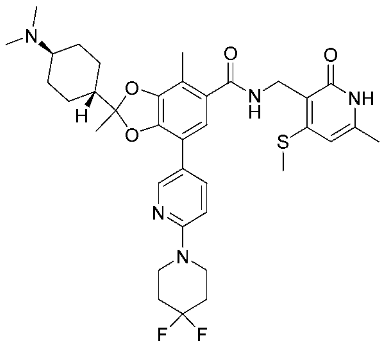

The same reaction as in Preparation Example 1 was performed except that 2-(4,4-difluoropiperidin-1-yl)-5-(4,4,5,5-tetramethyl-1,3,2-dioxaborolan-2-yl)pyridine was used instead of (2S,6R)-2,6-dimethyl-4-(5-(4,4,5,5-tetramethyl-1,3,2-dioxaborolan-2-yl)pyridin-2-yl)morpholine in [Step 3] of Preparation Example 1 to obtain the title compound (25 mg).

$^1$H NMR ($D_2O$): 8.23 (s, 1H), 8.11 (s, 1H), 7.29 (d, 1H), 7.01 (s, 1H), 6.25 (s, 1H), 4.31 (s, 2H), 3.76 (m, 4H), 3.09 (m, 1H), 2.71 (s, 3H), 2.38 (s, 3H), 2.31 (m, 7H), 2.12 (m, 8H), 2.03 (s, 1H), 1.65 (s, 3H), 1.58 (m, 2H), 1.42 (m, 2H).

LC-MS (ESI, m/z) = 682.4 (M+H$^+$)

**Preparation Example 4. Synthesis of 2-(trans-4-(dimethylamino)cyclohexyl)-2,4-dimethyl-N-((6-methyl-4-(methylthio)-2-oxo-1,2-dihydropyridin-3-yl)methyl)-7-(2-(piperidin-1-yl)pyrimidin-5-yl)benzo[d][1,3]dioxole-5-carboxamide (compound 4)**

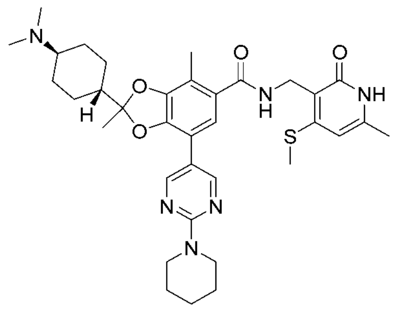

The same reaction as in Preparation Example 1 was performed except that 2-(piperidin-1-yl)-5-(4,4,5,5-tetramethyl-1,3,2-dioxaborolan-2-yl)pyrimidine was used instead of (2S,6R)-2,6-dimethyl-4-(5-(4,4,5,5-tetramethyl-1,3,2-dioxaborolan-2-yl)pyridin-2-yl)morpholine in [Step 3] of Preparation Example 1 to obtain the title compound (26 mg).

$^1$H NMR (Chloroform-d): 8.55 (s, 2H), 7.19 (t, 1H), 7.00 (s, 1H), 5.92 (s, 1H), 4.58 (d, 2H), 3.73 (t, 4H), 2.42 (s, 3H), 2.26 (d, 9H), 2.17 (s, 4 H), 1.95 (d, 4H), 1.79 (t, 1H), 1.64 (d, 2H), 1.54 (s, 7H), 1.21 (m, 4H).

LC-MS (ESI, m/z) = 647.4 (M+H$^+$)

**Preparation Example 5. Synthesis of 2-(trans-4-(dimethylamino)cyclohexyl)-2,4-dimethyl-N-((6-methyl-4-(methylthio)-2-oxo-1,2-dihydropyridin-3-yl)methyl)-7-(2-(pyrrolidin-1-yl)pyrimidin-5-yl)benzo[d][1,3]dioxole-5-carboxamide (compound 5)**

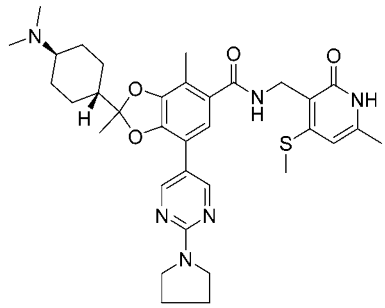

The same reaction as in Preparation Example 1 was performed except that 2-(pyrrolidin-1-yl)-5-(4,4,5,5-tetramethyl-1,3,2-dioxaborolan-2-yl)pyrimidine was used instead of (2S,6R)-2,6-dimethyl-4-(5-(4,4,5,5-tetramethyl-1,3,2-dioxaborolan-2-yl)pyridin-2-yl)morpholine in [Step 3] of Preparation Example 1 to obtain the title compound (70 mg).

$^1$H NMR (Methanol-d3): 8.85 (s, 1H), 7.23 (s, 1H), 6.36 (m, 1H), 4.51 (s, 2H), 3.70 (s 3H), 3.20 (s, 1H), 2.75 (s, 6H), 2.54 (m, 4H), 2.31 (s, 7H), 2.10 (m, 8H), 2.00 (m, 1H), 1.65 (s, 3H), 1.60 (m, 2H), 1.56 (m, 2H).

LC-MS (ESI, m/z) = 633.4 (M+H$^+$)

**Preparation Example 6. Synthesis of 2-(trans-4-(dimethylamino)cyclohexyl)-2,4-dimethyl-N-((6-methyl-4-(methylthio)-2-oxo-1,2-dihydropyridin-3-yl)methyl)-7-(4-(morpholinomethyl)phenyl)benzo[d][1,3]dioxole-5-carboxamide (compound 6)**

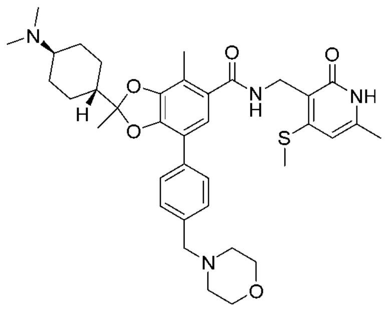

The same reaction as in Preparation Example 1 was performed except that 4-(4-(4,4,5,5-tetramethyl-1,3,2-dioxaborolan-2-yl)benzyl)morpholine was used instead of (2S,6R)-2,6-dimethyl-4-(5-(4,4,5,5-tetramethyl-1,3,2-dioxaborolan-2-yl)pyridin-2-yl)morpholine in [Step 3] of Preparation Example 1 to obtain the title compound (78 mg).
$^1$H NMR (Chloroform-d): 7.53(m, 2H), 7.27 (m, 2H), 7.25 (s, 1H), 5.90 (s, 1H), 4.64 (m 2H), 3.80 (6m 6H), 3.04 (s, 1H), 2.45 (s, 3H), 2.36 (s, 3H), 2.33 (s, 8H), 2.00 (m, 7H), 1.64 (m, 5H), 1.55 (m, 5H).
LC-MS (ESI, m/z) = 661.4 (M+H$^+$)

**Preparation Example 7. Synthesis of 2-(trans-4-(dimethylamino)cyclohexyl)-2,4-dimethyl-7-(1-methyl-1H-indol-5-yl)-N-((6-methyl-4-(methylthio)-2-oxo-1,2-dihydropyridin-3-yl)methyl)benzo[d][1,3]dioxole-5-carboxamide (compound 7)**

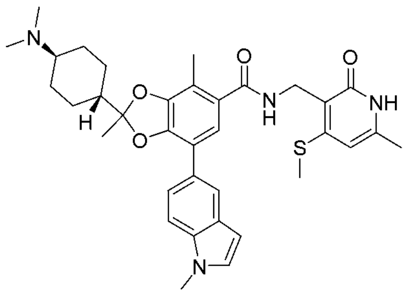

The same reaction as in Preparation Example 1 was performed except that 1-methyl-5-(4,4,5,5-tetramethyl-1,3,2-dioxaborolan-2-yl)-1H-indole was used instead of (2S,6R)-2,6-dimethyl-4-(5-(4,4,5,5-tetramethyl-1,3,2-dioxaborolan-2-yl)pyridin-2-yl)morpholine in [Step 3] of Preparation Example 1 to obtain the title compound (67 mg).
$^1$H NMR (Methanol-d3): 7.85 (s, 1H), 7.32 (d, 1H), 7.18 (d, 1H), 7.12 (s, 1H), 7.11 (d, 1H), 6.41 (d, 1H), 6.20 (s, 1H), 4.51 (s, 2H), 3.75 (s, 3H), 2.47 (s, 3H), 2.24 (m, 12H), 1.98 (dd, 4H), 1.80 (t, 1H), 1.58 (s, 3H), 1.26 (m, 5H).
LC-MS (ESI, m/z) = 615.4 (M+H$^+$)

**Preparation Example 8. Synthesis of 7-(2-(4,4-difluoropiperidin-1-yl)pyrimidin-5-yl)-2-(trans-4-(dimethylamino)cyclohexyl)-2,4-dimethyl-N-((6-methyl-4-(methylthio)-2-oxo-1,2-dihydropyridin-3-yl)methyl)benzo[d][1,3]dioxole-5-carboxamide (compound 8)**

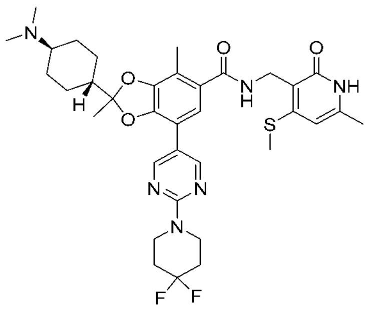

The same reaction as in Preparation Example 1 was performed except that 2-(4,4-difluoropiperidin-1-yl)-5-(4,4,5,5-tetramethyl-1,3,2-dioxaborolan-2-yl)pyrimidine was used instead of (2S,6R)-2,6-dimethyl-4-(5-(4,4,5,5-tetramethyl-1,3,2-dioxaborolan-2-yl)pyridin-2-yl)morpholine in [Step 3] of Preparation Example 1 to obtain the title compound (99 mg).
$^1$H NMR (Chloroform-d): 8.59 (s, 2H), 7.17 (t, 1H), 7.04(s, 1H), 5.92(s, 1H), 4.57(d, 2H), 3.91(t, 4H), 2.42 (s, 3H), 2.28 (m, 9H), 2.17 (s, 4H), 1.92 (m, 8H), 1.78 (t, 1H), 1.55 (s, 3H), 1.21 (m, 4H).
LC-MS (ESI, m/z) = 683.3 (M+H$^+$)

**Preparation Example 9. Synthesis of 2-(trans-4-(dimethylamino)cyclohexyl)-2,4-dimethyl-N-((6-methyl-4-(methylthio)-2-oxo-1,2-dihydropyridin-3-yl)methyl)-7-(4-morpholinophenyl)benzo[d][1,3]dioxole-5-carboxamide (compound 9)**

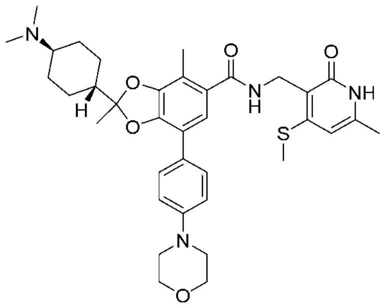

The same reaction as in Preparation Example 1 was performed except that 4-(4-(4,4,5,5-tetramethyl-1,3,2-dioxaborolan-2-yl)phenyl)morpholine was used instead of (2S,6R)-2,6-dimethyl-4-(5-(4,4,5,5-tetramethyl-1,3,2-dioxaborolan-2-yl)pyridin-2-yl)morpholine in [Step 3] of Preparation Example 1 to obtain the title compound (56 mg).
$^1$H NMR (Chloroform-d): 7.55 (m, 2H), 7.53 (t, 1H), 7.25 (s, 1H), 6.79 (d, 2H), 5.93 (s, 3H), 4.62 (d, 2H), 3.82 (m, 4H), 3.08 (m, 4H), 2.45 (s, 3H), 2.32 (m, 10H), 2.14 (s, 3H), 2.00 (m, 4H), 1.99 (m, 1H), 1.57 (s, 3H), 1.26 (m, 4H).
LC-MS (ESI, m/z) = 647.4 (M+H$^+$)

**Preparation Example 10. Synthesis of 2-(trans-4-(dimethylamino)cyclohexyl)-7-(4-fluorophenyl)-2,4-dimethyl-N-((6-methyl-4-(methylthio)-2-oxo-1,2-dihydropyridin-3-yl)methyl)benzo[d][1,3]dioxole-5-carboxamide (compound 10)**

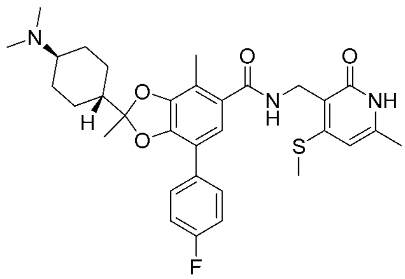

The same reaction as in Preparation Example 1 was performed except that 2-(4-fluorophenyl)-4,4,5,5-tetramethyl-1,3,2-dioxaborolane was used instead of (2S,6R)-2,6-dimethyl-4-(5-(4,4,5,5-tetramethyl-1,3,2-dioxaborolan-2-yl)pyridin-2-yl)morpholine in [Step 3] of Preparation Example 1 to obtain the title compound (78 mg).
$^1$H NMR (Chloroform-d): 7.58 (m, 2H), 7.19 (t, 1H), 7.08 (s, 1H), 7.00 (t, 2H), 5.92 (s, 1H), 4.60 (d, 2H), 2.44 (s, 3H), 2.31 (s, 3H), 2.25 (m, 10H), 2.09 (m, 4H), 1.96 (m, 4H), 1.80 (t, 1H), 1.57 (s, 3H), 1.23 (m, 4H).
LC-MS (ESI, m/z) = 580.3 (M+H$^+$)

**Preparation Example 11. Synthesis of 2-(trans-4-(dimethylamino)cyclohexyl)-2,4-dimethyl-N-((6-methyl-4-(methylthio)-2-oxo-1,2-dihydropyridin-3-yl)methyl)-7-(thiophen-3-yl)benzo[d][1,3]dioxole-5-carboxamide (compound 11)**

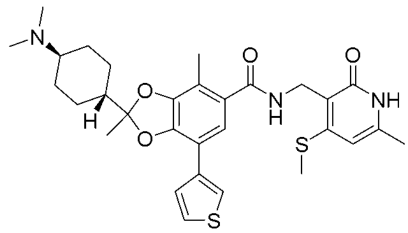

The same reaction as in Preparation Example 1 was performed except that 4,4,5,5-tetramethyl-2-(thiophen-3-yl)-1,3,2-dioxaborolane was used instead of (2S,6R)-2,6-dimethyl-4-(5-(4,4,5,5-tetramethyl-1,3,2-dioxaborolan-2-yl)pyridin-2-yl)morpholine in [Step 3] of Preparation Example 1 to obtain the title compound (30 mg).

$^1$H NMR (Chloroform-d): 7.59 (d, 1H), 7.39 (m, 1H), 7.25 (m, 1H), 7.23 (m, 1H), 7.19 (s, 1H), 5.92 (s, 1H), 4.60 (d, 2H), 2.43 (s, 3H), 2.29 (s, 3H), 2.24 (s, 6H), 2.14 (m, 4H), 1.97 (t, 4H), 1.80 (t, 1H), 1.58 (s, 3H), 1.23 (m, 4H).

LC-MS (ESI, m/z) = 568.3 (M+H$^+$)

**Preparation Example 12. Synthesis of 2-(trans-4-(dimethylamino)cyclohexyl)-7-(5-fluoro-2-methoxyphenyl)-2,4-dimethyl-N-((6-methyl-4-(methylthio)-2-oxo-1,2-dihydropyridin-3-yl)methyl)benzo[d][1,3]dioxole-5-carboxamide (compound 12)**

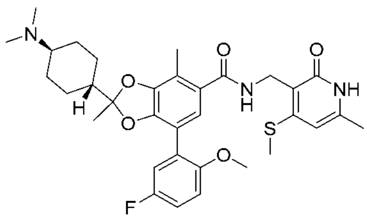

The same reaction as in Preparation Example 1 was performed except that 2-(5-fluoro-2-methoxyphenyl)-4,4,5,5-tetramethyl-1,3,2-dioxaborolane was used instead of (2S,6R)-2,6-dimethyl-4-(5-(4,4,5,5-tetramethyl-1,3,2-dioxaborolan-2-yl)pyridin-2-yl)morpholine in [Step 3] of Preparation Example 1 to obtain the title compound (35 mg).

$^1$H NMR (Chloroform-d): 7.02 (m, 1H), 6.98 (m, 2H), 6.92 (m, 1H), 6.79 (m, 1H), 5.91 (s, 1H), 4.58 (d, 2H), 3.68 (s, 3H), 2.44 (s, 3H), 2.32 (s, 2H), 2.25 (s, 6H), 2.13 (s, 4H), 1.94 (m, 3H), 1.79 (t, 1H), 1.53 (s, 3H), 1.20 (m, 4H).

LC-MS (ESI, m/z) = 610.3 (M+H$^+$)

**Preparation Example 13. Synthesis of 2-(trans-4-(dimethylamino)cyclohexyl)-7-(3,5-dimethylisoxazol-4-yl)-2,4-dimethyl-N-((6-methyl-4-(methylthio)-2-oxo-1,2-dihydropyridin-3-yl)methyl)benzo[d][1,3]dioxole-5-carboxamide (compound 13)**

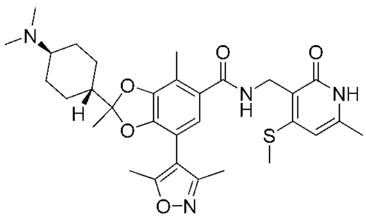

The same reaction as in Preparation Example 1 was performed except that 3,5-dimethyl-4-(4,4,5,5-tetramethyl-1,3,2-dioxaborolan-2-yl)isooxazole was used instead of (2S,6R)-2,6-dimethyl-4-(5-(4,4,5,5-tetramethyl-1,3,2-dioxaborolan-2-yl)pyridin-2-yl)morpholine in [Step 3] of Preparation Example 1 to obtain the title compound (80 mg).
$^1$H NMR (Chloroform-d): 7.01 (m, 1H), 6.74 (s, 1H), 5.97 (s, 1H), 4.57 (m, 2H), 2.45 (s, 3H), 2.28 (m, 5H), 2.25 (s, 6H), 2.19 (s, 3H), 2.16 (s, 2H), 2.12 (m, 1H), 1.92 (d, 4H), 1.76 (t, 1H), 1.51 (s, 2H), 1.18 (m, 4H).
LC-MS (ESI, m/z) = 581.4 (M+H$^+$)

**Preparation Example 14. Synthesis of 7-(2,6-difluoropyridin-3-yl)-2-(trans-4-(dimethylamino)cyclohexyl)-2,4-dimethyl-N-((6-methyl-4-(methylthio)-2-oxo-1,2-dihydropyridin-3-yl)methyl)benzo[d][1,3]dioxole-5-carboxamide (compound 14)**

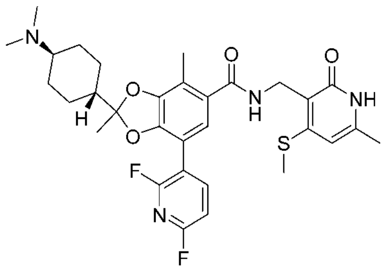

The same reaction as in Preparation Example 1 was performed except that 2,6-difluoro-3-(4,4,5,5-tetramethyl-1,3,2-dioxaborolan-2-yl)pyridine was used instead of (2S,6R)-2,6-dimethyl-4-(5-(4,4,5,5-tetramethyl-1,3,2-dioxaborolan-2-yl)pyridin-2-yl)morpholine in [Step 3] of Preparation Example 1 to obtain the title compound (63 mg).
$^1$H NMR (Methanol-d3): 8.15 (s, 1H), 7.05 (s, 2H), 6.42 (s, 1H), 4.52 (s, 2H), 2.82 (s, 1H), 2.32 (s, 6H), 2.26 (s, 3H), 2.13 (m, 5H), 2.10 (m, 5H), 1.61 (s, 6H), 1.60 (s, 3H), 1.55 (m, 2H), 1.53 (m, 4H).
LC-MS (ESI, m/z) = 599.0 (M+H$^+$)

**Preparation Example 15. Preparation of 7-(2-(2-oxa-7-azaspiro[3.5]nonan-7-yl)pyrimidin-5-yl)-2-(trans-4-(dimethylamino)cyclohexyl)-2,4-dimethyl-N-((6-methyl-4-(methylthio)-2-oxo-1,2-dihydropyridin-3-yl)methyl)benzo[d][1,3]dioxole-5-carboxamide (compound 15)**

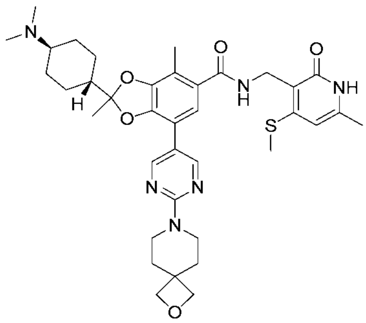

The same reaction as in Preparation Example 1 was performed except that 7-(5-(4,4,5,5-tetramethyl-1,3,2-dioxaborolan-2-yl)pyrimidin-2-yl)-2-oxa-7-azaspiro[3.5]nonane was used instead of (2S,6R)-2,6-dimethyl-4-(5-(4,4,5,5-tetramethyl-1,3,2-dioxaborolan-2-yl)pyridin-2-yl)morpholine in [Step 3] of Preparation Example 1 to obtain the title compound (63 mg).
$^1$H NMR (Chloroform-d): 8.58 (s, 1H), 7.11 (m, 1H), 7.04 (d, 1H), 5.98 (s, 1H), 4.61 (dd, 2H), 4.47 (s, 2H), 3.75 (s, 2H), 2.45 (m, 8H), 2.36 (s, 3H), 2.35 (s, 3H), 2.29 (m, 5H), 1.94 (m, 2H), 1.90 (m, 2H), 1.80 (s, 1H), 1.56 (m, 3H), 1.30 (m, 5H).
LC-MS (ESI, m/z) = 689.4 (M+H$^+$)

**Preparation Example 16. Synthesis of 2-(trans-4-(dimethylamino)cyclohexyl)-7-(6-(3,5-dimethylpiperidin-1-yl)pyridin-3-yl)-2,4-dimethyl-N-((6-methyl-4-(methylthio)-2-oxo-1,2-dihydropyridin-3-yl)methyl)benzo[d][1,3]dioxole-5-carboxamide (compound 16)**

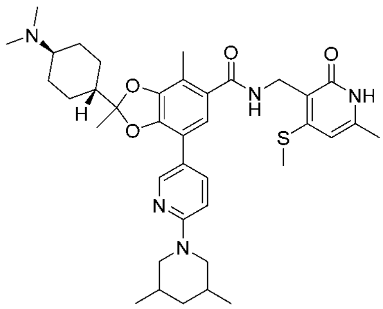

The same reaction as in Preparation Example 1 was performed except that 2-(3,5-dimethylpiperidin-1-yl)-5-(4,4,5,5-tetramethyl-1,3,2-dioxaborolan-2-yl)pyridine was used instead of (2S,6R)-2,6-dimethyl-4-(5-(4,4,5,5-tetramethyl-1,3,2-dioxaborolan-2-yl)pyridin-2-yl)morpholine in [Step 3] of Preparation Example 1 to obtain the title compound (30 mg).

$^1$H NMR (Chloroform-d): 8.52 (s, 1H), 7.67 (s, 1H), 7.25 (m, 1H), 7.06 (s, 1H), 6.62 (m, 1H), 5.94 (s, 1H), 4.62 (d, 2H), 4.24 (d, 2H), 2.45 (s, 3H), 2.39 (s, 6H), 2.30 (m, 5H), 2.30 (s, 3H), 2.29 (m, 4H), 2.17 (m, 2H), 1.57 (m, 2H), 1.56 (m, 3H), 1.28 (m, 4H), 0.94 (m, 6H).

LC-MS (ESI, m/z) = 675.1 (M+H$^+$)

**Preparation Example 17. Synthesis of 2-(trans-4-(dimethylamino)cyclohexyl)-2,4-dimethyl-N-((6-methyl-4-(methylthio)-2-oxo-1,2-dihydropyridin-3-yl)methyl)-7-(6-thiomorpholinopyridin-3-yl)benzo[d][1,3]dioxole-5-carboxamide (compound 17)**

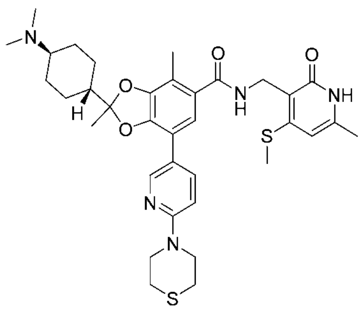

The same reaction as in Preparation Example 1 was performed except that 4-(5-(4,4,5,5-tetramethyl-1,3,2-dioxaborolan-2-yl)pyridin-2-yl)thiomorpholine was used instead of (2S,6R)-2,6-dimethyl-4-(5-(4,4,5,5-tetramethyl-1,3,2-dioxaborolan-2-yl)pyridin-2-yl)morpholine in [Step 3] of Preparation Example 1 to obtain the title compound (20 mg).

$^1$H NMR (Chloroform-d): 8.52(s, 1H), 7.70(d, 1H), 7.27(d, 1H), 6.52(d, 1H), 5.94(s, 1H), 4.61(m, 2H), 3.91(m, 4H), 2.61(s, 5H), 2.45(s, 3H), 2.39(m, 6H), 2.31(s, 3H), 2.16(s, 3H), 2.15(m, 4H), 2.04(m, 1H), 2.01(s, 3H), 1.29(s, 4H).

LC-MS (ESI, m/z) = 665.0 (M+H$^+$)

**Preparation Example 18. Synthesis of 2-(trans-4-(dimethylamino)cyclohexyl)-7-(furan-3-yl)-2,4-dimethyl-N-((6-methyl-4-(methylthio)-2-oxo-1,2-dihydropyridin-3-yl)methyl)benzo[d][1,3]dioxole-5-carboxamide (compound 18)**

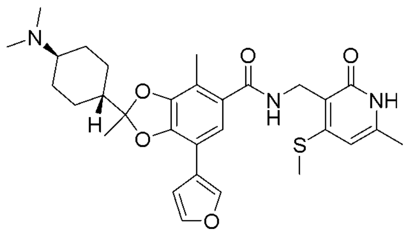

The same reaction as in Preparation Example 1 was performed except that 2-(furan-3-yl)-4,4,5,5-tetramethyl-1,3,2-dioxaborolane was used instead of (2S,6R)-2,6-dimethyl-4-(5-(4,4,5,5-tetramethyl-1,3,2-dioxaborolan-2-yl)pyridin-2-yl) morpholine in [Step 3] of Preparation Example 1 to obtain the title compound (73 mg).
$^1$H NMR (Chloroform-d): 7.81 (s, 1H), 7.36 (t, 1H), 7.19 (t, 1H), 7.06 (s, 1H), 6.66 (s, 1H), 5.95 (s, 1H), 4.59 (m, 2H), 2.44 (s, 3H), 2.24 (m, 9H), 2.18 (m, 4H), 1.95 (m, 4H), 1.80 (t, 1H), 1.58 (s, 3H), 1.21 (m, 4H).
LC-MS (ESI, m/z) = 552.3 (M+H$^+$)

**Preparation Example 19. Synthesis of 7-(5-chlorothiophen-2-yl)-2-(trans-4-(dimethylamino)cyclohexyl)-2,4-dimethyl-N-((6-methyl-4-(methylthio)-2-oxo-1,2-dihydropyridin-3-yl)methyl)benzo[d][1,3]dioxole-5-carboxamide (compound 19)**

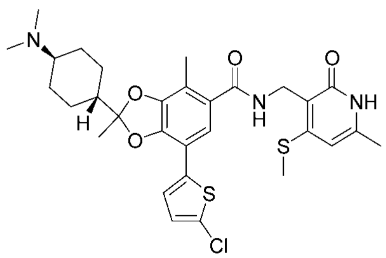

The same reaction as in Preparation Example 1 was performed except that 2-(5-chlorothiophen-2-yl)-4,4,5,5-tetramethyl-1,3,2-dioxaborolane was used instead of (2S,6R)-2,6-dimethyl-4-(5-(4,4,5,5-tetramethyl-1,3,2-dioxaborolan-2-yl)pyridin-2-yl)morpholine in [Step 3] of Preparation Example 1 to obtain the title compound (93 mg).
$^1$H NMR (Chloroform-d): 7.25 (m, 1H), 7.11 (d, 1H), 7.04 (s, 1H), 6.76 (d, 1H), 5.95 (s, 1H), 4.57 (t, 3H), 2.43 (t, 4H), 2.35 (s, 1H), 2.26 (m, 14H), 2.19 (m, 7H), 1.98 (m, 5H), 1.79 (t, 1H), 1.59 (m, 5H), 1.21 (m, 6H)
LC-MS (ESI, m/z) = 603.3 (M+H$^+$)

**Preparation Example 20. Synthesis of 7-(6-((2S,6R)-2,6-dimethylmorpholino)pyridin-3-yl)-2,4-dimethyl-2-(trans-4-(methyl(2,2,2-trifluoroethyl)amino)cyclohexyl)-N-((6-methyl-4-(methylthio)-2-oxo-1,2-dihydropyridin-3-yl)methyl)benzo[d][1,3]dioxole-5-carboxamide and hydrochloride thereof (compound 20 and hydrochloride thereof)**

<Reaction Formula 3>

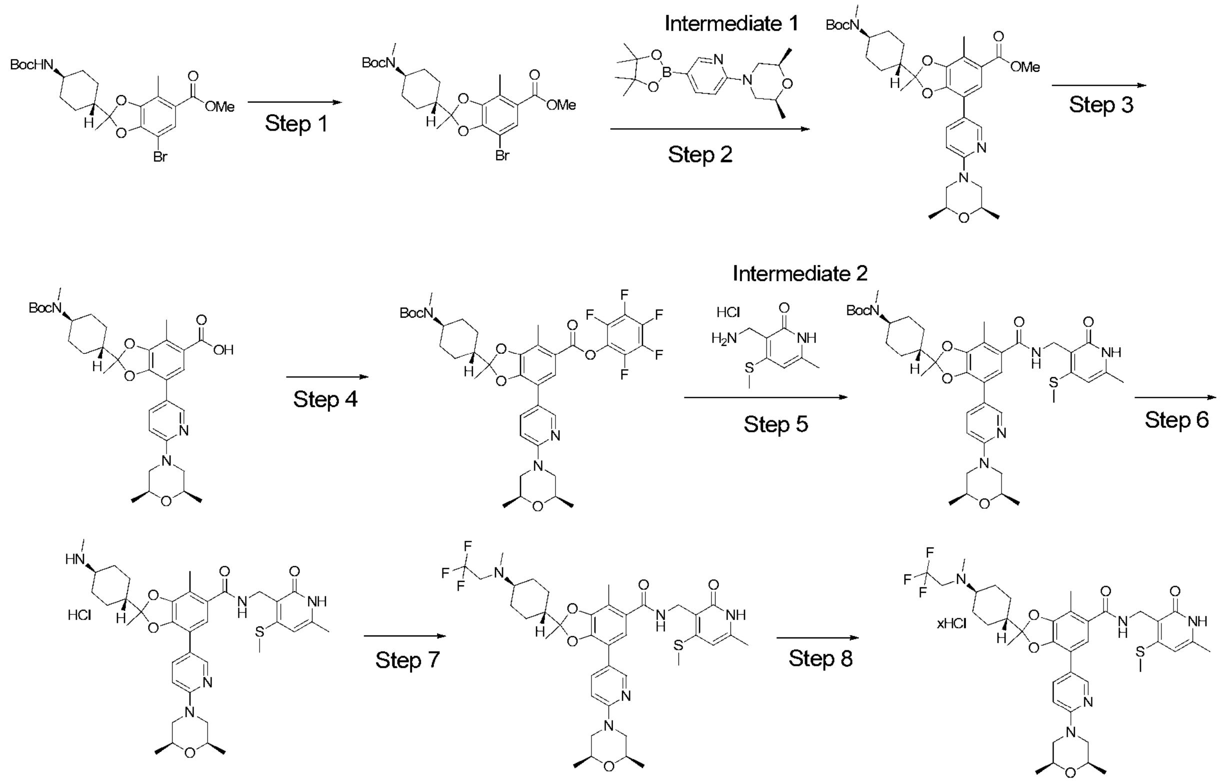


**[Step 1] Synthesis of methyl 7-bromo-2-(trans-4-(tert-butoxycarbonyl)(methyl)amino)cyclohexyl)-2,4-dimethylbenzo[d][1,3]dioxole-5-carboxylate**

Dimethylformamide (75 mL) was added to methyl 7-bromo-2-(trans-4((tert-butoxycarbonyl)amino)cyclohexyl)-2,4-dimethylbenzo[d][1,3]dioxole-5-carboxylate (15 g, 30.97 mmole), methyl iodide (16 mL) was added thereto, and an internal temperature was cooled to -5°C or less. Sodium hydride (3.372 g) was added, heated to room temperature, and stirred. When the reaction was completed, ethyl acetate (300 mL) was added and washed with the addition of purified water (300 mL). Magnesium sulfate was added thereto, filtered, and concentrated to obtain the title compound (16 g).

**[Step 2] Synthesis of methyl-2-(trans-4-((tert-butoxycarbonyl)(methyl)amino)cyclohexyl)-7-(6-((2S,6R)-2,6-dimethylmorpholino)pyridin-3-yl)-2,4-dimethoxybenzo[d][1,3]dioxole-5-carboxylate**

Dioxane (150 mL) and purified water (75 mL) were added to the compound (15 g, 30.10 mmole) obtained in above [Step 1]. Subsequently, (2S,6R)-2,6-dimethyl-4-(5-(4,4,5,5-tetramethyl-1,3,2-dioxaborolan-2-yl)pyridin-2-yl)morpholine (12.8 g) was added thereto, tetrakis(triphenylphosphine)palladium (3.58 g) was added thereto, and sodium carbonate (13.1 g) was added thereto, heated and stirred. When the reaction was completed, hexane was added to filter the precipitated solid using silica/celite, and the filtrate was washed with purified water (450 mL). Magnesium sulfate was added thereto, filtered, and concentrated to obtain the title compound (18 g).

**[Step 3] Synthesis of 2-(trans-4-((tert-butoxycarbonyl)(methyl)amino)cyclohexyl)-7-(6-((2S,6R)-2,6-dimethylmorpholino)pyridin-3-yl)-2,4-dimethylbenzo[d][1,3]dioxole-5-carboxylic acid**

Tetrahydrofuran (150 mL) and methanol (75 mL) were added to the compound (18 g, 29.6 mmole) obtained in above [Step 2]. A 2 N-sodium hydroxide aqueous solution (74 mL) was added thereto, heated, and stirred. When the reaction was completed, a 2 N-hydrochloric acid aqueous solution (74 mL) was added thereto and ethyl acetate (350 mL) was added thereto. Then, purified water (350 mL) was added thereto to perform washing, and magnesium sulfate was added thereto, filtered, and then concentrated to obtain the title compound (15.6 g).

**[Step 4] Synthesis of pentafluorophenyl-2-(trans-4-((tert-butoxycarbonyl)(methyl)amino)cyclohexyl)-7-(6-((2S,6R)-2,6-dimethylmorpholino)pyridin-3-yl)-2,4-dimethylbenzo[1,3] dioxole-5-carboxylate**

Dimethylformamide (80 mL) was added to the compound (15.6 g, 26.3 mmole) obtained in above [Step 3], and

triethylamine (15 mL) was added thereto. Then, pentafluorophenyl trifluoroacetate (7.1 mL) was added dropwise thereto and stirred at room temperature. When the reaction was completed, the following reaction was performed.

**[Step 5] Synthesis of tert-butyl(trans-4-(7-(6-((2S,6R)-2,6-dimethylmorpholino)pyridin-3-yl)-2,4-dimethyl-5-(((6-methyl-4-(methylthio)-2-oxo-1,2-dihydropyridin-3-yl)methyl)carbamyl)benzo[d][1,3]dioxole-2-yl)cyclohexyl)(methyl)carbamate**

Triethylamine (11 mL) was added to the reaction solution reacted in above [Step 4]. Then, 3-(aminomethyl)-6-methyl-4-(methylthio)pyridin-2(1H)-one hydrochloride (7.6 g) was added and stirred at an internal temperature of 50°C. When the reaction was completed, the reaction product was added dropwise to purified water (936 mL), and the resulting solid was filtered. The solid was dissolved in methylene chloride (312 mL) to separate an aqueous layer. Then, magnesium sulfate was added thereto, filtered, and concentrated to obtain the title compound (20 g).

**[Step 6] Synthesis of 7-(6-((2S,6R)-2,6-dimethylmorpholino)pyridin-3-yl)-2,4-dimethyl-N-((6-methyl-4-(methylthio)-2-oxo-1,2-dihydropyridin-3-yl-methyl-2-(trans-4-(methylamino)cyclohexyl)benzo[d][1,3]dioxole-5-carboxylate hydrochloride**

A 1 M-hydrochloric acid aqueous solution (100 mL) was added to the compound (20 g, 26.2 mmole) obtained in above [Step 5], and the resulting mixture was heated and stirred. When the reaction was completed, the resulting mixture was concentrated, dissolved in methanol (100 mL), and slowly added dropwise to ethyl acetate (400 mL). After that, the resulting mixture was filtered and dried at an internal temperature of 60°C to obtain the title compound (21.6 g).

**[Step 7] Synthesis of 7-(6-((2S,6R)-2,6-dimethylmorpholino)pyridin-3-yl)-2,4-dimethyl-2-(trans-4-(methyl(2,2,2-trifluoroethyl)amino)cyclohexyl)-N-((6-methyl-4-(methylthio)-2-oxo-1,2-dihydropyridin-3-yl)methyl)benzo[d][1,3]dioxole-5-carboxamide (compound 20)**

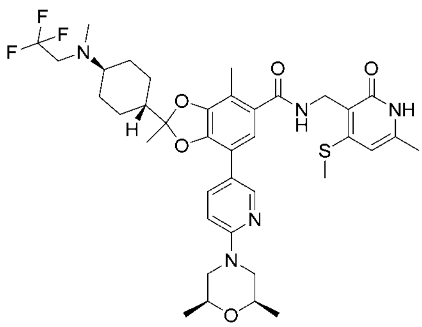

Acetonitrile (238 mL) was added to the compound (21.6 g, 29.4 mmole) obtained in above [Step 6] and sodium bicarbonate (20 g) was added thereto. 2,2,2-trifluoroethyl trifluoromethanesulfonate (10.6 mL) was added dropwise thereto, heated and stirred. When the reaction was completed, the resulting mixture was concentrated, ethyl acetate (432 mL) was added, and purified water (432 mL) was added to perform washing. Magnesium sulfate was added thereto, filtered, and then concentrated. After that, the resulting product was dissolved in isopropyl alcohol (130 mL), and hexane (151 mL) was added dropwise thereto to filter the resulting crystal, and dried at an internal temperature of 60°C to obtain the title compound (21 g).

$^1$H NMR (Chloroform-d): 8.54 (d, 1H), 7.70 (d, 1H), 7.22 (t, 1H), 7.07 (s, 1H), 6.56 (d, 1H), 5.91 (s, 1H), 4.60 (d, 2H), 4.01 (d, 2H), 3.67 (m, 2H), 2.95 (m, 2H), 2.51 (m, 2H), 2.41 (m. 8H), 2.30 (s, 3H), 2.15 (s, 3H), 1.99 (m, 2H), 1.88 (m, 2H), 1.78 (m, 1H), 1.56 (s, 3H), 1.25 (m, 12H).

LC-MS (ESI, m/z) = 745.4 ($M+H^+$)

**[Step 8] Synthesis of 7-(6-((2S,6R)-2,6-dimethylmorpholino)pyridin-3-yl)-2,4-dimethyl-2-(trans-4-(methyl(2,2,2-trifluoroethyl)amino)cyclohexyl)-N-((6-methyl-4-(methylthio)-2-oxo-1,2-dihydropyridin-3-yl)methyl)benzo[d][1,3]dioxole-5-carboxamide hydrochloride (hydrochloride of compound 20)**

A 1 M-hydrochloric acid aqueous solution (105 mL) was added to the compound (21 g, 28.2 mmole) obtained in above [Step 7], and the resulting mixture was heated and stirred. An internal temperature was cooled to room temperature, and isopropyl alcohol (105 mL) was added dropwise and stirred. The resulting crystal was filtered and dried at an internal

temperature of 60°C to obtain the title compound (20 g).

**Preparation Example 21. Synthesis of 2,4-dimethyl-2-(trans-4-(methyl(2,2,2-trifluoroethyl)amino)cyclohexyl)-N-((6-methyl-4-(methylthio)-2-oxo-1,2-dihydropyridin-3-yl)methyl)-7-(2-(piperidin-1-yl)pyrimidin-5-yl)benzo[d][1,3]dioxole-5-carboxamide (compound 21)**

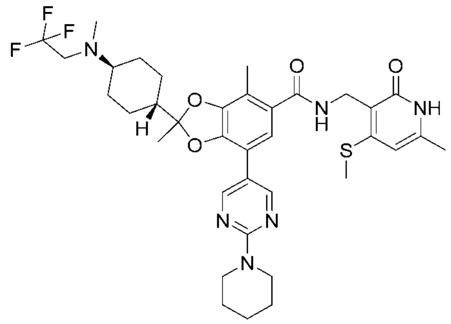

The same reaction as in Preparation Example 20 was performed except that 2-(piperidin-1-yl)-5-(4,4,5,5-tetramethyl-1,3,2-dioxaborolan-2-yl)pyrimidine was used instead of (2S,6R)-2,6-dimethyl-4-(5-(4,4,5,5-tetramethyl-1,3,2-dioxaborolan-2-yl)pyridin-2-yl)morpholine in [Step 2] of Preparation Example 20 to obtain the title compound (78 mg).

$^1$H NMR (DMSO-$d_6$): 11.50 (s, 1H), 8.62 (d, 2H), 7.91 (m, 1H), 7.05 (s, 1H), 6.05 (s, 1H), 4.27 (m, 2H), 3.74 (m, 4H), 3.06 (m, 2H), 2.47 (m, 4H), 2.29 (s, 3H), 2.17 (m, 6H), 1.85 (m. 3H), 1.80 (s, 2H), 1.60 (s, 2H), 1.55 (s, 3H), 1.48 (m, 4H), 1.17 (m, 4H).

LC-MS (ESI, m/z) = 716.3 (M+H$^+$)

**Preparation Example 22. Synthesis of 7-(2-(dimethylamino)pyrimidin-5-yl)-2,4-dimethyl-2-(trans-4-(methyl(2,2,2-trifluoroethyl)amino)cyclohexyl)-N-((6-methyl-4-(methylthio)-2-oxo-1,2-dihydropyridin-3-yl)methyl)benzo[d][1,3]dioxole-5-carboxamide (compound 22)**

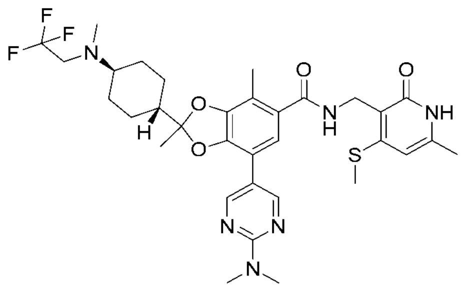

The same reaction as in Preparation Example 20 was performed except that N,N-dimethyl-5-(4,4,5,5-tetramethyl-1,3,2-dioxaborolan-2-yl)pyrimidin-2-amine was used instead of (2S,6R)-2,6-dimethyl-4-(5-(4,4,5,5-tetramethyl-1,3,2-dioxaborolan-2-yl)pyridin-2-yl)morpholine in [Step 2] of Preparation Example 20 to obtain the title compound (66 mg).

$^1$H NMR (DMSO-$d_6$): 11.50 (s, 1H), 8.65 (d, 2H), 7.91 (m, 1H), 7.05 (s, 1H), 6.05 (s, 1H), 3.08 (s, 6H), 3.06 (m, 2H), 2.46 (m, 4H), 2.28 (s, 2H), 2.17 (s, 3H), 2.13 (s, 3H), 1.85 (m. 3H), 1.80 (s, 2H), 1.55 (s, 3H), 1.18 (m, 4H).

LC-MS (ESI, m/z) = 625.3 (M+H$^+$)

**Preparation Example 23. Synthesis of 7-(2-((2S,6R)-2,6-dimethylmorpholino)pyrimidin-5-yl)-2,4-dimethyl-2-(trans-4-(methyl(2,2,2-trifluoroethyl)amino)cyclohexyl)-N-((6-methyl-4-(methylthio)-2-oxo-1,2-dihydropyridin-3-yl)methyl)benzo[d][1,3]dioxole-5-carboxamide (compound 23)**

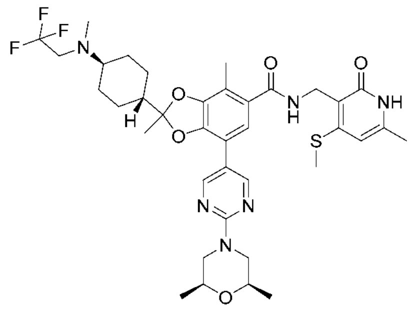

The same reaction as in Preparation Example 20 was performed except that (2S,6R)-2,6-dimethyl-4-(5-(4,4,5,5-tetramethyl-1,3,2-dioxaborolan-2-yl)pyrimidin-2-yl)morpholine was used instead of (2S,6R)-2,6-dimethyl-4-(5-(4,4,5,5-tetramethyl-1,3,2-dioxaborolan-2-yl)pyridin-2-yl)morpholine in [Step 2] of Preparation Example 20 to obtain the title compound (50 mg).

$^{1}H$ NMR (Chloroform-d): 8.60 (s, 2H), 8.41(s, 1H), 7.08 (s, 1H), 7.03 (s, 1H), 5.95 (s, 1H), 4.60 (m, 2H), 4.54 (m, 3H), 3.63 (m, 3H), 2.92 (m, 2H), 2.62 (m, 3H), 2.44 (m, 3H), 2.43 (m, 7H), 2.29 (s, 3H), 2.21 (m, 4H), 1.80 (m, 1H), 1.55 (s, 3H), 1.20 (m, 7H).

LC-MS (ESI, m/z) = 745.4 (M+H$^{+}$)

**Preparation Example 24. Synthesis of 7-(6-((2S,6R)-2,6-dimethylmorpholino)pyridin-3-yl)-2-(trans-4-((2-methoxyethyl)(methyl)amino)cyclohexyl)-2,4-dimethyl-N-((6-methyl-4-(methylthio)-2-oxo-1,2-dihydropyridin-3-yl)methyl)benzo[d][1,3]dioxole-5-carboxamide (compound 24)**

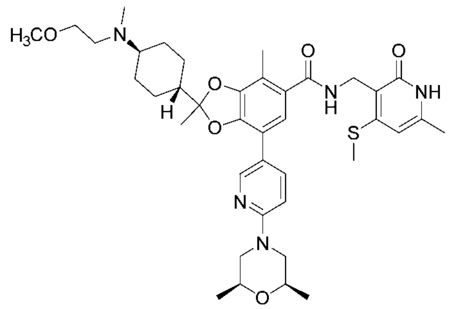

The same reaction as in Preparation Example 20 was performed except that 1-bromo-2-methoxyethane was used instead of 2,2,2-trifluoroethyl trifluoromethanesulfonate in [Step 7] of Preparation Example 20 to obtain the title compound (48 mg).

$^{1}H$ NMR (Chloroform-d): 8.50 (s, 1H), 7.72 (d, 1H), 7.15 (m, 1H), 7.07 (s, 1H), 6.59 (d, 1H), 5.96 (s, 1H), 4.62 (d, 2H), 4.04 (d, 2H), 3.70 (m, 2H), 3.68 (t, 2H), 3.49 (s, 3H), 2.53 (s, 2H), 2.51 (s, 3H), 2.45 (s, 3H), 2.37 (s, 3H), 2.30 (s, 3H), 2.23 (m, 4H), 2.19 (m, 1H), 1.58 (s, 3H), 1.26 (m, 10H).

LC-MS (ESI, m/z) = 720.5 (M+H$^{+}$)

**Preparation Example 25. Synthesis of 7-(2-((2S,6R)-2,6-dimethylmorpholino)pyrimidin-5-yl)-2-(trans-4-((2-methoxyethyl)(methyl)amino)cyclohexyl)-2,4-dimethyl-N-((6-methyl-4-(methylthio)-2-oxo-1,2-dihydropyridin-3-yl)methyl)benzo[d][1,3]dioxole-5-carboxamide (compound 25)**

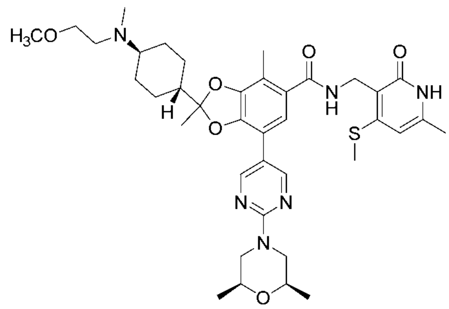

The same reaction as in Preparation Example 20 was performed except that (2S,6R)-2,6-dimethyl-4-(5-(4,4,5,5-tetramethyl-1,3,2-dioxaborolan-2-yl)pyrimidin-2-yl)morpholine was used instead of (2S,6R)-2,6-dimethyl-4-(5-(4,4,5,5-tetramethyl-1,3,2-dioxaborolan-2-yl)pyridin-2-yl)morpholine in [Step 2] of Preparation Example 20 and 1-bromo-2-methoxyethane was used instead of 2,2,2-trifluoroethyl trifluoromethanesulfonate in [Step 7] to obtain the title compound (27 mg).

$^1$H NMR (DMSO-$d_6$): 11.49 (s, 1H), 8.65 (s, 2H), 7.91 (t, 1H), 7.05 (s, 1H), 6.05 (s, 1H), 4.51 (d, 1H), 4.27 (d, 2H), 3.52 (m, 2H), 3.30 (s, 2H), 3.29 (s, 3H), 2.47 (m, 6H), 2.46 (s, 1H), 2.45 (s, 6H), 2.40 (m, 2H), 2.17 (m, 2H), 2.13 (s, 3H), 1.13 (m, 4H), 1.11 (m, 6H).

LC-MS (ESI, m/z) = 721.4 (M+H$^+$)

**Preparation Example 26. Synthesis of 7-(2-(dimethylamino)pyrimidin-5-yl)-2-(trans-4-((2-methoxyethyl)(methyl)amino)cyclohexyl)-2,4-dimethyl-N-((6-methyl-4-(methylthio)-2-oxo-1,2-dihydropyridin-3-yl)methyl)benzo[d][1,3]dioxole-5-carboxamide (compound 26)**

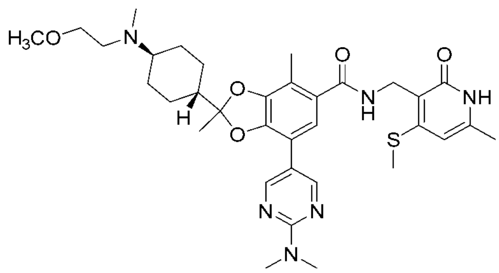

The same reaction as in Preparation Example 20 was performed except that N,N-dimethyl-5-(4,4,5,5-tetramethyl-1,3,2-dioxaborolan-2-yl)pyrimidin-2-amine was used instead of (2S,6R)-2,6-dimethyl-4-(5-(4,4,5,5-tetramethyl-1,3,2-dioxaborolan-2-yl)pyridin-2-yl)morpholine in [Step 2] of Preparation Example 20 and 1-bromo-2-methoxyethane was used instead of 2,2,2-trifluoroethyl trifluoromethanesulfonate in [Step 7] to obtain the title compound (19 mg).

$^1$H NMR (DMSO-$d_6$): 11.49 (s, 1H), 8.63 (s, 2H), 7.91 (s, 1H), 7.05 (s, 1H), 6.05 (s, 1H), 4.27 (m, 2H), 3.29 (s, 3H), 3.12 (s, 6H), 2.48 (s, 3H), 2.47 (s, 1H), 2.46 (s, 3H), 2.45 (s, 6H), 2.41 (m, 2H), 2.16 (m, 2H), 2.13 (m, 2H), 1.55 (m, 3H).

LC-MS (ESI, m/z) = 651.4 (M+H$^+$)

**Preparation Example 27. Synthesis of 7-(6-(4,4-difluoropiperidin-1-yl)pyridin-3-yl)-2-(trans-4-((2-methoxyethyl)(methyl)amino)cyclohexyl)-2,4-dimethyl-N-((6-methyl-4-(methylthio)-2-oxo-1,2-dihydropyridin-3-yl)methyl)benzo[d][1,3]dioxole-5-carboxamide (compound 27)**

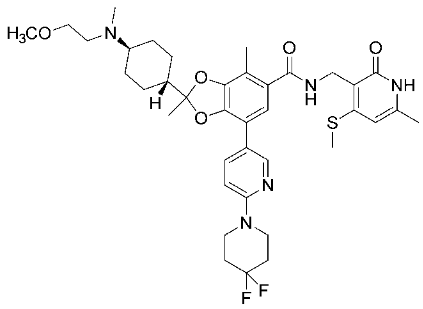

The same reaction as in Preparation Example 20 was performed except that 2-(4,4-difluoropiperidin-1-yl)-5-(4,4,5,5-tetramethyl-1,3,2-dioxaborolan-2-yl)pyridine was used instead of (2S,6R)-2,6-dimethyl-4-(5-(4,4,5,5-tetramethyl-1,3,2-dioxaborolan-2-yl)pyridin-2-yl)morpholine in [Step 2] of Preparation Example 20 and 1-bromo-2-methoxyethane was used instead of 2,2,2-trifluoroethyl trifluoromethanesulfonate in [Step 7] to obtain the title compound (30 mg).

$^1$H NMR (Chloroform-d): 8.50 (s, 1H), 7.67 (d, 1H), 7.29 (m, 1H), 6.57 (d, 1H), 5.89 (s, 1H), 4.58 (d, 2H), 3.65 (t, 3H), 3.39 (t, 2H), 3.28 (s, 3H), 2.57 (t, 2H), 2.40 (s, 3H), 2.38 (s, 1H), 2.28 (s, 3H), 2.24 (s, 3H), 2.11(s, 3H), 1.95 (m, 2H), 1.93 (s, 1H), 1.54 (s, 3H), 1.21 (t, 3H).

LC-MS (ESI, m/z) = 727.0 (M+H$^+$)

**Preparation Example 28. Synthesis of 2-(trans-4-((2-methoxyethyl)(methyl)amino)cyclohexyl)-7-(6-methoxypyridin-3-yl)-2,4-dimethyl-N-((6-methyl-4-(methylthio)-2-oxo-1,2-dihydropyridin-3-yl)methyl)benzo[d][1,3]dioxole-5-carboxamide (compound 28)**

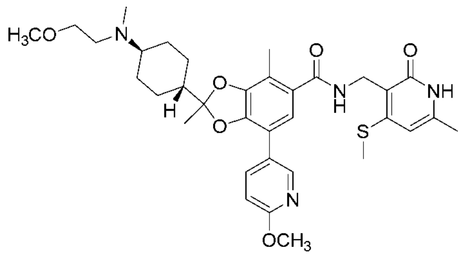

The same reaction as in Preparation Example 20 was performed except that 2-methoxy-5-(4,4,5,5-tetramethyl-1,3,2-dioxaborolan-2-yl)pyridine was used instead of (2S,6R)-2,6-dimethyl-4-(5-(4,4,5,5-tetramethyl-1,3,2-dioxaborolan-2-yl)pyridin-2-yl)morpholine in [Step 2] of Preparation Example 20 and 1-bromo-2-methoxyethane was used instead of 2,2,2-trifluoroethyl trifluoromethanesulfonate in [Step 7] to obtain the title compound (56 mg).

$^1$H NMR (Chloroform-d): 4.60 (d, 2H), 3.89 (s, 3H), 3.42 (t, 2H), 3.31 (s, 3H), 2.61 (t, 2H), 2.43 (s, 3H), 2.30 (s, 1H), 2.27 (s, 3H), 2.12 (s, 3H), 1.96 (m, 2H), 1.88 (m, 2H), 1.76 (m, 1H), 1.55 (s, 3H), 1.22 (t, 3H).

LC-MS (ESI, m/z) = 637.4 (M+H$^+$)

**Preparation Example 29. Synthesis of 7-(6-((2S,6R)-2,6-dimethylmorpholino)pyridin-3-yl)-2-(trans-4-(((S)-2-hydroxypropyl)(methyl)amino)cyclohexyl)-2,4-dimethyl-N-((6-methyl-4-(methylthio)-2-oxo-1,2-dihydropyridin-3-yl)methyl)benzo[d][1,3]dioxole-5-carboxamide (compound 29)**

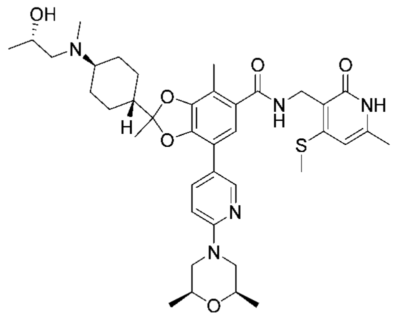

The same reaction as in Preparation Example 20 was performed except that (S)-1-chloro-2-propanol was used instead of 2,2,2-trifluoroethyl trifluoromethanesulfonate in [Step 7] of Preparation Example 20 to obtain the title compound (34 mg).

$^{1}H$ NMR (Chloroform-d): 8.50 (d, 1H), 7.69 (d, 1H), 7.13 (m, 1H), 7.05 (s, 1H), 6.58 (d, 1H), 5.94 (s, 1H), 4.57 (s, 2H), 4.18 (s, 1H), 4.01 (dd, 2H), 3.69 (m, 2H), 2.65 (m, 4H), 2.50 (t, 2H), 2.44 (s, 3H), 2.26 (s, 3H), 2.19 (s, 5H), 2.04 (m, 2H), 1.83 (t, 1H), 1.55 (s, 3H), 1.40 (m, 4H), 1.24 (d, 8H), 1.16 (d, 3H).

LC-MS (ESI, m/z) = 720.5 $(M+H^+)$

**Preparation Example 30. Synthesis of 7-(6-((2S,6R)-2,6-dimethylmorpholino)pyridin-3-yl)-2,4-dimethyl-2-(trans-4-(methyl(2-propoxyethyl)amino)cyclohexyl)-N-((6-methyl-4-(methylthio)-2-oxo-1,2-dihydropyridin-3-yl)methyl)benzo[d][1,3]dioxole-5-carboxamide (compound 30)**

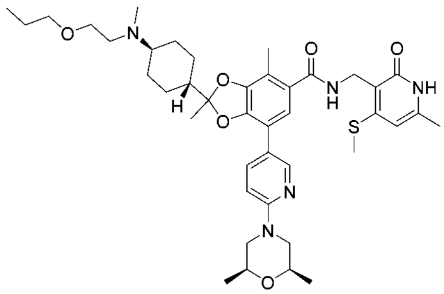

The same reaction as in Preparation Example 20 was performed except that 2-chloroethyl propyl ether was used instead of 2,2,2-trifluoroethyl trifluoromethanesulfonate in [Step 7] of Preparation Example 20 to obtain the title compound (28 mg).

$^{1}H$ NMR (Chloroform-d): 8.50 (s, 1H), 7.70 (dd, 1H), 7.19 (t, 1H), 7.06 (s, 1H), 6.54 (d, 1H), 5.92 (s, 1H), 4.59 (d, 2H), 3.98 (d, 2H), 3.79 (m, 2H), 3.51 (t, 2H), 3.35 (t, 2H), 2.69 (t, 2H), 2.50 (t, 3H), 2.43 (s, 3H), 2.33 (s, 3H), 2.28 (s, 3H), 2.14 (s, 3H), 1.92 (m, 4H), 1.79 (m, 1H), 1.54 (m, 5H), 1.24 (d, 11H), 0.86 (t, 3H).

LC-MS (ESI, m/z) = 749.3 $(M+H^+)$

**Preparation Example 31. Synthesis of 2-(trans-4-((2,2-dimethoxyethyl)(methyl)amino)cyclohexyl)-7-(6-((2S,6R)-2,6-dimethylmorpholino)pyridin-3-yl)-2,4-dimethyl-N-((6-methyl-4-(methylthio)-2-oxo-1,2-dihydropyridin-3-yl)methyl)benzo[d][1,3]dioxole-5-carboxamide (compound 31)**

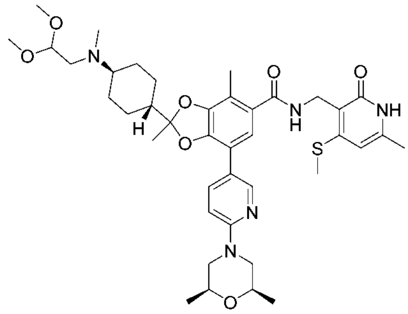

The same reaction as in Preparation Example 20 was performed except that bromoacetaldehyde dimethyl acetal was used instead of 2,2,2-trifluoroethyl trifluoromethanesulfonate in [Step 7] of Preparation Example 20 to obtain the title compound (53 mg).

$^1$H NMR (Chloroform-d): 8.53 (s, 1H), 7.69 (d, 1H), 7.19 (m, 1H), 7.06 (d, 1H), 6.56 (m, 1H), 5.92 (s, 1H), 4.59 (s, 2H), 4.48 (s, 1H), 3.98 (d, 2H), 3.67 (m, 2H), 3.33 (d, 6H), 2.58 (s, 2H), 2.48 (t, 3H), 2.42 (s, 3H), 2.35 (s, 3H), 2.28 (d, 3H), 2.14 (s, 3H), 1.91 (d, 4H), 1.78 (s, 1H), 1.54 (d, 3H), 1.23 (m, 12H).

LC-MS (ESI, m/z) = 750.5 (M+H$^+$)

**Preparation Example 32. Synthesis of 2-(trans-4-(((1,3-dioxolan-2-yl)methyl)(methyl)amino)cyclohexyl)-7-(6-((2S,6R)-2,6-dimethylmorpholino)pyridin-3-yl)-2,4-dimethyl-N-((6-methyl-4-(methylthio)-2-oxo-1,2-dihydropyridin-3-yl)methyl)benzo[d][1,3]dioxole-5-carboxamide (compound 32)**

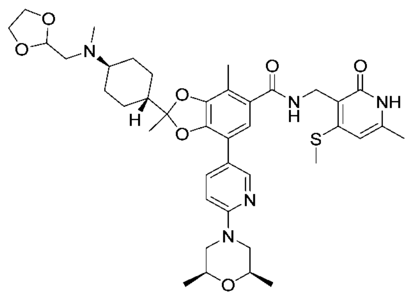

The same reaction as in Preparation Example 20 was performed except that 2-bromomethyl-1,3-dioxolane was used instead of 2,2,2-trifluoroethyl trifluoromethanesulfonate in [Step 7] of Preparation Example 20 to obtain the title compound (40 mg).

$^1$H NMR (Chloroform-d): 8.52 (d, 1H), 7.69 (m, 1H), 7.18 (t, 1H), 7.06 (s, 1H), 6.55 (d, 1H), 5.93 (s, 1H), 5.00 (t, 1H), 4.59 (d, 2H), 3.99 (m, 3H), 3.82 (m, 1H), 3.68 (m, 2H), 2.71 (d, 1H), 2.50 (m, 2H), 2.43 (s, 5H), 2.28 (s, 3H), 2.15 (s, 3H), 1.97 (m, 4H), 1.79 (d, 1H), 1.54 (s, 3H), 1.25 (m, 11H).

LC-MS (ESI, m/z) = 748.5 (M+H$^+$)

**Preparation Example 33. Synthesis of 7-(6-((2S,6R)-2,6-dimethylmorpholino)pyridin-3-yl)-2,4-dimethyl-N-((6-methyl-4-(methylthio)-2-oxo-1,2-dihydropyridin-3-yl)methyl)-2-(1-(2,2,2-trifluoroethyl)piperidin-4-yl)benzo[d][1,3]dioxole-5-carboxamide and hydrochloride thereof (compound 33 and hydrochloride thereof)**

<Reaction Formula 4>

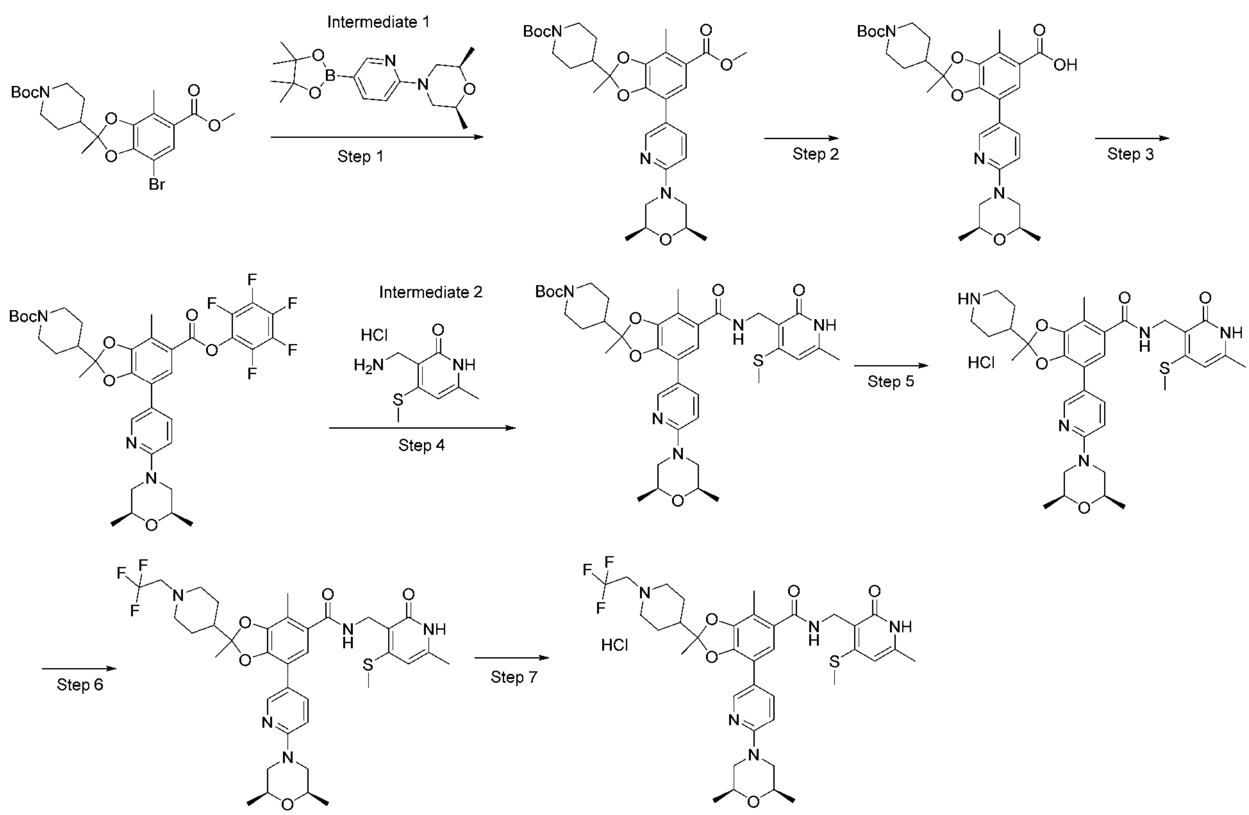


**[Step 1] Synthesis of tert-butyl 4-(7-(6-((2S,6R)-2,6-dimethylmorpholino)pyridin-3-yl)-5-(methoxycarbonyl)-2,4-dimethylbenzo[d][1,3]dioxole-2-yl)pyridin-1-carboxylate**

Dioxane (200 mL) was added to tert-butyl 4-(7-bromo-5-(methoxycarbonyl)-2,4-dimethylbenzo[d][1,3]dioxole-2-yl)piperidin-1-carboxylate (20 g, 44.2 mmole), and purified water (100 mL) was added thereto. Subsequently, (2S,6R)-2,6-dimethyl-4-(5-(4,4,5,5-tetramethyl-1,3,2-dioxaborolan-2-yl)pyridin-2-yl)morpholine (18.3 g) was added thereto, tetrakis(triphenylphosphine)palladium (5.11 g) was added thereto, and sodium carbonate (14 g) was added thereto, heated and stirred. When the reaction was completed, hexane (200 mL) and purified water (100 mL) were added to filter the precipitated solid using silica/celite, and the filtrate was washed with purified water (200 mL). Magnesium sulfate was added thereto, filtered, and concentrated to obtain the title compound (16 g).

**[Step 2] Synthesis of 2-(1-(tert-butoxycarbonyl)piperidin-4-yl)-7-(6-((2S,6R)-2,6-dimethylmorpholino)pyridin-3-yl)-2,4-dimethylbenzo[d] [1,3]dioxole-5-carboxylic acid**

Tetrahydrofuran (128 mL) and purified water (64 mL) were added to the compound (16 g, 27.5 mmole) obtained in above [Step 1]. A 2 N-sodium hydroxide aqueous solution (69 mL) was added thereto, heated, and stirred. When the reaction was completed, a 2 N-hydrochloric acid aqueous solution (69 mL) was added thereto and ethyl acetate (128 mL) was added thereto. Then, purified water was added twice at a rate of 300 mL to perform washing, and magnesium sulfate was added thereto, filtered, and then concentrated to obtain the title compound (14.2 g).

**[Step 3] Synthesis of tert-butyl 4-(7-(6-((2S,6R)-2,6-dimethylmorpholino)pyridin-3-yl)-2,4-dimethyl-5-((pentafluorophenoxy)carbonyl)benzo[d][1,3]dioxole-2-yl)piperidin-1-carboxylate**

Dimethylformamide (70 mL) was added to the compound (14.2 g, 25.1 mmole) obtained in above [Step 2], and triethylamine (14 mL) was added thereto. Then, pentafluorophenyl trifluoroacetate (6.8 mL) was added dropwise thereto and stirred at room temperature. When the reaction was completed, the following reaction was performed.

**[Step 4] Synthesis of tert-butyl 4-(7-(6-((2S,6R)-2,6-dimethylmorpholino)pyridin-3-yl)-2,4-dimethyl-5-(((6-methyl-4-(methylthio)-2-oxo-1,2-dihydropyridin-3-yl)methyl)carbamoyl)benzo[d][1,3]dioxole-2-yl)piperidin-1-carboxylate**

Triethylamine (10 mL) was added to the reaction solution reacted in above [Step 3]. Then, 3-(aminomethyl)-6-methyl-4-(methylthio)pyridin-2(1H)-one hydrochloride (7.2 g) was added and stirred at an internal temperature of 50°C. When the reaction was completed, the reaction product was added dropwise to purified water (710 mL), and the resulting solid was filtered. The solid was dissolved in methylene chloride (284 mL) to separate an aqueous layer. Then, magnesium sulfate was added thereto, filtered, and concentrated to obtain the title compound (16.9 g).

**[Step 5] Synthesis of 7-(6-((2S,6R)-2,6-dimethylmorpholino)pyridin-3-yl)-2,4-dimethyl-N-((6-methyl-4-(methylthio)-2-oxo-1,2-dihydropyridin-3-yl)methyl)2-(piperidin-4-yl)benzo[d][1,3]dioxole-5-carboxamide hydrochloride**

1 M-hydrochloric acid (ethanol aqueous solution, 85 mL) was added to the compound (16.9 g, 23 mmole) obtained in above [Step 4], and the resulting mixture was heated and stirred. When the reaction was completed, the reaction solution was added dropwise to ethyl acetate (338 mL), and the resulting solid was filtered and dried at an internal temperature of 60°C to obtain the title compound (15.4 g).

**[Step 6] Synthesis of 7-(6-((2S,6R)-2,6-dimethylmorpholino)pyridin-3-yl)-2,4-dimethyl-N-((6-methyl-4-(methylthio)-2-oxo-1,2-dihydropyridin-3-yl)methyl)-2-(1-(2,2,2-trifluoroethyl)piperidin-4-yl)benzo[d][1,3]dioxole-5-carboxamide (compound 33)**

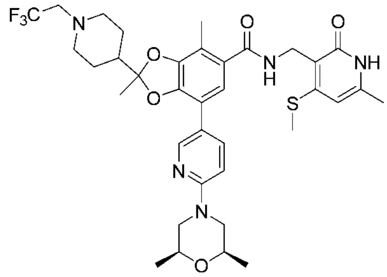

Acetonitrile (308 mL) was added to the compound (15.4 g, 22 mmole) obtained in above [Step 5] and sodium bicarbonate (15.5 g) was added thereto. Trifluoroethyl trifluoromethanesulfonate (8.2 mL) was added dropwise thereto, and the resulting mixture was heated and stirred. When the reaction was completed, the resulting mixture was concentrated, ethyl acetate (308 mL) was added, and purified water (462 mL) was added to perform washing. Magnesium sulfate was added thereto, filtered, and then concentrated. Then, isopropyl alcohol (92 mL) was added, heated and dissolved. Hexane (100 mL) was added dropwise and the resulting crystal was filtered and dried at an internal temperature of 60°C to obtain the title compound (11 g).

$^1$H NMR (Chloroform-d): 8.42 (s, 1H), 7.60 (d, 1H), 7.33 (s, 1H), 6.98 (s, 1H), 6.46 (d, 1H), 5.82 (s, 1H), 4.47 (s, 2H), 3.89 (d, 2H), 3.57 (s, 2H), 2.85 (m, 4H), 2.32(m, 5H), 2.20 (s, 5H), 2.02 (s, 3H), 1.72 (d, 3H), 1.49 (s, 5H), 1.13 (d, 6H).

LC-MS (ESI, m/z) = 716.4 (M+H$^+$)

**[Step 7] Synthesis of 7-(6-((2S,6R)-2,6-dimethylmorpholino)pyridin-3-yl)-2,4-dimethyl-N-((6-methyl-4-(methylthio)-2-oxo-1,2-dihydropyridin-3-yl)methyl)-2-(1-(2,2,2-trifluoroethyl)piperidin-4-yl)benzo[d][1,3]dioxole-5-carboxamide hydrochloride (hydrochloride of compound 33)**

A 1 M-hydrochloric acid aqueous solution (100 mL) was added to the compound (11 g, 14.8 mmole) obtained in above [Step 6], and the resulting mixture was heated and stirred. An internal temperature was cooled to room temperature, and ethyl acetate (230 mL) was added dropwise and stirred. The resulting crystal was filtered and dried at an internal temperature of 60°C to obtain the title compound (11 g).

**Preparation Example 34. Synthesis of 2,4-dimethyl-N-((6-methyl-4-(methylthio)-2-oxo-1,2-dihydropyridin-3-yl)methyl)-7-(6-thiomorpholinopyridin-3-yl)-2-(1-(2,2,2-trifluoroethyl)piperidin-4-yl)benzo[d][1,3]dioxole-5-carboxamide (compound 34)**

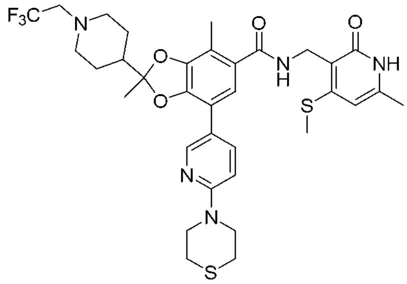

The same reaction as in Preparation Example 33 was performed except that 4-(5-(4,4,5,5-tetramethyl-1,3,2-dioxaborolan-2-yl)pyridin-2-yl)thiomorpholine was used instead of (2S,6R)-2,6-dimethyl-4-(5-(4,4,5,5-tetramethyl-1,3,2-dioxaborolan-2-yl)pyridin-2-yl)morpholine in [Step 1] of Preparation Example 33 to obtain the title compound (88 mg).

$^1$H NMR (Chloroform-d): 8.55 (s, 1H), 7.76 (s, 1H), 7.13 (s, 1H), 6.56 (s, 1H), 5.94 (s, 1H), 4.61 (d, 2H), 3.95 (m, 4H), 3.01 (d, 2H), 2.96 (m, 2H), 2.65 (m, 4H), 2.46 (s, 3H), 2.33 (m, 5H), 2.18 (s, 3H), 1.82 (m, 3H), 1.59 (m, 5H).

LC-MS (ESI, m/z) = 704.3 (M+H$^+$)

**Preparation Example 35. Synthesis of 7-(2-(dimethylamino)pyrimidin-5-yl)-2,4-dimethyl-N-((6-methyl-4-(methylthio)-2-oxo-1,2-dihydropyridin-3-yl)methyl)-2-(1-(2,2,2-trifluoroethyl)piperidin-4-yl)benzo[d][1,3]dioxole-5-carboxamide (compound 35)**

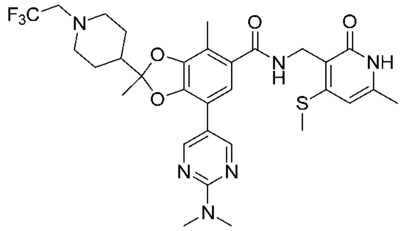

The same reaction as in Preparation Example 33 was performed except that N,N-dimethyl-5-(4,4,5,5-tetramethyl-1,3,2-dioxaborolan-2-yl)pyrimidin-2-amine was used instead of (2S,6R)-2,6-dimethyl-4-(5-(4,4,5,5-tetramethyl-1,3,2-dioxaborolan-2-yl)pyridin-2-yl)morpholine in [Step 1] of Preparation Example 33 to obtain the title compound (21 mg).

$^1$H NMR (Chloroform-d): 8.64 (s, 1H), 7.28 (s, 1H), 7.07 (S, 1H), 5.94 (s, 1H), 4.61 (d, 2H), 3.19 (s, 6H), 2.96 (m, 2H), 2.94 (m, 2H), 2.45 (s, 3H), 2.31 (m, 5H), 2.29 (s, 3H), 1.81 (m, 2H), 1.79 (s, 6H).

LC-MS (ESI, m/z) = 647.3 (M+H$^+$)

**Preparation Example 36. Synthesis of 7-(6-methoxypyridin-3-yl)-2,4-dimethyl-N-((6-methyl-4-(methylthio)-2-oxo-1,2-dihydropyridin-3-yl)methyl)-2-(1-(2,2,2-trifluoroethyl)piperidin-4-yl)benzo[d][1,3]dioxole-5-carboxamide (compound 36)**

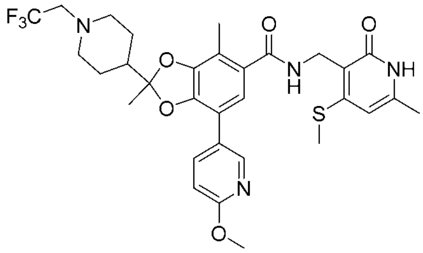

The same reaction as in Preparation Example 33 was performed except that 2-methoxy-5-(4,4,5,5-tetramethyl-1,3,2-dioxaborolan-2-yl)pyridine was used instead of (2S,6R)-2,6-dimethyl-4-(5-(4,4,5,5-tetramethyl-1,3,2-dioxaborolan-2-yl)pyridin-2-yl)morpholine in [Step 1] of Preparation Example 33 to obtain the title compound (20 mg).

$^1$H NMR (Chloroform-d): 8.45 (s, 1H), 7.78 (d, 1H), 7.22 (s, 1H), 7.07 (s, 1H), 6.69 (d, 1H), 5.92 (s, 1H), 4.58 (d, 2H), 3.89 (d, 3H), 3.00 (d, 2H), 2.93 (m, 2H), 2.43 (s, 3H), 2.29 (m, 5H), 2.12 (s, 3H), 1.80 (m, 3H), 1.57 (m, 5H).

LC-MS (ESI, m/z) = 633.4 ($M+H^+$)

**Preparation Example 37. Synthesis of 7-(6-(3,5-dimethylpiperidin-1-yl)pyridin-3-yl)-2,4-dimethyl-N-((6-methyl-4-(methylthio)-2-oxo-1,2-dihydropyridin-3-yl)methyl)-2-(1-(2,2,2-trifluoroethyl)piperidin-4-yl)benzo[d][1,3]dioxole-5-carboxamide (compound 37)**

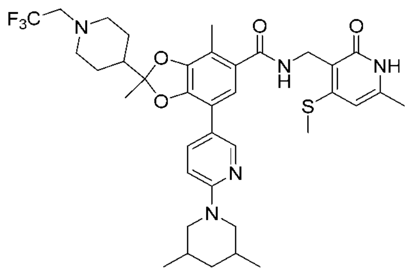

The same reaction as in Preparation Example 33 was performed except that 2-(3,5-dimethylpiperidin-1-yl)-5-(4,4,5,5-tetramethyl-1,3,2-dioxaborolan-2-yl)pyridine was used instead of (2S,6R)-2,6-dimethyl-4-(5-(4,4,5,5-tetramethyl-1,3,2-dioxaborolan-2-yl)pyridin-2-yl)morpholine in [Step 1] of Preparation Example 33 to obtain the title compound (47 mg).

$^1$H NMR (Chloroform-d): 8.51 (s, 1H), 7.68 (m, 1H), 7.25 (m, 1H), 7.08 (s, 1H), 6.60 (m, 1H), 5.92 (d, 1H), 4.60 (s, 2H), 4.20 (d, 2H), 2.99 (m, 2H), 2.93 (m, 2H), 2.43 (d, 3H), 2.29 (d, 7H), 2.15 (d, 3H), 1.80 (s, 4H), 1.56 (m, 7H), 1.24 (s, 1H), 0.93 (m, 7H), 0.79 (m, 1H).

LC-MS (ESI, m/z) = 714.5 ($M+H^+$)

**Preparation Example 38. Synthesis of 7-(2-((2S,6R)-2,6-dimethylmorpholino)pyrimidin-5-yl)-2,4-dimethyl-N-((6-methyl-4-(methylthio)-2-oxo-1,2-dihydropyridin-3-yl)methyl)-2-(1-(2,2,2-trifluoroethyl)piperidin-4-yl)benzo[d][1,3]dioxole-5-carboxamide (compound 38)**

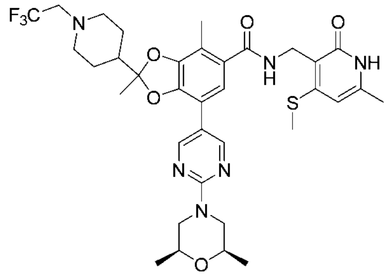

The same reaction as in Preparation Example 33 was performed except that (2S,6R)-2,6-dimethyl-4-(5-(4,4,5,5-tetramethyl-1,3,2-dioxaborolan-2-yl)pyrimidin-2-yl)morpholine was used instead of (2S,6R)-2,6-dimethyl-4-(5-(4,4,5,5-tetramethyl-1,3,2-dioxaborolan-2-yl)pyridin-2-yl)morpholine in [Step 1] of Preparation Example 33 to obtain the title compound (37 mg).

$^1$H NMR (Chloroform-d): 8.59 (s, 2H), 7.11 (t, 1H), 7.03 (s, 1H), 5.96 (s, 1H), 4.59 (d, 2H), 4.50 (dd, 2H), 3.61 (m, 2H), 3.00 (d, 2H), 2.95 (m, 2H), 2.59 (t, 2H), 2.44 (s, 3H), 2.28 (m, 5H), 2.21 (s, 3H), 1.79 (m, 3H), 1.56 (s, 5H), 1.23 (d, 9H).

LC-MS (ESI, m/z) = 717.4 (M+H$^+$)

**Preparation Example 39. Synthesis of 2,4-dimethyl-N-((6-methyl-4-(methylthio)-2-oxo-1,2-dihydropyridin-3-yl)methyl)-7-(2-(piperidin-1-yl)pyrimidin-5-yl)-2-(1-(2,2,2-trifluoroethyl)piperidin-4-yl)benzo[d][1,3]dioxole-5-carboxamide (compound 39)**

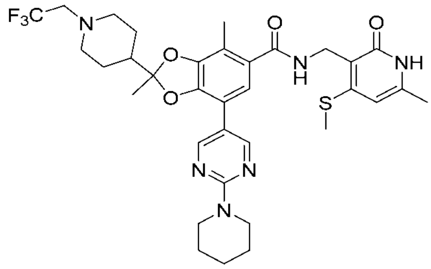

The same reaction as in Preparation Example 33 was performed except that 2-(piperidin-1-yl)-5-(4,4,5,5-tetramethyl-1,3,2-dioxaborolan-2-yl)pyrimidine was used instead of (2S,6R)-2,6-dimethyl-4-(5-(4,4,5,5-tetramethyl-1,3,2-dioxaborolan-2-yl)pyridin-2-yl)morpholine in [Step 1] of Preparation Example 33 to obtain the title compound (55 mg).

$^1$H NMR (Chloroform-d): 8.57 (s, 2H), 7.23 (m, 1H), 7.03 (s, 1H), 5.94 (s, 1H), 4.58 (d, 2H), 3.75 (t, 4H), 2.99 (d, 2H), 2.93 (m, 2H), 2.43 (s, 3H), 2.29 (m, 5H), 2.19 (s, 3H), 1.80 (m, 3H), 1.65 (m, 2H), 1.57 (s, 9H).

LC-MS (ESI, m/z) = 687.3 (M+H$^+$)

**Preparation Example 40. Synthesis of 7-(6-((2S,6R)-2,6-dimethylmorpholino)pyridin-3-yl)-2-(1-((S)-2-hydroxypropyl)piperidin-4-yl)-2,4-dimethyl-N-((6-methyl-4-(methylthio)-2-oxo-1,2-dihydropyridin-3-yl)methyl)benzo[d][1,3]dioxole-5-carboxamide (compound 40)**

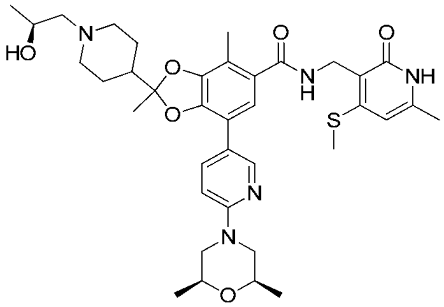

The same reaction as in Preparation Example 33 was performed except that (S)-1-chloro-2-propanol was used instead of 2,2,2-trifluoroethyl trifluoromethanesulfonate in [Step 6] of Preparation Example 33 to obtain the title compound (66 mg).

$^1$H NMR (Chloroform-d): 8.49 (t, 1H), 7.74 (d, 1H), 7.21 (s, 1H), 7.06(s, 1H), 6.59(d, 1H), 5.95(s, 1H), 4.58(s, 2H), 4.01(dd, 3H), 3.68(m, 2H), 2.50 (t, 4H), 2.44 (s, 4H), 2.28 (s, 3H), 2.18 (s, 3H), 1.88 (m, 4H), 1.58 (s, 3H), 1.24 (d, 9H), 1.13 (d, 3H).

LC-MS (ESI, m/z) = 692.4 (M+H$^+$)

**Preparation Example 41. Synthesis of 2-(1-(2,2-dimethoxyethyl)piperidin-4-yl)-7-(6-((2S,6R)-2,6-dimethylmorpholino)pyridin-3-yl)-2,4-dimethyl-N-((6-methyl-4-(methylthio)-2-oxo-1,2-dihydropyridin-3-yl)methyl)benzo[d][1,3]dioxole-5-carboxamide (compound 41)**

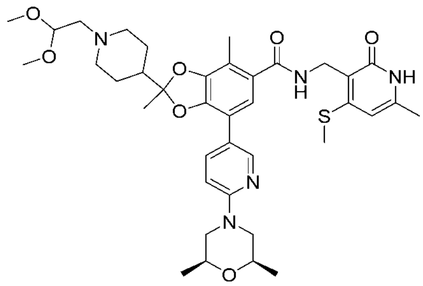

The same reaction as in Preparation Example 33 was performed except that bromoacetaldehyde dimethyl acetal was used instead of 2,2,2-trifluoroethyl trifluoromethanesulfonate in [Step 6] of Preparation Example 33 to obtain the title compound (201 mg).

$^1$H NMR (Chloroform-d): 8.50 (s, 1H), 7.67 (dd, 1H), 7.20 (t, 1H), 7.05(s, 1H), 6.53 (d, 1H), 5.92 (s, 1H), 4.59 (d, 2H), 4.51 (t, 1H), 3.98-4.00 (m, 2H), 3.66-3.67 (m, 2H), 3.31 (s, 6H), 3.06 (d, 2H), 2.48-2.53 (m, 4H), 2.42 (s, 3H), 2.27 (s, 3H), 2.13 (s, 3H), 2.05 (t, 2H), 1.76-1.80 (m, 3H), 1.64-1.66 (m, 2H), 1.55(s, 3H), 1.24 (d, 8H).

LC-MS (ESI, m/z) = 722.4 (M+H$^+$)

**Preparation Example 42. Synthesis of 2-(4-(dimethylamino)cyclohexyl)-2,4-dimethyl-N-((6-methyl-4-(methylthio)-2-oxo-1,2-dihydropyridin-3-yl)methyl)-7-(thiophen-3-yl)benzo[d][1,3]dioxole-5-carboxamide (compound 42)**

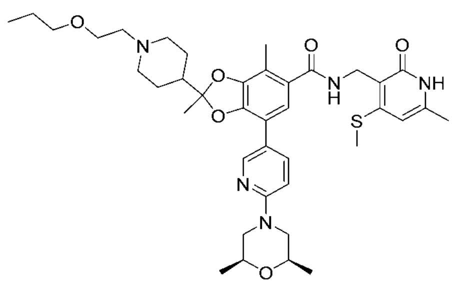

The same reaction as in Preparation Example 33 was performed except that 2-chloroethyl propyl ether was used instead of 2,2,2-trifluoroethyl trifluoromethanesulfonate in [Step 6] of Preparation Example 33 to obtain the title compound (220 mg).

$^1$H NMR (Chloroform-d): 7.59 (d, 1H), 7.39 (m, 1H), 7.25 (m, 1H), 7.23 (m, 1H), 7.19 (s, 1H), 5.92 (s, 1H), 4.60 (d, 2H), 2.43 (s, 3H), 2.29 (s, 3H), 2.24 (s, 6H), 2.14 (m, 4H), 1.97 (t, 4H), 1.80 (t, 1H), 1.58 (s, 3H), 1.23 (m, 4H).

LC-MS (ESI, m/z) = 568.3 (M+H$^+$)

**Preparation Example 43. Synthesis of 2-(4-(dimethylamino)cyclohexyl)-7-(5-fluoro-2-methoxyphenyl)-2,4-dimethyl-N-((6-methyl-4-(methylthio)-2-oxo-1,2-dihydropyridin-3-yl)methyl)benzo[d][1,3]dioxole-5-carboxamide (compound 43)**

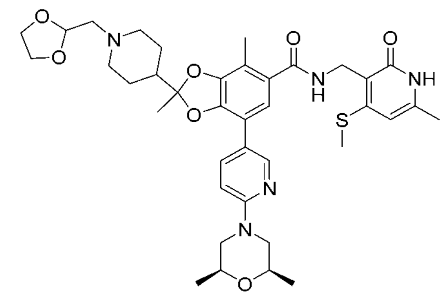

The same reaction as in Preparation Example 33 was performed except that 2-bromomethyl-1,3-dioxolane was used instead of 2,2,2-trifluoroethyl trifluoromethanesulfonate in [Step 6] of Preparation Example 33 to obtain the title compound (124 mg).

$^{1}H$ NMR (Chloroform-d): 7.02 (m, 1H), 6.98 (m, 2H), 6.92 (m, 1H), 6.79 (m, 1H), 5.91 (s, 1H), 4.58 (d, 2H), 3.68 (s, 3H), 2.44 (s, 3H), 2.32 (s, 2H), 2.25 (s, 6H), 2.13 (s, 4H), 1.94 (m, 3H), 1.79 (t, 1H), 1.53 (s, 3H), 1.20 (m, 4H).

LC-MS (ESI, m/z) = 610.3 ($M+H^+$)

**Preparation Example 44. Synthesis of stereoisomer B of 7-(6-((2S,6R)-2,6-dimethylmorpholino)pyridin-3-yl)-2,4-dimethyl-N-((6-methyl-4-(methylthio)-2-oxo-1,2-dihydropyridin-3-yl)methyl)-2-(1-(2,2,2-trifluoroethyl)piperidin-4-yl)benzo[d][1,3]dioxole-5-carboxamide (compound 44)**

Stereoisomer B of

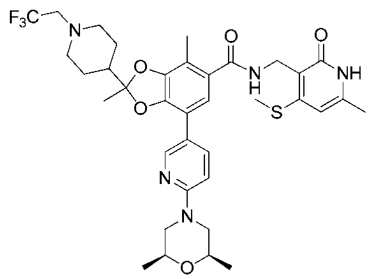

(

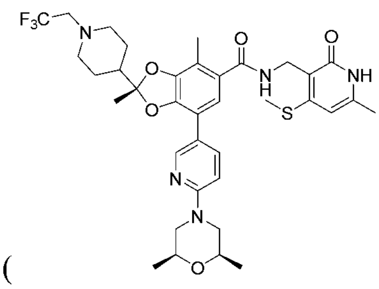

or

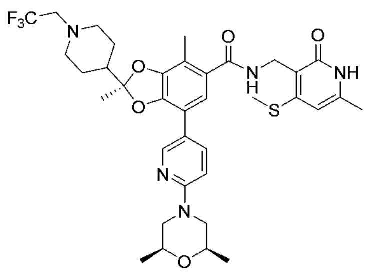

)

The same processes as in [Steps 1 to 6] of above Preparation Example 33 were performed except that tert-butyl (R)-4-(7-bromo-5-(methoxycarbonyl)-2,4-dimethylbenzo[d][1,3]dioxole-2-yl)piperidin-1-carboxylate (3 g, 6.38 mmole), which is stereospecific, was used as a starting material in [Step 1] instead of tert-butyl 4-(7-bromo-5-(methoxycarbonyl)-2,4-dimethylbenzo[d][1,3]dioxole-2-yl)piperidin-1-carboxylate, so as to obtain the title compound (3.3 g, 4.60 mmole).

Said tert-butyl (R)-4-(7-bromo-5-(methoxycarbonyl)-2,4-dimethylbenzo[d][1,3]dioxole-2-yl)piperidin-1-carboxylate used (

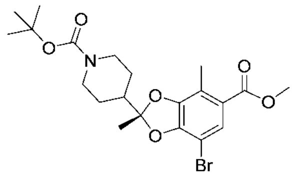

) was measured at RT 6.01 min, having a chiral purity of 99.3% and a specific optical rotation of $[\alpha]_D^{25}$ = +4.0 (c=1, chloroform) when the purity of isomers was measured by liquid chromatography under the following conditions.

- Detector: UV absorption spectrophotometer (measurement wavelength: 270 nm)
- Column: Chiralpak OX-3 or an equivalent thereto (ex: Chiralpak OX-3, particle size 3 μm, 4.6 mm × 15 cm)
- Column temperature: 25°C
- Mobile phase: a mixed solution of hexane/ethanol (98:2, v/v)
- Flux: 1.0 mL/min
- Analysis time: 15 minutes

$^1$H NMR (DMSO-$d_6$): 11.5 (s, 1H), 8.43 (s, 1H), 7.95 (t, 1H), 7.80 (s, 1H), 7.02 (s, 1H), 6.90 (d, 1H), 6.05 (s, 1H), 4.27 (d, 2H), 4.13 (d, 2H), 3.53-3.59 (m, 2H), 3.06-3.10 (m, 2H), 2.92 (d, 2H), 2.39-2.43 (m, 5H), 2.38-2.39 (m, 1H), 2.24-2.30 (m, 2H), 2.17 (s, 3H), 2.13 (s, 3H), 1.81-1.88 (m, 1H), 1.67-1.73 (m, 2H), 1.56 (s, 3H), 1.32-1.40 (m, 2H), 1.13 (s, 3H), 1.12 (s, 3H).

LC-MS (ESI, m/z) = 716.8 ($M+H^+$)
Specific optical rotation $[\alpha]_D^{25}$ = -55.0000 (c=0.1, methanol)

**Preparation Example 45. Synthesis of stereoisomer A of 7-(6-((2S,6R)-2,6-dimethylmorpholino)pyridin-3-yl)-2,4-dimethyl-N-((6-methyl-4-(methylthio)-2-oxo-1,2-dihydropyridin-3-yl)methyl)-2-(1-(2,2,2-trifluoroethyl)piperidin-4-yl)benzo[d][1,3]dioxole-5-carboxamide (compound 45)**

Stereoisomer A of

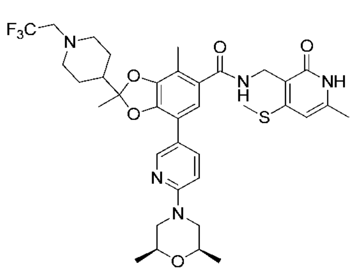

(

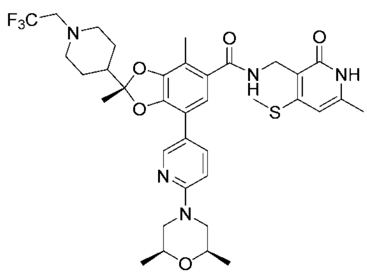

or

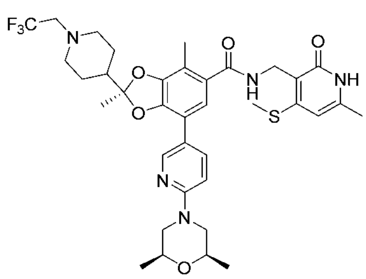

)

The same processes as in [Steps 1 to 6] of above Preparation Example 33 were performed except that tert-butyl (S)-4-(7-bromo-5-(methoxycarbonyl)-2,4-dimethylbenzo[d][1,3]dioxole-2-yl)piperidin-1-carboxylate (3 g, 6.38 mmole), which is stereospecific, was used as a starting material in [Step 1] instead of tert-butyl 4-(7-bromo-5-(methoxycarbonyl)-2,4-dimethylbenzo[d][1,3]dioxole-2-yl)piperidin-1-carboxylate, so as to obtain the title compound (3.2 g, 4.47 mmole).

Said tert-butyl (S)-4-(7-bromo-5-(methoxycarbonyl)-2,4-dimethylbenzo[d][1,3]dioxole-2-yl)piperidin-1-carbox-

ylate used (

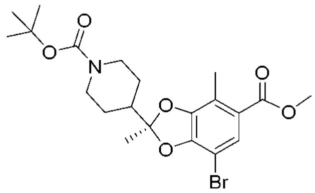

) was measured at RT 5.24 min, having a chiral purity of 99.8% and a specific optical rotation of $[\alpha]_D^{25}$ = -3.6 (c=1, chloroform) when the purity of isomers was measured by liquid chromatography under the following conditions.

- Detector: UV absorption spectrophotometer (measurement wavelength: 270 nm)
- Column: Chiralpak OX-3 or an equivalent thereto (ex: Chiralpak OX-3, particle size 3 μm, 4.6 mm × 15 cm)
- Column temperature: 25°C
- Mobile phase: a mixed solution of hexane/ethanol (98:2, v/v)
- Flux: 1.0 mL/min
- Analysis time: 15 minutes

$^1$H NMR (DMSO-$d_6$): 11.5 (s, 1H), 8.43 (s, 1H), 7.95 (t, 1H), 7.80 (s, 1H), 7.02 (s, 1H), 6.90 (d, 1H), 6.05 (s, 1H), 4.27 (d, 2H), 4.13 (d, 2H), 3.53-3.59 (m, 2H), 3.06-3.10 (m, 2H), 2.92 (d, 2H), 2.36-2.40 (m, 5H), 2.34-2.36 (m, 1H), 2.22-2.30 (m, 2H), 2.17 (s, 3H), 2.13 (s, 3H), 1.81-1.88 (m, 1H), 1.67-1.73 (m, 2H), 1.56 (s, 3H), 1.32-1.40 (m, 2H), 1.13 (s, 3H), 1.12 (s, 3H)
LC-MS (ESI, m/z) = 716.8 (M+H$^+$)
Specific optical rotation $[\alpha]_D^{25}$ = +55.7000 (c=0.1, methanol)

**Preparation Example 46. Synthesis of 2-(1-(2,2-difluoroethyl)piperidin-4-yl)-7-(6-((2S,6R)-2,6-dimethylmorpholino)pyridin-3-yl)-2,4-dimethyl-N-((6-methyl-4-(methylthio)-2-oxo-1,2-dihydropyridin-3-yl)methyl)benzo[d][1,3]dioxole-5-carboxamide (compound 46)**

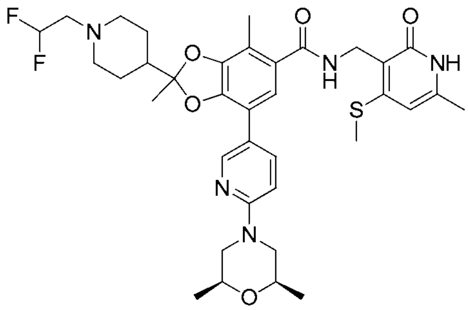

The same processes as in [Steps 1 to 5] of above Preparation Example 33 were performed to obtain 7-(6-((2S,6R)-2,6-dimethylmorpholino)pyridin-3-yl)-2,4-dimethyl-N-((6-methyl-4-(methylthio)-2-oxo-1,2-dihydropyridin-3-yl)methyl)2-(piperidin-4-yl)benzo[d][1,3]dioxole-5-carboxamide hydrochloride of [Step 5].

Acetonitrile (10 mL), sodium carbonate (0.43 g), and 2-iodo-1,1-difluoroethane (0.12 mL) were added to the obtained hydrochloride (0.5 g, 0.70 mmole), and stirred at an internal temperature of 100°C for 24 hours. After cooling, dichloromethane (5 mL) was added thereto, filtered, concentrated, and separated into layers with dichloromethane (10 mL) and purified water (10 mL), and an oil layer was dried with magnesium sulfate ($MgSO_4$) and then concentrated. The resulting crystal was filtered with ethanol (2 mL) and hexane (20 mL) and dried at an internal temperature of 60°C to obtain the title compound (0.3 g).

$^1$H NMR (DMSO-$d_6$): 11.5 (s, 1H), 8.36 (s, 1H), 8.19 (t, 1H ), 8.00 (s, 1H), 7.23 (s, 1H), 7.09 (s, 1H), 6.61 (t, 1H), 6.06 (s, 1H), 4.26-4.28 (m, 4H), 3.62-3.64 (m, 4H), 3.52-3.62 (m, 2H), 3.06-3.07 (m, 2H), 2.61-2.65 (m, 2H), 2.41 (s, 3H), 2.22 (s, 3H), 2.14 (s, 3H), 1.91-1.93 (m, 2H), 1.88-1.91 (m, 3H), 1.60 (s, 3H), 1.14 (s, 3H), 1.13 (s, 3H).

LC-MS (ESI, m/z) = 698.4 (M+H$^+$)

**Preparation Example 47. Synthesis of stereoisomer B of 2-(1-(2,2-difluoroethyl)piperidin-4-yl)-7-(6-((2S,6R)-2,6-dimethylmorpholino)pyridin-3-yl)-2,4-dimethyl-N-((6-methyl-4-(methylthio)-2-oxo-1,2-dihydropyridin-3-yl)methyl)benzo[d][1,3]dioxole-5-carboxamide (compound 47)**

Stereoisomer B of

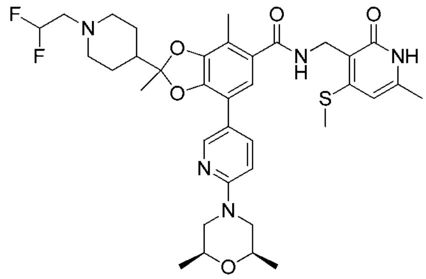

(

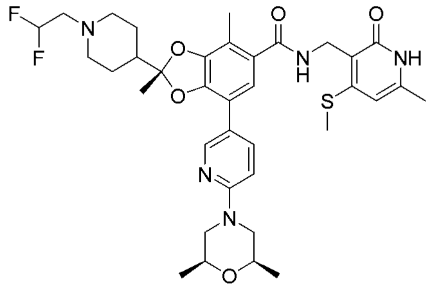

or

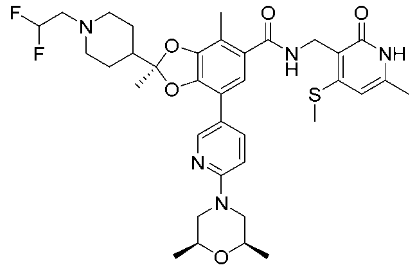

)

The same reaction as in above Preparation Example 46 was performed except that tert-butyl (R)-4-(7-bromo-5-(methoxycarbonyl)-2,4-dimethylbenzo[d][1,3]dioxole-2-yl)piperidin-1-carboxylate (3 g, 6.38 mmole), which is stereospecific, was used in [Step 1] instead of tert-butyl 4-(7-bromo-5-(methoxycarbonyl)-2,4-dimethylbenzo[d][1,3] dioxole-2-yl)piperidin-1-carboxylate, so as to obtain the title compound (0.2 g).

$^1$H NMR (DMSO-$d_6$): 11.5 (s, 1H), 8.44 (s, 1H), 7.94 (t, 1H), 7.79 (s, 1H), 7.02 (s, 1H), 6.90 (d, 1H), 6.04-6.05 (m, 2H), 4.26 (d, 2H), 4.13 (d, 2H), 3.55-3.56 (m, 2H), 2.89-2.92 (m, 2H), 2.60-2.69 (m, 2H), 2.39 (s, 3H), 2.36-2.38 (m, 2H), 2.16 (s, 3H), 2.13 (s, 3H), 2.07-2.12 (m, 2H), 1.80-1.86 (m, 1H), 1.65-1.72 (m, 2H), 1.56 (s, 3H), 1.35-1.40 (m, 2H), 1.13 (s, 3H), 1.12 (s, 3H).

LC-MS (ESI, m/z) = 698.6 (M+H$^+$)
Specific optical rotation $[\alpha]_D^{25}$ = -58.1500 (c=0.1, methanol)

**Preparation Example 48. Synthesis of stereoisomer A of 2-(1-(2,2-difluoroethyl)piperidin-4-yl)-7-(6-((2S,6R)-2,6-dimethylmorpholino)pyridin-3-yl)-2,4-dimethyl-N-((6-methyl-4-(methylthio)-2-oxo-1,2-dihydropyridin-3-yl)methyl)benzo[d][1,3]dioxole-5-carboxamide (compound 48)**

Stereoisomer A of

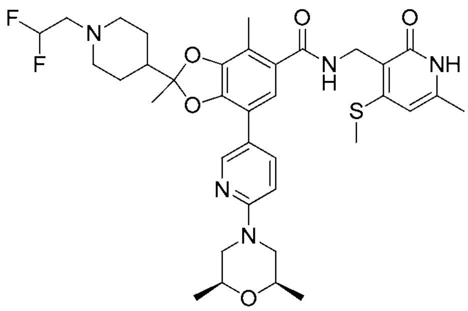

(

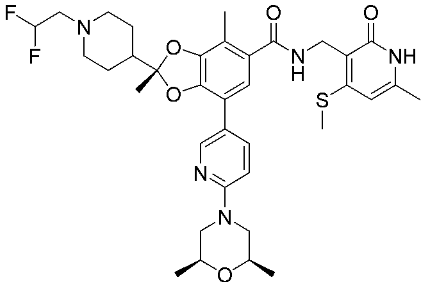

or

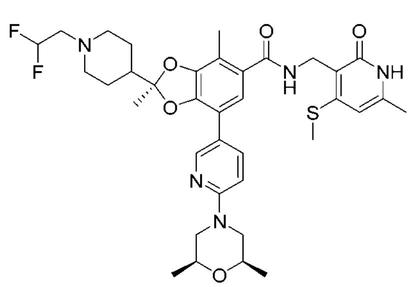

)

The same reaction as in above Preparation Example 46 was performed except that tert-butyl (S)-4-(7-bromo-5-(methoxycarbonyl)-2,4-dimethylbenzo[d][1,3]dioxole-2-yl)piperidin-1-carboxylate (3 g, 6.38 mmole), which is stereospecific, was used in [Step 1] instead of tert-butyl 4-(7-bromo-5-(methoxycarbonyl)-2,4-dimethylbenzo[d][1,3]dioxole-2-yl)piperidin-1-carboxylate, so as to obtain the title compound (0.2 g).

$^1$H NMR (DMSO-$d_6$): 11. 5(s, 1H), 8.44 (s, 1H), 7.94 (t, 1H), 7.79 (s, 1H), 7.02 (s, 1H), 6.90 (d, 1H), 6.04-6.05 (m, 2H), 4.26 (d, 2H), 4.13 (d, 2H), 3.57-3.58 (m, 2H), 2.89-2.92 (m, 2H), 2.60-2.69 (m, 2H), 2.40 (s, 3H), 2.36-2.38 (m, 2H), 2.16 (s, 3H), 2.13 (s, 3H), 2.07-2.12 (m, 2H), 1.80-1.86 (m, 1H), 1.65-1.72 (m, 2H), 1.56 (s, 3H), 1.35-1.40 (m, 2H), 1.13 (s, 3H), 1.12 (s, 3H).

LC-MS (ESI, m/z) = 698.6 (M+H$^+$)

Specific optical rotation $[\alpha]_D^{25}$ = +65.5667 (c=0.1, methanol)

**<u>Preparation Example 49. Synthesis of stereoisomer B of 7-(6-((2S,6R)-2,6-dimethylmorpholino)pyridin-3-yl)-2-(1-ethylpiperidin-4-yl)-2,4-dimethyl-N-((6-methyl-4-(methylthio)-2-oxo-1,2-dihydropyridin-3-yl)methyl)benzo[d][1,3]dioxole-5-carboxamide (compound 49)</u>**

Stereoisomer B of

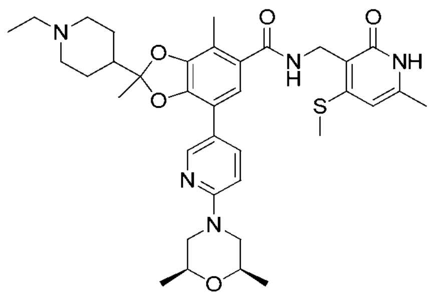

(

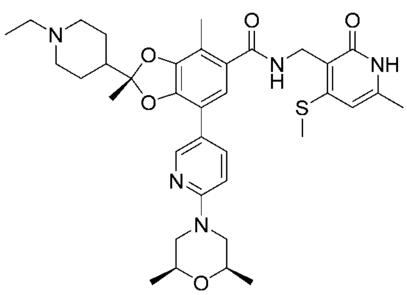

or

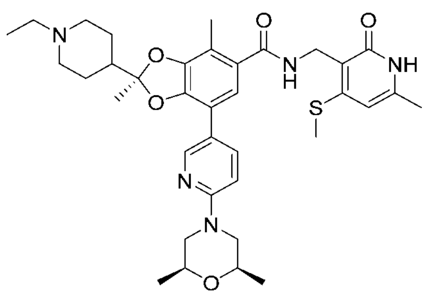

)

The same reaction as in above Preparation Example 47 was performed except that iodoethane (0.11 mL) was used instead of 2-iodo-1,1-difluoroethane in [Step 6] to obtain the title compound (0.1 g).

$^1$H NMR (DMSO-$d_6$): 11.5 (s, 1H), 8.48 (s, 1H), 7.96 (t, 1H), 7.82 (d, 1H), 7.05 (s, 1H), 6.90 (d, 1H), 6.05 (s, 1H),

4.27 (d, 2H), 4.14 (d, 2H), 3.55-3.59 (m, 2H), 3.40-3.47 (m, 2H), 3.00-3.05 (m, 2H), 2.80-2.86 (m, 2H), 2.47 (s, 3H), 2.41-2.44 (m, 2H), 2.18 (s, 3H), 2.12 (s, 3H), 1.87-1.99 (m, 3H), 1.56-1.66 (m, 5H), 1.12-1.13 (m, 6H), 1.12 (s, 3H).

LC-MS (ESI, m/z) = 662.6 ($M+H^+$)
Specific optical rotation $[\alpha]_D^{25}$ = -55.4000 (c=0.1, methanol)

**Preparation Example 50. Synthesis of stereoisomer A of 7-(6-((2S,6R)-2,6-dimethylmorpholino)pyridin-3-yl)-2-(1-ethylpiperidin-4-yl)-2,4-dimethyl-N-((6-methyl-4-(methylthio)-2-oxo-1,2-dihydropyridin-3-yl)methyl)benzo[d][1,3]dioxole-5-carboxamide (compound 50)**

Stereoisomer A of

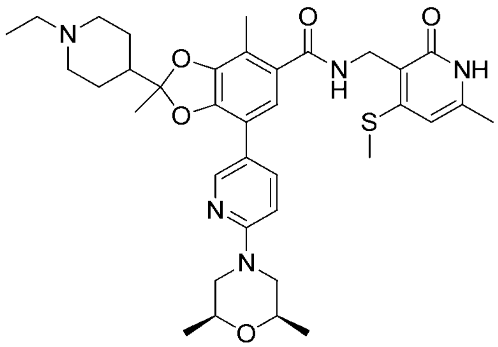

(

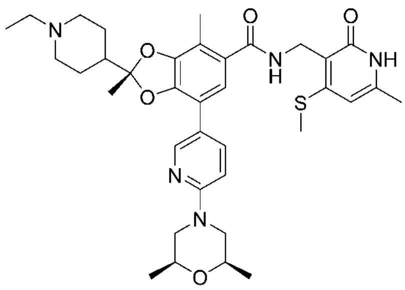

or

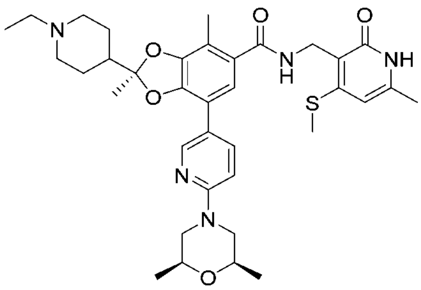

)

The same reaction as in above Preparation Example 48 was performed except that iodoethane (0.11 mL) was used instead of 2-iodo-1,1-difluoroethane in [Step 6] to obtain the title compound (0.1 g).

$^1$H NMR (DMSO-$d_6$): 11.5 (s, 1H), 8.49 (s, 1H), 7.96 (t, 1H), 7.82 (d, 1H), 7.05 (s, 1H), 6.90 (d, 1H), 6.05 (s, 1H), 4.27 (d, 2H), 4.14 (d, 2H), 3.55-3.57 (m, 2H), 3.40-3.49 (m, 2H), 3.00-3.06 (m, 2H), 2.80-2.86 (m, 2H), 2.47 (s, 3H), 2.41-2.43 (m, 2H), 2.18 (s, 3H), 2.13 (s, 3H), 1.90-1.99 (m, 3H), 1.55-1.65 (m, 5H), 1.12-1.13 (m, 6H), 1.12 (s, 3H).

LC-MS (ESI, m/z) = 662.6 ($M+H^+$)
Specific optical rotation $[\alpha]_D^{25}$ = +67.8834 (c=0.1, methanol)

**Preparation Example 51. Synthesis of stereoisomer B of 7-(6-((2S,6R)-2,6-dimethylmorpholino)pyridin-3-yl)-2-(1-isopropylpiperidin-4-yl)-2,4-dimethyl-N-((6-methyl-4-(methylthio)-2-oxo-1,2-dihydropyridin-3-yl)methyl)benzo[d][1,3]dioxole-5-carboxamide (compound 51)**

Stereoisomer B of

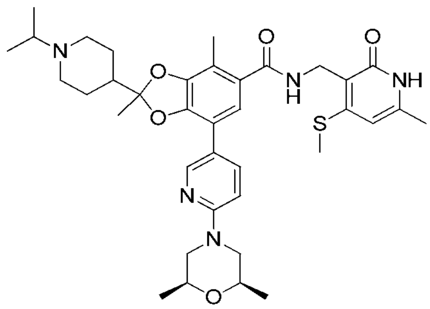

(

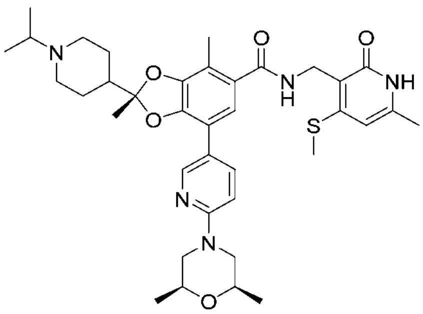

or

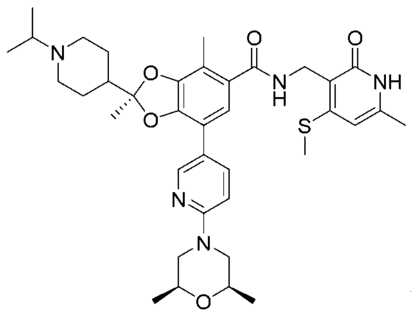

)

The same reaction as in above Preparation Example 47 was performed except that 2-iodo propane (0.13 mL) was used instead of 2-iodo-1,1-difluoroethane in [Step 6] to obtain the title compound (0.2 g).

$^1$H NMR (DMSO-$d_6$): 11.5 (s, 1H), 8.46 (s, 1H), 7.95 (t, 1H), 7.81 (d, 1H), 7.03 (s, 1H), 6.89 (d, 1H), 6.05 (s, 1H), 4.27 (d, 2H), 4.13 (d, 2H), 3.56-3.58 (m, 2H), 3.02-3.10 (m, 2H), 2.41 (s, 3H), 2.38-2.40 (m, 2H), 2.36-2.37 (m, 2H), 2.17 (s, 3H), 2.13 (s, 3H), 2.00-2.10 (m, 2H), 1.80-1.84 (m, 2H), 1.58 (s, 3H), 1.45-1.48 (m, 2H), 1.13 (s, 3H), 1.12 (s, 3H), 1.02 (s, 6H).

LC-MS (ESI, m/z) = 676.6 (M+H$^+$)
Specific optical rotation $[\alpha]_D^{25}$ = -61.1000 (c=0.1, methanol)

**Preparation Example 52. Synthesis of stereoisomer A of 7-(6-((2S,6R)-2,6-dimethylmorpholino)pyridin-3-yl)-2-(1-isopropylpiperidin-4-yl)-2,4-dimethyl-N-((6-methyl-4-(methylthio)-2-oxo-1,2-dihydropyridin-3-yl) methyl)benzo[d][1,3]dioxole-5-carboxamide (compound 52)**

Stereoisomer A of

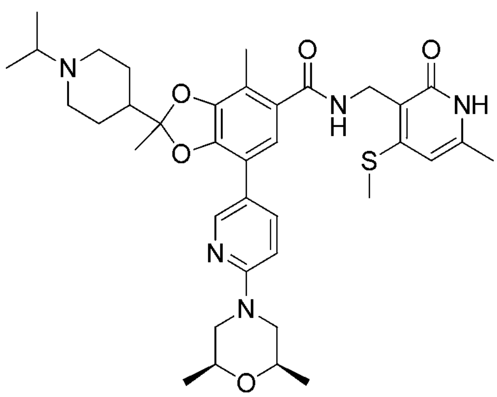

(

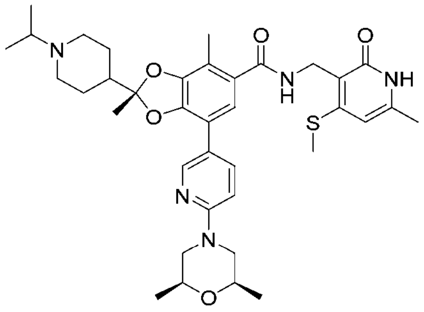

or

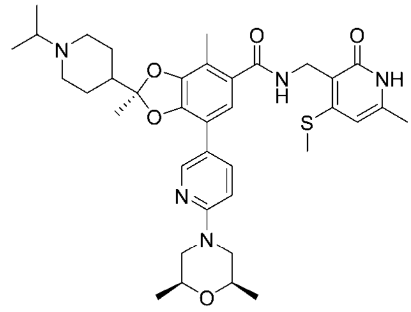

)

The same reaction as in above Preparation Example 48 was performed except that 2-iodo propane (0.13 mL) was used instead of 2-iodo-1,1-difluoroethane in [Step 6] to obtain the title compound (0.2 g).

$^{1}H$ NMR (DMSO-$d_6$): 11.5 (s, 1H), 8.46 (s, 1H), 7.95 (t, 1H), 7.80 (d, 1H), 7.02 (s, 1H), 6.90 (d, 1H), 6.05 (s, 1H), 4.27 (d, 2H), 4.14 (d, 2H), 3.56-3.58 (m, 2H), 2.83-2.88 (m, 2H), 2.41 (s, 3H), 2.38-2.40 (m, 2H), 2.36-2.37 (m, 2H), 2.17 (s, 3H), 2.13 (s, 3H), 2.00-2.10 (m, 2H), 1.72-1.81 (m, 2H), 1.57 (s, 3H), 1.38-1.43 (m, 2H), 1.13 (s, 3H), 1.12 (s, 3H), 0.95 (s, 6H).

LC-MS (ESI, m/z) = 676.6 ($M+H^+$)
Specific optical rotation $[\alpha]_D^{25}$ = +67.5167 (c=0.1, methanol)

**Preparation Example 53. Synthesis of stereoisomer B of 2-(1-cyclohexylpiperidin-4-yl)-7-(6-((2S,6R)-2,6-dimethylmorpholino)pyridin-3-yl)-2,4-dimethyl-N-((6-methyl-4-(methylthio)-2-oxo-1,2-dihydropyridin-3-yl)methyl)benzo[d][1,3]dioxole-5-carboxamide (compound 53)**

Stereoisomer B of

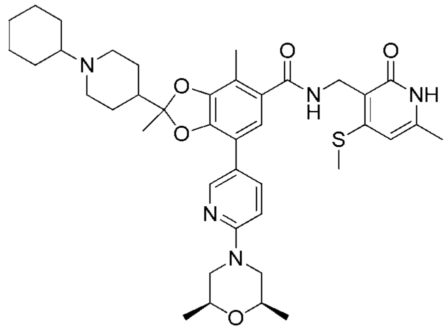

(

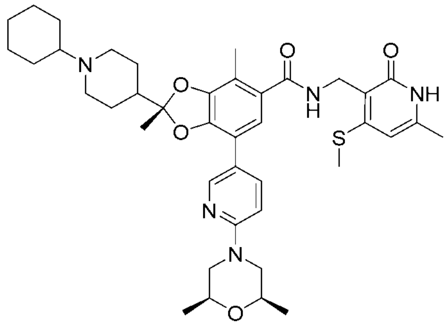

or

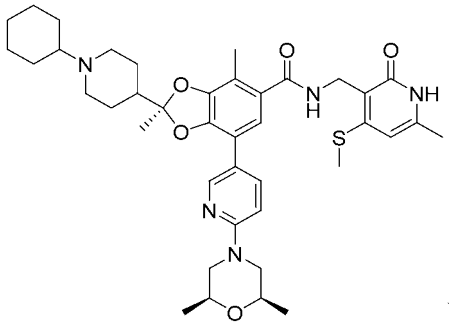

)

The same reaction as in above Preparation Example 47 was performed except that iodo cyclohexane (0.17 mL) was used instead of 2-iodo-1,1-difluoroethane in [Step 6] to obtain the title compound (0.2 g).

$^{1}H$ NMR (DMSO-$d_6$): 11.5 (s, 1H), 8.49 (s, 1H), 7.96 (t, 1H), 7.82 (d, 1H), 7.05 (s, 1H), 6.90 (d, 1H), 6.05 (s, 1H),

4.27 (d, 2H), 4.14 (d, 2H), 3.55-3.59 (m, 2H), 3.38-3.41 (m, 2H), 3.10-3.12 (m, 2H), 2.89-2.91 (m, 2H), 2.41 (s, 3H), 2.36-2.40 (m, 4H), 2.18 (s, 3H), 2.13 (s, 3H), 1.87-1.95 (m, 4H), 1.74-1.79 (m, 2H), 1.60 (s, 3H), 1.54-1.58 (m, 2H), 1.28-1.34 (m, 2H), 1.20-1.26 (m, 2H), 1.13 (s, 3H), 1.12 (s, 3H).

LC-MS (ESI, m/z) = 716.6 $(M+H^+)$
Specific optical rotation $[\alpha]_D^{25}$ = -62.5333 (c=0.1, methanol)

**Preparation Example 54. Synthesis of stereoisomer A of 2-(1-cyclohexylpiperidin-4-yl)-7-(6-((2S,6R)-2,6-dimethylmorpholino)pyridin-3-yl)-2,4-dimethyl-N-((6-methyl-4-(methylthio)-2-oxo-1,2-dihydropyridin-3-yl)methyl)benzo[d][1,3]dioxole-5-carboxamide (compound 54)**

Stereoisomer A of

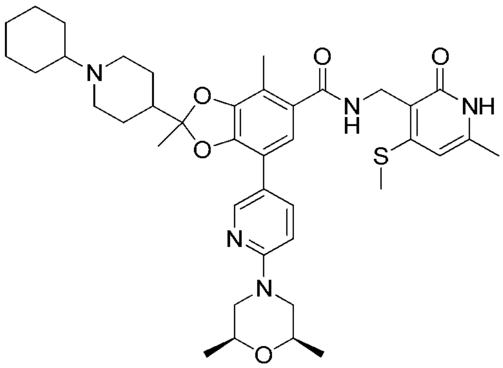

(

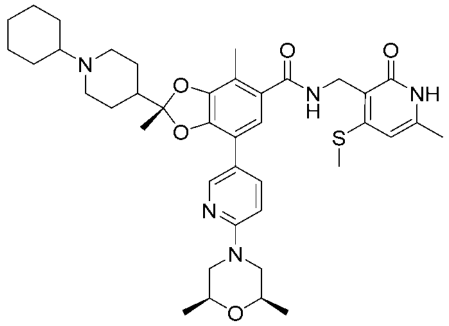

or

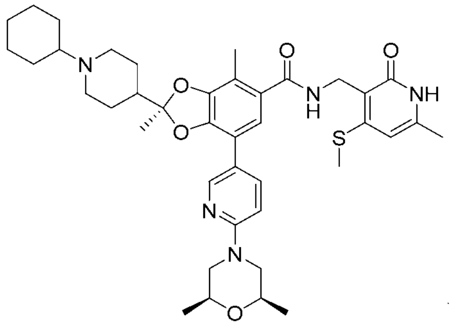

)

The same reaction as in above Preparation Example 48 was performed except that iodo cyclohexane (0.17 mL) was used instead of 2-iodo-1,1-difluoroethane in [Step 6] to obtain the title compound (0.2 g).

$^1$H NMR (DMSO-$d_6$): 11.5 (s, 1H), 8.49 (s, 1H), 7.96 (t, 1H), 7.82 (d, 1H), 7.05 (s, 1H), 6.90 (d, 1H), 6.05 (s, 1H), 4.27 (d, 2H), 4.15 (d, 2H), 3.54-3.58 (m, 2H), 3.38-3.41 (m, 2H), 3.10-3.12 (m, 2H), 2.93-3.60 (m, 2H), 2.41 (s, 3H), 2.38-2.40 (m, 4H), 2.19 (s, 3H), 2.13 (s, 3H), 1.87-1.95 (m, 4H), 1.74-1.79 (m, 2H), 1.60 (s, 3H), 1.56-1.58 (m, 2H), 1.28-1.34 (m, 2H), 1.20-1.26 (m, 2H), 1.13 (s, 3H), 1.12 (s, 3H).

LC-MS (ESI, m/z) = 716.6 $(M+H^+)$
Specific optical rotation $[\alpha]_D^{25}$ = +67.3834 (c=0.1, methanol)

**Preparation Example 55. Synthesis of stereoisomer B of 7-(6-((2S,6R)-2,6-dimethylmorpholino)pyridin-3-yl)-2,4-dimethyl-N-((6-methyl-4-(methylthio)-2-oxo-1,2-dihydropyridin-3-yl)methyl)-2-(1-phenethylpiperidin-4-yl)benzo[d][1,3]dioxole-5-carboxamide (compound 55)**

Stereoisomer B of

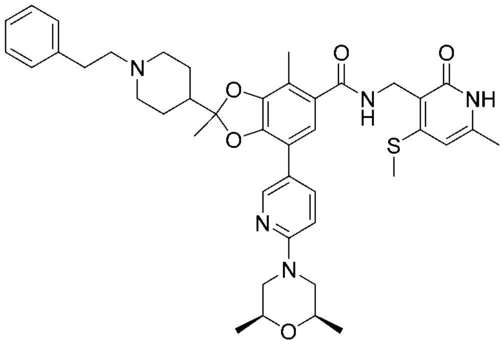

(

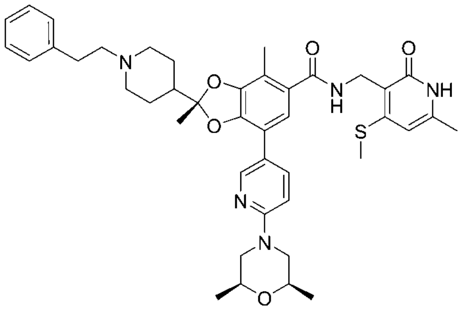

or

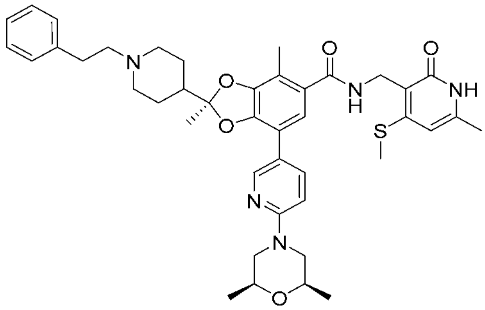

)

The same reaction as in above Preparation Example 47 was performed except that iodo cyclohexane (0.17 mL) was used instead of 2-iodo-1,1-difluoroethane in [Step 6] to obtain the title compound (0.2 g).

$^1$H NMR (DMSO-$d_6$): 11.5 (s, 1H), 8.46 (s, 1H), 7.95 (t, 1H), 7.81 (d, 1H), 7.20-7.30 (m, 2H), 7.10-7.20 (m, 3H), 7.03 (s, 1H), 6.90 (d, 1H), 6.05 (s, 1H), 4.27 (d, 2H), 4.14 (d, 2H), 3.53-3.58 (m, 2H), 3.01-3.10 (m, 2H), 2.70-2.97 (m, 2H), 2.41 (s, 3H), 2.38-2.40 (m, 2H), 2.17 (s, 3H), 2.13 (s, 3H), 1.93-1.95 (m, 2H), 1.87-1.91 (m, 2H), 1.81-1.85 (m, 2H), 1.70-1.81 (m, 2H), 1.58 (s, 3H), 1.38-1.48 (m, 2H), 1.13 (s, 3H), 1.12 (s, 3H).

LC-MS (ESI, m/z) = 738.6 (M+H$^+$)
Specific optical rotation $[\alpha]_D^{25}$ = -82.3667 (c=0.1, methanol)

**Preparation Example 56. Synthesis of stereoisomer A of 7-(6-((2S,6R)-2,6-dimethylmorpholino)pyridin-3-yl)-2,4-dimethyl-N-((6-methyl-4-(methylthio)-2-oxo-1,2-dihydropyridin-3-yl)methyl)-2-(1-phenethylpiperidin-4-yl)benzo[d][1,3]dioxole-5-carboxamide (compound 56)**

Stereoisomer A of

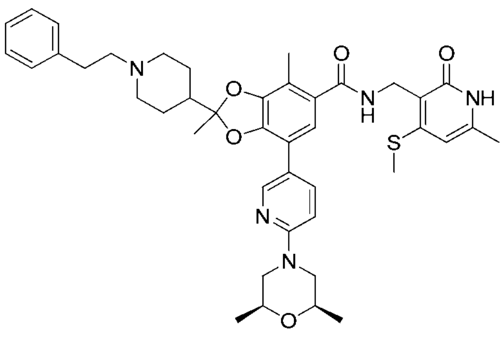

(

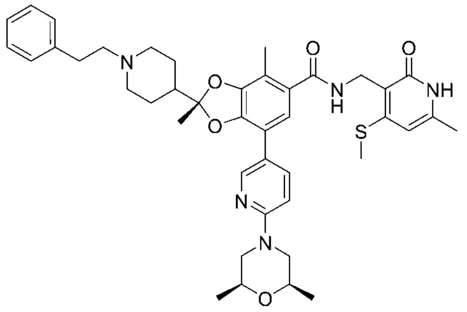

or

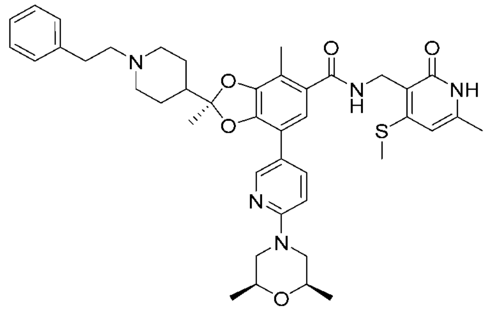

)

The same reaction as in above Preparation Example 48 was performed except that iodo cyclohexane (0.17 mL) was used instead of 2-iodo-1,1-difluoroethane in [Step 6] to obtain the title compound (0.2 g).

$^1$H NMR (DMSO-$d_6$): 11.5 (s, 1H), 8.45 (s, 1H), 7.95 (t, 1H), 7.81 (d, 1H), 7.21-7.22 (m, 2H), 7.03-7.18 (m, 3H), 7.02 (s, 1H), 6.91 (d, 1H), 6.05 (s, 1H), 4.27 (d, 2H), 4.14 (d, 2H), 3.56-3.57 (m, 2H), 2.92-2.99 (m, 2H), 2.66-2.69 (m, 2H), 2.41 (s, 3H), 2.38-2.40 (m, 2H), 2.17 (s, 3H), 2.13 (s, 3H), 1.87-1.91 (m, 2H), 1.85-1.87 (m, 2H), 1.81-1.85 (m, 2H), 1.70-1.81 (m, 2H), 1.58 (s, 3H), 1.38-1.48 (m, 2H), 1.13 (s, 3H), 1.12 (s, 3H).

LC-MS (ESI, m/z) = 738.6 (M+H$^+$)
Specific optical rotation $[\alpha]_D^{25}$ = +78.3167 (c=0.1, methanol)

**Preparation Example 57. Synthesis of stereoisomer B of 7-(6-((2S,6R)-2,6-dimethylmorpholino)pyridin-3-yl)-2,4-dimethyl-N-((6-methyl-4-(methylthio)-2-oxo-1,2-dihydropyridin-3-yl)methyl)-2-(1-(4,4,4-trifluorobutyl)piperidin-4-yl)benzo[d][1,3]dioxole-5-carboxamide (compound 57)**

Stereoisomer B of

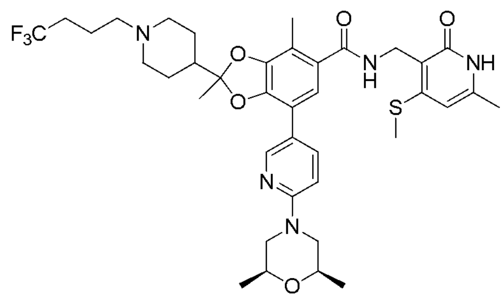

(

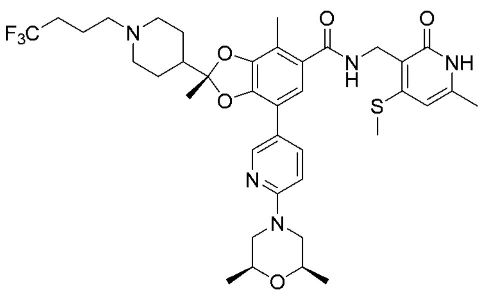

or

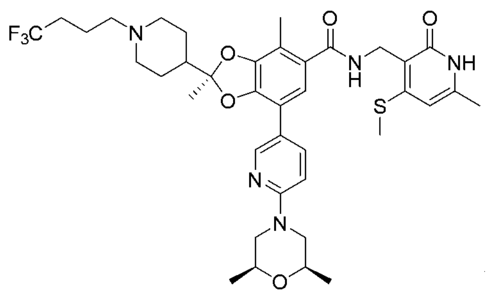

)

The same reaction as in above Preparation Example 47 was performed except that 1,1,1-trifluoro-4-iodobutane (0.17 mL) was used instead of 2-iodo-1,1-difluoroethane in [Step 6] to obtain the title compound (0.2 g).

$^1$H NMR (DMSO-$d_6$): 11.5 (s, 1H), 8.44 (s, 1H), 7.95 (t, 1H), 7.80 (d, 1H), 7.03 (s, 1H), 6.89 (d, 1H), 6.05 (s, 1H), 4.27 (d ,2H), 4.14 (d, 2H), 3.55-3.58 (m, 2H), 2.82-2.89 (m, 2H), 2.41 (s, 3H), 2.34-2.41 (m, 3H), 2.28-3.34 (m, 2H), 2.18-2.28 (m, 2H), 2.17 (s, 3H), 2.13 (s, 3H), 1.80-1.90 (m, 2H), 1.68-1.74 (m, 2H), 1.58-1.63 (m, 2H), 1.57 (s, 3H), 1.30-1.41 (m, 2H), 1.13 (s, 3H), 1.12 (s, 3H).

LC-MS (ESI, m/z) = 744.7 (M+H+)
Specific optical rotation $[\alpha]_D^{25}$ = -57.7500 (c=0.1, methanol)

**Preparation Example 58. Synthesis of stereoisomer A of 7-(6-((2S,6R)-2,6-dimethylmorpholino)pyridin-3-yl)-2,4-dimethyl-N-((6-methyl-4-(methylthio)-2-oxo-1,2-dihydropyridin-3-yl)methyl)-2-(1-(4,4,4-trifluorobutyl)piperidin-4-yl)benzo[d][1,3]dioxole-5-carboxamide (compound 58)**

Stereoisomer A of

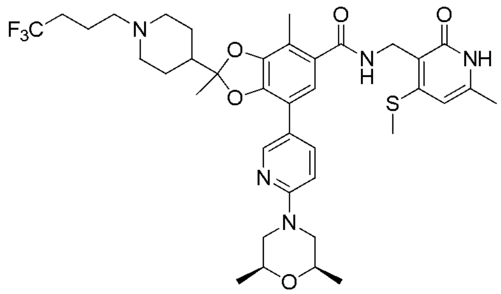

(

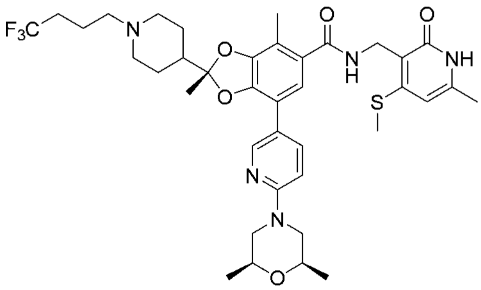

or

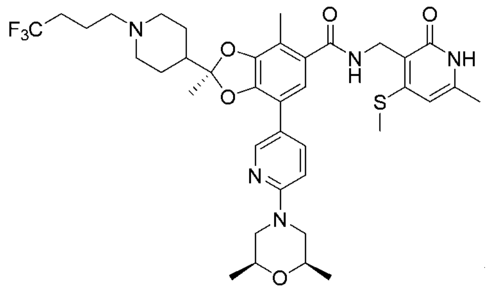

)

The same reaction as in above Preparation Example 48 was performed except that 1,1,1-trifluoro-4-iodobutane (0.17 mL) was used instead of 2-iodo-1,1-difluoroethane in [Step 6] to obtain the title compound (0.2 g).

$^1$H NMR (DMSO-$d_6$): 11.5 (s, 1H), 8.44 (s, 1H), 7.95 (t, 1H), 7.80 (d, 1H), 7.03 (s, 1H), 6.89 (d, 1H), 6.05 (s, 1H), 4.27 (d ,2H), 4.13 (d, 2H), 3.56-3.57 (m, 2H), 2.82-2.89 (m, 2H), 2.41 (s, 3H), 2.35-2.41 (m, 3H), 2.28-3.34 (m, 2H), 2.17-2.28 (m, 2H), 2.17 (s, 3H), 2.13 (s, 3H), 1.80-1.90 (m, 2H), 1.68-1.74 (m, 2H), 1.57-1.63 (m, 2H), 1.56 (s, 3H), 1.30-1.41 (m, 2H), 1.13 (s, 3H), 1.12 (s, 3H).

LC-MS (ESI, m/z) = 744.6 (M+H+)
Specific optical rotation $[\alpha]_D^{25}$ = +76.4834 (c=0.1, methanol)

**Preparation Example 59. Synthesis of stereoisomer B of 7-(6-((2S,6R)-2,6-dimethylmorpholino)pyridin-3-yl)-2,4-dimethyl-N-((6-methyl-4-(methylthio)-2-oxo-1,2-dihydropyridin-3-yl)methyl)-2-(1-(2,2,2-trifluoroacetyl)piperidin-4-yl)benzo[d][1,3]dioxole-5-carboxamide (compound 59)**

Stereoisomer B of

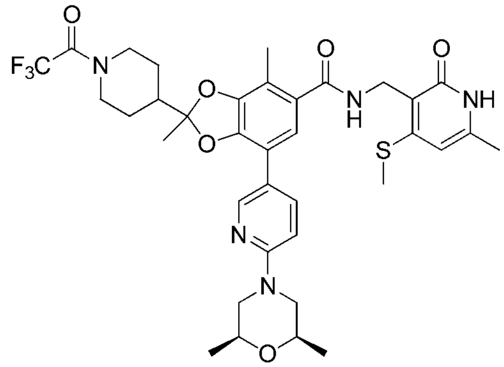

(

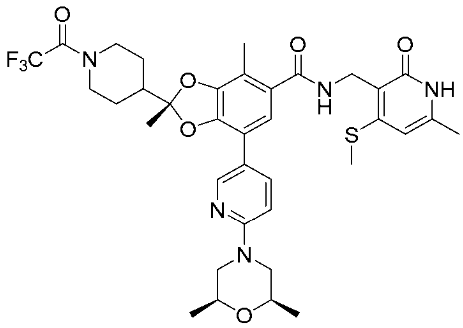

or

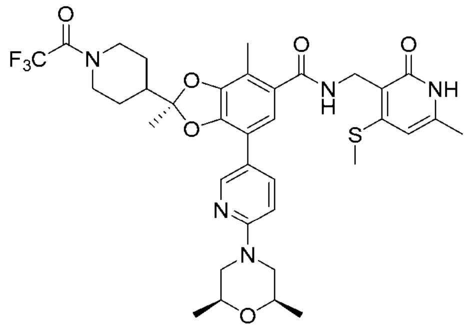

)

The same reaction as in above Preparation Example 47 was performed except that phenyl trifluoro acetate (0.15 mL) was used instead of 2-iodo-1,1-difluoroethane in [Step 6] to obtain the title compound (0.2 g).

$^{1}H$ NMR (DMSO-$d_6$): 11.5 (s, 1H), 8.44 (s, 1H), 7.95 (t, 1H), 7.80 (d, 1H), 7.04 (s, 1H), 6.88 (d, 1H), 6.05 (s, 1H), 4.36 (d, 1H), 4.27 (d, 2H), 4.13 (d, 2H), 3.88 (d, 1H), 3.55-3.57 (m, 2H), 3.21 (t, 1H), 2.83 (t, 1H), 2.41 (s, 3H), 2.32-2.38 (m, 2H), 2.21-2.30 (m, 1H), 2.17 (s, 3H), 2.13 (s, 3H), 1.88-1.95 (m, 2H). 1.57 (s, 3H), 1.27-1.34 (m, 2H) 1.13 (s, 3H). 1.12 (s, 3H).

LC-MS (ESI, m/z) = 730.5 ($M+H^+$)
Specific optical rotation $[\alpha]_D^{25}$ = -91.4000 (c=0.1, methanol)

**Preparation Example 60. Synthesis of stereoisomer A of 7-(6-((2S,6R)-2,6-dimethylmorpholino)pyridin-3-yl)-2,4-dimethyl-N-((6-methyl-4-(methylthio)-2-oxo-1,2-dihydropyridin-3-yl)methyl)-2-(1-(2,2,2-trifluoroacetyl)piperidin-4-yl)benzo[d][1,3]dioxole-5-carboxamide (compound 60)**

Stereoisomer A of

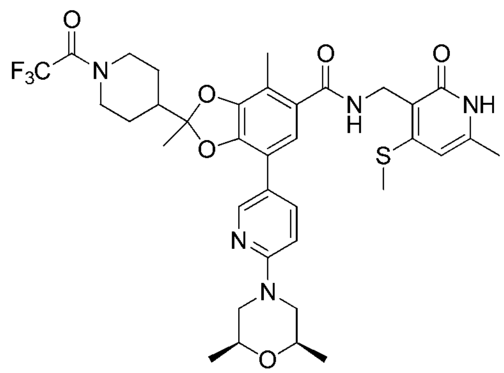

(

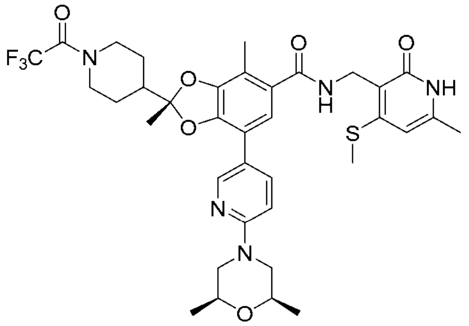

or

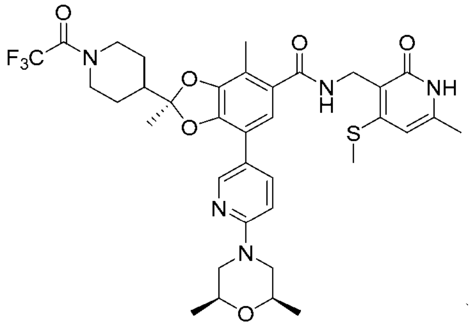

)

The same reaction as in above Preparation Example 48 was performed except that phenyl trifluoro acetate (0.15 mL) was used instead of 2-iodo-1,1-difluoroethane in [Step 6] to obtain the title compound (0.2 g).

$^1$H NMR (DMSO-$d_6$): 11.5 (s, 1H), 8.44 (s, 1H), 7.95 (t, 1H), 7.81 (d, 1H), 7.04 (s, 1H), 6.89 (d, 1H), 6.05 (s, 1H), 4.35 (d, 1H), 4.27 (d, 2H), 4.14 (d, 2H), 3.89 (d, 1H), 3.55-3.58 (m, 2H), 3.21 (t, 1H), 2.83 (t, 1H), 2.41 (s, 3H), 2.32-2.38 (m, 2H), 2.21-2.30 (m, 1H), 2.17 (s, 3H), 2.13 (s, 3H), 1.88-1.95 (m, 2H). 1.57 (s, 3H), 1.27-1.35 (m, 2H) 1.13 (s, 3H). 1.12 (s, 3H).

LC-MS (ESI, m/z) = 730.5 (M+H$^+$)

Specific optical rotation $[\alpha]_D^{25}$ = +66.5167 (c=0.1, methanol)

**Preparation Example 61. Synthesis of 2,4-dimethyl-N-((6-methyl-4-(methylthio)-2-oxo-1,2-dihydropyridin-3-yl)methyl)-7-(6-morpholinopyridin-3-yl)-2-(1-(2,2,2-trifluoro)piperidin-4-yl)benzo[d][1,3]dioxole-5-carboxamide (compound 61)**

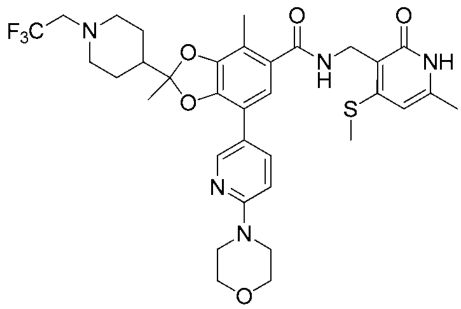

The same processes as in [Steps 1 to 5] were performed except that 4-(5-(4,4,5,5-tetramethyl-1,3,2-dioxaborolan-2-yl)pyridin-2-yl)morpholine was used in [Step 1] of above Preparation Example 33 instead of (2S,6R)-2,6-dimethyl-4-(5-(4,4,5,5-tetramethyl-1,3,2-dioxaborolan-2-yl)pyridin-2-yl)morpholine (intermediate 1), so as to obtain 2,4-dimethyl-N-((6-methyl-4-(methylthio)-2-oxo-1,2-dihydropyridin-3-yl)methyl)-7-(6-morpholinopyridin-3-yl)-2-(piperidin-4-yl)benzo[d][1,3]dioxole-5-carboxamide hydrochloride.

Acetonitrile (10 mL), sodium carbonate (0.43 g), and trifluoroethyl trifluoromethanesulfonate (0.20 mL) were added to the obtained hydrochloride (0.5 g, 0.70 mmole), and stirred at an internal temperature of 100°C for 24 hours. After cooling, dichloromethane (5 mL) was added, filtered and concentrated, and the crystal produced by using ethanol (2 mL) and hexane (20 mL) was filtered, and dried at an internal temperature of 60°C to obtain the title compound (0.3 g).

$^1$H NMR (DMSO-$d_6$): 11.5 (s, 1H), 8.44 (d, 1H), 7.95 (t, 1H), 7.82 (d, 1H), 7.04 (s, 1H), 6.89 (d, 1H), 6.04 (s, 1H), 4.26 (d, 2H), 3.62-3.64 (m, 4H), 3.43-3.45 (m, 4H), 3.02-3.12 (m, 2H), 2.91-2.98 (m, 2H), 2.41 (s, 3H), 2.20-2.28 (m, 2H), 2.26 (s, 3H), 2.22 (s, 3H), 1.81-1.88 (m, 1H), 1.68-1.72 (m, 2H), 1.56 (s, 3H), 1.35-1.40 (m, 2H).

LC-MS (ESI, m/z) = 688.4 (M+H$^+$)

**Preparation Example 62. Synthesis of stereoisomer B of 2,4-dimethyl-N-((6-methyl-4-(methylthio)-2-oxo-1,2-dihydropyridin-3-yl)methyl)-7-(6-morpholinopyridin-3-yl)-2-(1-(2,2,2-trifluoro)piperidin-4-yl)benzo[d][1,3]dioxole-5-carboxamide (compound 62)**

Stereoisomer B of

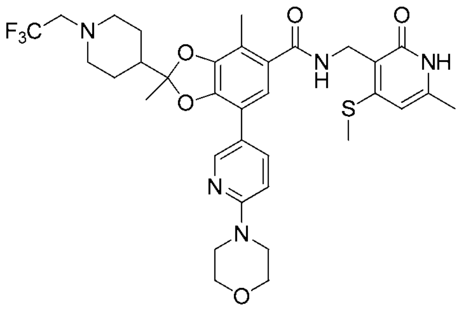

(

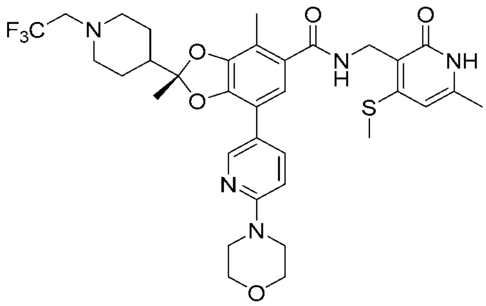

or

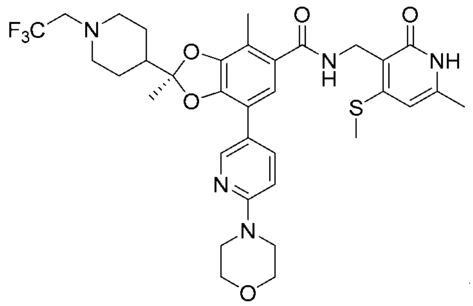

)

The same reaction as in above Preparation Example 61 was performed except that tert-butyl (R)-4-(7-bromo-5-(methoxycarbonyl)-2,4-dimethylbenzo[d][1,3]dioxole-2-yl)piperidin-1-carboxylate (3 g, 6.38 mmole), which is stereospecific, was used as a starting material in [Step 1] instead of tert-butyl 4-(7-bromo-5-(methoxycarbonyl)-2,4-dimethylbenzo[d][1,3]dioxole-2-yl)piperidin-1-carboxylate, so as to obtain the title compound (0.3 g).

$^1H$ NMR (DMSO-$d_6$): 11.5 (s, 1H), 8.45 (d, 1H), 7.95 (t, 1H), 7.81 (d, 1H), 7.03 (s, 1H), 6.89 (d, 1H), 6.04 (s, 1H), 4.26 (d, 2H), 3.65-3.66 (m, 4H), 3.44-3.45 (m, 4H), 3.06-3.12 (m, 2H), 2.90-2.98 (m, 2H), 2.40 (s, 3H), 2.20-2.28 (m, 2H), 2.26 (s, 3H), 2.22 (s, 3H), 1.81-1.88 (m, 1H), 1.68-1.72 (m, 2H), 1.56 (s, 3H), 1.35-1.40 (m, 2H).

LC-MS (ESI, m/z) = 688.5 ($M+H^+$)
Specific optical rotation $[\alpha]_D^{25}$ = -52.9667 (c=0.1, methanol)

**Preparation Example 63. Synthesis of stereoisomer A of 2,4-dimethyl-N-((6-methyl-4-(methylthio)-2-oxo-1,2-dihydropyridin-3-yl)methyl)-7-(6-morpholinopyridin-3-yl)-2-(1-(2,2,2-trifluoro)piperidin-4-yl)benzo[d][1,3]dioxole-5-carboxamide (compound 63)**

Stereoisomer A of

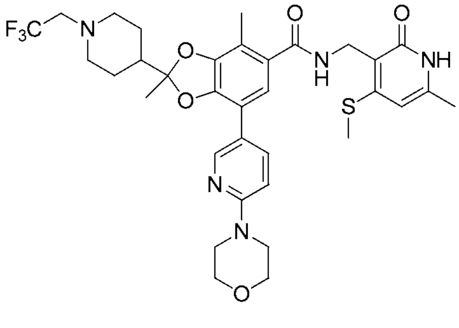

(

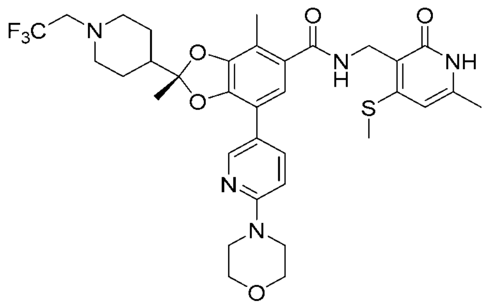

or

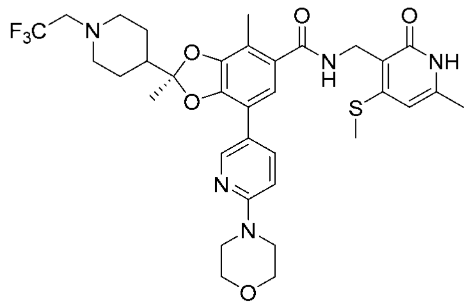

)

The same reaction as in above Preparation Example 61 was performed except that tert-butyl (S)-4-(7-bromo-5-(methoxycarbonyl)-2,4-dimethylbenzo[d][1,3]dioxole-2-yl)piperidin-1-carboxylate (3 g, 6.38 mmole), which is stereospecific, was used as a starting material in [Step 1] instead of tert-butyl 4-(7-bromo-5-(methoxycarbonyl)-2,4-dimethylbenzo[d][1,3]dioxole-2-yl)piperidin-1-carboxylate, so as to obtain the title compound (0.3 g).

$^1$H NMR (DMSO-$d_6$): 11.5 (s, 1H), 8.45 (d, 1H), 7.95 (t, 1H), 7.81 (d, 1H), 7.03 (s, 1H), 6.89 (d, 1H), 6.04 (s, 1H), 4.26 (d, 2H), 3.65-3.67 (m, 4H), 3.44-3.46 (m, 4H), 3.10-3.12 (m, 2H), 2.91-2.94 (m, 2H), 2.42 (s, 3H), 2.39-2.40 (m, 2H), 2.26 (s, 3H), 2.22 (s, 3H), 1.81-1.88 (m, 1H), 1.68-1.72 (m, 2H), 1.56 (s, 3H), 1.35-1.40 (m, 2H).

LC-MS (ESI, m/z) = 688.5 ($M+H^+$)
Specific optical rotation $[\alpha]_D^{25}$ = +73.2333 (c=0.1, methanol)

**Preparation Example 64. Synthesis of stereoisomer B of 2-(1-(2,2-difluoroethyl)piperidin-4-yl)-2,4-dimethyl-N-((6-methyl-4-(methylthio)-2-oxo-1,2-dihydropyridin-3-yl)methyl)-7-(6-morpholinopyridin-3-yl)benzo[d][1,3]dioxole-5-carboxamide (compound 64)**

Stereoisomer B of

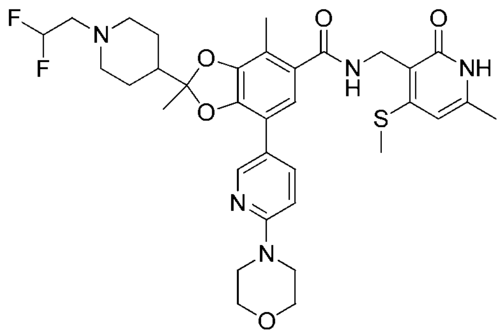

(

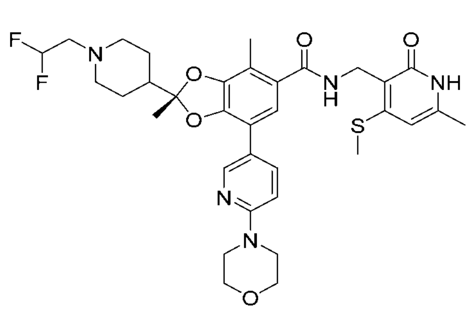

or

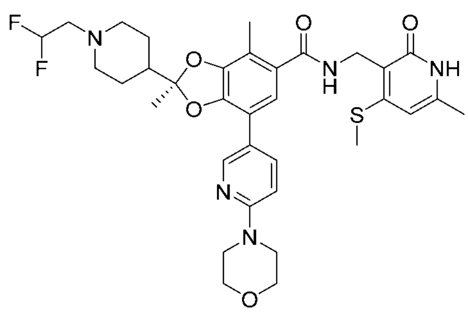

)

The same reaction as in above Preparation Example 62 was performed except that 2-iodo-1,1-difluoroethane (0.12 mL) was used instead of 2,2,2-trifluoroethyl trifluoromethanesulfonate in [Step 6] to obtain the title compound (0.2 g).

$^1H$ NMR (DMSO-$d_6$): 11.5 (s, 1H), 8.45 (d, 1H), 7.95 (t, 1H), 7.81 (d, 1H), 7.03 (s, 1H), 6.89 (d, 1H), 6.04-6.05 (m, 2H), 4.26 (d, 2H), 3.57-3.66 (m, 4H), 3.45-3.46 (m, 4H), 2.88-2.89 (m, 2H), 2.64-2.65 (m, 2H), 2.40 (s, 3H), 2.17 (s, 3H), 2.13 (s, 3H), 2.06-2.09 (m, 2H), 1.82-1.84 (m, 1H), 1.60-1.62 (m, 2H), 1.57 (s, 3H), 1.32-1.40 (m, 2H).

LC-MS (ESI, m/z) = 670.5 (M+H$^+$)
Specific optical rotation $[\alpha]_D^{25}$ = -61.8334 (c=0.1, methanol)

**Preparation Example 65. Synthesis of stereoisomer A of 2-(1-(2,2-difluoroethyl)piperidin-4-yl)-2,4-dimethyl-N-((6-methyl-4-(methylthio)-2-oxo-1,2-dihydropyridin-3-yl)methyl)-7-(6-morpholinopyridin-3-yl)benzo[d][1,3]dioxole-5-carboxamide (compound 65)**

Stereoisomer A of

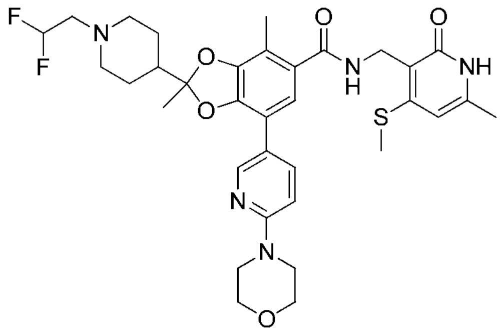

(

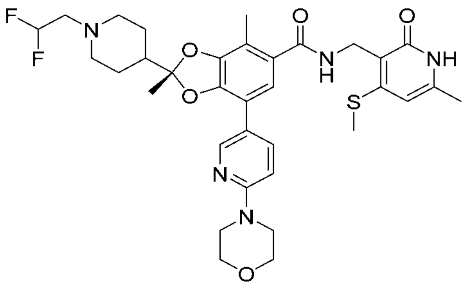

or

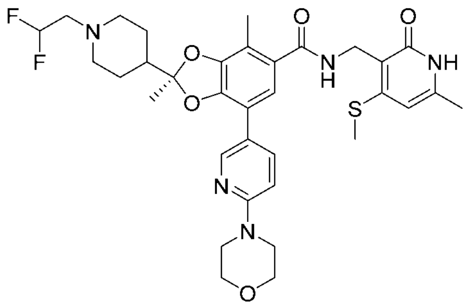

)

The same reaction as in above Preparation Example 63 was performed except that 2-iodo-1,1-difluoroethane (0.12 mL) was used instead of 2,2,2-trifluoroethyl trifluoromethanesulfonate in [Step 6] to obtain the title compound (0.2 g).

$^1H$ NMR (DMSO-$d_6$): 11.5 (s, 1H), 8.46 (d, 1H), 7.95 (t, 1H), 7.81 (d, 1H), 7.03 (s, 1H), 6.89 (d, 1H), 6.04-6.05 (m, 2H), 4.26 (d, 2H), 3.66-3.68 (m, 4H), 3.44-3.46 (m, 4H), 2.89-2.92 (m, 2H), 2.61-2.65 (m, 2H), 2.40 (s, 3H), 2.17 (s, 3H), 2.13 (s, 3H), 2.05-2.09 (m, 2H), 1.80-1.85 (m, 1H), 1.68-1.70 (m, 2H), 1.57 (s, 3H), 1.30-1.38 (m, 2H).

LC-MS (ESI, m/z) = 670.5 (M+H$^+$)
Specific optical rotation $[\alpha]_D^{25}$ = +57.7334 (c=0.1, methanol)

**Preparation Example 66. Synthesis of stereoisomer B of 2-(1-ethylpiperidin-4-yl)-2,4-dimethyl-N-((6-methyl-4-(methylthio)-2-oxo-1,2-dihydropyridin-3-yl)methyl)-7-(6-morpholinopyridin-3-yl)benzo[d][1,3]dioxole-5-carboxamide (compound 66)**

Stereoisomer B of

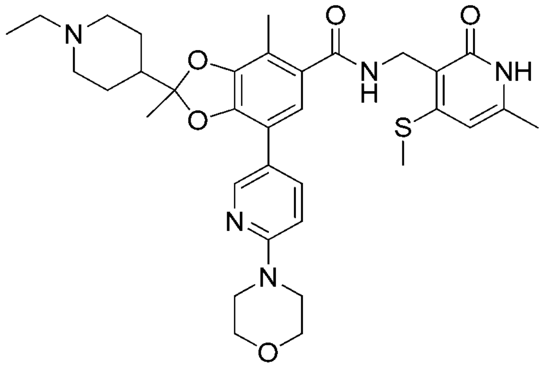

(

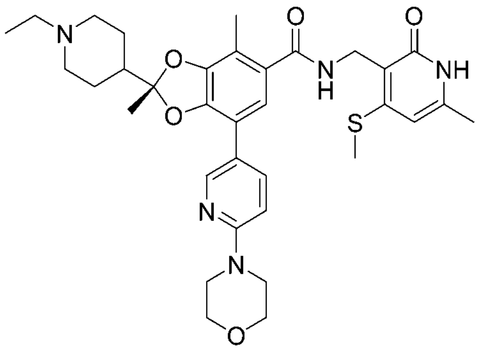

or

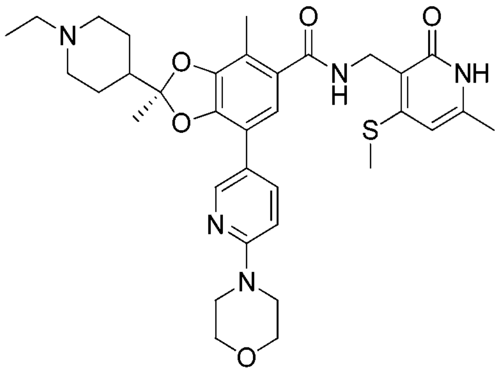

)

The same reaction as in above Preparation Example 62 was performed except that iodoethane (0.11 mL) was used instead of 2,2,2-trifluoroethyl trifluoromethanesulfonate in [Step 6] to obtain the title compound (0.1 g).

$^1H$ NMR (DMSO-$d_6$): 11.5 (s, 1H), 8.45 (s, 1H), 7.95 (t, 1H), 7.82 (d, 1H), 7.03 (s, 1H), 6.89 (d, 1H), 6.04 (s, 1H), 4.26 (d, 2H), 3.66-3.68 (m, 4H), 3.44-3.46 (m, 4H), 2.88-2.89 (m, 2H), 2.40 (s, 3H), 2.22-2.28 (m, 2H), 2.17(s, 3H), 2.13 (s, 3H), 1.74-1.90 (m, 3H), 1.69-1.71 (m, 2H), 1.56 (s, 3H), 1.32-1.37 (m, 2H), 0.93 (t, 3H).

LC-MS (ESI, m/z) = 634.6 (M+H$^+$)

**Preparation Example 67. Synthesis of stereoisomer A of 2-(1-ethylpiperidin-4-yl)-2,4-dimethyl-N-((6-methyl-4-(methylthio)-2-oxo-1,2-dihydropyridin-3-yl)methyl)-7-(6-morpholinopyridin-3-yl)benzo[d][1,3]dioxole-5-carboxamide (compound 67)**

Stereoisomer A of

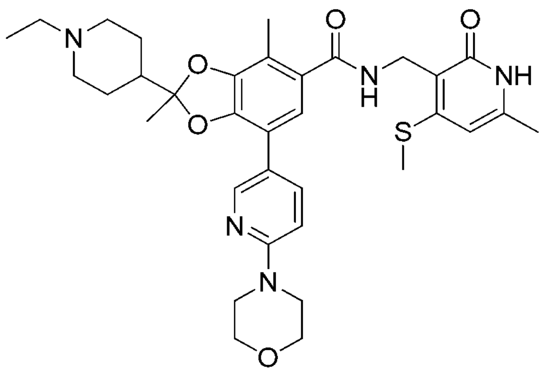

(

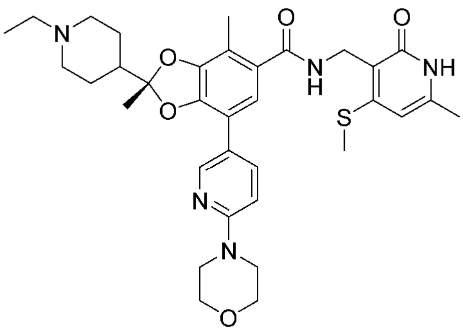

or

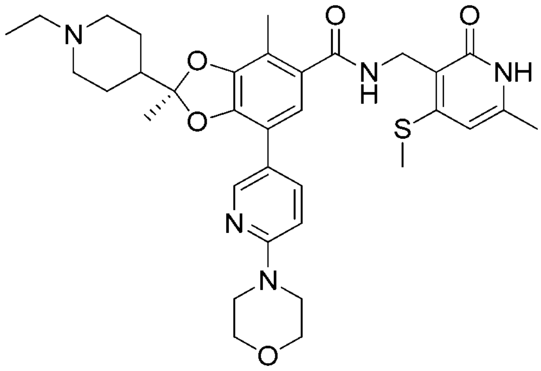

)

The same reaction as in above Preparation Example 63 was performed except that iodoethane (0.11 mL) was used instead of 2,2,2-trifluoroethyl trifluoromethanesulfonate in [Step 6] to obtain the title compound (0.1 g).

$^1$H NMR (DMSO-$d_6$): 11.5 (s, 1H), 8.46 (s, 1H), 7.95 (t, 1H), 7.82 (d, 1H), 7.03 (s, 1H), 6.89 (d, 1H), 6.05 (s, 1H) 4.26 (d, 2H), 3.66-3.67 (m, 4H), 3.45-3.46 (m, 4H), 2.95-2.98 (m, 2H), 2.44 (s, 3H), 2.32-2.40 (m, 2H), 2.17(s, 3H), 2.13 (s, 3H), 1.83-1.89 (m, 3H), 1.71-1.79 (m, 2H), 1.57 (s, 3H), 1.37-1.41 (m, 2H), 0.97 (t, 3H).

LC-MS (ESI, m/z) = 634.6 (M+H$^+$)

**<u>Preparation Example 68. Synthesis of 2,4-dimethyl-N-((6-methyl-4-(methylthio)-2-oxo-1,2-dihydropyridin-3-yl)methyl)-7-(2-morpholinopyrimidin-5-yl)-2-(1-(2,2,2-trifluoro)piperidin-4-yl)benzo[d][1,3]dioxole-5-carboxamide (compound 68)</u>**

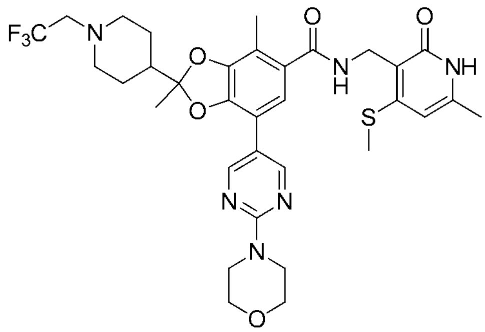

The same processes as in [Steps 1 to 5] were performed except that 4-(5-(4,4,5,5-tetramethyl-1,3,2-dioxaborolan-2-yl)pyrimidin-2-yl)morpholine was used in [Step 1] of above Preparation Example 33 instead of (2S,6R)-2,6-dimethyl-4-(5-(4,4,5,5-tetramethyl-1,3,2-dioxaborolan-2-yl)pyridin-2-yl)morpholine (intermediate 1), so as to obtain 2,4-dimethyl-N-((6-methyl-4-(methylthio)-2-oxo-1,2-dihydropyridin-3-yl)methyl)-7-(2-morpholinopyridin-5-yl)-2-(piperidin-4-yl)benzo[d][1,3]dioxole-5-carboxamide hydrochloride of [Step 5].

Acetonitrile (10 mL), sodium carbonate (0.43 g), and trifluoroethyl trifluoromethanesulfonate (0.20 mL) were added to the obtained hydrochloride (0.5 g, 0.70 mmole), and stirred at an internal temperature of 100°C for 24 hours. After cooling, dichloromethane (5 mL) was added, filtered and concentrated, and the crystal produced by using ethanol (2 mL) and hexane (20 mL) was filtered, and dried at an internal temperature of 60°C to obtain the title compound (0.3 g).

$^1$H NMR (DMSO-$d_6$): 11.5 (s, 1H), 8.66 (s, 2H), 7.90 (t, 1H), 7.06 (s, 1H), 6.04 (s, 1H), 4.26 (d, 2H), 3.65-3.69 (m, 4H), 3.62-3.64 (m, 4H), 3.07-3.10 (m, 2H), 2.90-2.92 (m, 2H), 2.40 (s, 3H), 2.22-2.28 (m, 2H), 2.19 (s, 3H), 2.12 (s, 3H), 1.81-1.87 (m, 1H), 1.68-1.72 (m, 2H), 1.56 (s, 3H), 1.35-1.38 (m, 2H).

LC-MS (ESI, m/z) = 689.5 (M+H$^+$)

**<u>Preparation Example 69. Synthesis of stereoisomer B of 2,4-dimethyl-N-((6-methyl-4-(methylthio)-2-oxo-1,2-dihydropyridin-3-yl)methyl)-7-(2-morpholinopyrimidine-5-yl)-2-(1-(2,2,2-trifluoro)piperidin-4-yl)benzo [d][1,3] dioxole-5-carboxamide (compound 69)</u>**

Stereoisomer B of

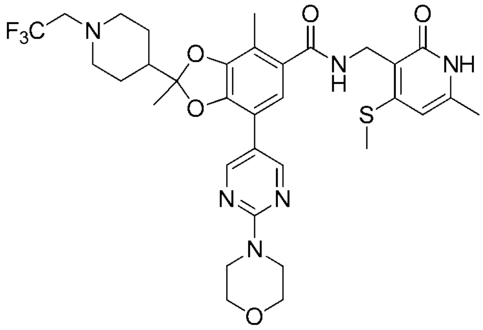

(

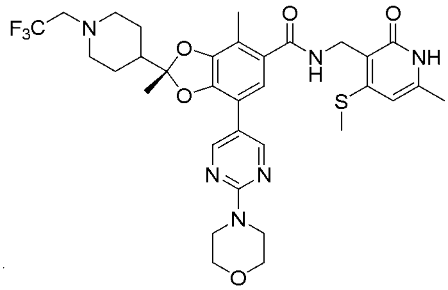

or

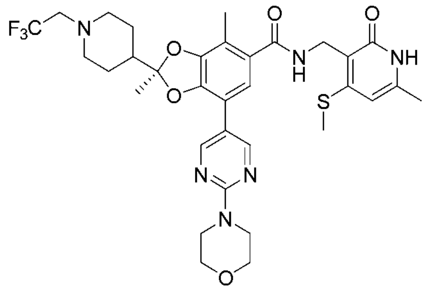

)

The same reaction as in above Preparation Example 68 was performed except that tert-butyl (R)-4-(7-bromo-5-(methoxycarbonyl)-2,4-dimethylbenzo[d][1,3]dioxole-2-yl)piperidin-1-carboxylate (3 g, 6.38 mmole), which is stereospecific, was used as a starting material in [Step 1] instead of tert-butyl 4-(7-bromo-5-(methoxycarbonyl)-2,4-dimethylbenzo[d][1,3]dioxole-2-yl)piperidin-1-carboxylate, so as to obtain the title compound (0.3 g).

$^1$H NMR (DMSO-$d_6$): 11.5 (s, 1H), 8.67 (s, 2H), 7.90 (t, 1H), 7.07 (s, 1H), 6.04 (s, 1H), 4.26 (d, 2H), 3.68-3.70 (m, 4H), 3.63-3.64 (m, 4H), 3.08-3.10 (m, 2H), 2.91-2.92 (m, 2H), 2.40 (s, 3H), 2.22-2.28 (m, 2H), 2.19 (s, 3H), 2.12 (s, 3H), 1.81-1.88 (m, 1H), 1.68-1.71 (m, 2H), 1.56 (s, 3H), 1.35-1.38 (m, 2H).

LC-MS (ESI, m/z) = 689.5 (M+H$^+$)
Specific optical rotation $[\alpha]_D^{25}$ = -50.8167 (c=0.1, methanol)

**<u>Preparation Example 70. Synthesis of stereoisomer A of 2,4-dimethyl-N-((6-methyl-4-(methylthio)-2-oxo-1,2-dihydropyridin-3-yl)methyl)-7-(2-morpholinopyrimidine-5-yl)-2-(1-(2,2,2-trifluoro)piperidin-4-yl)benzo[d][1,3]dioxole-5-carboxamide (compound 70)</u>**

Stereoisomer A of

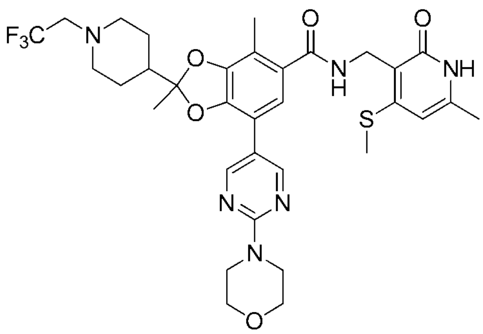

(

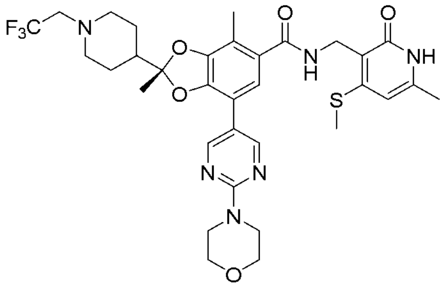

or

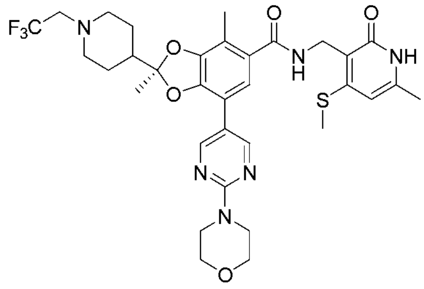

)

The same reaction as in above Preparation Example 68 was performed except that tert-butyl (S)-4-(7-bromo-5-(methoxycarbonyl)-2,4-dimethylbenzo[d][1,3]dioxole-2-yl)piperidin-1-carboxylate (3 g, 6.38 mmole), which is stereospecific, was used as a starting material in [Step 1] instead of tert-butyl 4-(7-bromo-5-(methoxycarbonyl)-2,4-dimethylbenzo[d][1,3]dioxole-2-yl)piperidin-1-carboxylate, so as to obtain the title compound (0.3 g).

$^1$H NMR (DMSO-$d_6$): 11.5 (s, 1H), 8.67 (s, 2H), 7.90 (t, 1H), 7.06 (s, 1H), 6.04 (s, 1H), 4.26 (d, 2H), 3.68-3.70 (m, 4H), 3.63-3.64 (m, 4H), 3.08-3.12 (m, 2H), 2.92-2.98 (m, 2H), 2.40 (s, 3H), 2.20-2.30 (m, 2H), 2.16 (s, 3H), 2.12 (s, 3H), 1.80-1.86 (m, 1H), 1.64-1.72 (m, 2H), 1.56 (s, 3H), 1.34-1.36 (m, 2H).

LC-MS (ESI, m/z) = 689.5 ($M+H^+$)
Specific optical rotation $[\alpha]_D^{25}$ = +62.5000 (c=0.1, methanol)

**Preparation Example 71. Synthesis of stereoisomer B of 2-(1-(2,2-difluoroethyl)piperidin-4-yl)-2,4-dimethyl-N-((6-methyl-4-(methylthio)-2-oxo-1,2-dihydropyridin-3-yl)methyl)-7-(2-morpholinopyrimidin-5-yl)benzo[d][1,3]dioxole-5-carboxamide (compound 71)**

Stereoisomer B of

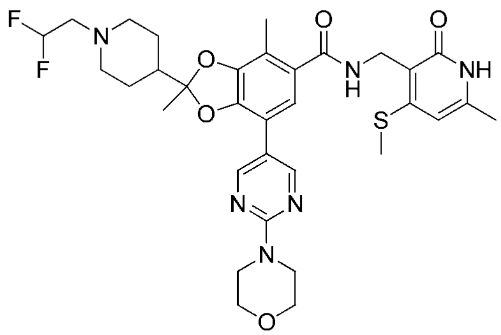

(

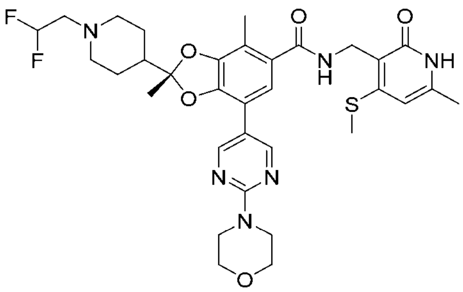

or

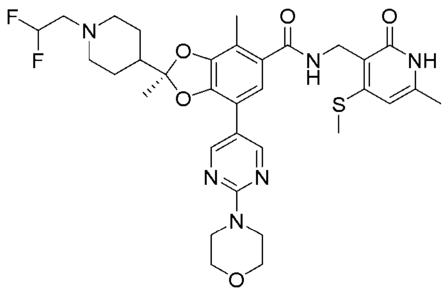

)

The same reaction as in above Preparation Example 69 was performed except that 2-iodo-1,1-difluoroethane (0.12 mL) was used instead of 2,2,2-trifluoroethyl trifluoromethanesulfonate in [Step 6] to obtain the title compound (0.2 g).

$^1$H NMR (DMSO-$d_6$): 11.5 (s, 1H), 8.67 (s, 2H), 7.91 (t, 1H), 7.06 (s, 1H), 6.04-6.05 (m, 2H), 4.27 (d, 2H), 3.68-3.70 (m, 4H), 3.63-3.64 (m, 4H), 2.89-2.91 (m, 2H), 2.62-2.65 (m, 2H), 2.40 (s, 3H), 2.17 (s, 3H), 2.13 (s, 3H), 2.07-2.09 (m, 2H),

1.81-1.86 (m, 1H), 1.68-1.73 (m, 2H), 1.57 (s, 3H), 1.32-1.40 (m, 2H).

LC-MS (ESI, m/z) = 671.5 ($M+H^+$)
Specific optical rotation $[\alpha]_D^{25}$ = -66.4167 (c=0.1, methanol)

**Preparation Example 72. Synthesis of stereoisomer A of 2-(1-(2,2-difluoroethyl)piperidin-4-yl)-2,4-dimethyl-N-((6-methyl-4-(methylthio)-2-oxo-1,2-dihydropyridin-3-yl)methyl)-7-(2-morpholinopyrimidin-5-yl)benzo[d][1,3]dioxole-5-carboxamide (compound 72)**

Stereoisomer A of

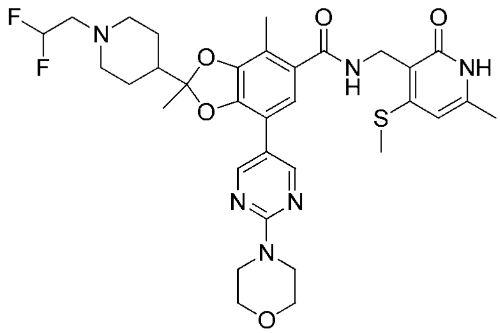

(

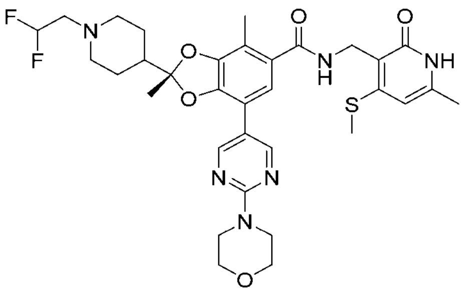

or

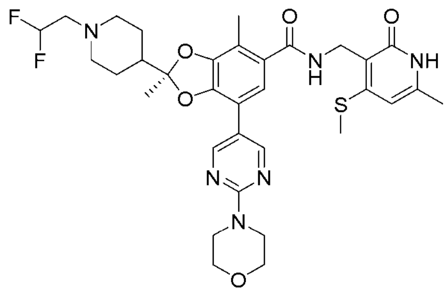

)

The same reaction as in above Preparation Example 70 was performed except that 2-iodo-1,1-difluoroethane (0.12 mL) was used instead of 2,2,2-trifluoroethyl trifluoromethanesulfonate in [Step 6] to obtain the title compound (0.2 g).

$^1H$ NMR (DMSO-$d_6$): 11.5 (s, 1H), 8.67 (s, 2H), 7.91 (t, 1H), 7.06 (s, 1H), 6.05-6.06 (m, 2H), 4.27 (d, 2H), 3.68-3.70 (m, 4H), 3.63-3.64 (m, 4H), 2.87-2.90 (m, 2H), 2.62-2.65 (m, 1H), 2.40 (s, 3H), 2.17 (s, 3H), 2.13 (s, 3H), 2.07-2.09 (m, 1H), 1.68-1.73 (m, 2H), 1.57 (s, 3H), 1.32-1.40 (m, 2H).

LC-MS (ESI, m/z) = 671.5 ($M+H^+$)
Specific optical rotation $[\alpha]_D^{25}$ = +55.1000 (c=0.1, methanol)

**Preparation Example 73. Synthesis of stereoisomer B of 2-(1-ethylpiperidin-4-yl)-2,4-dimethyl-N-((6-methyl-4-(methylthio)-2-oxo-1,2-dihydropyridin-3-yl)methyl)-7-(2-morpholinopyrimidin-5-yl)benzo[d][1,3]dioxole-5-carboxamide (compound 73)**

Stereoisomer B of

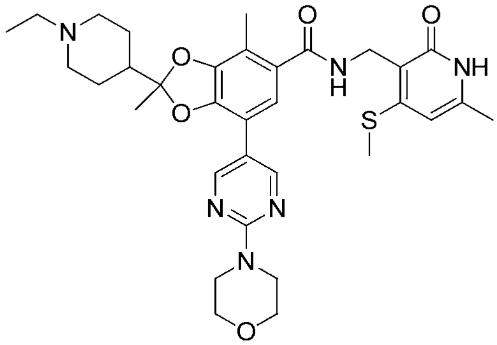

(

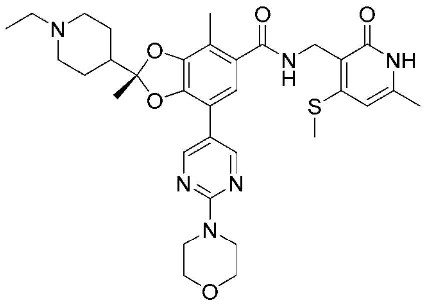

or

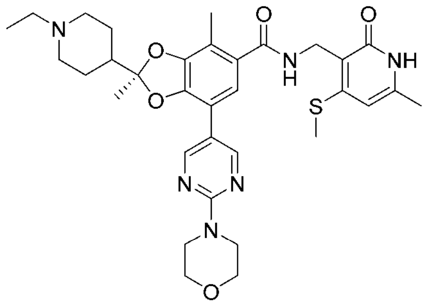

)

The same reaction as in above Preparation Example 69 was performed except that iodoethane (0.11 mL) was used instead of 2,2,2-trifluoroethyl trifluoromethanesulfonate in [Step 6] to obtain the title compound (0.1 g).

$^1$H NMR (DMSO-$d_6$): 11.5 (s, 1H), 8.70 (s, 2H), 7.92 (t, 1H), 7.10 (s, 1H), 6.05 (s, 1H), 4,27 (d, 2H), 3.70-3.72 (m, 4H), 3.63-3.65 (m, 4H), 3.40-3.51 (m, 2H), 3.25-3.27 (m, 2H), 3.01-3.05 (m, 2H), 2.82-2.88 (m, 2H), 2.41 (s, 3H), 2.20 (s, 3H), 2.13 (s, 3H), 1.92-2.00 (m, 2H), 1.61 (s, 3H), 1.54-1.57 (m, 1H), 1.14-1.16 (t, 3H).

LC-MS (ESI, m/z) = 635.6 (M+H$^+$)
Specific optical rotation $[\alpha]_D^{25}$ = -48.7834 (c=0.1, methanol)

**Preparation Example 74. Synthesis of stereoisomer A of 2-(1-ethylpiperidin-4-yl)-2,4-dimethyl-N-((6-methyl-4-(methylthio)-2-oxo-1,2-dihydropyridin-3-yl)methyl)-7-(2-morpholinopyrimidin-5-yl)benzo[d][1,3]dioxole-5-carboxamide (compound 74)**

Stereoisomer A of

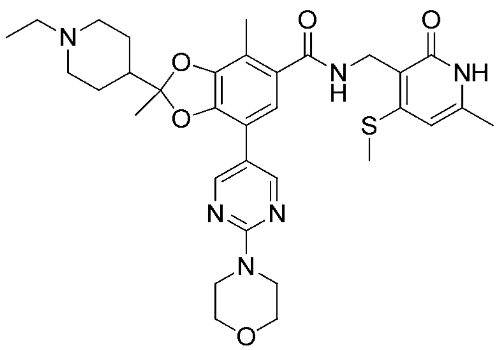

(

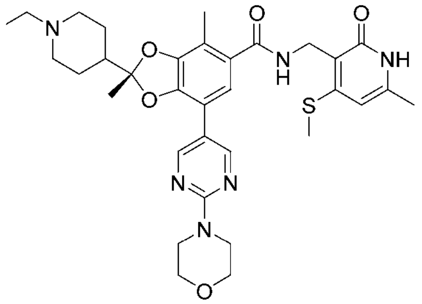

or

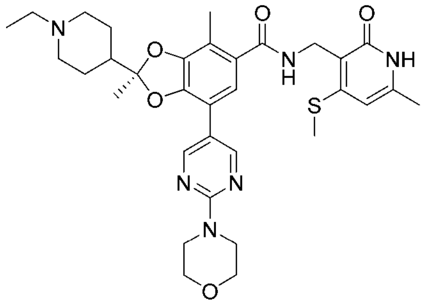

)

The same reaction as in above Preparation Example 70 was performed except that iodoethane (0.11 mL) was used instead of 2,2,2-trifluoroethyl trifluoromethanesulfonate in [Step 6] to obtain the title compound (0.1 g).

$^1$H NMR (DMSO-$d_6$): 11.5 (s, 1H), 8.71 (s, 2H), 7.92 (t, 1H), 7.10 (s, 1H), 6.06 (s, 1H), 4,27 (d, 2H), 3.70-3.72 (m, 4H), 3.63-3.65 (m, 4H), 3.40-3.51 (m, 2H), 3.25-3.29 (m, 2H), 3.01-3.05 (m, 2H), 2.82-2.88 (m, 2H), 2.41 (s, 3H), 2.19 (s, 3H), 2.13 (s, 3H), 1.90-1.98 (m, 2H), 1.60 (s, 3H), 1.53-1.59 (m, 1H), 1.15-1.20 (t, 3H).

LC-MS (ESI, m/z) = 635.6 (M+H$^+$)
Specific optical rotation $[\alpha]_D^{25}$ = +56.6834 (c=0.1, methanol)

**Preparation Example 75. Synthesis of 7-(2-(4-ethylpiperazin-1-yl)pyrimidin-5-yl)-2,4-dimethyl-N-((6-methyl-4-(methylthio)-2-oxo-1,2-dihydropyridin-3-yl)methyl)-2-(1-(2,2,2-trifluoroethyl)piperidin-4-yl)benzo[d][1,3]dioxole-5-carboxamide (compound 75)**

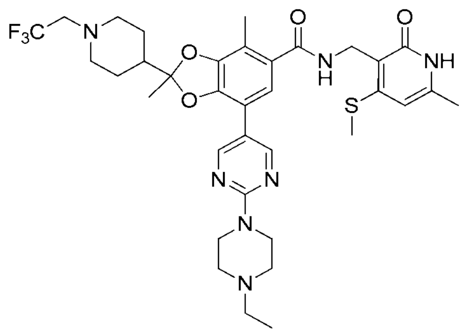

The same processes as in [Steps 1 to 5] were performed except that 2-(4-ethylpiperazin-1-yl)-5-(4,4,5,5-tetramethyl-1,3,2-dioxaborolan-2-yl)pyrimidine was used in [Step 1] of above Preparation Example 33 instead of (2S,6R)-2,6-dimethyl-4-(5-(4,4,5,5-tetramethyl-1,3,2-dioxaborolan-2-yl)pyridin-2-yl)morpholine (intermediate 1), so as to obtain 7-(2-(4-ethylpiperazin-1-yl)pyrimidin-5-yl)-2,4-dimethyl-N-((6-methyl-4-(methylthio)-2-oxo-1,2-dihydropyridin-3-yl)methyl)-2-(pyridin-4-yl)benzo[d][1,3]dioxole-5-carboxamide hydrochloride of [Step 5].

Acetonitrile (10 mL), sodium carbonate (0.43 g), and trifluoroethyl trifluoromethanesulfonate (0.20 mL) were added to the obtained hydrochloride (0.5 g, 0.70 mmole), and stirred at an internal temperature of 100°C for 24 hours. After cooling, dichloromethane (5 mL) was added, filtered and concentrated, and the crystal produced by using ethanol (2 mL) and hexane (20 mL) was filtered, and dried at an internal temperature of 60°C to obtain the title compound (0.2 g).

$^1$H NMR (DMSO-$d_6$): 11.5 (s, 1H), 8.65 (s, 2H), 7.91 (t, 1H), 7.05 (s, 1H), 6.05 (s, 1H), 4.27 (d, 2H), 3.66-3.70 (m, 4H), 3.06-3.12 (m, 2H), 2.91-2.93 (m, 2H), 2.40 (s, 3H), 2.36-2.40 (m, 4H), 2.30-2.34 (m, 2H), 2.22-2.28 (m, 2H), 2.17 (s, 3H), 2.13 (s, 3H), 1.82-1.87 (m, 1H), 1.68-1.70 (m, 2H), 1.56 (s, 3H), 1.34-1.38 (m, 2H), 1.00 (t, 3H).

LC-MS (ESI, m/z) = 716.4 (M+H$^+$)

**Preparation Example 76. Synthesis of stereoisomer B of 7-(2-(4-ethylpiperazin-1-yl)pyrimidin-5-yl)-2,4-dimethyl-N-((6-methyl-4-(methylthio)-2-oxo-1,2-dihydropyridin-3-yl)methyl)-2-(1-(2,2,2-trifluoroethyl)piperidin-4-yl)benzo[d][1,3]dioxole-5-carboxamide (compound 76)**

Stereoisomer B of

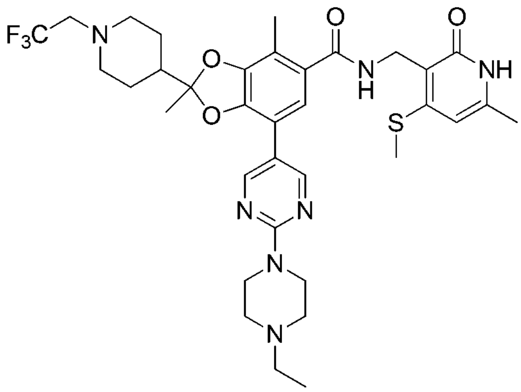

(

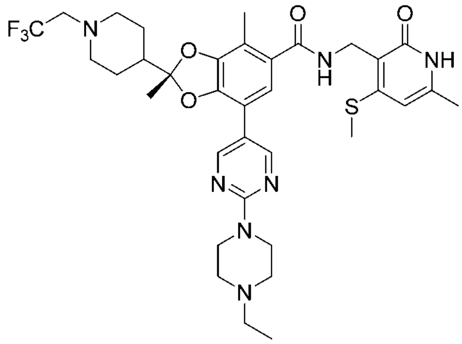

or

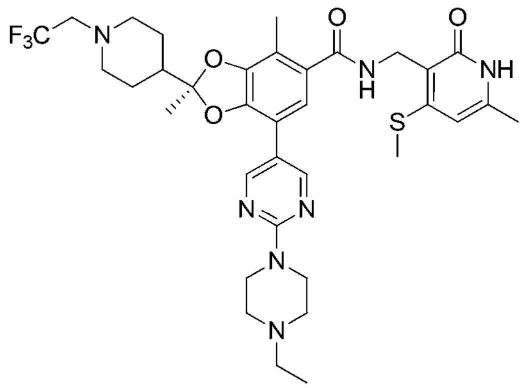

)

The same reaction as in above Preparation Example 75 was performed except that tert-butyl (R)-4-(7-bromo-5-(methoxycarbonyl)-2,4-dimethylbenzo[d][1,3]dioxole-2-yl)piperidin-1-carboxylate (3 g, 6.38 mmole), which is stereospecific, was used as a starting material in [Step 1] instead of tert-butyl 4-(7-bromo-5-(methoxycarbonyl)-2,4-dimethylbenzo[d][1,3]dioxole-2-yl)piperidine-1-carboxylate, so as to obtain the title compound (0.2 g).

$^1H$ NMR (DMSO-$d_6$): 11.5 (s, 1H), 8.64 (s, 2H), 7.92 (s, 1H), 7.05 (s, 1H), 6.05 (s, 1H), 4.27 (d, 2H), 3.75-3.70 (m, 4H), 3.06-3.10 (m, 2H), 2.91-2.93 (m, 2H), 2.41 (s, 3H), 2.36-2.40 (m, 4H), 2.30-2.34 (m, 2H), 2.21-2.28 (m, 2H), 2.17 (s, 3H), 2.13 (s, 3H), 1.82-1.87 (m, 1H), 1.68-1.70 (m, 2H), 1.56 (s, 3H), 1.35-1.38 (m, 2H), 1.00 (t, 3H).

LC-MS (ESI, m/z) = 716.6 (M+H$^+$)

## Preparation Example 77. Synthesis of stereoisomer A of 7-(2-(4-ethylpiperazin-1-yl)pyrimidin-5-yl)-2,4-dimethyl-N-((6-methyl-4-(methylthio)-2-oxo-1,2-dihydropyridin-3-yl)methyl)-2-(1-(2,2,2-trifluoroethyl)piperidin-4-yl)benzo[d][1,3]dioxole-5-carboxamide (compound 77)

Stereoisomer A of

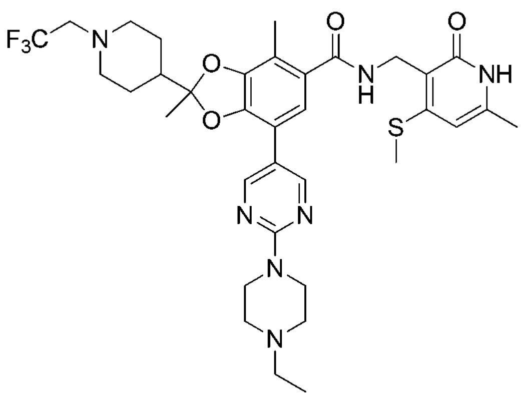

(

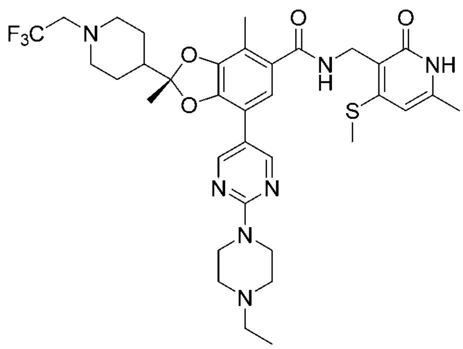

or

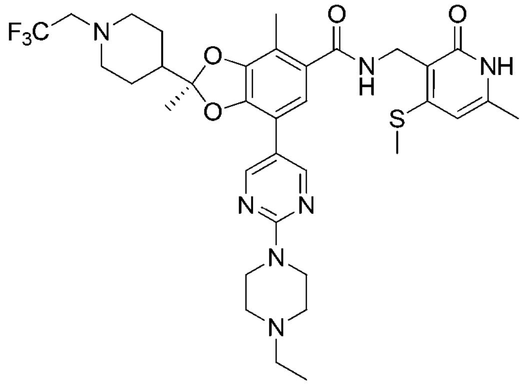

)

The same reaction as in above Preparation Example 75 was performed except that tert-butyl (S)-4-(7-bromo-5-(methoxycarbonyl)-2,4-dimethylbenzo[d][1,3]dioxole-2-yl)piperidin-1-carboxylate (3 g, 6.38 mmole), which is stereospecific, was used as a starting material in [Step 1] instead of tert-butyl 4-(7-bromo-5-(methoxycarbonyl)-2,4-dimethylbenzo[d][1,3]dioxole-2-yl)piperidin-1-carboxylate, so as to obtain the title compound (0.2 g).

$^1$H NMR (DMSO-$d_6$): 11.5 (s, 1H), 8.64 (s, 2H), 7.92 (s, 1H), 7.06 (s, 1H), 6.05 (s, 1H), 4.27 (d, 2H), 3.73-3.74 (m, 4H), 3.06-3.12 (m, 2H), 2.91-2.93 (m, 2H), 2.41 (s, 3H), 2.34-2.43 (m, 4H), 2.26-2.34 (m, 2H), 2.20-2.26 (m, 2H), 2.17 (s, 3H), 2.13 (s, 3H), 1.82-1.87 (m, 1H), 1.68-1.70 (m, 2H), 1.56 (s, 3H), 1.35-1.39 (m, 2H), 1.01 (t, 3H).

LC-MS (ESI, m/z) = 716.6 (M+H$^+$)

**Preparation Example 78. Synthesis of 7-(2-(ethyl(methyl)amino)pyridin-5-yl)-2,4-dimethyl-N-((6-methyl-4-(methylthio)-2-oxo-1,2-dihydropyridin-3-yl)methyl)-2-(1-(2,2,2-trifluoroethyl)piperidin-4-yl)benzo[d][1,3]dioxole-5-carboxamide (compound 78)**

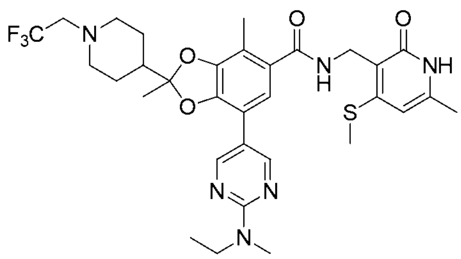

The same processes as in [Steps 1 to 5] were performed except that N-ethyl-N-methyl-5-(4,4,5,5-tetramethyl-1,3,2-dioxaborolan-2-yl)pyrimidin-2-amine was used in [Step 1] of above Preparation Example 33 instead of (2S,6R)-2,6-dimethyl-4-(5-(4,4,5,5-tetramethyl-1,3,2-dioxaborolan-2-yl)pyridin-2-yl)morpholine (intermediate 1), so as to obtain 7-(2-(ethyl(methyl)amino)pyrimidin-5-yl)-2,4-dimethyl-N-((6-methyl-4-(methylthio)-2-oxo-1,2-dihydropyridin-3-yl)methyl)-2-(piperidin-4-yl)benzo[d][1,3]dioxole-5-carboxamide hydrochloride of [Step 5].

Acetonitrile (10 mL), sodium carbonate (0.43 g), and trifluoroethyl trifluoromethanesulfonate (0.20 mL) were added to the obtained hydrochloride (0.5 g, 0.73 mmole), and stirred at an internal temperature of 100°C for 24 hours. After cooling, dichloromethane (5 mL) was added, filtered and concentrated, and the crystal produced by using ethanol (2 mL) and hexane (20 mL) was filtered, and dried at an internal temperature of 60°C to obtain the title compound (0.3 g).

$^1$H NMR (DMSO-$d_6$): 11.5(s, 1H), 8.62(s, 2H), 7.90 (d, 1H), 7.05 (s, 1H), 6.05 (s, 1H), 4.26 (d, 2H), 3.62-3.63 (m, 2H), 3.06-3.10 (m, 5H), 2.91-2.92 (m, 2H), 2.40 (s, 3H), 2.24-2.28 (m, 2H), 2.17 (s, 3H), 2.13 (s, 3H), 1.83-1.88 (m, 1H), 1.69-1.72 (m, 2H), 1.56 (s, 3H), 1.36-1.38 (m, 2H), 1.07 (t, 3H).

LC-MS (ESI, m/z) = 661.5 (M+H$^+$)

**Preparation Example 79. Synthesis of stereoisomer B of 7-(2-(ethyl(methyl)amino)pyridin-5-yl)-2,4-dimethyl-N-((6-methyl-4-(methylthio)-2-oxo-1,2-dihydropyridin-3-yl)methyl)-2-(1-(2,2,2-trifluoroethyl)piperidin-4-yl)benzo[d][1,3]dioxole-5-carboxamide (compound 79)**

Stereoisomer B of

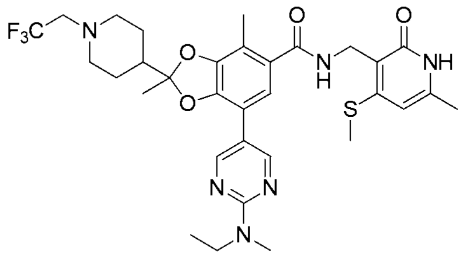

(

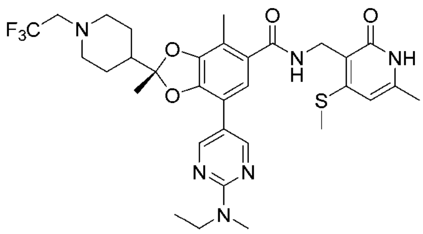

or

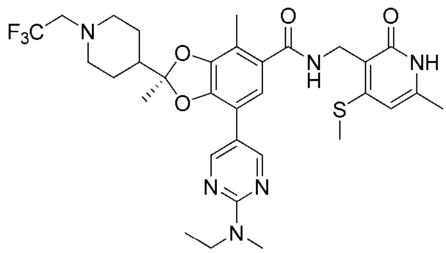

)

The same reaction as in above Preparation Example 78 was performed except that tert-butyl (R)-4-(7-bromo-5-(methoxycarbonyl)-2,4-dimethylbenzo[d][1,3]dioxole-2-yl)piperidin-1-carboxylate (3 g, 6.38 mmole), which is stereospecific, was used as a starting material in [Step 1] instead of tert-butyl 4-(7-bromo-5-(methoxycarbonyl)-2,4-dimethylbenzo[d][1,3]dioxole-2-yl)piperidin-1-carboxylate, so as to obtain the title compound (0.3 g).

$^1H$ NMR (DMSO-$d_6$): 11.5(s, 1H), 8.62(s, 2H), 7.90 (t, 1H), 7.05 (s, 1H), 6.05 (s, 1H), 4.26 (d, 2H), 3.62-3.65 (m, 2H), 3.06-3.10 (m, 5H), 2.91-2.92 (m, 2H), 2.40 (s, 3H), 2.24-2.28 (m, 2H), 2.17 (s, 3H), 2.13 (s, 3H), 1.82-1.84 (m, 1H), 1.69-1.70 (m, 2H), 1.56 (s, 3H), 1.36-1.40 (m, 2H), 1.07 (t, 3H).

LC-MS (ESI, m/z) = 661.5 ($M+H^+$)
Specific optical rotation $[\alpha]_D^{25}$ = -49.3500 (c=0.1, methanol)

**Preparation Example 80. Synthesis of stereoisomer A of 7-(2-(ethyl(methyl)amino)pyridin-5-yl)-2,4-dimethyl-N-((6-methyl-4-(methylthio)-2-oxo-1,2-dihydropyridin-3-yl)methyl)-2-(1-(2,2,2-trifluoroethyl)piperidin-4-yl)benzo[d][1,3]dioxole-5-carboxamide (compound 80)**

Stereoisomer A of

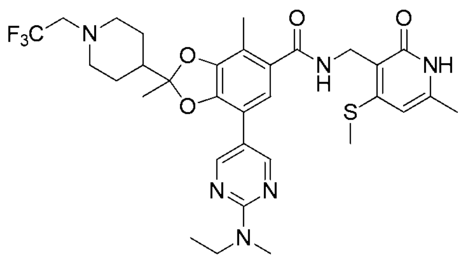

(

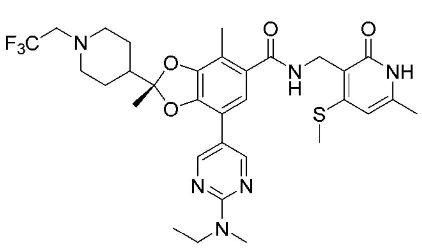

or

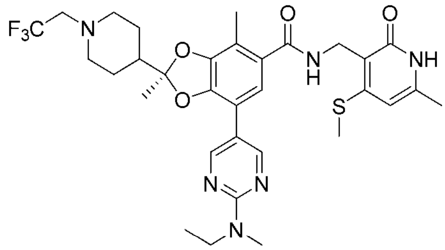

)

The same reaction as in above Preparation Example 78 was performed except that tert-butyl (S)-4-(7-bromo-5-(methoxycarbonyl)-2,4-dimethylbenzo[d][1,3]dioxole-2-yl)piperidin-1-carboxylate (3 g, 6.38 mmole), which is stereospecific, was used as a starting material in [Step 1] instead of tert-butyl 4-(7-bromo-5-(methoxycarbonyl)-2,4-dimethylbenzo[d][1,3]dioxole-2-yl)piperidin-1-carboxylate, so as to obtain the title compound (0.3 g).

$^1$H NMR (DMSO-$d_6$): 11.5(s, 1H), 8.62(s, 2H), 7.90 (t, 1H), 7.05 (s, 1H), 6.05 (s, 1H), 4.26 (d, 2H), 3.62-3.65 (m, 2H), 3.06-3.12 (m, 5H), 2.91-2.92 (m, 2H), 2.41 (s, 3H), 2.24-2.28 (m, 2H), 2.17 (s, 3H), 2.13 (s, 3H), 1.83-1.88 (m, 1H), 1.69-1.72 (m, 2H), 1.56 (s, 3H), 1.36-1.38 (m, 2H), 1.07 (t, 3H).

LC-MS (ESI, m/z) = 661.5 (M+H$^+$)

Specific optical rotation $[\alpha]_D^{25}$ = +63.2167 (c=0.1, methanol)

<u>**Preparation Example 81. Synthesis of stereoisomer B of 2-(1-(2,2-difluoroethyl)piperidin-4-yl)-7-(2-(ethyl(methyl)amino)pyrimidin-5-yl)-2,4-dimethyl-N-((6-methyl-4-(methylthio)-2-oxo-1,2-dihydropyridin-3-yl)methyl)benzo[d][1,3]dioxole-5-carboxamide (compound 81)**</u>

Stereoisomer B of

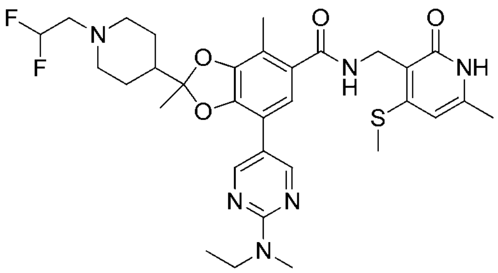

(

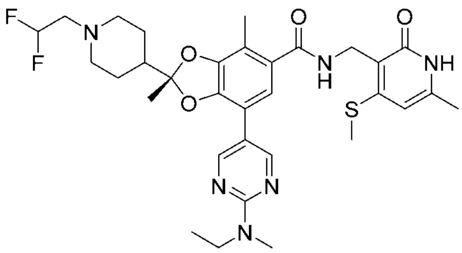

or

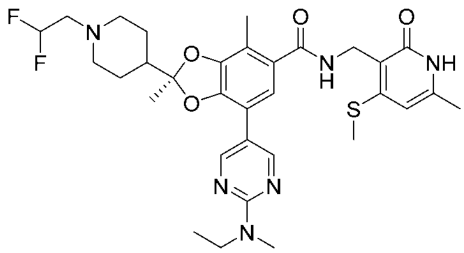

)

The same reaction as in above Preparation Example 79 was performed except that 2-iodo-1,1-difluoroethane (0.13 mL) was used instead of 2,2,2-trifluoroethyl trifluoromethanesulfonate in [Step 6] to obtain the title compound (0.2 g).

$^1$H NMR (DMSO-$d_6$): 11.5 (s, 1H), 8.63 (s, 2H), 7.90 (t, 1H), 7.04 (s, 1H), 6.04-6.05 (m, 2H), 4.26 (d, 2H), 3.61-3.64 (m, 2H), 3.07 (s, 3H), 2.91-2.92 (m, 2H), 2.62-2.65 (m, 2H), 2.40 (s, 3H), 2.17 (s, 3H), 2.13 (s, 3H), 2.06 (t, 2H), 1.80-1.81 (m, 1H), 1.68-1.69 (m, 2H), 1.56 (s, 3H), 1.35-1.36 (m, 2H), 1.08 (t, 3H).

LC-MS (ESI, m/z) = 643.5 (M+H$^+$)

Specific optical rotation $[\alpha]_D^{25}$ = -52.7667 (c=0.1, methanol)

**Preparation Example 82. Synthesis of stereoisomer A of 2-(1-(2,2-difluoroethyl)piperidin-4-yl)-7-(2-(ethyl(methyl)amino)pyrimidin-5-yl)-2,4-dimethyl-N-((6-methyl-4-(methylthio)-2-oxo-1,2-dihydropyridin-3-yl)methyl)benzo[d][1,3]dioxole-5-carboxamide (compound 82)**

Stereoisomer A of

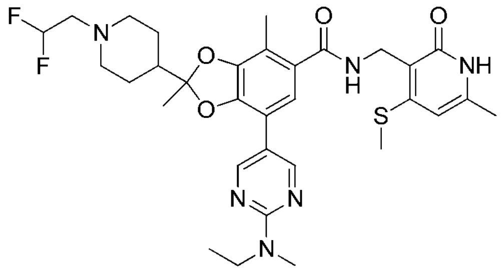

(

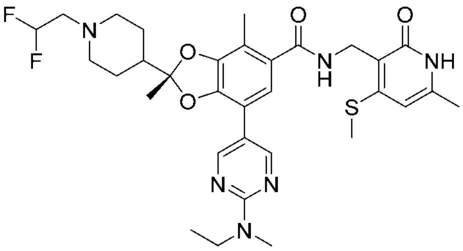

or

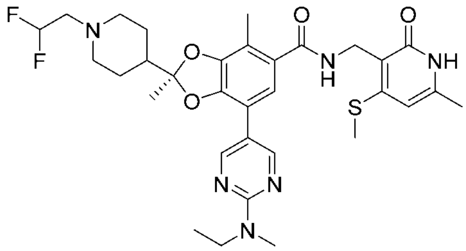

)

The same reaction as in above Preparation Example 80 was performed except that 2-iodo-1,1-difluoroethane (0.13 mL) was used instead of 2,2,2-trifluoroethyl trifluoromethanesulfonate in [Step 6] to obtain the title compound (0.2 g).

$^1$H NMR (DMSO-$d_6$): 11.5 (s, 1H), 8.62 (s, 2H), 7.91 (t, 1H), 7.05 (s, 1H), 6.04-6.05 (m, 2H), 4.26 (d, 2H), 3.62-3.65 (m, 2H), 3.08 (s, 3H), 2.89-2.92 (m, 2H), 2.62-2.78 (m, 2H), 2.41 (s, 3H), 2.17 (s, 3H), 2.13 (s, 3H), 2.06 (t, 2H), 1.81-1.86 (m, 1H), 1.68-1.69 (m, 2H), 1.57 (s, 3H), 1.35-1.39 (m, 2H), 1.07 (t, 3H).

LC-MS (ESI, m/z) = 643.5 (M+H$^+$)
Specific optical rotation $[\alpha]_D^{25}$ = +62.1000 (c=0.1, methanol)

**Preparation Example 83. Synthesis of stereoisomer B of 7-(2-(ethyl(methyl)amino)pyrimidin-5-yl)-2-(1-ethylpiperidin-4-yl)-2,4-dimethyl-N-((6-methyl-4-(methylthio)-2-oxo-1,2-dihydropyridin-3-yl)methyl)benzo[d][1,3]dioxole-5-carboxamide (compound 83)**

Stereoisomer B of

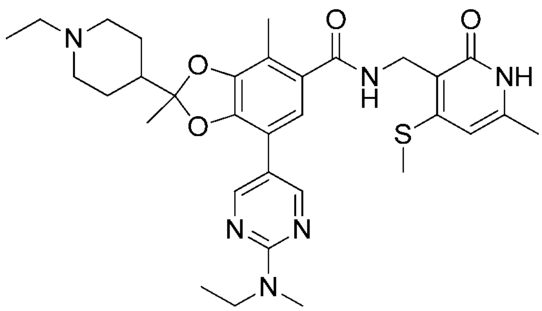

(

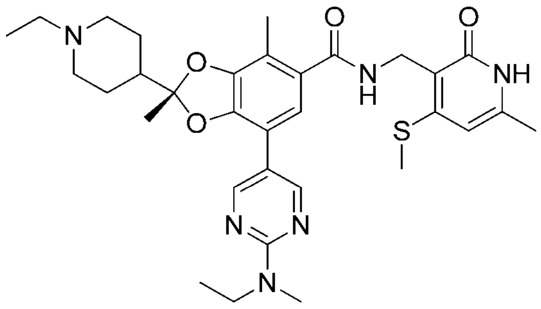

or

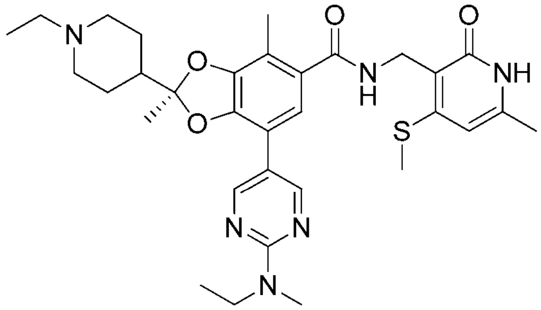

)

The same reaction as in above Preparation Example 79 was performed except that iodoethane (0.12 mL) was used instead of 2,2,2-trifluoroethyl trifluoromethanesulfonate in [Step 6] to obtain the title compound (0.1 g).

$^1$H NMR (DMSO-$d_6$): 11.5 (s, 1H), 8.63 (s, 2H), 7.92 (t, 1H), 7.08 (s, 1H), 6.06 (s, 1H), 4.28 (d, 2H), 3.61-3.64 (m, 2H), 3.40-3.47 (m, 2H), 3.08 (s, 3H), 2.96-3.05 (m ,2H), 2.61-2.90 (m, 2H), 2.41 (s, 3H), 2.19 (s, 3H), 2.13 (s, 3H), 1.91-1.96 (m, 3H), 1.60 (s, 3H), 1.55-1.57 (m, 2H), 1.14 (t, 3H), 1.07 (t, 3H).

LC-MS (ESI, m/z) = 607.5 (M+H$^+$)

**<u>Preparation Example 84. Synthesis of stereoisomer A of 7-(2-(ethyl(methyl)amino)pyrimidin-5-yl)-2-(1-ethylpiperidin-4-yl)-2,4-dimethyl-N-((6-methyl-4-(methylthio)-2-oxo-1,2-dihydropyridin-3-yl)methyl)benzo[d][1,3]dioxole-5-carboxamide (compound 84)</u>**

Stereoisomer A of

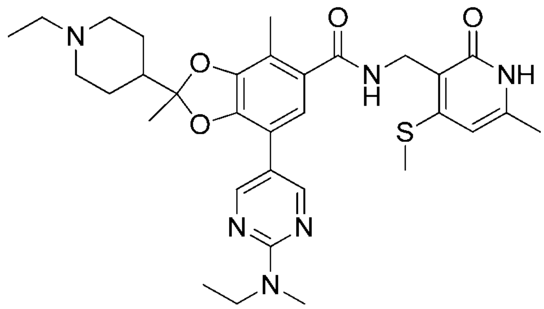

(

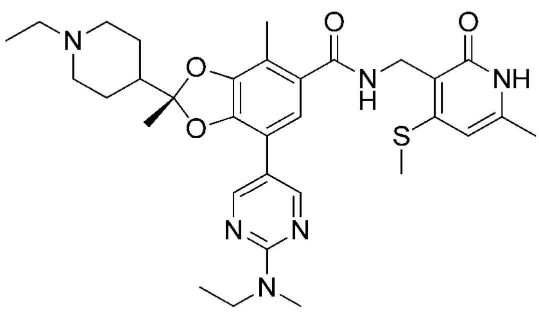

or

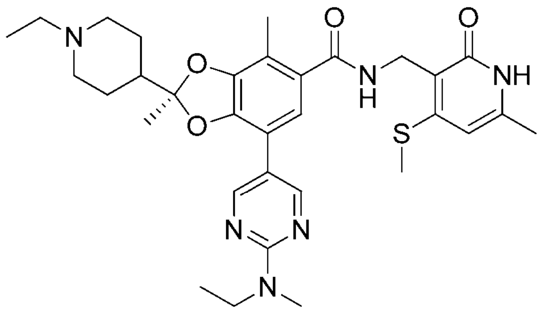

)

The same reaction as in above Preparation Example 80 was performed except that iodoethane (0.12 mL) was used instead of 2,2,2-trifluoroethyl trifluoromethanesulfonate in [Step 6] to obtain the title compound (0.1 g).

$^1$H NMR (DMSO-$d_6$): 11.5 (s, 1H), 8.63 (s, 2H), 7.92 (t, 1H), 7.08 (s, 1H), 6.06 (s, 1H), 4.27 (d, 2H), 3.63-3.66 (m, 2H), 3.43-3.55 (m, 2H), 3.08 (s, 3H), 3.03-3.06 (m ,2H), 2.80-2.96 (m, 2H), 2.41 (s, 3H), 2.21 (s, 3H), 2.13 (s, 3H), 1.93-2.00 (m, 3H), 1.60 (s, 3H), 1.28-1.32 (m, 2H), 1.11-1.28 (m, 3H), 1.07 (t, 3H).

LC-MS (ESI, m/z) = 607.5 (M+H$^+$)

**Preparation Example 85. Synthesis of 7-(6-(ethyl(methyl)amino)pyridin-3-yl)-2,4-dimethyl-N-((6-methyl-4-(methylthio)-2-oxo-1,2-dihydropyridin-3-yl)methyl)-2-(1-(2,2,2-trifluoro)piperidin-4-yl)benzo[d] [1,3] dioxole-5-carboxamide (compound 85)**

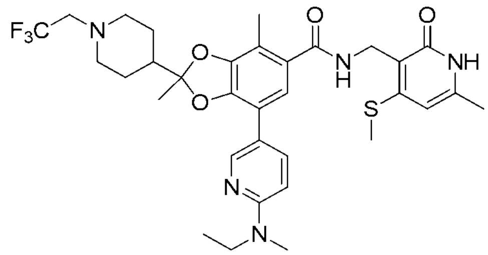

The same processes as in [Steps 1 to 5] were performed except that N-ethyl-N-methyl-5-(4,4,5,5-tetramethyl-1,3,2-dioxaborolan-2-yl)pyridin-2-amine was used in [Step 1] of above Preparation Example 33 instead of (2S,6R)-2,6-dimethyl-4-(5-(4,4,5,5-tetramethyl-1,3,2-dioxaborolan-2-yl)pyridin-2-yl)morpholine (intermediate 1), so as to obtain 7-(6-(ethyl(methyl)amino)pyridin-3-yl)-2,4-dimethyl-N-((6-methyl-4-(methylthio)-2-oxo-1,2-dihydropyridin-3-yl) methyl)-2-(piperidin-4-yl)benzo[d][1,3]dioxole-5-carboxamide hydrochloride of [Step 5].

Acetonitrile (10 mL), sodium carbonate (0.43 g), and 2,2,2-trifluoroethyl trifluoromethanesulfonate (0.20 mL) were added to the obtained hydrochloride (0.5 g, 0.73 mmole), and stirred at an internal temperature of 100°C for 24 hours. After cooling, dichloromethane (5 mL) was added, filtered and concentrated, and the crystal produced by using ethanol (2 mL) and hexane (20 mL) was filtered, and dried at an internal temperature of 60°C to obtain the title compound (0.3 g).

$^1$H NMR (DMSO-$d_6$): 11.6 (s, 1H), 8.41 (s, 1H), 7.92 (t, 1H), 7.76 (d, 1H), 7.01 (s, 1H), 6.65 (d, 1H), 6.03 (s, 1H), 4.25 (d, 2H), 3.51-3.53 (m, 2H), 3.05-3.11 (m, 2H), 2.96 (s, 3H), 2.91-2.93 (m, 2H), 2.41 (s, 3H), 2.26-2.28 (m, 2H), 2.16 (s, 3H), 2.13 (s, 3H), 1.82-1.86 (m, 1H), 1.69-1.71 (m, 2H), 1.56 (s, 3H), 1.36-1.41 (m, 2H), 1.04 (t, 3H).

LC-MS (ESI, m/z) = 660.5 (M+H$^+$)

**Preparation Example 86. Synthesis of stereoisomer B of 7-(6-(ethyl(methyl)amino)pyridin-3-yl)-2,4-dimethyl-N-((6-methyl-4-(methylthio)-2-oxo-1,2-dihydropyridin-3-yl)methyl)-2-(1-(2,2,2-trifluoroethyl)piperidin-4-yl)benzo[d][1,3]dioxole-5-carboxamide (compound 86)**

Stereoisomer B of

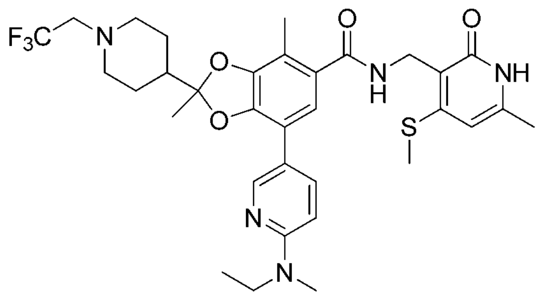

(

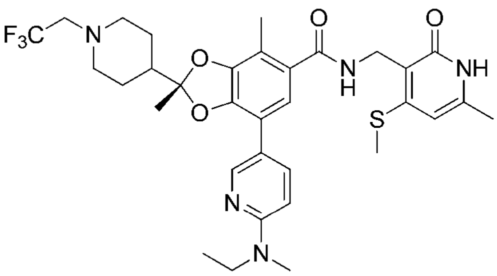

or

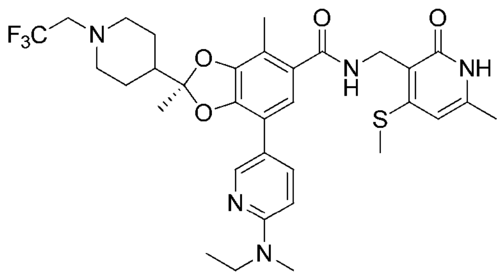

)

The same reaction as in above Preparation Example 85 was performed except that tert-butyl (R)-4-(7-bromo-5-(methoxycarbonyl)-2,4-dimethylbenzo[d][1,3]dioxole-2-yl)piperidin-1-carboxylate (3 g, 6.38 mmole), which is stereospecific, was used as a starting material in [Step 1] instead of tert-butyl 4-(7-bromo-5-(methoxycarbonyl)-2,4-dimethylbenzo[d][1,3]dioxole-2-yl)piperidin-1-carboxylate, so as to obtain the title compound (0.3 g).

$^1H$ NMR (DMSO-$d_6$): 11.5 (s, 1H), 8.41 (s, 1H), 7.93 (t, 1H), 7.76 (d, 1H), 7.01 (s, 1H), 6.65 (d, 1H), 6.04 (s, 1H), 4.26 (d, 2H), 3.52-3.55 (m, 2H), 3.06-3.12 (m, 2H), 2.96 (s, 3H), 2.91-2.93 (m, 2H), 2.41 (s, 3H), 2.27-2.29 (m, 2H), 2.16 (s, 3H), 2.13 (s, 3H), 1.82-1.86 (m, 1H), 1.69-1.71 (m, 2H), 1.56 (s, 3H), 1.36-1.41 (m, 2H), 1.04 (t, 3H).

LC-MS (ESI, m/z) = 660.5 (M+H$^+$)
Specific optical rotation $[\alpha]_D^{25}$ = -61.9834 (c=0.1, methanol)

**Preparation Example 87. Synthesis of stereoisomer A of 7-(6-(ethyl(methyl)amino)pyridin-3-yl)-2,4-dimethyl-N-((6-methyl-4-(methylthio)-2-oxo-1,2-dihydropyridin-3-yl)methyl)-2-(1-(2,2,2-trifluoroethyl)piperidin-4-yl)benzo[d][1,3]dioxole-5-carboxamide (compound 87)**

Stereoisomer A of

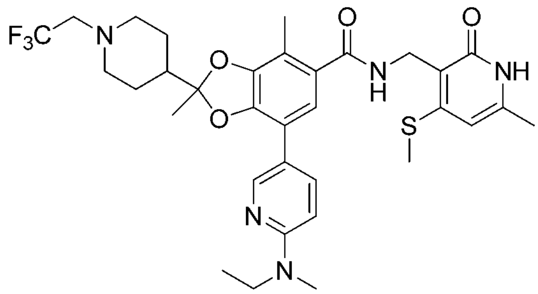

(

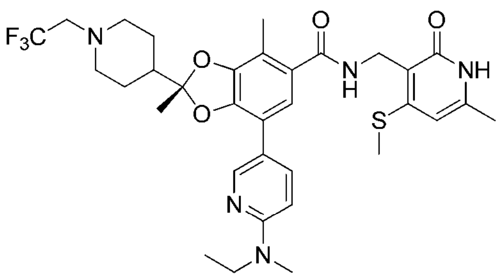

or

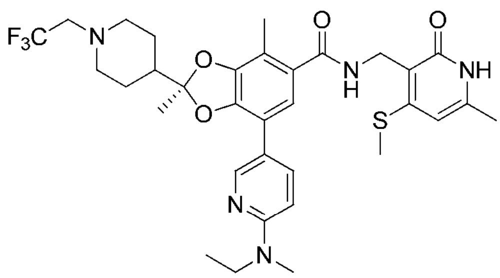

)

The same reaction as in above Preparation Example 85 was performed except that tert-butyl (S)-4-(7-bromo-5-(methoxycarbonyl)-2,4-dimethylbenzo[d][1,3]dioxole-2-yl)piperidin-1-carboxylate (3 g, 6.38 mmole), which is stereospecific, was used as a starting material in [Step 1] instead of tert-butyl 4-(7-bromo-5-(methoxycarbonyl)-2,4-dimethylbenzo[d][1,3]dioxole-2-yl)piperidin-1-carboxylate, so as to obtain the title compound (0.3 g).

$^1H$ NMR (DMSO-$d_6$): 11.5 (s, 1H), 8.41 (s, 1H), 7.93 (t, 1H), 7.76 (d, 1H), 7.01 (s, 1H), 6.65 (d, 1H), 6.05 (s, 1H), 4.26 (d, 2H), 3.51-3.56 (m, 2H), 3.08-3.12 (m, 2H), 2.97 (s, 3H), 2.91-2.94 (m, 2H), 2.41 (s, 3H), 2.26-2.28 (m, 2H), 2.16 (s, 3H), 2.13 (s, 3H), 1.82-1.87 (m, 1H), 1.69-1.73 (m, 2H), 1.56 (s, 3H), 1.36-1.41 (m, 2H), 1.03 (t, 3H).

LC-MS (ESI, m/z) = 660.5 (M+H$^+$)

**Preparation Example 88. Synthesis of stereoisomer B of 2-(1-(2,2-difluoroethyl)piperidin-4-yl)-7-(6-(ethyl(methyl)amino)pyridin-3-yl)-2,4-dimethyl-N-((6-methyl-4-(methylthio)-2-oxo-1,2-dihydropyridin-3-yl)methyl)benzo[d][1,3]dioxole-5-carboxamide (compound 88)**

Stereoisomer B of

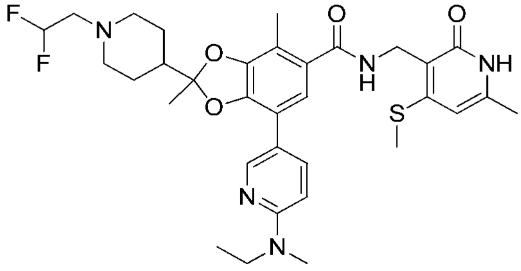

(

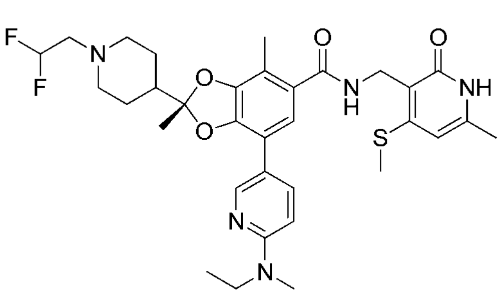

or

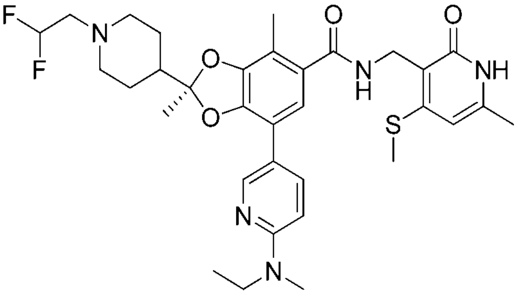

)

The same reaction as in above Preparation Example 86 was performed except that 2-iodo-1,1-difluoroethane (0.13 mL) was used instead of 2,2,2-trifluoroethyl trifluoromethanesulfonate in [Step 6] to obtain the title compound (0.2 g).

$^1$H NMR (DMSO-$d_6$): 11.5 (s, 1H), 8.40 (s, 1H), 7.94 (t, 1H), 7.74 (d, 1H), 7.01 (s, 1H), 6.66 (d, 1H), 5.95-6.07 (m, 3H), 4.26 (d, 2H), 3.52-3.55 (m, 2H) 2.97 (s, 3H), 2.90-2.92 (m, 2H), 2.62-2.67 (m, 2H), 2.41 (s, 3H), 2.16 (s, 3H), 2.13 (s, 3H), 2.07-2.10 (m, 2H). 1.80-1.84 (m, 1H), 1.68-1.71 (m, 2H), 1.56 (s, 3H), 1.35-1.42 (m, 2H), 1.04 (t, 3H).

LC-MS (ESI, m/z) = 642.5 (M+H$^+$)

**Preparation Example 89. Synthesis of stereoisomer B of 7-(6-(ethyl(methyl)amino)pyridin-3-yl)-2-(1-ethylpiperidin-4-yl)-2,4-dimethyl-N-((6-methyl-4-(methylthio)-2-oxo-1,2-dihydropyridin-3-yl)methyl)benzo[d][1,3]dioxole-5-carboxamide (compound 89)**

Stereoisomer B of

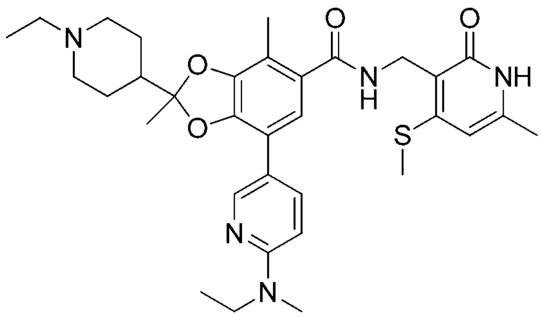

(

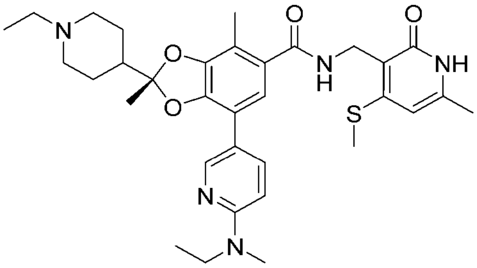

or

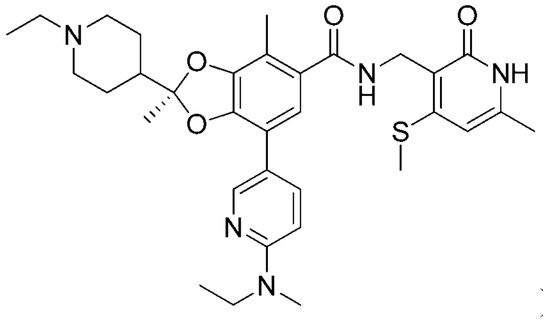

)

The same reaction as in above Preparation Example 86 was performed except that iodoethane (0.12 mL) was used instead of 2,2,2-trifluoroethyl trifluoromethanesulfonate in [Step 6] to obtain the title compound (0.1 g).

$^1$H NMR (DMSO-$d_6$): 11.5 (s, 1H), 8.46 (s, 1H), 7.95 (t, 1H), 7.77 (d, 1H), 7.03 (s, 1H), 6.65 (d, 1H), 6.05 (s, 1H), 4.26 (d, 2H), 3.54-3.56 (m, 2H), 3.47-3.49 (m, 2H), 3.01-3.04 (m, 2H), 2.97 (s, 3H), 2.84-2.90 (m, 2H), 2.41 (s, 3H), 2.19-2.20 (m, 1H), 2.18 (s, 3H), 2.13 (s, 3H), 1.94-2.01 (m, 2H), 1.60-1.65 (m, 5H), 1.16 (t, 3H), 1.04 (t, 3H).

LC-MS (ESI, m/z) = 606.5 (M+H$^+$)

**Preparation Example 90. Synthesis of 2,4-dimethyl-N-((6-methyl-4-(methylthio)-2-oxo-1,2-dihydropyridin-3-yl)methyl)-7-(4-(piperidin-1-yl)phenyl)-2-(1-(2,2,2-trifluoroethyl)piperidin-4-yl)benzo[d][1,3]dioxole-5-carboxamide (compound 90)**

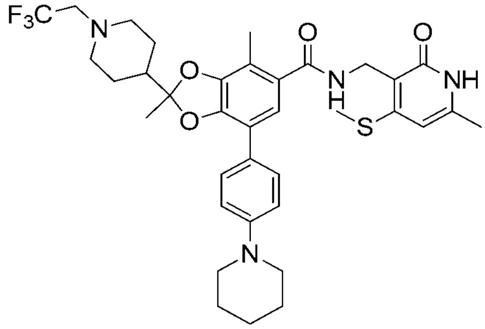

The same processes as in [Steps 1 to 5] were performed except that 1-(4-(4,4,5,5-tetramethyl-1,3,2-dioxaborolan-2-yl)phenyl)piperidine was used in [Step 1] of above Preparation Example 33 instead of (2S,6R)-2,6-dimethyl-4-(5-(4,4,5,5-tetramethyl-1,3,2-dioxaborolan-2-yl)pyridin-2-yl)morpholine (intermediate 1), so as to obtain 2,4-dimethyl-N-((6-methyl-4-(methylthio)-2-oxo-1,2-dihydropyridin-3-yl)methyl)-7-(4-(piperidin-1-yl)phenyl)-2-(piperidin-4-yl)benzo[d][1,3]dioxole-5-carboxamide hydrochloride of [Step 5].

Acetonitrile (10 mL), sodium carbonate (0.43 g), and 2,2,2-trifluoroethyl trifluoromethanesulfonate (0.20 mL) were added to the obtained hydrochloride (0.5 g, 0.73 mmole), and stirred at an internal temperature of 100°C for 24 hours. After cooling, dichloromethane (5 mL) was added, filtered and concentrated, and the crystal produced by using ethanol (2 mL) and hexane (20 mL) was filtered, and dried at an internal temperature of 60°C to obtain the title compound (0.3 g).

$^1$H NMR (DMSO-$d_6$): 11.5 (s, 1H), 7.95 (t, 1H), 7.50 (d, 2H), 7.01 (s, 1H), 6.91 (d, 2H), 6.04 (s, 1H), 4.26 (d, 2H), 3.14-3.16 (m, 4H), 3.04-3.14 (m, 2H), 2.91-2.96 (m, 2H), 2.41 (s, 3H), 2.20-2.28 (m, 2H), 2.16 (s, 3H), 2.13 (s, 3H), 1.82-1.86 (m, 1H), 1.66-1.73 (m, 2H), 1.55-1.58 (m, 4H), 1.55 (s, 3H), 1.49-1.54 (m, 2H), 1.35-1.40 (m, 2H).

LC-MS (ESI, m/z) = 685.5 (M+H$^+$)

**Preparation Example 91. Synthesis of 2,4-dimethyl-N-((6-methyl-4-(methylthio)-2-oxo-1,2-dihydropyridin-3-yl)methyl)-7-(6-(piperidin-1-yl)pyridin-3-yl)-2-(1-(2,2,2-trifluoroethyl)piperidin-4-yl)benzo[d][1,3]dioxole-5-carboxamide (compound 91)**

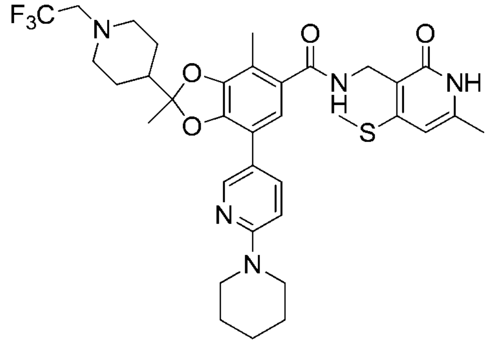

The same processes as in [Steps 1 to 5] were performed except that 2-(piperidin-1-yl)-5-(4,4,5,5-tetraamyl-1,3,2-dioxaborolan-2-yl)pyridine was used in [Step 1] of above Preparation Example 33 instead of (2S,6R)-2,6-dimethyl-4-(5-(4,4,5,5-tetramethyl-1,3,2-dioxaborolan-2-yl)pyridin-2-yl)morpholine (intermediate 1), so as to obtain 2,4-dimethyl-N-((6-methyl-4-(methylthio)-2-oxo-1,2-dihydropyridin-3-yl)methyl)-7-(6-(piperidin-1-yl)pyridin-3-yl)-2-(piperidin-4-yl)benzo[d][1,3]dioxole-5-carboxamide hydrochloride of [Step 5].

Acetonitrile (10 mL), sodium carbonate (0.43 g), and 2,2,2-trifluoroethyl trifluoromethanesulfonate (0.20 mL) were added to the obtained hydrochloride (0.5 g, 0.73 mmole), and stirred at an internal temperature of 100°C for 24 hours. After cooling, dichloromethane (5 mL) was added, filtered and concentrated, and the crystal produced by using ethanol (2 mL) and hexane (20 mL) was filtered, and dried at an internal temperature of 60°C to obtain the title compound (0.3 g).

$^1$H NMR (DMSO-$d_6$): 11.5 (s, 1H), 8.40 (s, 1H), 7.94 (t, 1H), 7.81 (d, 1H), 7.02 (s, 1H), 6.89 (d, 1H), 6.05 (d, 1H), 4.26 (d, 2H), 3.51-3.54 (m, 4H), 3.09 (d, 2H), 2.93 (d, 2H), 2.41 (s, 3H), 2.22-2.30 (m, 2H), 2.16 (s, 3H), 2.13 (s, 3H), 1.80-1.90 (m, 1H), 1.69-1.71 (d, 2H), 1.56-1.57 (m, 2H), 1.56 (s, 3H), 1.51-1.52 (m, 4H), 1.34-1.40 (m, 2H).

LC-MS (ESI, m/z) = 686.4 ($M+H^+$)

**Preparation Example 92. Synthesis of 2,4-dimethyl-N-((6-methyl-4-(methylthio)-2-oxo-1,2-dihydropyridin-3-yl)methyl)-7-(2-thiomorpholinopyrimidin-5-yl)-2-(1-(2,2,2-trifluoroethyl)piperidin-4-yl)benzo[d][1,3]dioxole-5-carboxamide (compound 92)**

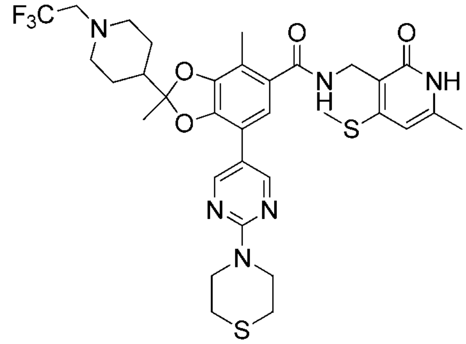

The same processes as in [Steps 1 to 5] were performed except that 4-(5-(4,4,5,5-tetramethyl-1,3,2-dioxaborolan-2-yl)pyrimidin-2-yl)thiomorpholine was used in [Step 1] of above Preparation Example 33 instead of (2S,6R)-2,6-dimethyl-4-(5-(4,4,5,5-tetramethyl-1,3,2-dioxaborolan-2-yl)pyridin-2-yl)morpholine (intermediate 1), so as to obtain 2,4-dimethyl-N-((6-methyl-4-(methylthio)-2-oxo-1,2-dihydropyridin-3-yl)methyl)-2-(piperidin-4-yl)-7-(6-thiomorpholinopyridin-3-yl)benzo[d][1,3]dioxole-5-carboxamide hydrochloride of [Step 5].

Acetonitrile (10 mL), sodium carbonate (0.43 g), and 2,2,2-trifluoroethyl trifluoromethanesulfonate (0.20 mL) were added to the obtained hydrochloride (0.5 g, 0.73 mmole), and stirred at an internal temperature of 100°C for 24 hours. After cooling, dichloromethane (5 mL) was added, filtered and concentrated, and the crystal produced by using ethanol (2 mL) and hexane (20 mL) was filtered, and dried at an internal temperature of 60°C to obtain the title compound (0.3 g).

$^1$H NMR (DMSO-$d_6$): 11.5 (s. 1H), 8.70 (s, 2H), 7.91 (t, 1H), 7.06 (s, 1H), 6.04 (s, 1H), 4.24-4.28 (m, 2H), 4.06-4.08 (m, 4H), 3.08-3.10 (m, 2H), 2.88-2.95 (m, 2H), 2.58-2.60 (m, 4H) 2.41 (s. 3H), 2.21-2.30 (m, 2H), 2.17 (s, 3H), 2.13 (s, 3H), 1.78-1.88 (m, 1H), 1.65-1.71 (m, 2H), 1.56 (s, 3H), 1.34-1.38 (m, 2H).

LC-MS (ESI, m/z) = 705.8 ($M+H^+$)

**Preparation Example 93. Synthesis of 2',2'-difluoro-2,7-dimethyl-N-((6-methyl-4-(methylthio)-2-oxo-1,2-dihydropyridin-3-yl)methyl)-2-(1-(2,2,2-trifluoroethyl)piperidin-4-yl)-[4,5'-bibenzo[d][1,3]dioxole]-6-carboxamide (compound 93)**

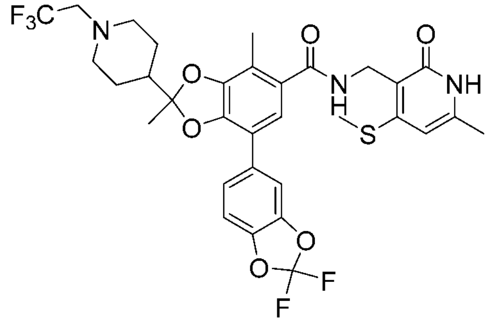

The same processes as in [Steps 1 to 5] were performed except that 2-(2,2-difluorobenzo[d][1,3]dioxole-5-yl)-4,4,5,5-tetramethyl-1,3,2-dioxaborolane was used in [Step 1] of above Preparation Example 33 instead of (2S,6R)-2,6-dimethyl-4-(5-(4,4,5,5-tetramethyl-1,3,2-dioxaborolan-2-yl)pyridin-2-yl)morpholine (intermediate 1), so as to obtain 2',2'-difluoro-2,7-dimethyl-N-((6-methyl-4-(methylthio)-2-oxo-1,2-dihydropyridin-3-yl)methyl)-2-(piperidin-4-yl)-[4,5'-bibenzo[d][1,3]dioxole-6-carboxamide hydrochloride of [Step 5].

Acetonitrile (10 mL), sodium carbonate (0.43 g), and 2,2,2-trifluoroethyl trifluoromethanesulfonate (0.20 mL) were added to the obtained hydrochloride (0.5 g, 0.73 mmole), and stirred at an internal temperature of 100°C for 24 hours. After cooling, dichloromethane (5 mL) was added, filtered and concentrated, and the crystal produced by using ethanol (2 mL) and hexane (20 mL) was filtered, and dried at an internal temperature of 60°C to obtain the title compound (0.3 g).

$^1$H NMR (DMSO-$d_6$): 11.5 (s, 1H), 7.95 (t, 1H), 7.67 (s, 1H), 7.49-7.51 (m, 1H), 7.46-7.47 (m, 1H), 7.07 (s, 1H), 6.05 (s, 1H), 4.27 (d, 2H), 3.06-3.12 (m, 2H), 2.89-2.95 (m, 2H), 2.41 (s, 3H), 2.25-2.31 (m, 2H), 2.19 (s, 3H), 2.13 (s, 3H), 1.83-1.88 (m, 1H), 1.69-1.71 (m, 2H), 1.58 (s, 3H), 1.35-1.40 (m, 2H).

LC-MS (ESI, m/z) = 682.7 (M+H$^+$)

**Preparation Example 94. Synthesis of 2,7-dimethyl-N-((6-methyl-4-(methylthio)-2-oxo-1,2-dihydropyridin-3-yl)methyl)-2-(1-(2,2,2-trifluoroethyl)piperidin-4-yl)-[4,5'-bibenzo[d][1,3]dioxole]-6-carboxamide (compound 94)**

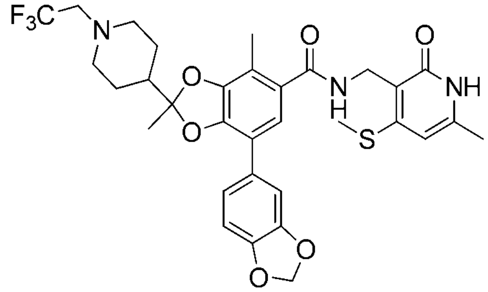

The same processes as in [Steps 1 to 5] were performed except that 2-(benzo[d][1,3]dioxole-5-yl)-4,4,5,5-tetramethyl-1,3,2-dioxaborolane was used in [Step 1] of above Preparation Example 33 instead of (2S,6R)-2,6-dimethyl-4-(5-(4,4,5,5-tetramethyl-1,3,2-dioxaborolan-2-yl)pyridin-2-yl)morpholine (intermediate 1), so as to obtain 2,7-dimethyl-N-((6-methyl-4-(methylthio)-2-oxo-1,2-dihydropyridin-3-yl)methyl)-2-(piperidin-4-yl)-[4,5'-bibenzo[d][1,3]dioxole-6-carboxamide hydrochloride of [Step 5].

Acetonitrile (10 mL), sodium carbonate (0.43 g), and 2,2,2-trifluoroethyl trifluoromethanesulfonate (0.20 mL) were added to the obtained hydrochloride (0.5 g, 0.73 mmole), and stirred at an internal temperature of 100°C for 24 hours. After cooling, dichloromethane (5 mL) was added, filtered and concentrated, and the crystal produced by using ethanol (2 mL) and hexane (20 mL) was filtered, and dried at an internal temperature of 60°C to obtain the title compound (0.3 g).

$^1$H NMR (DMSO-$d_6$): 11.5 (s, 1H), 7.96 (t, 1H), 7.21 (s, 1H), 7.19-7.20 (m, 1H), 7.01 (s, 1H), 6.97-6.98 (m, 1H), 6.04 (s, 1H), 6.01 (s, 2H), 4.26 (d, 2H), 3.06-3.13 (m, 2H), 2.91-2.94 (m, 2H), 2.41 (s, 3H), 2.21-2.30 (m, 2H), 2.17 (s, 3H),

2.13 (s, 3H), 1.82-1.87 (m, 1H), 1.68-1.72 (m, 2H), 1.56 (s, 3H), 1.32-1.38 (m, 2H).

LC-MS (ESI, m/z) = 646.7 (M+H+)

**Preparation Example 95. Synthesis of 2-(1-(2,2-difluoroethyl)piperidin-4-yl)-2,7-dimethyl-N-((6-methyl-4-(methylthio)-2-oxo-1,2-dihydropyridin-3-yl)methyl)-[4,5'-bibenzo[d][1,3]dioxole]-6-carboxamide (compound 95)**

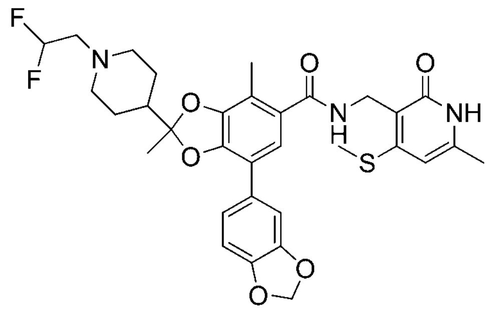

Acetonitrile (10 mL), sodium carbonate (0.43 g), and 2-iodo-1,1-difluoroethane (0.09 mL) were added to 2,7-dimethyl-N-((6-methyl-4-(methylthio)-2-oxo-1,2-dihydropyridin-3-yl)methyl)-2-(piperidin-4-yl)-[4,5'-bibenzo[d][1,3]dioxole]-6-carboxamide hydrochloride (0.5 g, 0.73 mmole) obtained in the same process as in [Steps 1 to 5] of above Preparation Example 94, and stirred at an internal temperature of 100°C for 24 hours. After cooling, dichloromethane (5 mL) was added, filtered and concentrated, and the crystal produced by using ethanol (2 mL) and hexane (20 mL) was filtered, and dried at an internal temperature of 60°C to obtain the title compound (0.3 g).

$^1$H NMR (DMSO-$d_6$): 11.5 (s, 1H), 7.96 (t, 1H), 7.21 (s, 1H), 7.19-7.20 (m, 1H), 7.01 (s, 1H), 6.97-6.98 (m, 1H), 6.04 (s, 1H), 6.01 (t, 1H), 4.26 (d, 2H), 2.89-2.92 (m, 2H), 2.61-2.68 (m, 2H), 2.46 (s, 3H), 2.17 (s, 3H), 2.13 (s, 3H), 2.05-2.10 (m, 2H), 1.83-1.84 (m, 1H), 1.67-1.70 (m, 2H), 1.57 (s, 3H), 1.34-1.39 (m, 2H).

LC-MS (ESI, m/z) = 628.5 (M+H+)

**Preparation Example 96. Synthesis of 7-(benzofuran-5-yl)-2,4-dimethyl-N-((6-methyl-4-(methylthio)-2-oxo-1,2-dihydropyridin-3-yl)methyl)-2-(1-(2,2,2-trifluoroethyl)piperidin-4-yl)benzo[d][1,3]dioxole-5-carboxamide (compound 96)**

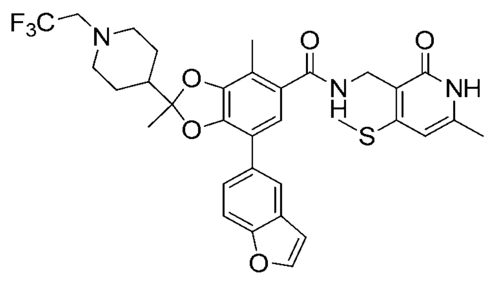

The same processes as in [Steps 1 to 5] were performed except that 2-(benzofuran-5-yl)-4,4,5,5-tetramethyl-1,3,2-dioxaborolane was used in [Step 1] of above Preparation Example 33 instead of (2S,6R)-2,6-dimethyl-4-(5-(4,4,5,5-tetramethyl-1,3,2-dioxaborolan-2-yl)pyridin-2-yl)morpholine (intermediate 1), so as to obtain 7-(benzofuran-5-yl)-2,4-dimethyl-N-((6-methyl-4-(methylthio)-2-oxo-1,2-dihydropyridin-3-yl)methyl)-2-(piperidin-4-yl) benzo[d][1,3]dioxole-5-carboxamide hydrochloride of [Step 5].

Acetonitrile (10 mL), sodium carbonate (0.43 g), and 2,2,2-trifluoroethyl trifluoromethanesulfonate (0.20 mL) were added to the obtained hydrochloride (0.5 g, 0.73 mmole), and stirred at an internal temperature of 100°C for 24 hours. After cooling, dichloromethane (5 mL) was added, filtered and concentrated, and the crystal produced by using ethanol (2 mL) and hexane (20 mL) was filtered, and dried at an internal temperature of 60°C to obtain the title compound (0.3 g).

$^1$H NMR (DMSO-$d_6$): $^1$H NMR (DMSO-$d_6$): 11.5 (s, 1H), 7.99 (s, 2H), 7.91 (s, 1H), 7.61-7.63 (m, 1H), 7.60-7.61 (m, 1H), 7.09 (s, 1H), 6.99 (s, 1H), 6.04 (s, 1H), 4.27 (d, 2H), 3.06-3.10 (m, 2H), 2.92-2.94 (m, 2H), 2.41 (s, 3H), 2.26-2.30

(m, 2H), 2.19 (s, 3H), 2.13 (s, 3H), 1.84-1.89 (m, 2H), 1.71-1.74 (m, 2H), 1.58 (s, 3H), 1.38-1.41 (m, 2H).
LC-MS (ESI, m/z) = 642.6 (M+H$^+$)

**Preparation Example 97. Synthesis of 7-(benzofuran-5-yl)-2-(1-(2,2-difluoroethyl)piperidin-4-yl)-2,4-dimethyl-N-((6-methyl-4-(methylthio)-2-oxo-1,2-dihydropyridin-3-yl)methyl)benzo[d][1,3]dioxole-5-carboxamide (compound 97)**

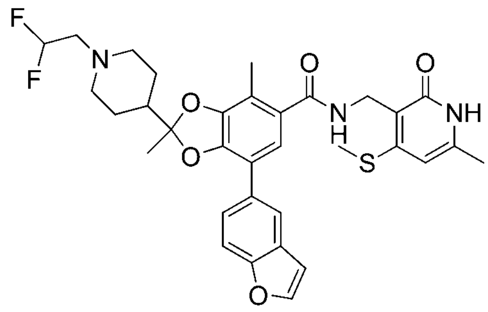

Acetonitrile (10 mL), sodium carbonate (0.43 g), and 2-iodo-1,1-difluoroethane (0.09 mL) were added to 7-(benzofuran-5-yl)-2,4-dimethyl-N-((6-methyl-4-(methylthio)-2-oxo-1,2-dihydropyridin-3-yl)methyl)-2-(piperidin-4-yl) benzo[d][1,3]dioxole-5-carboxamide hydrochloride (0.5 g, 0.73 mmole) obtained in the same process as in [Steps 1 to 5] of above Preparation Example 96, and stirred at an internal temperature of 100°C for 24 hours. After cooling, dichloromethane (5 mL) was added, filtered and concentrated, and the crystal produced by using ethanol (2 mL) and hexane (20 mL) was filtered, and dried at an internal temperature of 60°C to obtain the title compound (0.3 g).
$^1$H NMR (DMSO-$d_6$): 11.5 (s, 1H), 7.99 (s, 2H), 7.90 (s, 1H), 7.62-7.63 (m, 1H), 7.60-7.61 (m, 1H), 7.08 (s, 1H), 6.99 (s, 1H), 5.94-6.06 (m, 2H), 4.27 (d, 2H), 2.91 (d, 2H), 2.60-2.67 (m, 2H), 2.41 (s, 3H), 2.19 (s, 3H), 2.13 (s, 3H), 2.06-2.10 (m, 2H), 1.83-1.87 (m, 1H), 1.69-1.72 (m, 2H), 1.58 (s, 3H), 1.33-1.41 (m, 2H).
LC-MS (ESI, m/z) = 624.6 (M+H$^+$)

**Preparation Example 98. Synthesis of 7-(6-(butyl(methyl)amino)pyridin-3-yl)-2,4-dimethyl-N-((6-methyl-4-(methylthio)-2-oxo-1,2-dihydropyridin-3-yl)methyl)-2-(1-(2,2,2-trifluoroethyl)piperidin-4-yl)benzo[d][1,3]dioxole-5-carboxamide (compound 98)**

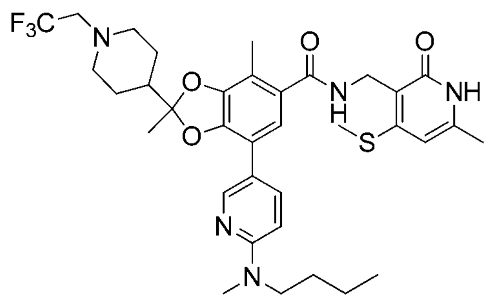

The same processes as in [Steps 1 to 5] were performed except that N-butyl-N-methyl-5-(4,4,5,5-tetramethyl-1,3,2-dioxaborolan-2-yl)pyridin-2-amine was used in [Step 1] of above Preparation Example 33 instead of (2S,6R)-2,6-dimethyl-4-(5-(4,4,5,5-tetramethyl-1,3,2-dioxaborolan-2-yl)pyridin-2-yl)morpholine (intermediate 1), so as to obtain 7-(6-(butyl(methyl)amino)pyridin-3-yl)-2,4-dimethyl-N-((6-methyl-4-(methylthio)-2-oxo-1,2-dihydropyridin-3-yl) methyl)-2-(piperidin-4-yl)benzo[d][1,3]dioxole-5-carboxamide hydrochloride of [Step 5].
Acetonitrile (10 mL), sodium carbonate (0.43 g), and 2,2,2-trifluoroethyl trifluoromethanesulfonate (0.20 mL) were added to the obtained hydrochloride (0.5 g, 0.73 mmole), and stirred at an internal temperature of 100°C for 24 hours. After cooling, dichloromethane (5 mL) was added, filtered and concentrated, and the crystal produced by using ethanol (2 mL) and hexane (20 mL) was filtered, and dried at an internal temperature of 60°C to obtain the title compound (0.3 g).
$^1$H NMR (DMSO-$d_6$): 11.5 (s, 1H), 8.40 (s, 1H), 7.93 (t, 1H), 7.74 (d, 1H), 7.01 (s, 1H), 6.64 (d, 1H), 6.05 (s, 1H), 4.26 (d, 2H), 3.48 (t, 2H), 3.06-3.12 (m, 2H), 2.97 (s, 3H), 2.94 (d, 2H), 2.41 (s, 3H), 2.27 (t, 2H), 2.17 (s, 3H), 2.13 (s, 3H),

1.84 (t, 1H), 1.70 (d, 2H), 1.56 (s, 3H), 1.46-1.50 (m, 2H), 1.32-1.43 (m, 2H), 1.24-1.28 (m, 2H), 0.88 (t, 3H).

LC-MS (ESI, m/z) = 688.8 ($M+H^+$)

**Preparation Example 99. Synthesis of 7-(6-(butyl(methyl)amino)pyridin-3-yl)-2,4-dimethyl-N-((6-methyl-4-(methylthio)-2-oxo-1,2-dihydropyridin-3-yl)methyl)-2-(1-(2,2,2-trifluoroethyl)piperidin-4-yl)benzo[d][1,3]dioxole-5-carboxamide (compound 99)**

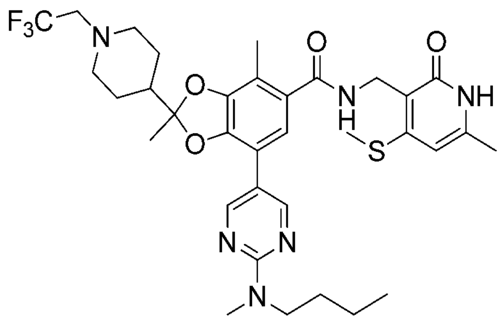

The same processes as in [Steps 1 to 5] were performed except that N-butyl-N-methyl-5-(4,4,5,5-tetramethyl-1,3,2-dioxaborolan-2-yl)pyrimidin-2-amine was used in [Step 1] of above Preparation Example 33 instead of (2S,6R)-2,6-dimethyl-4-(5-(4,4,5,5-tetramethyl-1,3,2-dioxaborolan-2-yl)pyridin-2-yl)morpholine (intermediate 1), so as to obtain 7-(2-(butyl(methyl)amino)pyrimidin-5-yl)-2,4-dimethyl-N-((6-methyl-4-(methylthio)-2-oxo-1,2-dihydropyridin-3-yl)methyl)-2-(piperidin-4-yl)benzo[d][1,3]dioxole-5-carboxamide hydrochloride of [Step 5].

Acetonitrile (10 mL), sodium carbonate (0.43 g), and 2,2,2-trifluoroethyl trifluoromethanesulfonate (0.20 mL) were added to the obtained hydrochloride (0.5 g, 0.73 mmole), and stirred at an internal temperature of 100°C for 24 hours. After cooling, dichloromethane (5 mL) was added, filtered and concentrated, and the crystal produced by using ethanol (2 mL) and hexane (20 mL) was filtered, and dried at an internal temperature of 60°C to obtain the title compound (0.3 g).

$^1H$ NMR (DMSO-$d_6$): 11.5 (s, 1H), 8.62 (s, 2H), 7.90 (t, 1H), 7.05 (s, 1H), 6.05 (s, 1H), 4.26 (d, 2H), 3.58 (t, 2H), 3.06-3.10 (m, 5H), 2.92 (d, 2H), 2.41 (s, 3H), 2.27 (t, 2H), 2.18 (s, 3H), 2.13 (s, 3H), 1.85 (t, 1H), 1.70 (d, 2H), 1.57 (s, 3H), 1.49-1.53 (m, 2H), 1.30-1.41 (m, 2H), 1.24-1.29 (m, 2H), 0.88 (t, 3H).

LC-MS (ESI, m/z) = 689.8 ($M+H^+$)

**Preparation Example 100. Synthesis of 2-(trans-4-aminocyclohexyl)-7-(6-((2S,6R)-2,6-dimethylmorpholino)pyridin-3-yl)-2,4-dimethyl-N-((6-methyl-4-(methylthio)-2-oxo-1,2-dihydropyridin-3-yl)methyl)benzo[d][1,3]dioxole-5-carboxamide hydrochloride (compound 100)**

<Reaction Formula 5>

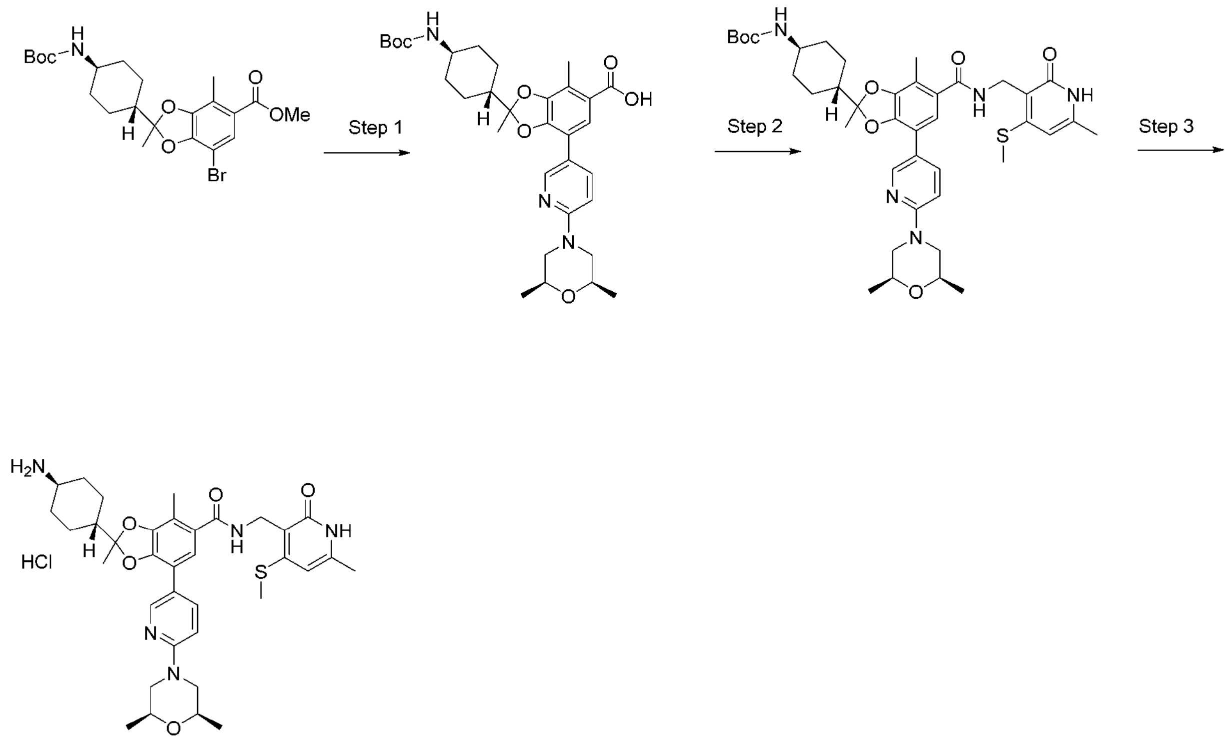


**[Step 1] Synthesis of 2-((trans-4-((tert-butoxycarbonyl)amino)cyclohexyl)-7-(6-((2S,6R)-2,6-dimethoxymorpholino)pyridin-3-yl)-2,4-dimethylbenzo[d][1,3] dioxole-5-carboxylic acid**

Dioxane (50 mL) was added to methyl 7-bromo-2-((trans-4-((tert-butoxycarbonyl)amino)cyclohexyl)-2,4-dimethylbenzo[d][1,3]dioxole-5-carboxylate (10 g, 20.64 mmole), and purified water (25 mL) was added thereto. Subsequently, (2S,6R)-2,6-dimethyl-4-(5-(4,4,5,5-tetramethyl-1,3,2-dioxaborolan-2-yl)pyridin-2-yl)morpholine (8.54 g) was added thereto, tetrakis(triphenylphosphine)palladium (0.48 g) was added thereto, sodium carbonate (4.37 g) was added thereto, and the resulting solution was stirred at 90°C for four hours. When the reaction was completed, ethyl acetate (50 mL) was added to filter the precipitated solid using celite, and washed with ethyl acetate (25 mL). After concentration under reduced pressure, ethyl acetate (80 mL) and purified water (80 mL) were injected and stirred for 30 minutes. The reaction solution was allowed to stand to separate layers, and anhydrous sodium sulfate and activated carbon were added to an organic layer and stirred for one hour. The reaction solution was filtered and then concentrated to obtain methyl 2-(trans-4-((tert-butoxycarbonyl)amino)cyclohexyl)-7-(6-((2S,6R)-2,6-dimethoxymorpholino)pyridin-3-yl)-2,4-dimethylbenzo[d][1,3]dioxole-5-carboxylate intermediate, which was then subjected to the following reaction without a separate purification process.

The obtained intermediate was dissolved in tetrahydrofuran (72 mL) and methanol (36 mL), and then a 2 N sodium hydroxide aqueous solution (46.5 mL) was injected thereto and stirred under reflux for four hours. The reaction product was concentrated under reduced pressure, ethyl acetate (70 mL) and purified water (30 mL) were injected into the residue, and the pH was adjusted to 4.0 with a 20% hydrochloric acid aqueous solution (13 mL). The reaction product was allowed to stand and separated into layers, and anhydrous sodium sulfate and activated carbon were injected into an organic layer and stirred for one hour. The reaction product was filtered and washed with dichloromethane (20 mL). The filtered organic material was concentrated under reduced pressure, and acetonitrile (90 mL) was injected into the residue, stirred under reflux for two hours, and cooled. The resulting solid was filtered, washed with acetonitrile (30 mL), and dried under reduced pressure to obtain 2-(trans-4-((tert-butoxycarbonyl)amino)cyclohexyl)-7-(6-((2S,6R)-2,6-dimethoxymorpholino)pyridin-3-yl)-2,4-dimethylbenzo[d][1,3]dioxole-5-carboxylic acid (9.9 g, 17.01 mmol) as the title compound.

**[Step 2] Synthesis of tert butyl(trans-4-(7-(6-((2S,6R)-2,6-dimethoxymorpholino)pyridin-3-yl)-2,4-dimethyl-5-(((6-methyl-4-(methylthio)-2-oxo-1,2-dihydropyridin-3-yl)methyl)carbamoyl)benzo[d][1,3]dioxole-2-yl) cyclohexyl)carbamate**

Dimethylformamide (18 mL) and triethylamine (9.5 mL) were added to the compound (9.9 g, 17.01 mmole) obtained in above [Step 1]. Then, pentafluorophenyl trifluoroacetate (3.04 mL) was added dropwise thereto and stirred at room temperature for three hours. Triethylamine (4.73 mL) was added to the reaction solution, and 3-(aminomethyl)-6-methyl-4-(methylthio)pyridin-2(1H)-one hydrochloride (4.5 g) was added and stirred at an internal temperature of 50°C. When the reaction was completed, the resulting mixture was concentrated under reduced pressure, the concentrated residue was dissolved in dichloromethane (40 mL) and purified water (40 mL) to separate an aqueous layer, purified water (30 mL) was injected into an organic layer, and the pH was adjusted to 3.5 with a 20% hydrochloric acid aqueous solution to separate the layers, thereby separating the organic layer. Anhydrous sodium sulfate and activated carbon were injected to the separated organic layer and stirred for one hour. The reaction solution was filtered, washed with dichloromethane (15 mL), and concentrated to perform the reaction of [Step 3] without a separate purification process.

**[Step 3] Synthesis of 2-(trans-4-aminocyclohexyl)-7-(6-((2S,6R)-2,6-dimethoxymorpholino)pyridin-3-yl)-2,4-dimethyl-N-((6-methyl-4-(methylthio)-2-oxo-1,2-dihydropyridin-3-yl)methyl)benzo[d][1,3]dioxole-5-carboxamide hydrochloride**

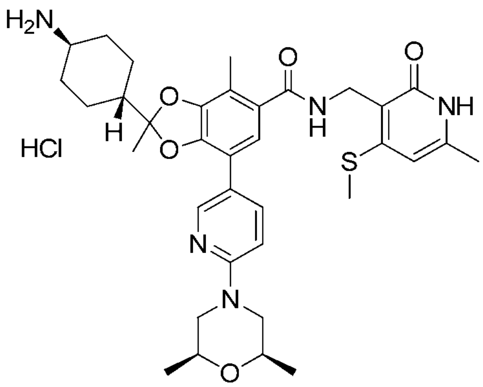

1 M-hydrochloric acid (ethanol aqueous solution, 100 mL) was added to the compound obtained in above [Step 2], and the resulting mixture was heated at 70°C and stirred. When the reaction was completed, the reaction solution was added dropwise to ethyl acetate (338 mL), and the resulting solid was filtered and dried at an internal temperature of 60°C to obtain the title compound (11.0 g, 16.98 mmol).

$^1H$ NMR (DMSO-$d_6$): 11.5 (s, 1H), 8.44 (s, 1H), 8.16 (br. s, 2H), 7.95 (t, 1H), 7.81 (d, 1H), 7.03 (s, 1H), 6.89 (d, 1H), 6.05 (s, 1H), 4.26 (d, 2H), 4.14 (d, 2H), 3.55-3.57 (m, 2H), 2.89-2.93 (m, 1H), 2.41 (s, 3H), 2.34-2.39 (m, 2H), 2.17 (s, 3H), 2.13 (s, 3H), 1.95-2.01 (m, 2H), 1.81-1.90 (m, 3H), 1.55 (s, 3H), 1.28-1.39 (m, 2H), 1.18-1.28 (m, 2H), 1.13 (s, 3H), 1.11 (s, 3H).

LC-MS (ESI, m/z) = 648.6 ($M+H^+$)

**Preparation Example 101. Synthesis of 7-(6-((2S,6R)-2,6-dimethylmorpholino)pyridin-3-yl)-2-(trans-4-(3-hydroxyazetidin-1-yl)cyclohexyl)-2,4-dimethyl-N-((6-methyl-4-(methylthio)-2-oxo-1,2-dihydropyridin-3-yl)methyl) benzo[d][1,3]dioxole-5-carboxamide (compound 101)**

<Reaction Formula 6>

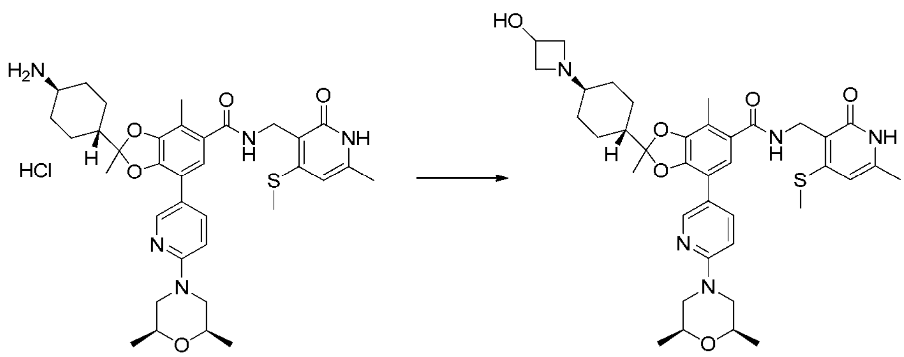

Dichloromethane and purified water were added to the compound (8 g, 12.3 mmole) obtained in [Step 3] of above Preparation Example 100. A 2 N-sodium hydroxide aqueous solution was added to adjust the pH 7 to 8 and separated into layers, and an oil layer was dried with sodium sulfate. After filtration and concentration, ethanol was added, and (R)-epichlorohydrin was added and heated. When the reaction was completed, calcium carbonate was added after cooling, heated to perform the reaction, cooled, filtrated, and separated into layers with dichloromethane and purified water. An oil layer was dried over sodium sulfate, filtered, concentrated, purified via column, and recrystallized with dichloromethane and ethyl acetate to obtain the title compound (5.4 g, 7.7 mmole).

$^1$H NMR (DMSO-$d_6$): 11.5 (s, 1H), 8.43 (s, 1H), 7.94 (t, 1H), 7.79 (d, 1H), 7.02 (s, 1H), 6.89 (d, 1H), 6.05 (s, 1H), 5.25 (br. s, 1H), 4.27 (d, 2H), 4.14 (d, 2H), 4.09-4.13 (m, 1H), 3.51-3.60 (m, 2H), 3.40-3.51 (m, 2H), 2.68-2.74 (m, 3H), 2.41 (s, 3H), 2.36-2.40 (m, 2H), 2.15 (s, 3H), 2.13 (s, 3H), 1.78-1.87 (m, 3H), 1.73-1.75 (m, 2H), 1.54 (s, 3H), 1.28-1.21 (m, 2H), 1.13 (s, 3H), 1.12 (s, 3H), 0.87-0.81 (m, 2H).

LC-MS (ESI, m/z) = 704.6 (M+H$^+$)

**Preparation Example 102. Synthesis of 7-(6-((2S,6R)-2,6-dimethylmorpholino)pyridin-3-yl)-2,4-dimethyl-N-((6-methyl-4-(methylthio)-2-oxo-1,2-dihydropyridin-3-yl)methyl)-2-(trans-4-(3-(2,2,2-trifluoroethyl)azetidin-1-yl)cyclohexyl)benzo[d][1,3]dioxole-5-carboxamide (compound 102)**

<Reaction Formula 7>

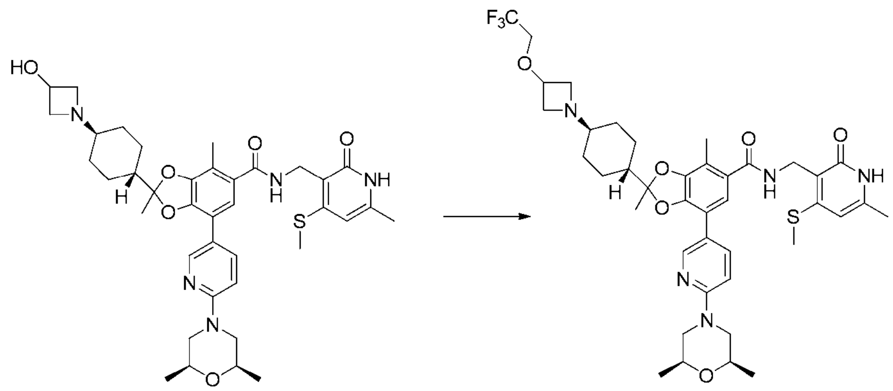

Dimethylformamide (20 mL) was added to the compound (300 mg, 0.42 mmole) obtained in above Preparation Example 101. Cesium carbonate (347 mg) and 2,2,2-trifluoroethyl trifluoromethanesulfonate (99 mg) were injected, stirred at 90°C for 15 hours, and distilled. The resulting mixture was separated into layers with dichloromethane and purified water, dried over anhydrous sodium sulfate, filtered, washed, and distilled under reduced pressure. Purification via column chromatography was performed to obtain the title compound (135 mg).

$^1$H NMR (DMSO-$d_6$): 11.5 (s, 1H), 8.42 (s, 1H), 8.09 (t, 1H), 7.76 (d, 1H), 7.01 (s, 1H), 6.90 (d, 1H), 6.89 (s, 1H), 4.88-4.92 (m, 2H), 4.38-4.39 (m, 2H), 4.30-4.31 (m, 1H), 4.20-4.30 (m, 2H), 4.13-4.15 (m, 2H), 3.55-3.57 (m, 3H), 2.60-2.66 (m, 2H), 2.36-2.43 (m, 2H), 2.35 (s, 3H), 2.16 (s, 3H), 1.79-1.81 (m, 7H), 1.55 (s, 3H), 1.13 (s, 3H), 1.12 (s, 6H), 1.01-1.08 (m, 2H).

LC-MS (ESI, m/z) = 786.6 (M+H$^+$)

**Preparation Example 103. Synthesis of 2-(trans-4-(3-(2,2-difluoroethoxy)azetidin-1-yl)cyclohexyl)-7-(6-((2S,6R)-2,6-dimethylmorpholino)pyridin-3-yl)-2,4-dimethyl-N-((6-methyl-4-(methylthio)-2-oxo-1,2-dihydropyridin-3-yl)methyl)benzo[d][1,3]dioxole-5-carboxamide (compound 103)**

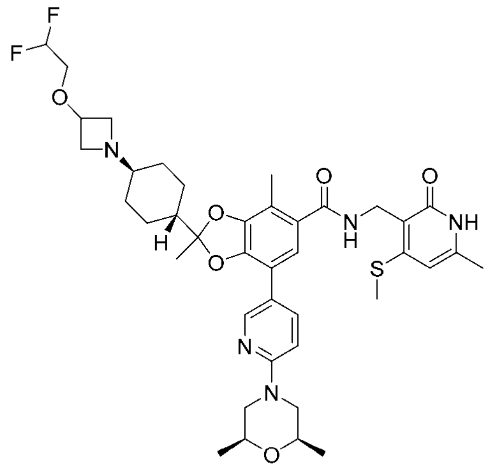

The same reaction as in Preparation Example 102 was performed by using the compound (300 mg, 0.43 mmole) obtained in above Preparation Example 101 except that 2-iodo-1,1-difluoroethane (0.04 mL) was used instead of 2,2,2-trifluoroethyl trifluoromethanesulfonate in Preparation Example 102 to obtain the title compound (71 mg, 0.09 mmole).

$^1$H NMR (DMSO-$d_6$): 11.5 (s, 1H), 8.42 (s, 1H), 8.06 (t, 1H), 7.77-7.79 (m, 1H), 7.01 (s, 1H), 6.90 (d, 1H), 6.85 (s, 1H), 6.33 (t, 1H), 6.05 (s, 1H), 4.50-4.51 (m, 2H), 4.30-4.38 (m, 3H), 4.13-4.15 (m, 2H), 4.06-4.07 (m, 1H), 3.55-3.57 (m, 2H), 3.43-3.45 (m, 2H), 2.60-2.66 (m, 2H), 2.45-2.47 (m, 2H), 2.36-2.43 (m, 2H), 2.35 (s, 3H), 2.15 (s, 3H), 1.79-1.81 (m, 1H), 1.75-1.78 (m, 4H), 1.72-1.75 (m, 2H), 1.54 (s, 3H), 1.13 (s, 3H), 1.12 (s, 6H), 0.83-0.86 (m, 2H).

LC-MS (ESI, m/z) = 768.7 (M+H$^+$)

**Preparation Example 104. Synthesis of 2-(trans-4-(3-(2,2-difluoroethoxy)azetidin-1-yl)cyclohexyl)-7-(6-((2S,6R)-2,6-dimethylmorpholino)pyridin-3-yl)-2,4-dimethyl-N-((6-methyl-4-(methylthio)-2-oxo-1,2-dihydropyridin-3-yl)methyl)benzo[d][1,3]dioxole-5-carboxamide (compound 104)**

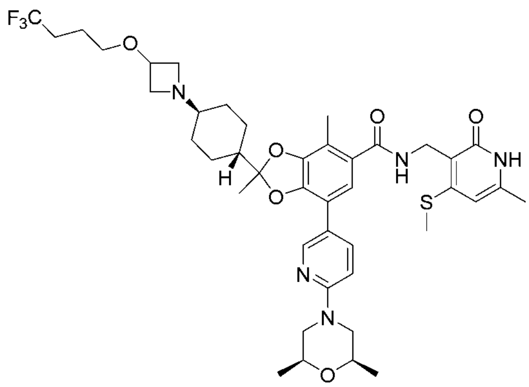

The same reaction as in Preparation Example 102 was performed by using the compound (300 mg, 0.43 mmole) obtained in above Preparation Example 101 except that 1,1,1-trifluoro-4-iodobutane (0.06 mL) was used instead of 2,2,2-trifluoroethyl trifluoromethanesulfonate in Preparation Example 102 to obtain the title compound (71 mg, 0.09 mmole).

$^1$H NMR (DMSO-$d_6$): 11.6 (s, 1H), 8.44 (s, 1H), 7.93 (t, 1H), 7.78-7.81 (m, 1H), 7.01 (d, 1H), 6.89 (d, 1H), 6.78 (s, 1H), 4.36-4.39 (m, 2H), 4.29-4.30 (m, 2H), 4.26-4.29 (m, 2H), 4.13-4.15 (m, 2H), 3.95-3.97 (m, 2H), 3.55-3.57 (m, 3H), 2.60-2.66 (m, 4H), 2.43-2.46 (m, 3H), 2.35-2.38 (m, 2H), 2.43 (s, 3H), 2.15 (s, 3H), 1.78-1.87 (m, 4H), 1.76-1.77 (m, 1H), 1.55 (s, 3H), 1.13 (s, 3H), 1.12 (s, 6H), 1.01-1.08 (m, 2H).

LC-MS (ESI, m/z) = 814.7 (M+H$^+$)

**Preparation Example 105. Synthesis of 7-(6-((2S,6R)-2,6-dimethylmorpholino)pyridin-3-yl)-2-(trans-4-(3-isopropoxyazetidin-1-yl)cyclohexyl)-2,4-dimethyl-N-((6-methyl-4-(methylthio)-2-oxo-1,2-dihydropyridin-3-yl)methyl)benzo[d][1,3]dioxole-5-carboxamide (compound 105)**

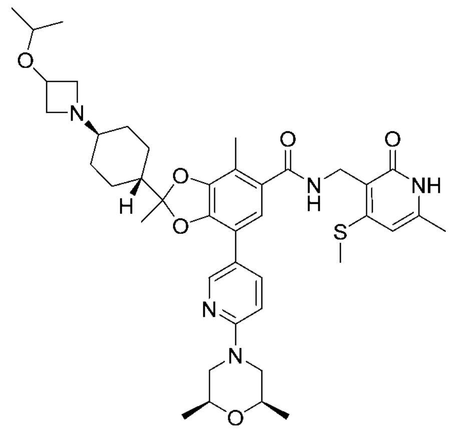

The same reaction as in Preparation Example 102 was performed by using the compound (300 mg, 0.43 mmole) obtained in above Preparation Example 101 except that 2-iodopropane (0.04 mL) was used instead of 2,2,2-trifluoroethyl trifluoromethanesulfonate in Preparation Example 102 to obtain the title compound (56 mg, 0.08 mmole).

$^1$H NMR (DMSO-$d_6$): 11.5 (s, 1H), 8.42 (s, 1H), 8.00 (t, 1H), 7.77 (d, 1H), 7.00 (s, 1H), 6.90 (d, 1H), 6.72 (s, 1H), 5.20-5.23 (m, 2H), 4.34 (d, 2H), 4.30-4.31 (m, 1H), 4.14 (d, 2H), 4.06-4.08 (m, 1H), 3.56-3.57 (m, 2H), 2.43-2.46 (m, 3H), 2.34-2.42 (m, 2H), 2.30 (s, 3H), 2.16 (s, 3H), 1.79-1.87 (m, 1H), 1.78-1.87 (m, 4H), 1.72-1.74 (m, 2H), 1.54 (s, 3H), 1.23 (s, 3H), 1.22 (s, 3H), 1.13 (s, 3H), 1.12 (s, 6H), 0.83-0.86 (m, 2H).

LC-MS (ESI, m/z) = 746.7 (M+H$^+$)

**Preparation Example 106. Synthesis of 7-(6-((2S,6R)-2,6-dimethylmorpholino)pyridin-3-yl)-2-(trans-4-(3-methoxyazetidin-1-yl)cyclohexyl)-2,4-dimethyl-N-((6-methyl-4-(methylthio)-2-oxo-1,2-dihydropyridin-3-yl)methyl)benzo[d][1,3]dioxole-5-carboxamide (compound 106)**

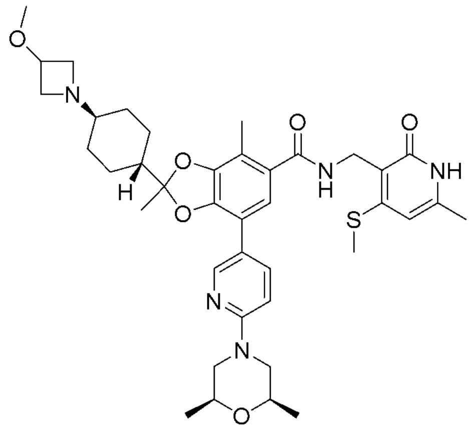

The same reaction as in Preparation Example 102 was performed by using the compound (300 mg, 0.43 mmole) obtained in above Preparation Example 101 except that iodomethane (0.03 mL) was used instead of 2,2,2-trifluoroethyl trifluoromethanesulfonate in Preparation Example 102 to obtain the title compound (10 mg, 0.02 mmole).

$^1$H NMR (DMSO-$d_6$): 11.5 (s. 1H), 8.43 (s, 1H), 7.94 (t, 1H), 7.80 (d, 1H), 7.02 (s, 1H), 6.89 (d, 1H), 6.05 (s, 1H), 4.24-4.27 (d, 2H), 4.14 (d, 2H), 3.45-3.60 (m, 2H), 3.36-3.37 (m, 1H), 2.86-2.90 (m, 2H), 2.61-2.64 (m, 2H), 2.58-2.59 (m, 1H), 2.41 (s, 3H), 2.34-2.39 (m, 2H), 2.28-2.33 (m, 2H), 2.18 (s, 3H), 2.16 (s, 3H), 2.13 (s, 3H), 1.84-1.89 (m, 2H), 1.72-1.82 (m, 3H), 1.55 (s, 3H), 1.14-1.20 (m, 2H), 1.13 (s, 3H), 1.12 (s, 3H).

LC-MS (ESI, m/z) = 718.5 (M+H$^+$)

**Preparation Example 107. Synthesis of stereoisomer B of 7-(6-((2S,6R)-2,6-dimethylmorpholino)pyridin-3-yl)-2,4-dimethyl-2-(trans-4-(methyl(2,2,2-trifluoroethyl)amino)cyclohexyl)-N-((6-methyl-4-(methylthio)-2-oxo-1,2-dihydropyridin-3-yl)methyl)benzo[d][1,3]dioxole-5-carboxamide (compound 107)**

Stereoisomer B of

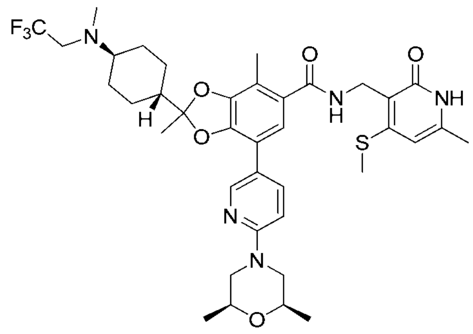

(

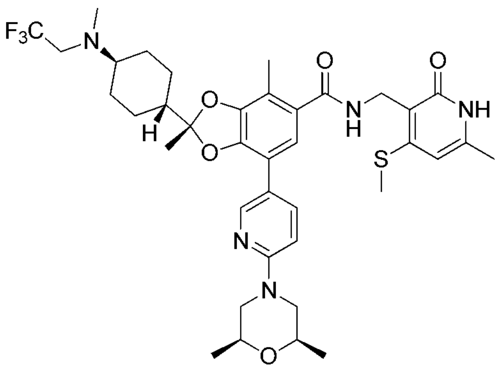

or

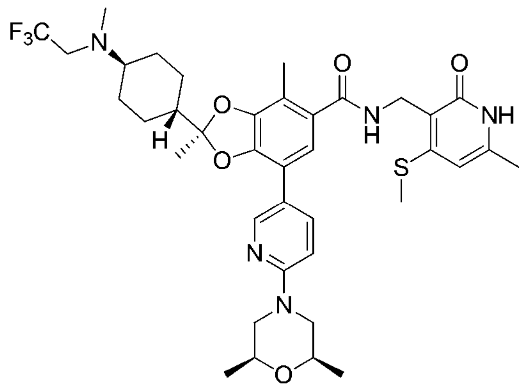

)

The same reaction as in [Step 1] to [Step 7] of Preparation Example 20 was performed except that methyl-(R)-7-bromo-2-(trans-4-((tert-butoxycarbonyl)amino)cyclohexyl)-2,4-dimethylbenzo[d][1,3]dioxole-5-carboxylate (0.5 g, 1.03 mmole), which is stereospecific, was used as a starting material in [Step 1] of above Preparation Example 20 instead of methyl-7-bromo-2-(trans-4-((tert-butoxycarbonyl)amino)cyclohexyl)-2,4-dimethylbenzo[d][1,3]dioxole-5-carboxylate, so as to obtain the title compound (0.2 g, 0.27 mmole).

$^1$H NMR (DMSO-$d_6$): 11.5 (s, 1H), 8.29 (d, 2H), 8.09 (s, 1H), 7.51 (d, 1H), 7.15 (s, 1H), 6.12 (s, 1H), 4.36 (d, 2H), 4.29 (s, 2H), 4.12-4.13 (m, 1H), 3.63-3.64 (m, 2H), 3.23-3.24 (m, 1H), 2.76-2.78 (m, 6H), 2.75 (s, 3H), 2.42 (s, 3H), 2.22 (s, 3H), 2.17-2.21 (m, 2H), 1.98 (s, 3H), 1.59 (s, 3H), 1.56-1.58 (m, 2H), 1.21-1.41 (m, 2H), 1.14 (s, 3H), 1.13 (s, 3H).

LC-MS (ESI, m/z) = 744.8 (M+H$^+$)

**Preparation Example 108. Synthesis of stereoisomer A of 7-(6-((2S,6R)-2,6-dimethylmorpholino)pyridin-3-yl)-2,4-dimethyl-2-(trans-4-(methyl(2,2,2-trifluoroethyl)amino)cyclohexyl)-N-((6-methyl-4-(methylthio)-2-oxo-1,2-dihydropyridin-3-yl)methyl)benzo[d][1,3]dioxole-5-carboxamide (compound 108)**

Stereoisomer A of

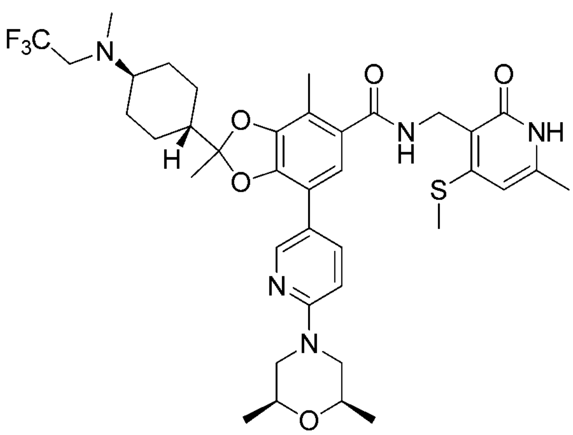

(

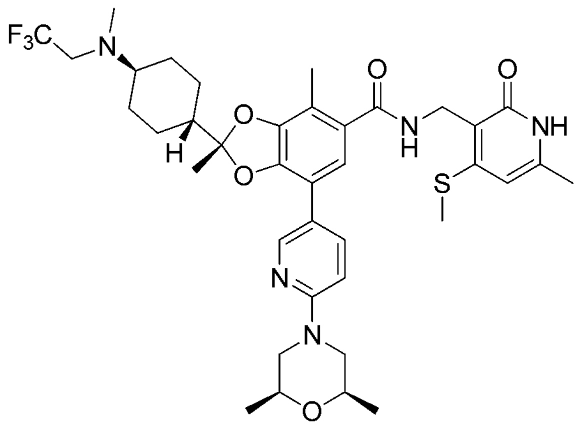

or

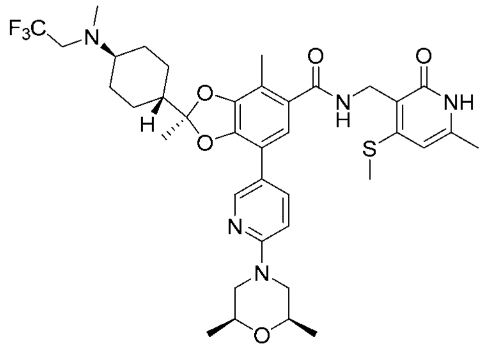

)

The same reaction as in [Step 1] to [Step 7] of Preparation Example 20 was performed except that methyl-(S)-7-bromo-2-(trans-4-((tert-butoxycarbonyl)amino)cyclohexyl)-2,4-dimethylbenzo[d][1,3]dioxole-5-carboxylate (0.5 g, 1.03 mmole), which is stereospecific, was used as a starting material in [Step 1] of above Preparation Example 20 instead of methyl-7-bromo-2-(trans-4-((tert-butoxycarbonyl)amino)cyclohexyl)-2,4-dimethylbenzo[d][1,3]dioxole-5-carboxylate, so as to obtain the title compound (0.2 g, 0.27 mmole).

$^1H$ NMR (DMSO-$d_6$): 11.5 (s, 1H), 8.29 (d, 2H), 8.09 (s, 1H), 7.50 (d, 1H), 7.15 (s, 1H), 6.12 (s, 1H), 4.36 (d, 2H), 4.29 (s, 2H), 4.12-4.13 (m, 1H), 3.64-3.67 (m, 2H), 3.21-3.23 (m, 1H), 2.76-2.75 (m, 6H), 2.43 (s, 3H), 2.20 (s, 3H), 2.19 (s, 3H), 2.16-2.18 (m, 2H), 1.97 (s, 3H), 1.59 (s, 3H), 1.42-1.58 (m, 2H), 1.21-1.32 (m, 2H), 1.14 (s, 3H), 1.13 (s, 3H).

LC-MS (ESI, m/z) = 744.8 (M+H$^+$)

**<u>Preparation Example 109. Synthesis of 2-(trans-4-((2,2-difluoroethyl(methyl)amino)cyclohexyl)-7-(6-((2S,6R)-2,6-dimethylmorpholino)pyridin-3-yl)-2,4-dimethyl-N-((6-methyl-4-(methylthio)-2-oxo-1,2-dihydropyridin-3-yl)methyl)benzo[d][1,3]dioxole-5-carboxamide (compound 109)</u>**

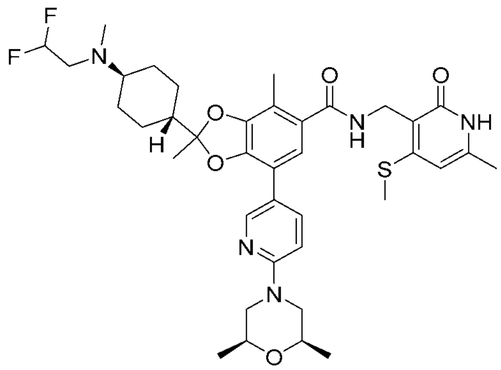

The reaction as in [Step 7] was performed by using the compound (0.5 g, 0.68 mmole) obtained through the same reaction as in [Steps 1 to 6] of above Preparation Example 20, except that 2-iodo-1,1-difluoroethane (0.12 ml) was used instead of 2,2,2-trifluoroethyl trifluoromethanesulfonate, so as to obtain the title compound (0.2 g).

$^1H$ NMR (DMSO-$d_6$): 11.5 (s, 1H), 8.44 (s, 1H), 7.94 (t, 1H), 7.80 (d, 1H), 7.02 (s, 1H), 6.89 (d, 1H), 6.04 (s, 1H), 5.91 (t, 1H), 4.27 (d, 2H), 4.13 (d, 2H), 3.52-3.57 (m, 2H), 2.66-2.73 (m, 2H), 2.41 (s, 3H), 2.36-2.40 (m, 2H), 2.30-2.36 (m, 1H), 2.22 (s, 3H), 2.16 (s, 3H), 2.13 (s, 3H), 1.85-1.88 (m, 2H), 1.76-1.85 (m, 1H), 1.68-1.72 (m, 2H), 1.54 (s, 3H), 1.14-1.18 (m, 4H), 1.13 (s, 3H), 1.12 (s, 3H).

LC-MS (ESI, m/z) = 726.6 (M+H$^+$)

**Preparation Example 110. Synthesis of 7-(6-((2S,6R)-2,6-dimethylmorpholino)pyridin-3-yl)-2-(trans-4-(isopropyl(methyl)amino)cyclohexyl)-2,4-dimethyl-N-((6-methyl-4-(methylthio)-2-oxo-1,2-dihydropyridin-3-yl)methyl)benzo[d][1,3]dioxole-5-carboxamide (compound 110)**

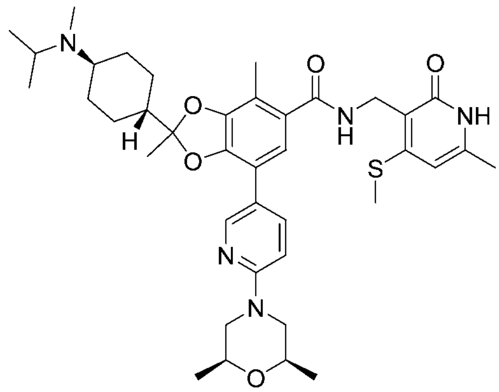

The reaction as in [Step 7] was performed by using the compound (0.5 g, 0.68 mmole) obtained through the same reaction as in [Steps 1 to 6] of above Preparation Example 20, except that 2-iodopropane (0.14 ml) was used instead of 2,2,2-trifluoroethyl trifluoromethanesulfonate, so as to obtain the title compound (0.1 g).

$^1$H NMR (DMSO-$d_6$): 11.5 (s, 1H), 8.44 (s, 1H), 7.94 (t, 1H), 7.81 (d, 1H), 7.02 (s, 1H), 6.89 (d, 1H), 6.05 (s, 1H), 4.27 (d, 2H), 4.14 (d, 2H), 3.54-3.59 (m, 2H), 2.86-2.90 (m, 1H), 2.41 (s, 3H), 2.28-2.40 (m, 2H), 2.32-2.40 (m, 1H4), 2.16 (s, 3H), 2.13 (s, 3H), 2.05 (s, 3H), 1.84-1.88 (m, 2H), 1.72-1.84 (m, 3H), 1.54 (s, 3H), 1.14-1.23 (m, 4H), 1.13 (s, 3H), 1.12 (s, 3H), 0.90 (s, 3H), 0.89 (s, 3H).

LC-MS (ESI, m/z) = 704.7 (M+H$^+$)

**Preparation Example 111. Synthesis of 7-(6-((2S,6R)-2,6-dimethylmorpholino)pyridin-3-yl)-2-(trans-4-(ethyl(methyl)amino)cyclohexyl)-2,4-dimethyl-N-((6-methyl-4-(methylthio)-2-oxo-1,2-dihydropyridin-3-yl)methyl)benzo[d][1,3]dioxole-5-carboxamide (compound 111)**

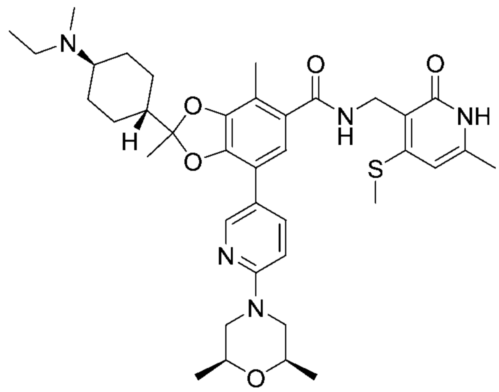

The reaction as in [Step 7] was performed by using the compound (0.5 g, 0.68 mmole) obtained through the same reaction as in [Steps 1 to 6] of above Preparation Example 20, except that 2-iodoethane (0.11 ml) was used instead of 2,2,2-trifluoroethyl trifluoromethanesulfonate, so as to obtain the title compound (0.1 g).

$^1$H NMR (DMSO-$d_6$): 11.5 (s, 1H), 8.46 (s, 1H), 7.94 (t, 1H), 7.81 (d, 1H), 7.04 (s, 1H), 6.89 (d, 1H), 6.05 (s, 1H), 4.26 (d, 2H), 4.14 (d, 2H), 3.53-4.57 (m, 2H), 2.62 (d, 3H), 2.41 (s, 3H), 2.36-2.40 (m, 2H), 2.34-2.36 (m, 1H), 2.17 (s, 3H), 2.13 (s, 3H), 1.87-1.1.97 (m, 3H), 1.57 (s ,3H),1.43-1.52 (m, 2H), 1.20-1.25 (m, 2H), 1.16-1.20 (m, 4H), 1.13 (s, 3H), 1.12 (s, 3H), 0.83 (t, 3H).

LC-MS (ESI, m/z) = 690.6 (M+H$^+$)

**Preparation Example 112. Synthesis of 7-(6-((2S,6R)-2,6-dimethylmorpholino)pyridin-3-yl)-2,4-dimethyl-2-(trans-4-(methyl(phenethyl)amino)cyclohexyl)-N-((6-methyl-4-(methylthio)-2-oxo-1,2-dihydropyridin-3-yl)methyl)benzo[d][1,3]dioxole-5-carboxamide (compound 112)**

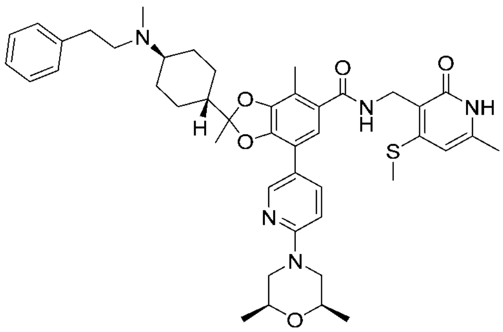

The reaction as in [Step 7] was performed by using the compound (0.5 g, 0.68 mmole) obtained through the same reaction as in [Steps 1 to 6] of above Preparation Example 20, except that 2-iodoethylbenzene (0.20 mL) was used instead of 2,2,2-trifluoroethyl trifluoromethanesulfonate, so as to obtain the title compound (0.2 g).

$^1$H NMR (DMSO-$d_6$): 11.5 (s, 1H), 8.43 (s, 1H), 7.94 (t, 1H), 7.80 (d, 1H), 7.18-7.22 (m, 2H), 7.15-7.18 (m, 3H), 7.01 (s, 1H), 6.90 (d, 1H), 6.05 (s, 1H), 4.27 (d, 2H), 4.14 (d, 2H), 3.54-2.59 (m, 2H), 2.51-2.63 (m, 2H), 2.45 (s, 3H), 2.41 (s, 3H), 2.36-2.40 (m, 2H), 2.34-2.36 (m, 1H), 2.18-2.22 (m, 2H), 2.16 (s, 3H), 2.13 (s, 3H), 1.81-1.88 (m, 2H), 1.70-1.78 (m, 3H), 1.54 (s, 3H), 1.16-1.29 (m, 4H), 1.13 (s, 3H), 1.12 (s, 3H).

LC-MS (ESI, m/z) = 766.7 (M+H$^+$)

**Preparation Example 113. Synthesis of 7-(6-((2S,6R)-2,6-dimethylmorpholino)pyridin-3-yl)-2,4-dimethyl-2-(trans-4-(methyl(phenethyl)amino)cyclohexyl)-N-((6-methyl-4-(methylthio)-2-oxo-1,2-dihydropyridin-3-yl)methyl)benzo[d][1,3]dioxole-5-carboxamide (compound 113)**

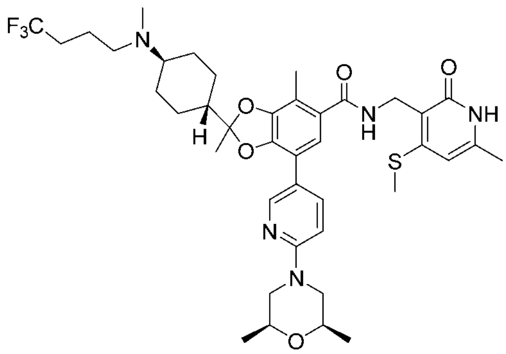

The reaction as in [Step 7] was performed by using the compound (0.5 g, 0.68 mmole) obtained through the same reaction as in [Steps 1 to 6] of above Preparation Example 20, except that 1,1,1-trifluoro4-iodobutane (0.17 mL) was used instead of 2,2,2-trifluoroethyl trifluoromethanesulfonate, so as to obtain the title compound (0.2 g).

$^1$H NMR (DMSO-$d_6$): 11.5 (s, 1H), 8.45 (s, 1H), 7.94 (t, 1H), 7.81 (d, 1H), 7.03 (s, 1H), 6.89 (d, 1H), 6.05 (s, 1H), 4.27 (d, 2H), 4.14 (d, 2H), 3.53-3.57 (m, 2H), 2.41 (s, 3H), 2.34-2.41 (m, 4H), 2.31-2.34 (m, 1H), 2.17 (s, 3H), 2.13 (s, 3H), 1.89-1.95 (m, 3H), 1.67-1.80 (m, 4H), 1.66-1.68 (m, 1H), 1.61-1.65 (m, 2H), 1.57 (s, 3H), 1.42-1.48 (m, 2H), 1.20-1.34 (m, 4H), 1.13 (s, 3H), 1.12 (s, 3H).

LC-MS (ESI, m/z) = 772.7 (M+H$^+$)

**Preparation Example 114. Synthesis of 2-(trans-4-(cyclohexyl(methyl)amino)cyclohexyl)-7-(6-((2S,6R)-2,6-dimethylmorpholino)pyridin-3-yl)-2,4-dimethyl-N-((6-methyl-4-(methylthio)-2-oxo-1,2-dihydropyridin-3-yl)methyl)benzo[d][1,3]dioxole-5-carboxamide (compound 114)**

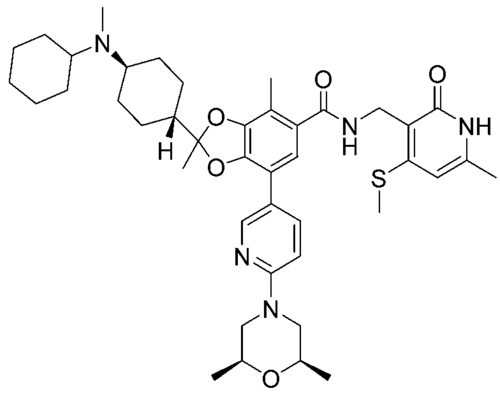

The reaction as in [Step 7] was performed by using the compound (0.5 g, 0.68 mmole) obtained through the same reaction as in [Steps 1 to 6] of above Preparation Example 20, except that cyclohexyl iodide (0.18 mL) was used instead of 2,2,2-trifluoroethyl trifluoromethanesulfonate, so as to obtain the title compound (0.05 g).

$^1$H NMR (DMSO-$d_6$): 11.5 (s, 1H), 8.45 (s, 1H), 7.95 (t, 1H), 7.82 (d, 1H), 7.01 (s, 1H), 6.90 (d, 1H), 6.05 (s, 1H), 4.27 (d, 2H), 4.14 (d, 2H), 3.54-3.58 (m, 2H), 3.38-3.41 (m, 2H), 3.10-3.12 (m, 2H), 2.93-3.60 (m, 2H), 2.41 (s, 3H), 2.34-2.41 (m, 2H), 2.31-2.34 (m, 1H), 2.17 (s, 3H), 2.13 (s, 3H), 1.89-1.95 (s, 3H), 1.67-1.80 (m, 4H), 1.66-1.68 (m, 1H), 1.61-1.65 (m, 2H), 1.57 (s, 3H), 1.55-1.56 (m, 2H), 1.42-1.48 (m, 2H), 1.28-1.34 (m, 2H), 1.13 (s, 3H), 1.12 (s, 3H).

LC-MS (ESI, m/z) = 744.7 (M+H$^+$)

**Preparation Example 115. Synthesis of 7-(6-((2S,6R)-2,6-dimethylmorpholino)pyridin-3-yl)-2,4-dimethyl-N-((6-methyl-4-(methylthio)-2-oxo-1,2-dihydropyridin-3-yl)methyl)-2-(trans-4-(2,2,2-trifluoro-N-methylacetamido)cyclohexyl)benzo[d][1,3]dioxole-5-carboxamide (compound 115)**

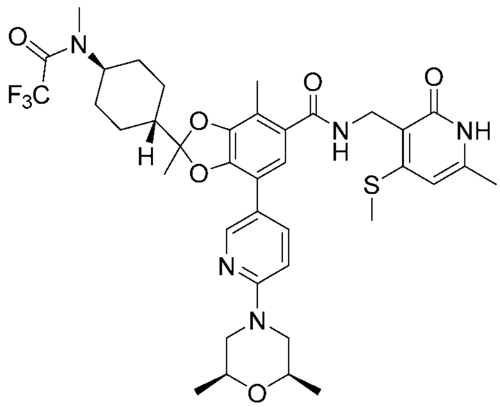

The reaction as in [Step 7] was performed by using the compound (0.5 g, 0.68 mmole) obtained through the same reaction as in [Steps 1 to 6] of above Preparation Example 20, except that phenyl trifluoroacetate (0.20 mL) was used instead of 2,2,2-trifluoroethyl trifluoromethanesulfonate, so as to obtain the title compound (0.2 g).

$^1$H NMR (DMSO-$d_6$): 11.5 (s, 1H), 8.45 (s, 1H), 7.95 (t, 1H), 7.82 (d, 1H), 7.04 (s, 1H), 6.90 (d, 1H), 6.05 (s, 1H), 4.27 (d, 2H), 4.14 (d, 2H), 3.53-3.57 (m, 2H), 2.89-2.91 (m, 2H), 2.41 (s, 3H), 2.34-2.41 (m, 3H), 2.17 (s, 3H), 2.13 (s, 3H), 1.89-1.95 (m, 3H), 1.66-1.68 (m, 1H), 1.61-1.65 (m, 2H), 1.57 (s, 3H), 1.20-1.34 (m, 4H), 1.13 (s, 3H), 1.12 (s, 3H).

LC-MS (ESI, m/z) = 758.6 (M+H$^+$)

**<Experimental Example>**

The cell line used in the experiment was purchased from American Type Culture Collection (ATCC).

**Experimental Example 1. Evaluation of inhibitory activity against EZH1 and EZH2**

In order to confirm the EZH1 and/or EZH2 activity inhibitory effect of the 1,3-benzodioxole derivative compounds represented by chemical formula I of the present invention, the activity was measured using an AlphaLISA test method.
The test substance dissolved in dimethylsulfoxide (DMSO) was diluted in a test solution (20 mM Tris pH 8.0, 0.1% BSA, 0.01% Triton X-100, 0.5 mM DTT) according to a final treatment concentration (5 nM or 10 nM) and mixed with EZH1 and EZH2, respectively, and then histone H3 peptide and S-adenosylmethionine (SAM) were added to initiate an enzyme reaction. An acceptor bead was added thereto after reaction for two hours and subjected to an additional reaction for one hour, after which a donor bead was added thereto. After an additional reaction for 30 minutes, the reaction was completed. An activity was measured using the Magellan program on a Spark10M instrument. DMSO was used as a control group, an inhibition rate of enzyme activity was calculated using a following equation, and the results thereof are shown in Table 1 below.

Activity inhibition rate (%) = (1-(Group treated with test substance /Control group))*100 <Calculation Formula>

[Table 1]

| | Enzyme inhibition % | | | | | Enzyme inhibition % | | | |
|---|---|---|---|---|---|---|---|---|---|
| | EZH1 | | EZH2 | | | EZH1 | | EZH2 | |
| Comp ound No. | 5nM | 10nM | 5nM | 10nM | Comp ound No. | 5nM | 10nM | 5nM | 10nM |
| **1** | 89.1 | | 98.7 | | **53** | 78.3 | 91.6 | 98.5 | 99.6 |
| **2** | 82.0 | | 92.9 | | **55** | 31.6 | 52.2 | 58.2 | 93.3 |
| **3** | 73.5 | | 66.5 | | **57** | 58.9 | 76.2 | 96.0 | 99.4 |
| **4** | 74.0 | | 77.4 | | **59** | 42.2 | 5.7 | 98.5 | 99.5 |
| **5** | 95.1 | | 99.5 | | **61** | 53.0 | 78.2 | 86.5 | 99.4 |
| **6** | 86.7 | | 98.2 | | **62** | 66.9 | 81.8 | 93.6 | 99.4 |
| **7** | 81.4 | | 96.0 | | **64** | 82.5 | 90.5 | 99.2 | 99.6 |
| **8** | 85.6 | | 96.1 | | **66** | 81.3 | 90.9 | 99.3 | 99.6 |
| **9** | 86.1 | | 98.6 | | **68** | 56.4 | 78.9 | 87.3 | 99.7 |
| **10** | 82.8 | | 98.7 | | **69** | 71.9 | 87.9 | 98.2 | 99.6 |
| **11** | 82.9 | | 98.9 | | **71** | 68.5 | 82.8 | 96.1 | 99.5 |
| **12** | 80.5 | | 99.2 | | **73** | 75.7 | 88.0 | 99.2 | 99.5 |
| **13** | 53.7 | | 97.6 | | **75** | 61.3 | 86.0 | 79.0 | 99.7 |
| **14** | 86.4 | | 97.6 | | **76** | 79.0 | 96.2 | 99.5 | 99.6 |
| **15** | 94.5 | | 99.8 | | **78** | 53.1 | 79.3 | 86.1 | 98.1 |
| **16** | 69.0 | | 92.4 | | **79** | 71.9 | 87.4 | 97.1 | 99.5 |
| **17** | 93.8 | | 99.3 | | **81** | 80.4 | 90.8 | 99.2 | 99.8 |
| **18** | 87.6 | | 99.8 | | **83** | 45.3 | 78.8 | 99.2 | 99.4 |
| **19** | 78.7 | | 98.7 | | **85** | 40.7 | 61.1 | 56.1 | 97.9 |
| **20** | 34.3 | | 26.1 | | **86** | 42.0 | 65.9 | 98.3 | 99.2 |
| **21** | 20.6 | | 11.3 | | **88** | 45.2 | 62.0 | 99.5 | 99.9 |
| **22** | 53.1 | | 81.0 | | **89** | 40.0 | 71.0 | 99.5 | 100 |
| **23** | 31.4 | | 34.2 | | **90** | 20.2 | 16.2 | 60.1 | 82.3 |

(continued)

| | Enzyme inhibition % | | | | | Enzyme inhibition % | | | |
|---|---|---|---|---|---|---|---|---|---|
| | EZH1 | | EZH2 | | | EZH1 | | EZH2 | |
| Comp ound No. | 5nM | 10nM | 5nM | 10nM | Comp ound No. | 5nM | 10nM | 5nM | 10nM |
| **24** | 35.1 | | 98.6 | | **91** | 24.5 | 41.0 | 47.4 | 71.6 |
| **25** | 82.4 | | 97.6 | | **92** | 27.2 | 43.2 | 80.1 | 91.8 |
| **26** | 87.3 | | 99.6 | | **93** | 16.8 | 12.7 | 32.2 | 37.9 |
| **27** | 80.2 | | 95.8 | | **94** | 25.3 | 28.5 | 65.1 | 82.0 |
| **28** | 77.3 | | 99.4 | | **95** | 38.1 | 46.0 | 83.2 | 91.1 |
| **29** | 91.6 | | 98.3 | | **96** | 19.2 | 14.8 | 58.4 | 78.0 |
| **30** | 80.4 | | 83.1 | | **97** | 25.9 | 20.5 | 74.7 | 82.3 |
| **31** | 67.3 | | 75.6 | | **98** | 30.9 | 17.6 | 52.2 | 48.9 |
| **32** | 74.1 | | 82.6 | | **99** | 37.1 | 42.9 | 62.4 | 76.5 |
| **33** | 37.6 | | 59.1 | | **100** | 30.0 | 68.1 | 96.3 | 100 |
| **34** | 24.9 | | 41.1 | | **101** | 38.8 | 78.1 | 98.7 | 100 |
| **35** | 67.7 | | 94.0 | | **103** | 10.7 | 20.5 | 27.1 | 72.5 |
| **36** | 57.4 | | 83.1 | | **104** | | 19.9 | 37.0 | 82.6 |
| **38** | 40.6 | | 57.4 | | **106** | 34.2 | 69.1 | 90.6 | 99.9 |
| **39** | 20.5 | | 62.0 | | **107** | 39.3 | | 41.7 | |
| **40** | 73.8 | | 94.6 | | **108** | 20.4 | | 4.3 | |
| **41** | 69.1 | | 96.8 | | **109** | 22.0 | 35.2 | 52.4 | 91.6 |
| **42** | 59.6 | | 90.5 | | **110** | 52.0 | 91.9 | 99.8 | 100 |
| **43** | 65.6 | | 94.6 | | **111** | 28.1 | 62.1 | 86.7 | 100 |
| **44** | 46.8 | 78.2 | 96.1 | 99.6 | **112** | 24.3 | 40.7 | 58.5 | 97.0 |
| **46** | 44.7 | 63.0 | 59.2 | 84.0 | **113** | 32.5 | 68.8 | 96.7 | 100 |
| **47** | 68.1 | 86.7 | 94.5 | 99.6 | **114** | 28.6 | 38.2 | 45.8 | 91.7 |
| **49** | 78.8 | 92.4 | 99.2 | 99.5 | **115** | 6.1 | 9.6 | 37.3 | 85.8 |
| **51** | 80.2 | 93.0 | 99.3 | 99.5 | | | | | |

As shown in above Table 1, it was confirmed that the compound of the present invention exhibits an excellent inhibitory effect on EZH1 and/or EZH2 enzyme activity.

## Experimental Example 2. Evaluation of inhibition of cancer cell proliferation

An inhibitory ability of the 1,3-benzodiazole derivative compounds represented by chemical formula I of the present invention on cancer cell proliferation was evaluated.

Among solid cancer cells, in G401 cells in which expression of SMARCB1, which is a member of switch/-sucrosenon-fermenting (SWI/SNF), is defective as a cell line of a rhabdoid tumor in which a therapeutic effect may be expected through dual inhibition of EZH1/2, and MKN-45 cells in which expression of ARID1A, which is a member of SWI/SNF, is defective as a gastric cancer cell line, a proliferation inhibitory ability of the 1,3-benzodioxole derivative compounds represented by chemical formula I of the present invention was evaluated.

In addition, as one example of solid cancer in which an overexpression of EZH2 and a consequent disease association therebetween is reported, a proliferation inhibitory activity of the 1,3-benzodioxole derivative compounds represented by chemical formula I of the present invention was evaluated in Lovo cells as a colorectal cancer cell line, SW-13 cells as an adrenal cancer cell line, MIA-PaCa-2 cells as a pancreatic cancer cell line, and NCI-H1299 cells as a lung cancer cell line.

Each cell line (G401 cell, MKN-45 cell, Lovo cell, SW-13 cell, MIA-PaCa-2, NCI-H1299) of Table 2 below was cultured using RPMI1640, McCoy's 5a, L-15 or DMEM containing 10% fetal bovine serum, and the cells were seeded into wells of a 96-well culture dish at 100 to 1,000 cells each, and a test substance 1000 nM was cultured for seven to 11 days after drug treatment. After the culture, the cells were measured according to a manufacturer's instructions using Cell Counting Kit-8, and a cell proliferation inhibitory effect (% inhibition rate) of the test substance was obtained. The results thereof are shown in Table 2 below.

The cell proliferation inhibitory effect (% inhibition rate) of the test substance was calculated using a following equation.

$$\% \text{ inhibition rate} = \{1-(\text{test group}/\text{control group})\} \text{ X } 100$$

[Table 2]

| | % inhibition rate | | | | | | | % inhibition rate | | | | | |
|---|---|---|---|---|---|---|---|---|---|---|---|---|---|
| | LoVo | G401 | MKN -45 | SW-13 | MIA-Paca2 | NCI-H129 9 | | LoVo | G401 | MKN -45 | SW-13 | MIA-Paca2 | NCI-H129 9 |
| Comp ound No. | 1000 nM | | | | | | Comp ound No. | 1000 nM | | | | | |
| **1** | ++ | ++ | + | ++ | + | + | **61** | +++ | +++ | ++ | + | + | ++ |
| **2** | ++ | ++ | + | ++ | + | + | **62** | +++ | +++ | ++ | ++ | + | ++ |
| **3** | ++ | ++ | + | ++ | + | ++ | **63** | ++ | + | ++ | + | + | + |
| **4** | +++ | ++ | + | ++ | + | + | **64** | +++ | +++ | ++ | ++ | + | + |
| **5** | ++ | ++ | + | ++ | + | + | **65** | ++ | + | ++ | + | + | + |
| **6** | ++ | ++ | + | ++ | + | + | **66** | ++ | +++ | ++ | + | + | + |
| **7** | ++ | ++ | ++ | ++ | + | + | **67** | + | + | + | + | + | + |
| **8** | ++ | ++ | ++ | ++ | + | + | **68** | +++ | +++ | ++ | ++ | + | + |
| **9** | ++ | ++ | + | ++ | + | + | **69** | +++ | +++ | ++ | ++ | + | + |
| **10** | +++ | ++ | + | ++ | + | + | **70** | +++ | + | ++ | + | + | + |
| **11** | +++ | ++ | + | ++ | + | ++ | **71** | +++ | +++ | ++ | ++ | + | ++ |
| **12** | ++ | ++ | + | ++ | + | + | **72** | +++ | + | ++ | ++ | + | + |
| **13** | ++ | ++ | + | + | + | + | **73** | ++ | +++ | ++ | ++ | + | + |
| **14** | +++ | ++ | + | ++ | + | + | **74** | ++ | + | + | + | + | + |
| **15** | +++ | ++ | ++ | ++ | + | + | **75** | +++ | +++ | ++ | ++ | + | + |
| **16** | +++ | ++ | ++ | +++ | + | + | **76** | +++ | +++ | ++ | ++ | + | + |
| **17** | +++ | ++ | + | ++ | + | ++ | **77** | ++ | + | + | + | + | + |
| **18** | +++ | ++ | + | ++ | + | + | **78** | +++ | +++ | ++ | + | + | + |
| **19** | +++ | ++ | ++ | ++ | + | + | **79** | +++ | +++ | ++ | ++ | + | ++ |
| **20** | +++ | ++ | ++ | + | + | + | **80** | +++ | + | ++ | + | + | + |
| **21** | +++ | + | + | + | + | + | **81** | +++ | +++ | ++ | ++ | + | + |
| **22** | +++ | ++ | ++ | + | + | + | **82** | +++ | ++ | + | + | + | + |
| **23** | +++ | ++ | + | + | + | + | **83** | +++ | +++ | + | ++ | + | + |
| **24** | +++ | ++ | + | ++ | + | + | **84** | + | + | + | + | + | + |

(continued)

| | % inhibition rate | | | | | | | % inhibition rate | | | | | |
|---|---|---|---|---|---|---|---|---|---|---|---|---|---|
| | LoVo | G401 | MKN -45 | SW-13 | MIA-Paca2 | NCI-H129 9 | | LoVo | G401 | MKN -45 | SW-13 | MIA-Paca2 | NCI-H129 9 |
| Comp ound No. | 1000 nM | | | | | | Comp ound No. | 1000 nM | | | | | |
| **25** | +++ | ++ | + | ++ | + | + | **85** | +++ | +++ | ++ | + | + | + |
| **26** | +++ | ++ | + | ++ | + | + | **86** | +++ | +++ | ++ | + | + | + |
| **27** | +++ | ++ | ++ | ++ | + | + | **87** | +++ | + | + | + | + | + |
| **28** | ++ | ++ | + | ++ | + | + | **88** | +++ | +++ | ++ | ++ | + | + |
| **29** | +++ | ++ | + | ++ | + | + | **89** | +++ | +++ | + | ++ | + | + |
| **30** | +++ | ++ | ++ | ++ | + | + | **90** | +++ | ++ | + | + | + | + |
| **31** | +++ | ++ | ++ | ++ | + | + | **91** | +++ | ++ | + | + | + | + |
| **32** | +++ | ++ | ++ | ++ | + | ++ | **92** | +++ | +++ | ++ | + | + | + |
| **33** | +++ | +++ | ++ | ++ | + | + | **93** | +++ | + | + | ++ | + | + |
| **34** | +++ | ++ | + | + | + | + | **94** | +++ | + | + | + | + | + |
| **35** | +++ | +++ | + | + | + | + | **95** | +++ | +++ | ++ | + | + | + |
| **36** | +++ | +++ | + | + | + | + | **96** | +++ | + | + | + | + | + |
| **37** | +++ | + | + | + | + | ++ | **97** | +++ | ++ | ++ | + | + | + |
| **38** | +++ | +++ | ++ | + | + | ++ | **98** | +++ | ++ | ++ | + | + | + |
| **39** | +++ | ++ | + | + | + | + | **99** | +++ | ++ | ++ | ++ | + | + |
| **40** | ++ | +++ | + | ++ | + | ++ | **100** | + | +++ | ++ | ++ | + | + |
| **41** | +++ | +++ | + | + | + | + | **101** | ++ | +++ | + | ++ | + | + |
| **42** | +++ | +++ | ++ | ++ | + | ++ | **102** | +++ | ++ | ++ | +++ | +++ | + |
| **43** | +++ | +++ | ++ | + | + | ++ | **103** | +++ | +++ | +++ | +++ | +++ | + |
| **44** | +++ | +++ | ++ | ++ | + | ++ | **104** | +++ | +++ | ++ | ++ | + | + |
| **45** | ++ | + | + | ++ | + | ++ | **105** | +++ | ++ | +++ | +++ | +++ | +++ |
| **46** | +++ | +++ | ++ | ++ | + | ++ | **106** | +++ | +++ | + | ++ | + | ++ |
| **47** | +++ | +++ | ++ | ++ | + | ++ | **107** | +++ | +++ | + | + | + | + |
| **48** | +++ | + | + | + | + | ++ | **108** | +++ | + | + | + | + | + |

(continued)

| | % inhibition rate | | | | | | | % inhibition rate | | | | | |
|---|---|---|---|---|---|---|---|---|---|---|---|---|---|
| | LoVo | G401 | MKN -45 | SW-13 | MIA-Paca2 | NCI-H129 9 | | LoVo | G401 | MKN -45 | SW-13 | MIA-Paca2 | NCI-H129 9 |
| Comp ound No. | 1000 nM | | | | | | Comp ound No. | 1000 nM | | | | | |
| **49** | +++ | ++ | + | ++ | + | ++ | **109** | +++ | +++ | ++ | ++ | + | + |
| **50** | + | + | + | + | + | ++ | **110** | +++ | +++ | + | ++ | + | + |
| **51** | +++ | +++ | + | ++ | + | ++ | **111** | +++ | +++ | + | ++ | + | + |
| **52** | + | + | + | + | + | + | **112** | +++ | +++ | ++ | ++ | + | + |
| **53** | +++ | +++ | + | ++ | + | ++ | **113** | +++ | +++ | + | ++ | + | + |
| **54** | ++ | ++ | + | ++ | + | + | **114** | +++ | +++ | + | ++ | + | + |
| **55** | +++ | +++ | ++ | ++ | + | ++ | **115** | +++ | ++ | + | + | + | + |
| **56** | +++ | ++ | + | + | + | + | | | | | | | |
| **57** | +++ | +++ | ++ | ++ | ++ | ++ | | | | | | | |
| **58** | ++ | + | + | + | + | + | | | | | | | |
| **59** | +++ | +++ | ++ | ++ | + | ++ | | | | | | | |
| **60** | +++ | + | + | + | + | + | | | | | | | |

Note) Cell proliferation inhibitory effect (% inhibition)
+++: ≥70%, ++: more than 30% and less than 70%, +: ≤30%

As shown in above Table 2, it was confirmed that the compound of the present invention effectively inhibits the proliferation of tumor cells, and thus may be advantageously used for the prevention or treatment of solid cancer.

While specific portions of the present invention have been described in detail above, it is apparent to those skilled in the art that such detailed descriptions are set forth to illustrate exemplary embodiments only, but are not construed to limit the scope of the present invention. Thus, it should be understood that the substantial scope of the present invention is defined by the accompanying claims and equivalents thereto.

## Claims

**1.** A pharmaceutical composition for preventing or treating enhancer of zeste homolog 1 (EZH1) or enhancer of zeste homolog 2 (EZH2) activity-associated diseases, comprising a compound represented by chemical formula I below, a stereoisomer thereof, a pharmaceutically acceptable salt thereof, a hydrate or solvate thereof as an active ingredient, wherein the EZH1 or EZH2 activity-associated disease is a solid cancer:

<Chemical Formula I>

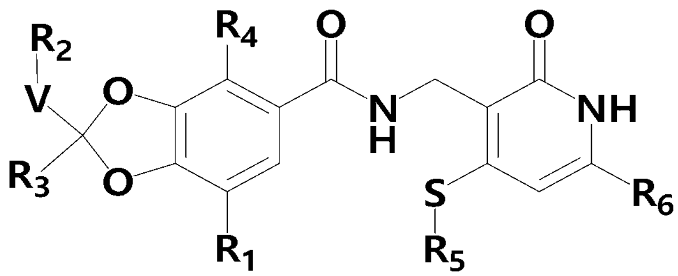

wherein in the chemical formula I,

$R_1$ is H; $C_1$-$C_6$ alkyl; $C_2$-$C_6$ alkenyl; $C_2$-$C_6$ alkynyl; $C_3$-$C_8$ cycloalkyl; $C_3$-$C_8$ cycloalkenyl; $C_1$-$C_6$ alkoxy; 3 to 12-membered heterocycloalkyl or heterocycloalkenyl comprising in a ring one to three heteroatoms independently selected from the group consisting of N, O, and S; 6 to 14-membered aryl; 5 to 12-membered heteroaryl comprising in a ring one to three heteroatoms independently selected from the group consisting of N, O and S; -(C=O)-($C_1$-$C_6$ alkyl); -CN; benzodioxolyl or halogen,
in the $R_1$, at least one H of $C_1$-$C_6$ alkyl; $C_3$-$C_8$ cycloalkyl; 3 to 12-membered heterocycloalkyl or heterocycloalkenyl comprising in a ring one to three heteroatoms independently selected from the group consisting of N, O, and S; 6 to 14-membered aryl; 5 to 12-membered heteroaryl comprising in a ring one to three heteroatoms independently selected from the group consisting of N, O and S or benzodioxolyl may be substituted or unsubstituted with Ra,
the Ra is $C_1$-$C_6$ alkyl; $C_1$-$C_6$ alkoxy; 3 to 12-membered heterocycloalkyl comprising in a ring one to three heteroatoms independently selected from the group consisting of N, O, and S; -NRxRy; or halogen,
at least one H of the Ra may be substituted or unsubstituted with Rb,
the Rb is $C_1$-$C_6$ alkyl; 3 to 12-membered heterocycloalkyl comprising in a ring one to three heteroatoms independently selected from the group consisting of N, O, and S; or halogen,
$R_2$ is H; $C_1$-$C_6$ alkyl; $C_3$-$C_8$ cycloalkyl; 3 to 12-membered heterocycloalkyl or heterocycloalkenyl comprising in a ring one to three heteroatoms independently selected from the group consisting of N, O, and S; 6 to 14-membered aryl; or 5 to 12-membered heteroaryl comprising in a ring one to three heteroatoms independently selected from the group consisting of N, O and S,
at least one H of the $R_2$ may be substituted or unsubstituted with Rc,
the Rc is $C_1$-$C_6$ alkyl; $C_3$-$C_8$ cycloalkyl; 3 to 12-membered heterocycloalkyl comprising in a ring one to three heteroatoms independently selected from the group consisting of N, O, and S; -NRpRq; -(HC=O); -(C=O)-($C_1$-$C_6$ alkyl); -S(=O)$_2$-($C_1$-$C_6$ alkyl); or halogen,
at least one H of the Rc may be substituted or unsubstituted with Rd,
the Rd is $C_1$-$C_6$ alkoxy which may be substituted or unsubstituted with halogen; 3 to 12-membered heterocycloalkyl comprising in a ring one to three heteroatoms independently selected from the group consisting of N, O, and S; 6 to 14-membered aryl; halogen; or -OH,
$R_3$ is H; or $C_1$-$C_6$ alkyl,
$R_4$ is H; $C_1$-$C_6$ alkyl; or halogen,
$R_5$ and $R_6$ are each independently H; or $C_1$-$C_6$ alkyl,

Rx and Ry are each independently H; or $C_1$-$C_6$ alkyl,
Rp and Rq are each independently H; $C_1$-$C_6$ alkyl, $C_3$-$C_8$ cycloalkyl or -(C=O)-($C_1$-$C_6$ alkyl), and
V is a single bond; or -($C_1$-$C_6$ alkylene)-.

**2.** The pharmaceutical composition of claim 1, wherein

$R_1$ is 6 to 14-membered aryl; 5 to 12-membered heteroaryl comprising in a ring one to three heteroatoms independently selected from the group consisting of N, O and S or benzodioxolyl,
at least one H of the $R_1$ may be substituted or unsubstituted with Ra,
the Ra is $C_1$-$C_6$ alkyl; $C_1$-$C_6$ alkoxy; 3 to 12-membered heterocycloalkyl comprising in a ring one to three heteroatoms independently selected from the group consisting of N, O, and S; -NRxRy; or halogen,
at least one H of the Ra may be substituted or unsubstituted with Rb,
the Rb is $C_1$-$C_6$ alkyl; 3 to 12-membered heterocycloalkyl comprising in a ring one to three heteroatoms independently selected from the group consisting of N, O, and S; or halogen,
$R_2$ is $C_3$-$C_8$ cycloalkyl; or 3 to 12-membered heterocycloalkyl comprising in a ring one to three heteroatoms independently selected from the group consisting of N, O, and S,
at least one H of the $R_2$ may be substituted or unsubstituted with Rc,
the Rc is $C_1$-$C_6$ alkyl; $C_3$-$C_8$ cycloalkyl; 3 to 12-membered heterocycloalkyl comprising in a ring one to three heteroatoms independently selected from the group consisting of N, O, and S; -NRpRq or -(C=O)-($C_1$-$C_6$ alkyl),
at least one H of the Rc may be substituted or unsubstituted with Rd,
the Rd is $C_1$-$C_6$ alkoxy which may be substituted or unsubstituted with halogen; 3 to 12-membered heterocycloalkyl comprising in a ring one to three heteroatoms independently selected from the group consisting of N, O, and S; 6 to 14-membered aryl; halogen; or -OH,
$R_3$ to $R_6$ are each independently $C_1$-$C_6$ alkyl,
Rx and Ry are each independently $C_1$-$C_6$ alkyl,
Rp and Rq are each independently H; $C_1$-$C_6$ alkyl, $C_3$-$C_8$ cycloalkyl or -(C=O)-(Ci-$C_6$ alkyl), and
V is a single bond.

**3.** The pharmaceutical composition of claim 2, wherein

$R_1$ is phenyl, pyridinyl, pyrimidinyl, indolyl, thiophenyl, isoxazolyl, furanyl, benzofuranyl or benzodioxolyl,
at least one H of the $R_1$ may be substituted or unsubstituted with Ra,
the Ra is methyl, methoxy, $-N(CH_3)_2$, $-N(CH_3)(CH_2CH_3)$, - $N(CH_3)(CH_2CH_2CH_2CH_3)$, morpholinyl, thiomorpholinyl, pyrrolidinyl, piperidinyl, piperazinyl,

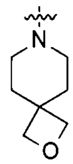

or halogen,
at least one H of the Ra may be substituted or unsubstituted with Rb,
the Rb is methyl, ethyl, morpholinyl or halogen,
$R_2$ is cyclohexyl or piperidinyl,
at least one H of the $R_2$ may be substituted or unsubstituted with Rc,
the Rc is methyl, ethyl, propyl, isopropyl, butyl, cyclohexyl, azetidinyl, $-NH_2$, - $N(CH_3)_2$, $-N(CH_3)(CH_2CH_3)$, $-N(CH_3)(CH_2CH_2CH_3)$, $-N(CH_3)(CH)(CH_3)_2$, - $N(CH_3)(CH_2CH_2CH_2CH_3)$, $-N(CH_3)(C_6H_{11})$, $-N(CH_3)(C=O)CH_3$ or $-(C=O)CH_3$,
at least one H of the Rc may be substituted or unsubstituted with Rd,
the Rd is methoxy, ethoxy which may be substituted or unsubstituted with halogen, propoxy, butoxy which may be substituted or unsubstituted with halogen, isopropoxy, dioxolanyl, phenyl, halogen or -OH, and
$R_3$ to $R_6$ are methyl.

**4.** The pharmaceutical composition of claim 1, wherein the compound represented by the chemical formula I is a compound described in a following table:

| Structure | Structure |
|---|---|
| 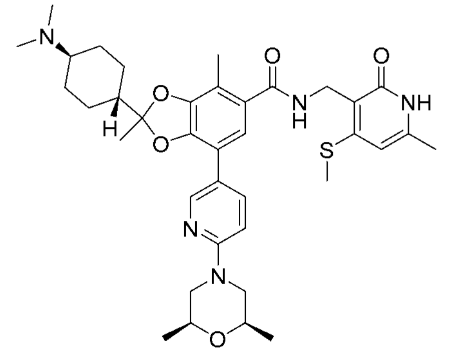 | 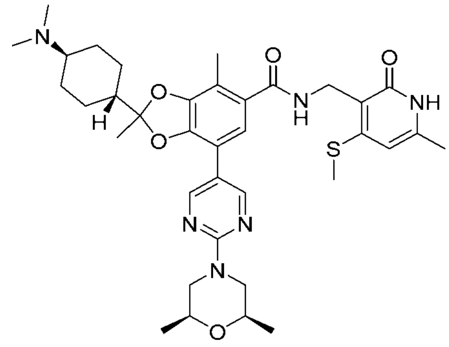 |
| 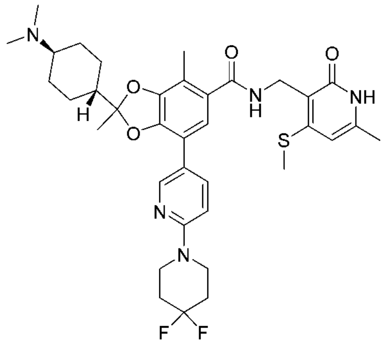 | 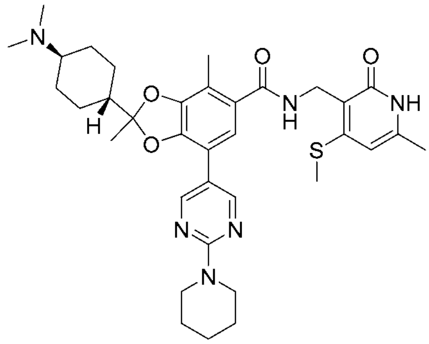 |
| 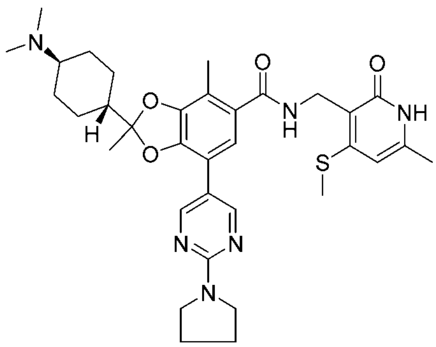 | 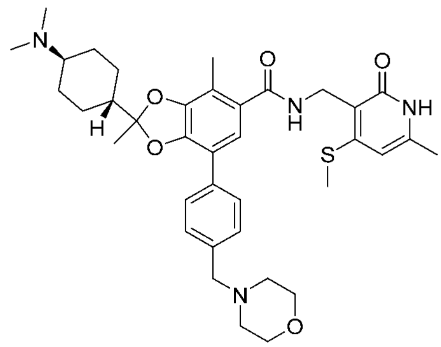 |
| 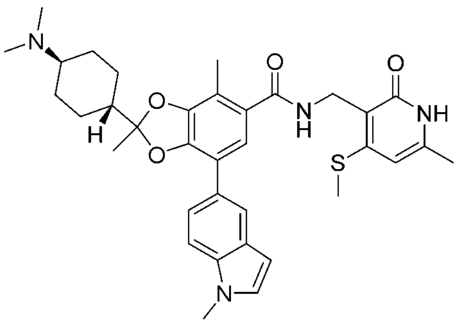 | 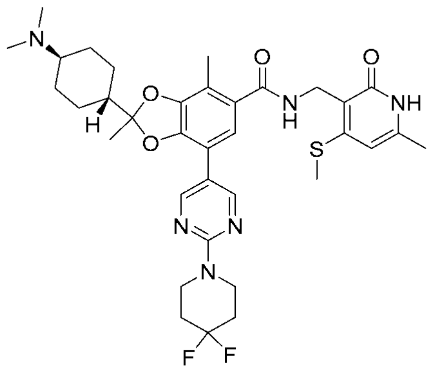 |

(continued)

| Structure | Structure |
|---|---|
| 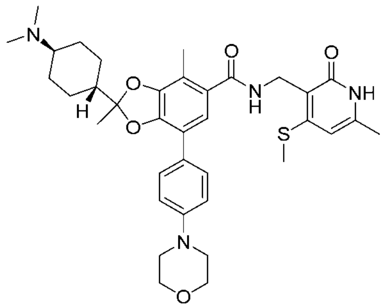 | 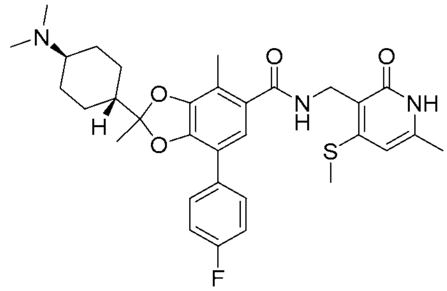 |
| 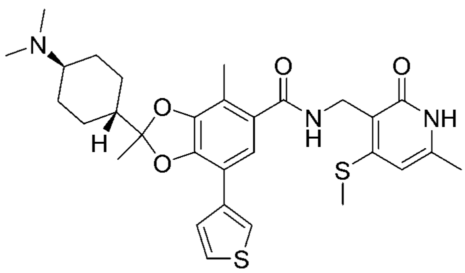 | 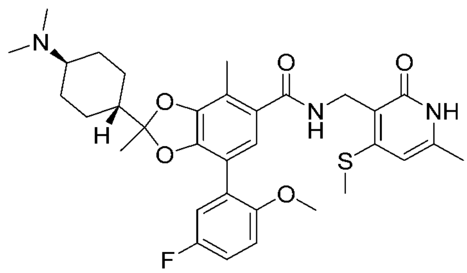 |
| 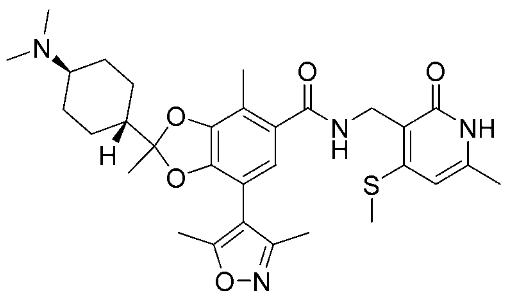 | 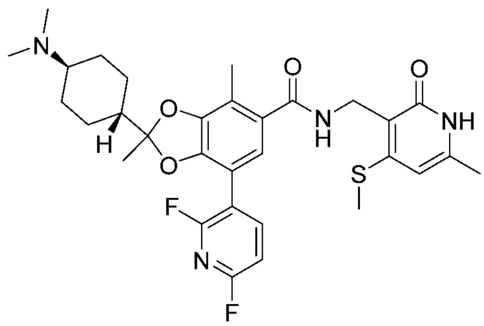 |
| 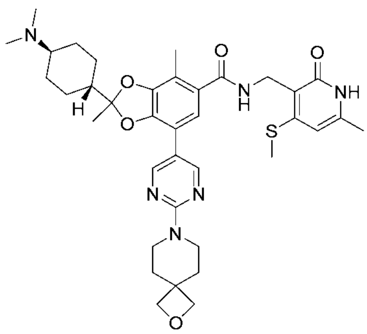 | 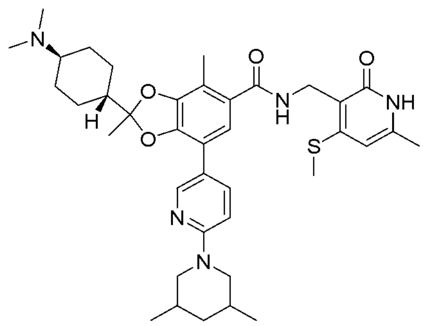 |
| 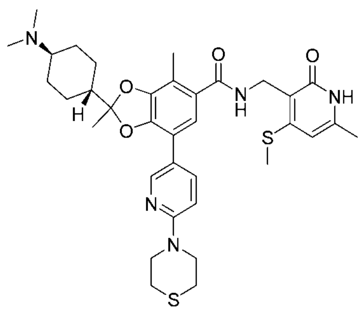 | 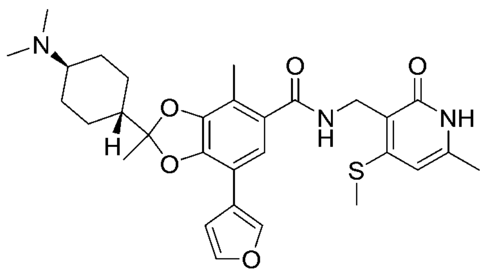 |

(continued)

| Structure | Structure |
|---|---|
| 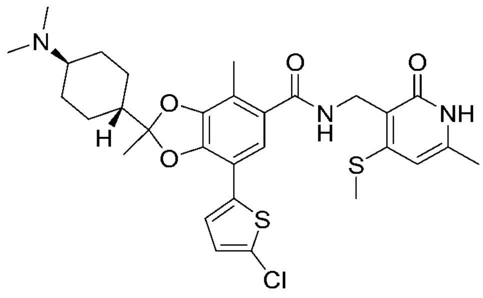 | 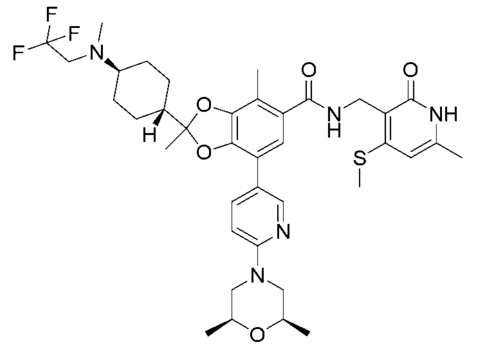 |
| 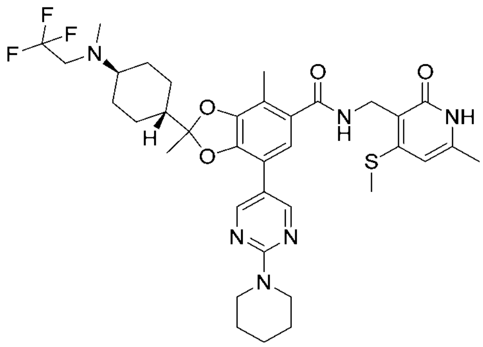 | 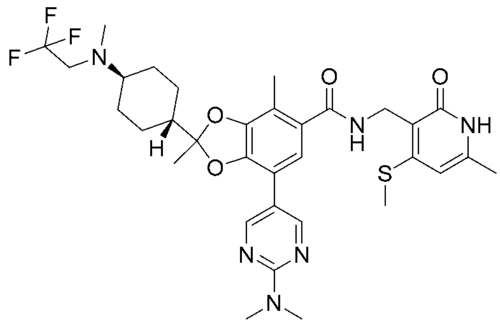 |
| 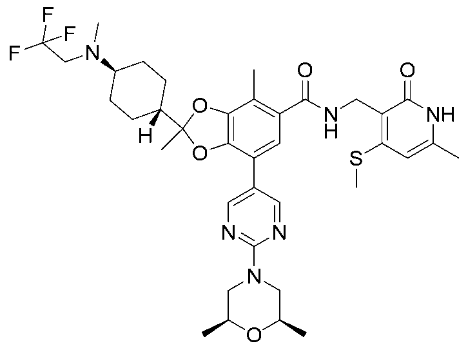 | 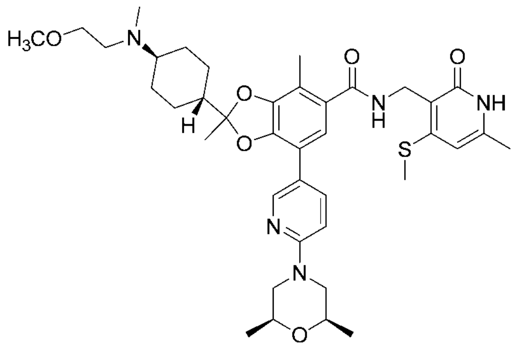 |
| 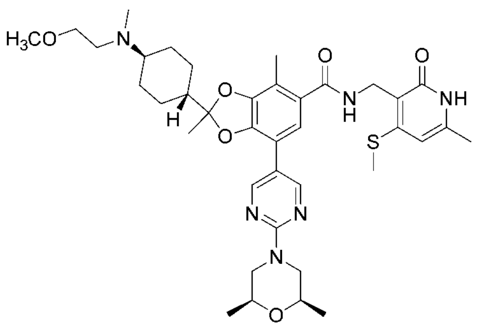 | 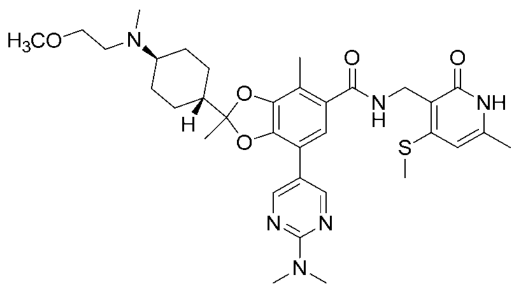 |

(continued)

| Structure | Structure |
|---|---|
| 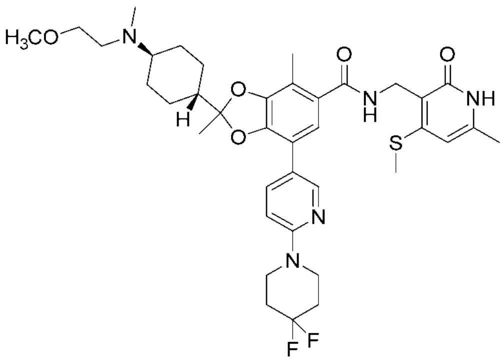 | 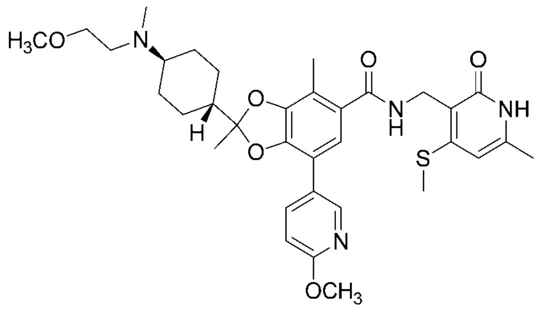 |
| 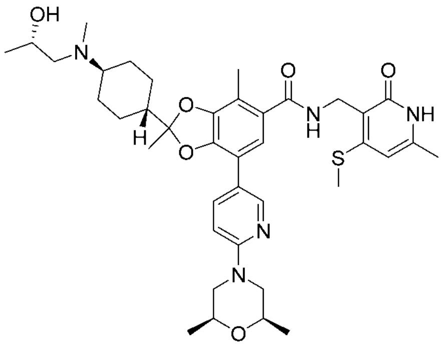 | 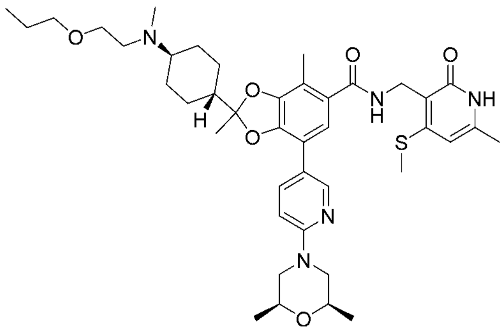 |
| 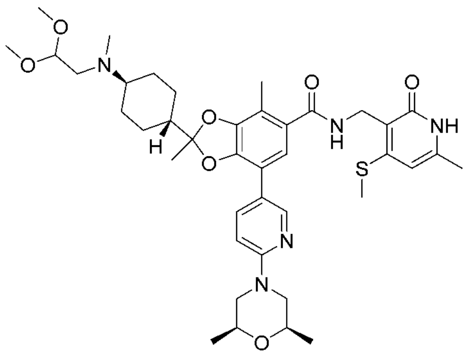 | 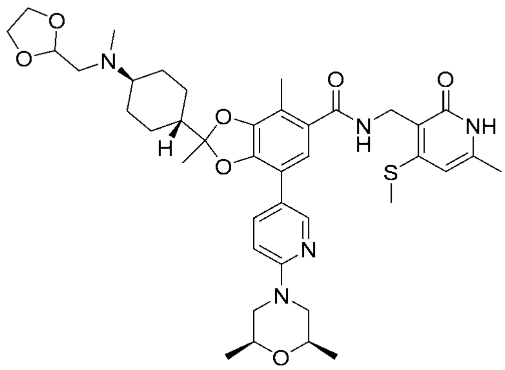 |
| 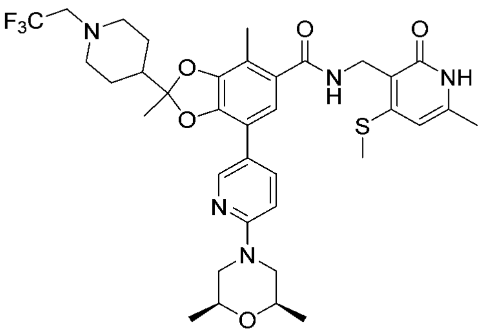 | 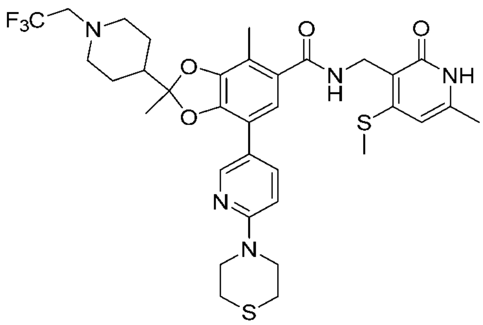 |

(continued)

| Structure | Structure |
|---|---|
| 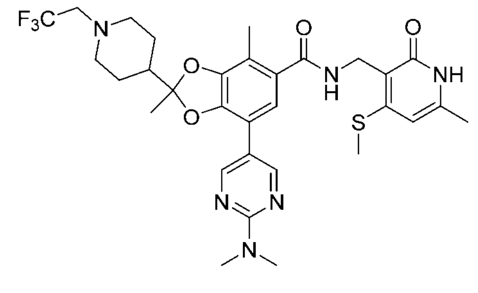 | 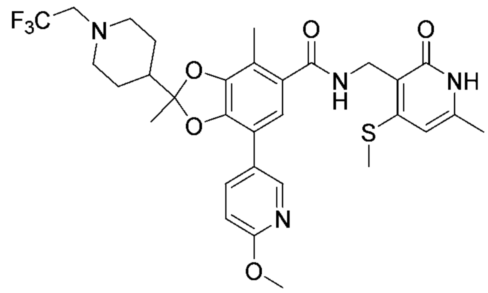 |
| 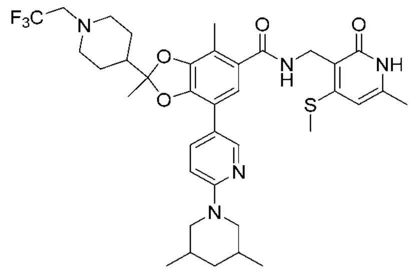 | 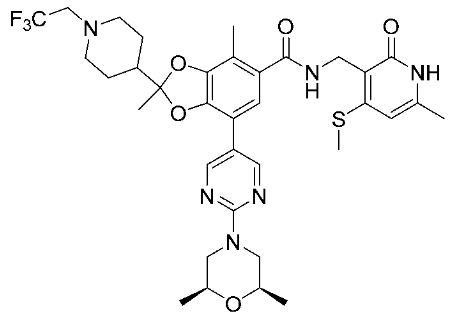 |
| 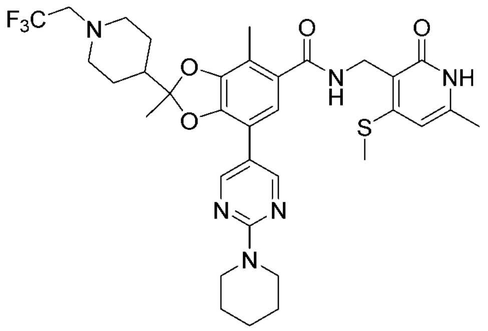 | 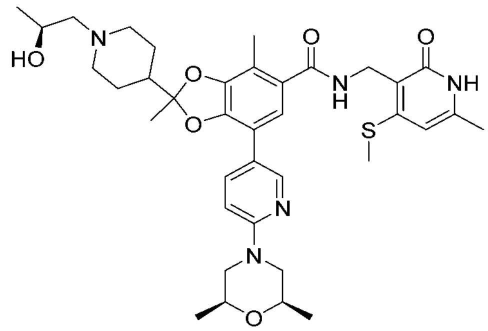 |
| 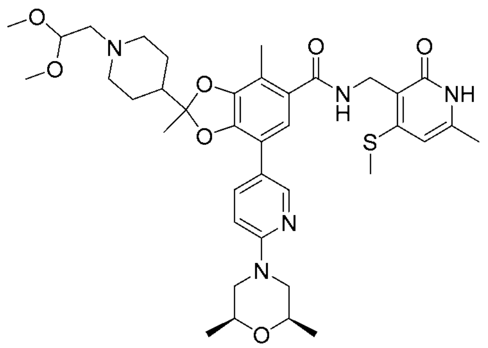 | 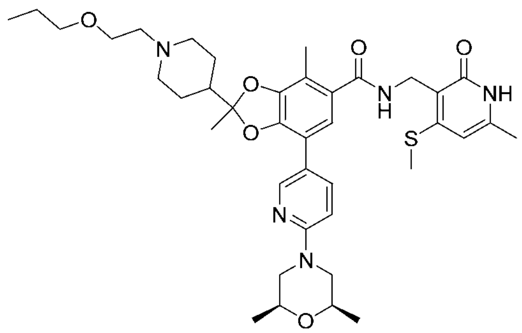 |
| 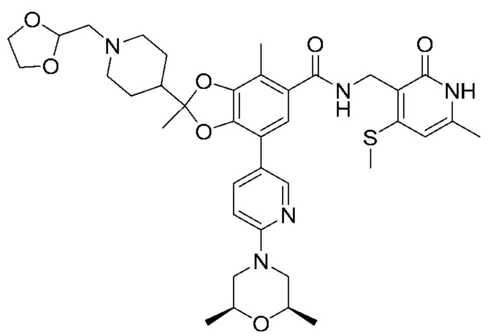 | 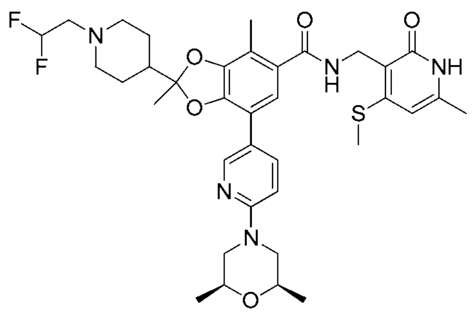 |

(continued)

| Structure | Structure |
|---|---|
| 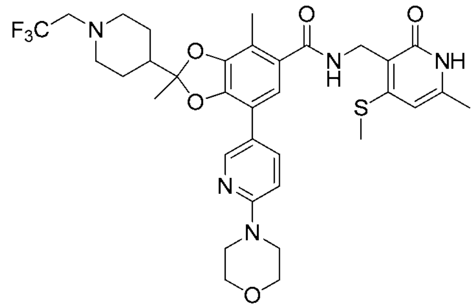 | 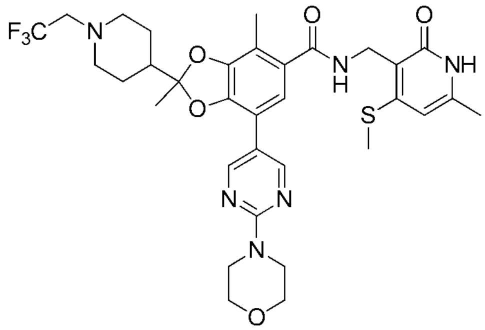 |
| 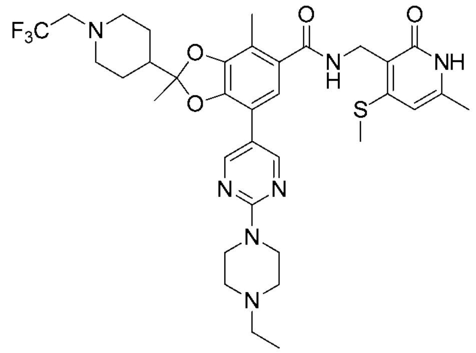 | 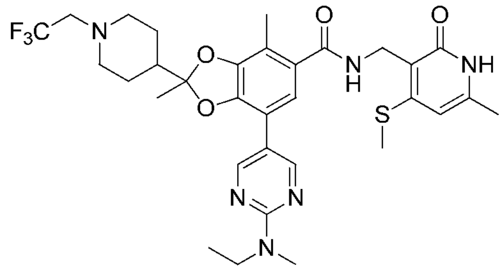 |
| 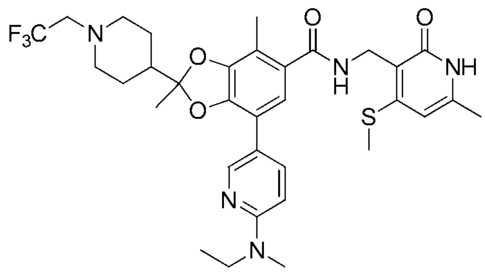 | 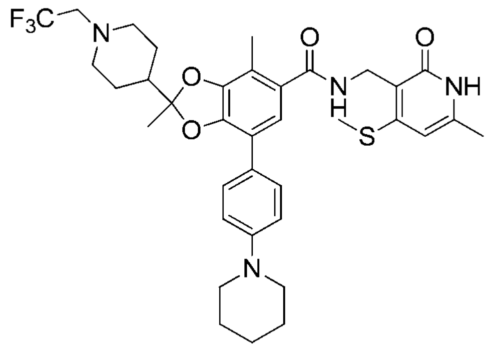 |
| 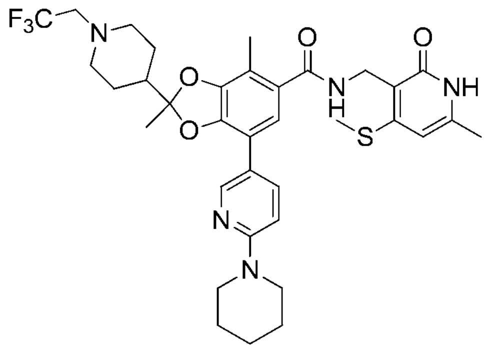 | 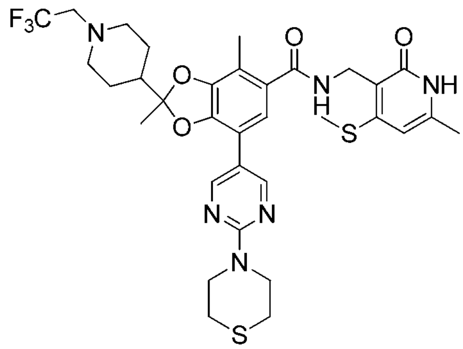 |

(continued)

| Structure | Structure |
|---|---|
| 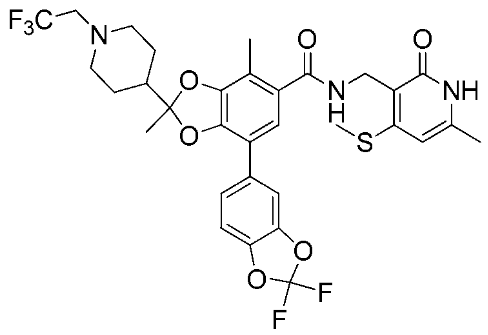 | 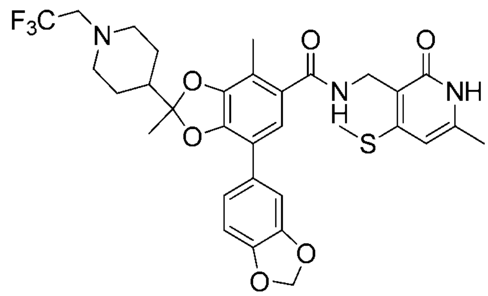 |
| 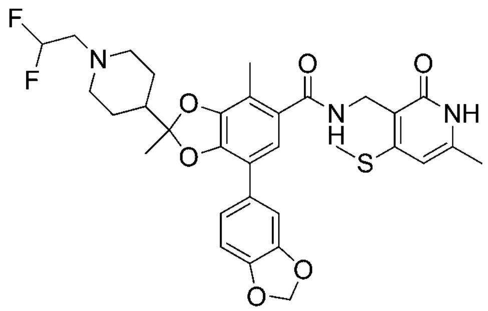 | 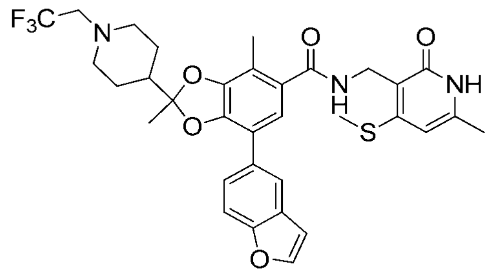 |
| 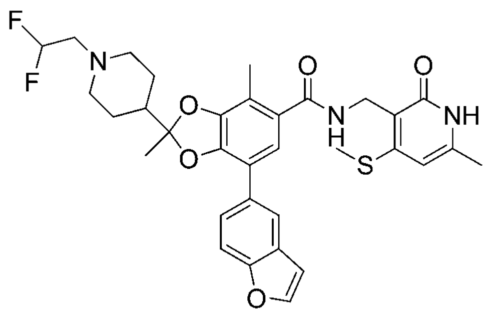 | 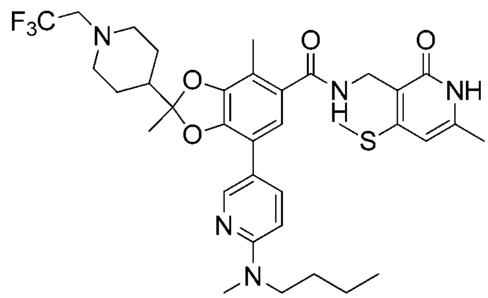 |
| 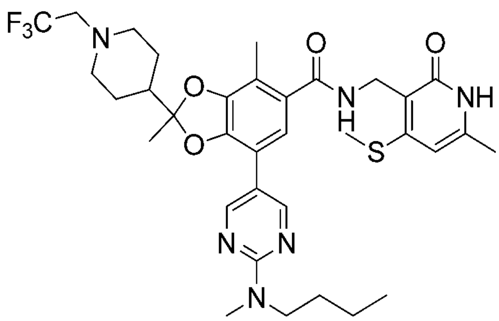 | 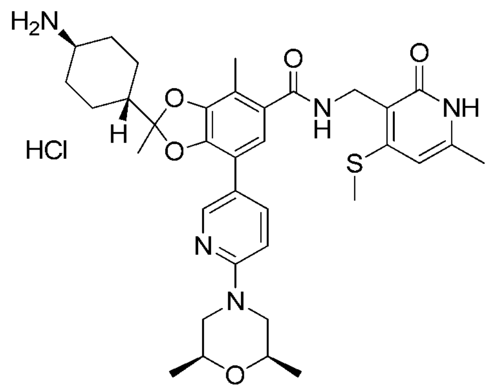 HCl |

(continued)

| Structure | Structure |
|---|---|
| 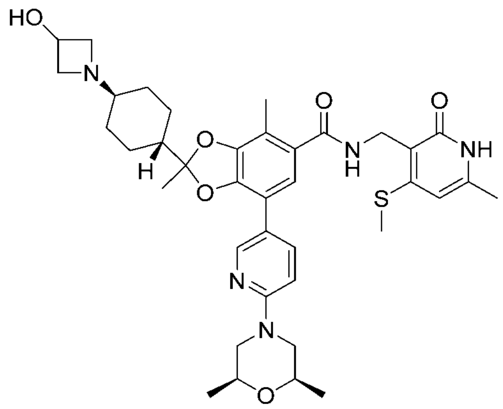 | 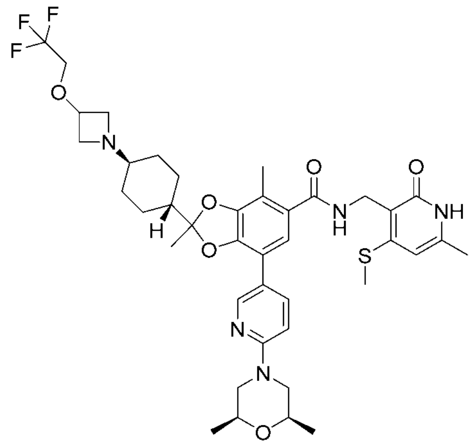 |
| 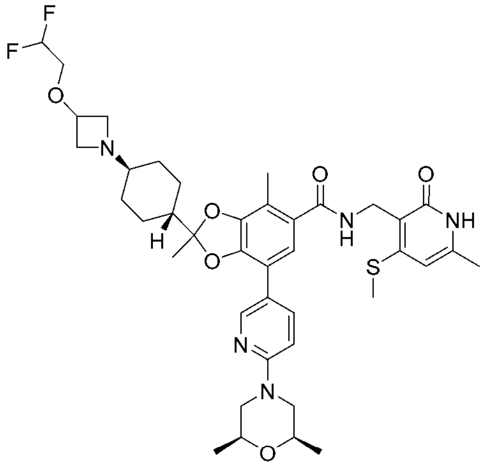 | 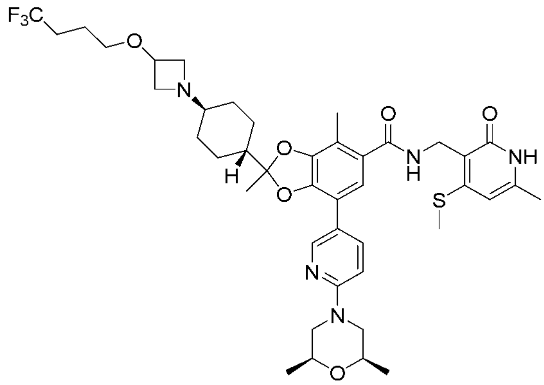 |
| 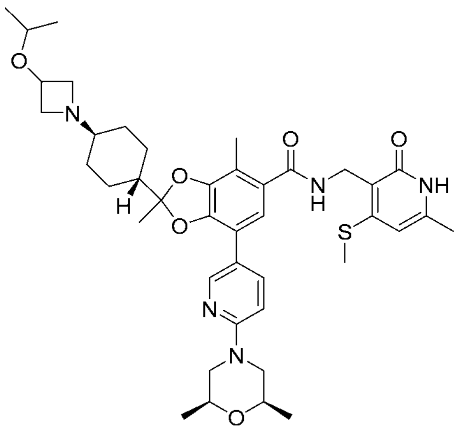 | 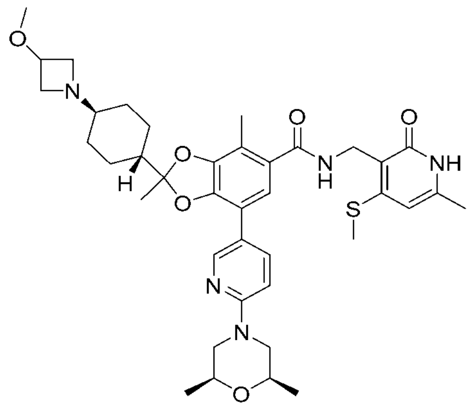 |

(continued)

| Structure | Structure |
|---|---|
| 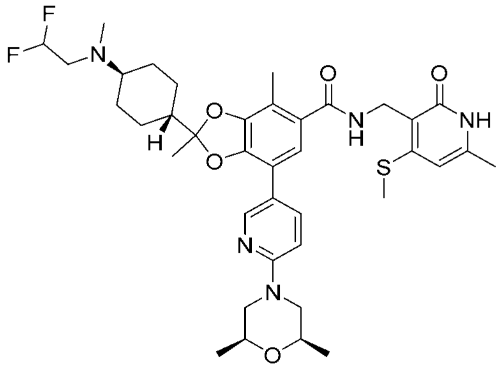 | 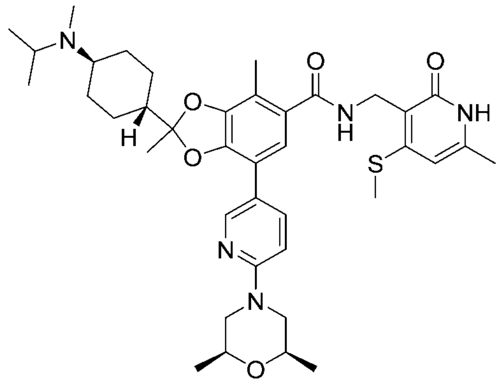 |
| 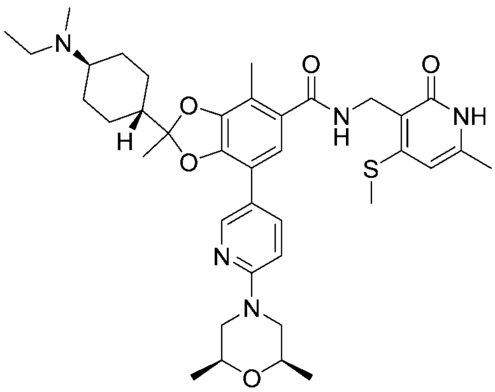 | 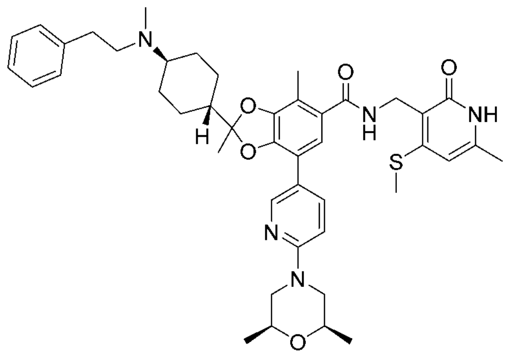 |
| 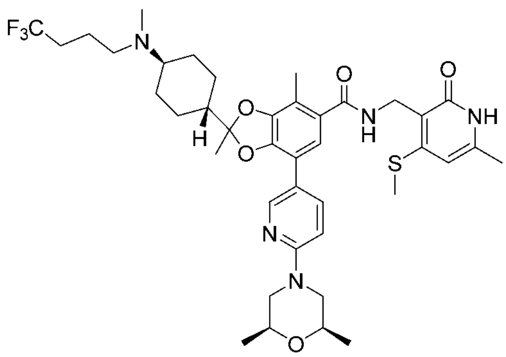 | 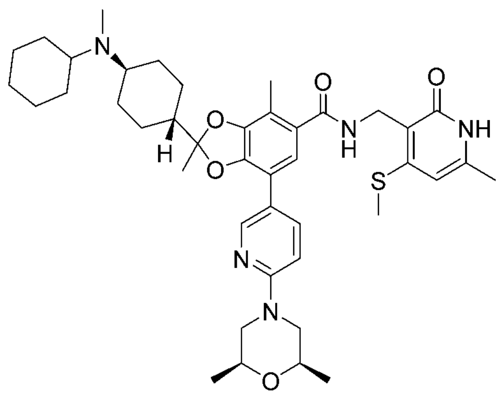 |
| 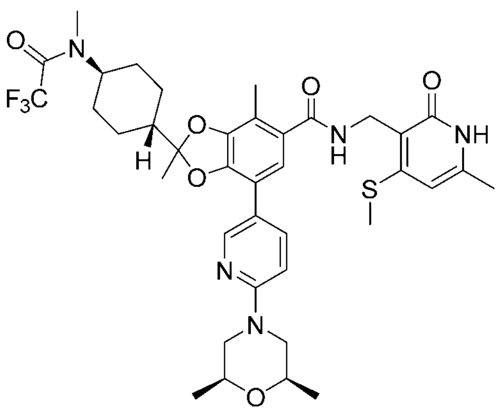 | 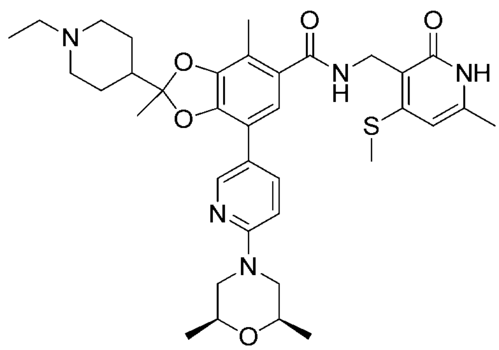 |

(continued)

| Structure | Structure |
|---|---|
| 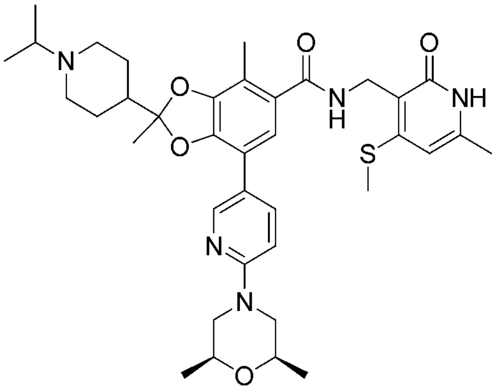 | 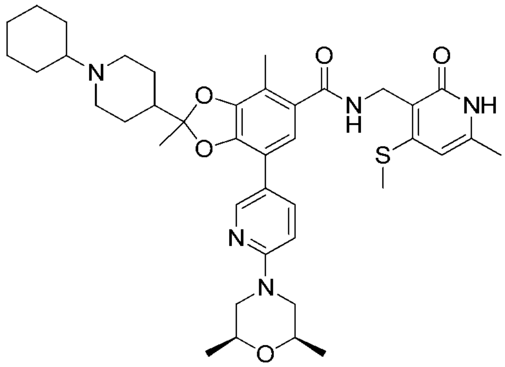 |
| 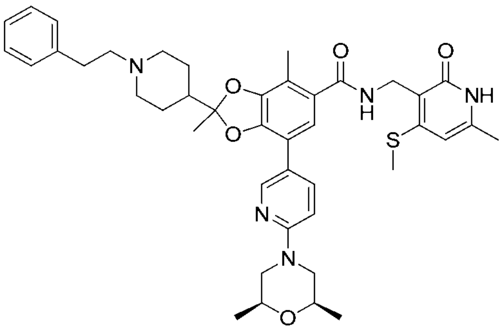 | 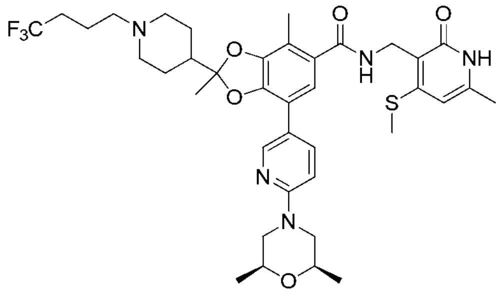 |
| 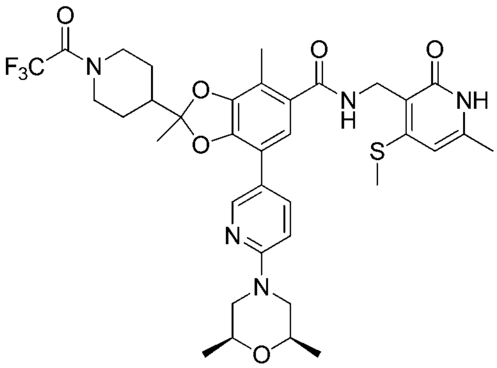 | 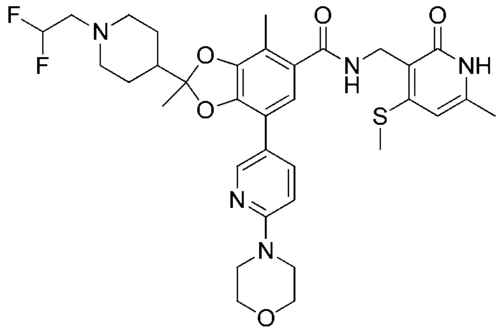 |
| 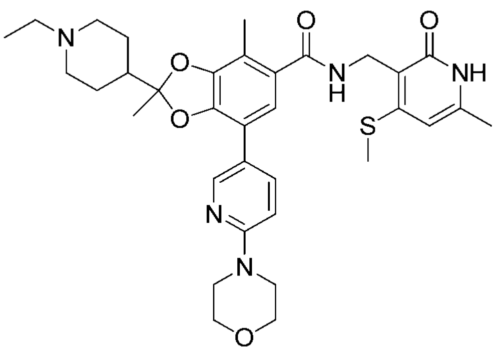 | 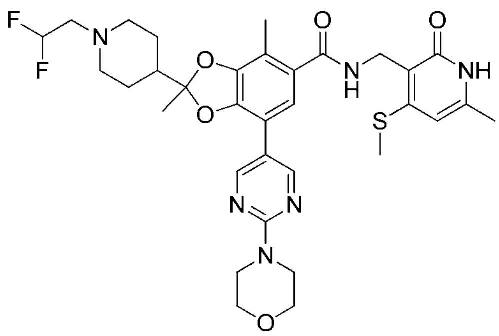 |

(continued)

| Structure | Structure |
|---|---|
| 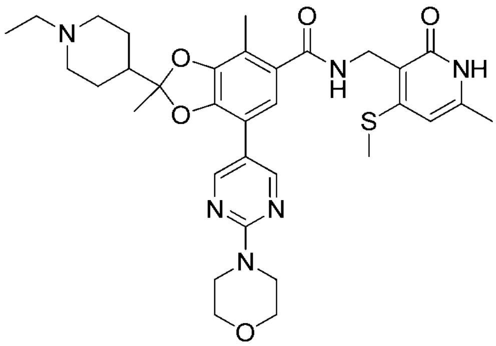 | 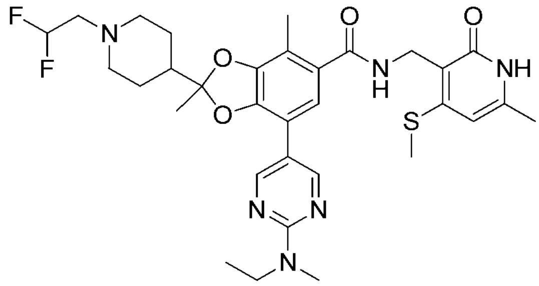 |
| 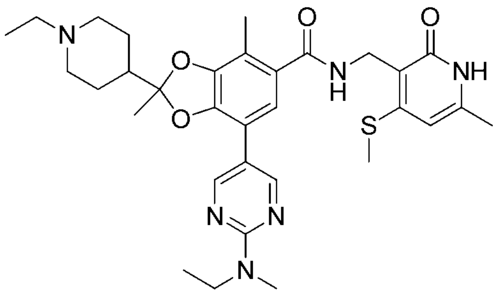 | 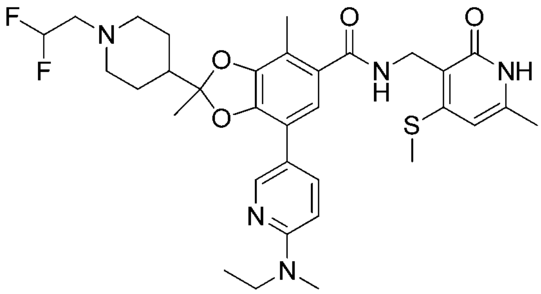 |
| 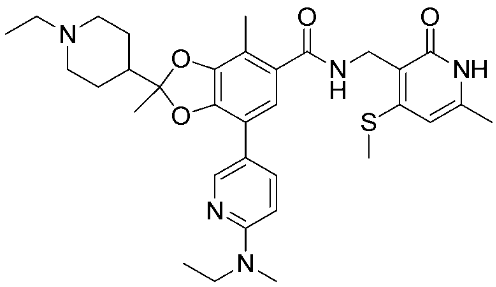 | 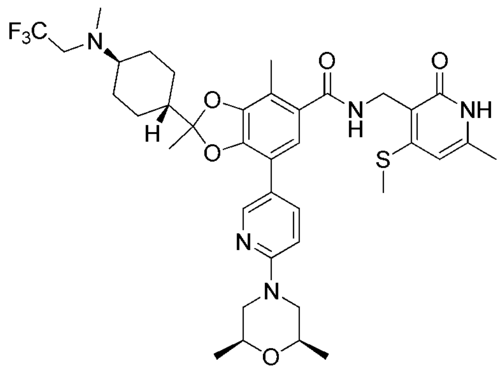 |

**5.** A pharmaceutical composition for preventing or treating enhancer of zeste homolog 1 (EZH1) or enhancer of zeste homolog 2 (EZH2) activity-associated diseases, comprising a following compound, a stereoisomer thereof, a pharmaceutically acceptable salt thereof, a hydrate or solvate thereof as an active ingredient, wherein
the EZH1 or EZH2 activity-associated disease is a solid cancer:

1) 2-(trans-4-(dimethylamino)cyclohexyl)-7-(6-((2S,6R)-2,6-dimethylmorpholino)pyridin-3-yl)-2,4-dimethyl-N-((6-methyl-4-(methylthio)-2-oxo-1,2-dihydropyridin-3-yl)methyl)benzo[d] [1 ,3]dioxole-5-carboxamide
2) 2-(trans-4-(dimethylamino)cyclohexyl)-7-(2-((2S,6R)-2,6-dimethylaminomorpholino)pyrimidin-5-yl)-2,4-dimethyl-N-((6-methyl-4-(methylthio)-2-oxo-1,2-dihydropyridin-3-yl)methyl)benzo[d][1,3]dioxole-5-carboxamide
3) 7-(6-(4,4-difluoropiperidin-1-yl)pyridin-3-yl)-2-(trans-4-(dimethylamino)cyclohexyl)-2,4-dimethyl-N-((6-methyl-4-(methylthio)-2-oxo-1,2-dihydropyridin-3-yl)methyl)benzo[d][1,3]dioxole-5-carboxamide
4) 2-(trans-4-(dimethylamino)cyclohexyl)-2,4-dimethyl-N-((6-methyl-4-(methylthio)-2-oxo-1,2-dihydropyridin-3-yl)methyl)-7-(2-(piperidin-1-yl)pyrimidin-5-yl)benzo[d][1,3]dioxole-5-carboxamide
5) 2-(trans-4-(dimethylamino)cyclohexyl)-2,4-dimethyl-N-((6-methyl-4-(methylthio)-2-oxo-1,2-dihydropyridin-3-yl)methyl)-7-(2-(pyrrolidin-1-yl)pyrimidin-5-yl)benzo[d][1,3]dioxole-5-carboxamide
6) 2-(trans-4-(dimethylamino)cyclohexyl)-2,4-dimethyl-N-((6-methyl-4-(methylthio)-2-oxo-1,2-dihydropyridin-3-yl)methyl)-7-(4-(morpholinomethyl)phenyl)benzo[d][1,3]dioxole-5-carboxamide
7) 2-(trans-4-(dimethylamino)cyclohexyl)-2,4-dimethyl-7-(1-methyl-1H-indol-5-yl)-N-((6-methyl-4-(methylthio)-2-oxo-1,2-dihydropyridin-3 - yl)methyl)benzo[d][1,3]dioxole-5-carboxamide
8) 7-(2-(4,4-difluoropiperidin-1-yl)pyrimidin-5-yl)-2-(trans-4-(dimethylamino)cyclohexyl)-2,4-dimethyl-N-((6-methyl-4-(methylthio)-2-oxo-1,2-dihydropyridin-3-yl)methyl)benzo[d][1,3]dioxole-5-carboxamide
9) 2-(trans-4-(dimethylamino)cyclohexyl)-2,4-dimethyl-N-((6-methyl-4-(methylthio)-2-oxo-1,2-dihydropyridin-3-yl)methyl)-7-(4-morpholinophenyl)benzo[d][1,3]dioxole-5-carboxamide
10) 2-(trans-4-(dimethylamino)cyclohexyl)-7-(4-fluorophenyl)-2,4-dimethyl-N-((6-methyl-4-(methylthio)-2-oxo-1,2-dihydropyridin-3-yl)methyl)benzo[d][1,3]dioxole-5-carboxamide
11) 2-(trans-4-(dimethylamino)cyclohexyl)-2,4-dimethyl-N-((6-methyl-4-(methylthio)-2-oxo-1,2-dihydropyridin-3-yl)methyl)-7-(thiophene-3-yl)benzo[d][1,3]dioxole-5-carboxamide
12) 2-(trans-4-(dimethylamino)cyclohexyl)-7-(5-fluoro-2-methoxyphenyl)-2,4-dimethyl-N-((6-methyl-4-(methylthio)-2-oxo-1,2-dihydropyridin-3-yl)methyl)benzo[d][1,3]dioxole-5-carboxamide
13) 2-(trans-4-(dimethylamino)cyclohexyl)-7-(3,5-dimethylisoxazol-4-yl)-2,4-dimethyl-N-((6-methyl-4-(methylthio)-2-oxo-1,2-dihydropyridin-3-yl)methyl)benzo[d][1,3]dioxole-5-carboxamide
14) 7-(2,6-difluoropyridin-3-yl)-2-(trans-4-(dimethylamino)cyclohexyl)-2,4-dimethyl-N-((6-methyl-4-(methylthio)-2-oxo-1,2-dihydropyridin-3-yl)methyl)benzo[d][1,3]dioxole-5-carboxamide
15) 7-(2-(2-oxa-7-azaspiro[3.5]nonan-7-yl)pyrimidin-5-yl)-2-(trans-4-(dimethylamino)cyclohexyl)-2,4-dimethyl-N-((6-methyl-4-(methylthio)-2-oxo-1,2-dihydropyridin-3-yl)methyl)benzo[d][1,3]dioxole-5-carboxamide
16) 2-(trans-4-(dimethylamino)cyclohexyl)-7-(6-(3,5-dimethylpiperidin-1-yl)pyridin-3-yl)-2,4-dimethyl-N-((6-methyl-4-(methylthio)-2-oxo-1,2-dihydropyridin-3-yl)methyl)benzo[d][1,3]dioxole-5-carboxamide
17) 2-(trans-4-(dimethylamino)cyclohexyl)-2,4-dimethyl-N-((6-methyl-4-(methylthio)-2-oxo-1,2-dihydropyridin-3-yl)methyl)-7-(6-thiomorpholinopyridin-3-yl)benzo[d][1,3]dioxole-5-carboxamide
18) 2-(trans-4-(dimethylamino)cyclohexyl)-7-(furan-3-yl)-2,4-dimethyl-N-((6-methyl-4-(methylthio)-2-oxo-1,2-dihydropyridin-3-yl)methyl)benzo[d][1,3]dioxole-5-carboxamide
19) 7-(5-chlorothiophen-2-yl)-2-(trans-4-(dimethylamino)cyclohexyl)-2,4-dimethyl-N-((6-methyl-4-(methylthio)-2-oxo-1,2-dihydropyridin-3-yl)methyl)benzo[d][1,3]dioxole-5-carboxamide
20) 7-(6-((2S,6R)-2,6-dimethylmorpholino)pyridin-3-yl)-2,4-dimethyl-2-(trans-4-(methyl(2,2,2-trifluoroethyl)amino)cyclohexyl)-N-((6-methyl-4-(methylthio)-2-oxo-1,2-dihydropyridin-3-yl)methyl)benzo[d][1,3]dioxole-5-carboxamide
21) 2,4-dimethyl-2-(trans-4-(methyl(2,2,2-trifluoroethyl)amino)cyclohexyl)-N-((6-methyl-4-(methylthio)-2-oxo-1,2-dihydropyridin-3-yl)methyl)-7-(2-(piperidin-1-yl)pyrimidin-5-yl)benzo[d][1,3]dioxole-5-carboxamide
22) 7-(2-(dimethylamino)pyrimidin-5-yl)-2,4-dimethyl-2-(trans-4-(methyl(2,2,2-trifluoroethyl)amino)cyclohexyl)-N-((6-methyl-4-(methylthio)-2-oxo-1,2-dihydropyridin-3-yl)methyl)benzo[d][1,3]dioxole-5-carboxamide
23) 7-(2-((2S,6R)-2,6-dimethylmorpholino)pyrimidin-5-yl)-2,4-dimethyl-2-(trans-4-(methyl(2,2,2-trifluoroethyl)amino)cyclohexyl)-N-((6-methyl-4-(methylthio)-2-oxo-1,2-dihydropyridin-3-yl)methyl)benzo[d][1,3]dioxole-5-carboxamide
24) 7-(6-((2S,6R)-2,6-dimethylmorpholino)pyridin-3-yl)-2-(trans-4-((2-methoxyethyl)(methyl)amino)cyclohexyl)-2,4-dimethyl-N-((6-methyl-4-(methylthio)-2-oxo-1,2-dihydropyridin-3-yl)methyl)benzo[d][1,3]dioxole-5-carboxamide
25) 7-(2-((2S,6R)-2,6-dimethylmorpholino)pyrimidin-5-yl)-2-(trans-4-((2-methoxyethyl)(methyl)amino)cyclohexyl)-2,4-dimethyl-N-((6-methyl-4-(methylthio)-2-oxo-1,2-dihydropyridin-3-yl)methyl)benzo[d][1,3]dioxole-5-carboxamide
26) 7-(2-(dimethylamino)pyrimidin-5-yl)-2-(trans-4-((2-methoxyethyl)(methyl)amino)cyclohexyl)-2,4-dimethyl-N-((6-methyl-4-(methylthio)-2-oxo-1,2-dihydropyridin-3-yl)methyl)benzo[d][1,3]dioxole-5-carboxamide
27) 7-(6-(4,4-difluoropiperidin-1-yl)pyridin-3-yl)-2-(trans-4-((2-methoxyethyl)(methyl)amino)cyclohexyl)-2,4-dimethyl-N-((6-methyl-4-(methylthio)-2-oxo-1,2-dihydropyridin-3-yl)methyl)benzo[d][1,3]dioxole-5-carboxamide
28) 2-(trans-4-((2-methoxyethyl)(methyl)amino)cyclohexyl)-7-(6-methoxypyridin-3-yl)-2,4-dimethyl-N-((6-methyl-4-(methylthio)-2-oxo-1,2-dihydropyridin-3-yl)methyl)benzo[d][1,3]dioxole-5-carboxamide
29) 7-(6-((2S,6R)-2,6-dimethylmorpholino)pyridin-3-yl)-2-(trans-4-(((S)-2-hydroxypropyl)(methyl)amino)cyclohexyl)-2,4-dimethyl-N-((6-methyl-4-(methylthio)-2-oxo-1,2-dihydropyridin-3-yl)methyl)benzo[d][1,3]dioxole-5-carboxamide
30) 7-(6-((2S,6R)-2,6-dimethylmorpholino)pyridin-3-yl)-2,4-dimethyl-2-(trans-4-(methyl(2-propoxyethyl)amino)cyclohexyl)-N-((6-methyl-4-(methylthio)-2-oxo-1,2-dihydropyridin-3-yl)methyl)benzo[d][1,3]dioxole-5-carboxamide
31) 2-(trans-4-((2,2-dimethoxyethyl)(methyl)amino)cyclohexyl)-7-(6-((2S,6R)-2,6-dimethylmorpholino)pyridin-3-yl)-2,4-dimethyl-N-((6-methyl-4-(methylthio)-2-oxo-1,2-dihydropyridin-3-yl)methyl)benzo[d][1,3]dioxole-5-carboxamide
32) 2-(trans-4-(((1,3-dioxolan-2-yl)methyl)(methyl)amino)cyclohexyl)-7-(6-((2S,6R)-2,6-dimethylmorpholino)pyridin-3-yl)-2,4-dimethyl-N-((6-methyl-4-(methylthio)-2-oxo-1,2-dihydropyridin-3-yl)methyl)benzo[d][1,3]dioxole-5-carboxamide
33) 7-(6-((2S,6R)-2,6-dimethylmorpholino)pyridin-3-yl)-2,4-dimethyl-N-((6-methyl-4-(methylthio)-2-oxo-1,2-dihydropyridin-3-yl)methyl)-2-(1-(2,2,2-trifluoroethyl)piperidin-4-yl)benzo[d][1,3]dioxole-5-carboxamide
34) 2,4-dimethyl-N-((6-methyl-4-(methylthio)-2-oxo-1,2-dihydropyridin-3-yl)methyl)-7-(6-thiomorpholinopyridin-3-yl)-2-(1-(2,2,2-trifluoroethyl)piperidin-4-yl)benzo[d][1,3]dioxole-5-carboxamide
35) 7-(2-(dimethylamino)pyrimidin-5-yl)-2,4-dimethyl-N-((6-methyl-4-(methylthio)-2-oxo-1,2-dihydropyridin-3-yl)methyl)-2-(1-(2,2,2-trifluoroethyl)piperidin-4-yl)benzo[d][1,3]dioxole-5-carboxamide
36) 7-(6-methoxypyridin-3-yl)-2,4-dimethyl-N-((6-methyl-4-(methylthio)-2-oxo-1,2-dihydropyridin-3-yl)methyl)-2-(1-(2,2,2-trifluoroethyl)piperidin-4-yl)benzo[d][1,3]dioxole-5-carboxamide
37) 7-(6-(3,5-dimethylpiperidin-1-yl)pyridin-3-yl)-2,4-dimethyl-N-((6-methyl-4-(methylthio)-2-oxo-1,2-dihydropyridin-3-yl)methyl)-2-(1-(2,2,2-trifluoroethyl)piperidin-4-yl)benzo[d][1,3]dioxole-5-carboxamide
38) 7-(2-((2S,6R)-2,6-dimethylmorpholino)pyrimidin-5-yl)-2,4-dimethyl-N-((6-methyl-4-(methylthio)-2-oxo-1,2-dihydropyridin-3-yl)methyl)-2-(1-(2,2,2-trifluoroethyl)piperidin-4-yl)benzo[d][1,3]dioxole-5-carboxamide
39) 2,4-dimethyl-N-((6-methyl-4-(methylthio)-2-oxo-1,2-dihydropyridin-3-yl)methyl)-7-(2-(piperidin-1-yl)pyrimidin-5-yl)-2-(1-(2,2,2-trifluoroethyl)piperidin-4-yl)benzo[d][1,3]dioxole-5-carboxamide
40) 7-(6-((2S,6R)-2,6-dimethylmorpholino)pyridin-3-yl)-2-(1-((S)-2-hydroxypropyl)piperidin-4-yl)-2,4-dimethyl-N-((6-methyl-4-(methylthio)-2-oxo-1,2-dihydropyridin-3-yl)methyl)benzo[d][1,3]dioxole-5-carboxamide
41) 2-(1-(2,2-dimethoxyethyl)piperidin-4-yl)-7-(6-((2S,6R)-2,6-dimethylmorpholino)pyridin-3-yl)-2,4-dimethyl-N-((6-methyl-4-(methylthio)-2-oxo-1,2-dihydropyridin-3-yl)methyl)benzo[d][1,3]dioxole-5-carboxamide
42) 2-(4-(dimethylamino)cyclohexyl)-2,4-dimethyl-N-((6-methyl-4-(methylthio)-2-oxo-1,2-dihydropyridin-3-yl)methyl)-7-(thiophen-3-yl)benzo[d][1,3]dioxole-5-carboxamide
43) 2-(4-(dimethylamino)cyclohexyl)-7-(5-fluoro-2-methoxyphenyl)-2,4-dimethyl-N-((6-methyl-4-(methylthio)-2-oxo-1,2-dihydropyridin-3-yl)methyl)benzo[d][1,3]dioxole-5-carboxamide
44) Stereoisomer B of 7-(6-((2S,6R)-2,6-dimethylmorpholino)pyridin-3-yl)-2,4-dimethyl-N-((6-methyl-4-(methylthio)-2-oxo-1,2-dihydropyridin-3-yl)methyl)-2-(1-(2,2,2-trifluoroethyl)piperidin-4-yl)benzo[d][1,3]dioxole-5-carboxamide
45) Stereoisomer A of 7-(6-((2S,6R)-2,6-dimethylmorpholino)pyridin-3-yl)-2,4-dimethyl-N-((6-methyl-4-(methylthio)-2-oxo-1,2-dihydropyridin-3-yl)methyl)-2-(1-(2,2,2-trifluoroethyl)piperidin-4-yl)benzo[d][1,3]dioxole-5-carboxamide
46) 2-(1-(2,2-difluoroethyl)piperidin-4-yl)-7-(6-((2S,6R)-2,6-dimethylmorpholino)pyridin-3-yl)-2,4-dimethyl-N-((6-methyl-4-(methylthio)-2-oxo-1,2-dihydropyridin-3-yl)methyl)benzo[d][1,3]dioxole-5-carboxamide
47) Stereoisomer B of 2-(1-(2,2-difluoroethyl)piperidin-4-yl)-7-(6-((2S,6R)-2,6-dimethylmorpholino)pyridin-3-yl)-2,4-dimethyl-N-((6-methyl-4-(methylthio)-2-oxo-1,2-dihydropyridin-3-yl)methyl)benzo[d][1,3]dioxole-5-carboxamide
48) Stereoisomer A of 2-(1-(2,2-difluoroethyl)piperidin-4-yl)-7-(6-((2S,6R)-2,6-dimethylmorpholino)pyridin-3-yl)-2,4-dimethyl-N-((6-methyl-4-(methylthio)-2-oxo-1,2-dihydropyridin-3-yl)methyl)benzo[d][1,3]dioxole-5-carboxamide
49) Stereoisomer B of 7-(6-((2S,6R)-2,6-dimethylmorpholino)pyridin-3-yl)-2-(1-ethylpiperidin-4-yl)-2,4-dimethyl-N-((6-methyl-4-(methylthio)-2-oxo-1,2-dihydropyridin-3-yl)methyl)benzo[d][1,3]dioxole-5-carboxamide
50) Stereoisomer A of 7-(6-((2S,6R)-2,6-dimethylmorpholino)pyridin-3-yl)-2-(1-ethylpiperidin-4-yl)-2,4-dimethyl-N-((6-methyl-4-(methylthio)-2-oxo-1,2-dihydropyridin-3-yl)methyl)benzo[d][1,3]dioxole-5-carboxamide
51) Stereoisomer B of 7-(6-((2S,6R)-2,6-dimethylmorpholino)pyridin-3-yl)-2-(1-isopropylpiperidin-4-yl)-2,4-dimethyl-N-((6-methyl-4-(methylthio)-2-oxo-1,2-dihydropyridin-3-yl)methyl)benzo[d][1,3]dioxole-5-carboxamide
52) Stereoisomer A of 7-(6-((2S,6R)-2,6-dimethylmorpholino)pyridin-3-yl)-2-(1-isopropylpiperidin-4-yl)-2,4-dimethyl-N-((6-methyl-4-(methylthio)-2-oxo-1,2-dihydropyridin-3-yl)methyl)benzo[d][1,3]dioxole-5-carboxamide
53) Stereoisomer B of 2-(1-cyclohexylpiperidin-4-yl)-7-(6-((2S,6R)-2,6-dimethylmorpholino)pyridin-3-yl)-2,4-dimethyl-N-((6-methyl-4-(methylthio)-2-oxo-1,2-dihydropyridin-3-yl)methyl)benzo[d][1,3]dioxole-5-carboxamide
54) Stereoisomer A of 2-(1-cyclohexylpiperidin-4-yl)-7-(6-((2S,6R)-2,6-dimethylmorpholino)pyridin-3-yl)-2,4-dimethyl-N-((6-methyl-4-(methylthio)-2-oxo-1,2-dihydropyridin-3-yl)methyl)benzo[d][1,3]dioxole-5-carboxamide
55) Stereoisomer B of 7-(6-((2S,6R)-2,6-dimethylmorpholino)pyridin-3-yl)-2,4-dimethyl-N-((6-methyl-4-(methylthio)-2-oxo-1,2-dihydropyridin-3-yl)methyl)-2-(1-phenethylpiperidin-4-yl)benzo[d][1,3]dioxole-5-carboxamide
56) Stereoisomer A of 7-(6-((2S,6R)-2,6-dimethylmorpholino)pyridin-3-yl)-2,4-dimethyl-N-((6-methyl-4-(methylthio)-2-oxo-1,2-dihydropyridin-3-yl)methyl)-2-(1-phenethylpiperidin-4-yl)benzo[d][1,3]dioxole-5-carboxamide
57) Stereoisomer B of 7-(6-((2S,6R)-2,6-dimethylmorpholino)pyridin-3-yl)-2,4-dimethyl-N-((6-methyl-4-(methylthio)-2-oxo-1,2-dihydropyridin-3-yl)methyl)-2-(1-(4,4,4-trifluorobutyl)piperidin-4-yl)benzo[d][1,3]dioxole-5-carboxamide

58) Stereoisomer A of 7-(6-((2S,6R)-2,6-dimethylmorpholino)pyridin-3-yl)-2,4-dimethyl-N-((6-methyl-4-(methylthio)-2-oxo-1,2-dihydropyridin-3-yl)methyl)-2-(1-(4,4,4-trifluorobutyl)piperidin-4-yl)benzo[d][1,3]dioxole-5-carboxamide
59) Stereoisomer B of 7-(6-((2S,6R)-2,6-dimethylmorpholino)pyridin-3-yl)-2,4-dimethyl-N-((6-methyl-4-(methylthio)-2-oxo-1,2-dihydropyridin-3-yl)methyl)-2-(1-(2,2,2-trifluoroacetyl)piperidin-4-yl)benzo[d][1,3]dioxole-5-carboxamide
60) Stereoisomer A of 7-(6-((2S,6R)-2,6-dimethylmorpholino)pyridin-3-yl)-2,4-dimethyl-N-((6-methyl-4-(methylthio)-2-oxo-1,2-dihydropyridin-3-yl)methyl)-2-(1-(2,2,2-trifluoroacetyl)piperidin-4-yl)benzo[d][1,3]dioxole-5-carboxamide
61) 2,4-dimethyl-N-((6-methyl-4-(methylthio)-2-oxo-1,2-dihydropyridin-3-yl)methyl)-7-(6-morpholinopyridin-3-yl)-2-(1-(2,2,2-trifluoro)piperidin-4-yl)benzo[d][1,3]dioxole-5-carboxamide
62) Stereoisomer B of 2,4-dimethyl-N-((6-methyl-4-(methylthio)-2-oxo-1,2-dihydropyridin-3-yl)methyl)-7-(6-morpholinopyridin-3-yl)-2-(1-(2,2,2-trifluoro)piperidin-4-yl)benzo[d][1,3]dioxole-5-carboxamide
63) Stereoisomer A of 2,4-dimethyl-N-((6-methyl-4-(methylthio)-2-oxo-1,2-dihydropyridin-3-yl)methyl)-7-(6-morpholinopyridin-3-yl)-2-(1-(2,2,2-trifluoro)piperidin-4-yl)benzo[d][1,3]dioxole-5-carboxamide
64) Stereoisomer B of 2-(1-(2,2-difluoroethyl)piperidin-4-yl)-2,4-dimethyl-N-((6-methyl-4-(methylthio)-2-oxo-1,2-dihydropyridin-3-yl)methyl)-7-(6-morpholinopyridin-3-yl)benzo[d][1,3]dioxole-5-carboxamide
65) Stereoisomer A of 2-(1-(2,2-difluoroethyl)piperidin-4-yl)-2,4-dimethyl-N-((6-methyl-4-(methylthio)-2-oxo-1,2-dihydropyridin-3-yl)methyl)-7-(6-morpholinopyridin-3-yl)benzo[d][1,3]dioxole-5-carboxamide
66) Stereoisomer B of 2-(1-ethylpiperidin-4-yl)-2,4-dimethyl-N-((6-methyl-4-(methylthio)-2-oxo-1,2-dihydropyridin-3-yl)methyl)-7-(6-morpholinopyridin-3-yl)benzo[d][1,3]dioxole-5-carboxamide
67) Stereoisomer A of 2-(1-ethylpiperidin-4-yl)-2,4-dimethyl-N-((6-methyl-4-(methylthio)-2-oxo-1,2-dihydropyridin-3-yl)methyl)-7-(6-morpholinopyridin-3-yl)benzo[d][1,3]dioxole-5-carboxamide
68) 2,4-dimethyl-N-((6-methyl-4-(methylthio)-2-oxo-1,2-dihydropyridin-3-yl)methyl)-7-(2-morpholinopyrimidin-5-yl)-2-(1-(2,2,2-trifluoro)piperidin-4-yl)benzo[d][1,3]dioxole-5-carboxamide
69) Stereoisomer B of 2,4-dimethyl-N-((6-methyl-4-(methylthio)-2-oxo-1,2-dihydropyridin-3-yl)methyl)-7-(2-morpholinopyrimidin-5-yl)-2-(1-(2,2,2-trifluoro)piperidin-4-yl)benzo[d][1,3]dioxole-5-carboxamide
70) Stereoisomer A of 2,4-dimethyl-N-((6-methyl-4-(methylthio)-2-oxo-1,2-dihydropyridin-3-yl)methyl)-7-(2-morpholinopyrimidin-5-yl)-2-(1-(2,2,2-trifluoro)piperidin-4-yl)benzo[d][1,3]dioxole-5-carboxamide
71) Stereoisomer B of 2-(1-(2,2-difluoroethyl)piperidin-4-yl)-2,4-dimethyl-N-((6-methyl-4-(methylthio)-2-oxo-1,2-dihydropyridin-3-yl)methyl)-7-(2-morpholinopyrimidin-5-yl)benzo[d][1,3]dioxole-5-carboxamide
72) Stereoisomer A of 2-(1-(2,2-difluoroethyl)piperidin-4-yl)-2,4-dimethyl-N-((6-methyl-4-(methylthio)-2-oxo-1,2-dihydropyridin-3-yl)methyl)-7-(2-morpholinopyrimidin-5-yl)benzo[d][1,3]dioxole-5-carboxamide
73) Stereoisomer B of 2-(1-ethylpiperidin-4-yl)-2,4-dimethyl-N-((6-methyl-4-(methylthio)-2-oxo-1,2-dihydropyridin-3-yl)methyl)-7-(2-morpholinopyrimidin-5-yl)benzo[d][1,3]dioxole-5-carboxamide
74) Stereoisomer A of 2-(1-ethylpiperidin-4-yl)-2,4-dimethyl-N-((6-methyl-4-(methylthio)-2-oxo-1,2-dihydropyridin-3-yl)methyl)-7-(2-morpholinopyrimidin-5-yl)benzo[d][1,3]dioxole-5-carboxamide
75) 7-(2-(4-ethylpiperazin-1-yl)pyrimidin-5-yl)-2,4-dimethyl-N-((6-methyl-4-(methylthio)-2-oxo-1,2-dihydropyridin-3-yl)methyl)-2-(1-(2,2,2-trifluoroethyl)piperidin-4-yl)benzo[d][1,3]dioxole-5-carboxamide
76) Stereoisomer B of 7-(2-(4-ethylpiperazin-1-yl)pyrimidin-5-yl)-2,4-dimethyl-N-((6-methyl-4-(methylthio)-2-oxo-1,2-dihydropyridin-3-yl)methyl)-2-(1-(2,2,2-trifluoroethyl)piperidin-4-yl)benzo[d][1,3]dioxole-5-carboxamide
77) Stereoisomer A of 7-(2-(4-ethylpiperazin-1-yl)pyrimidin-5-yl)-2,4-dimethyl-N-((6-methyl-4-(methylthio)-2-oxo-1,2-dihydropyridin-3-yl)methyl)-2-(1-(2,2,2-trifluoroethyl)piperidin-4-yl)benzo[d][1,3]dioxole-5-carboxamide
78) 7-(2-(ethyl(methyl)amino)pyridin-5-yl)-2,4-dimethyl-N-((6-methyl-4-(methylthio)-2-oxo-1,2-dihydropyridin-3-yl)methyl)-2-(1-(2,2,2-trifluoroethyl)piperidin-4-yl)benzo[d][1,3]dioxole-5-carboxamide
79) Stereoisomer B of 7-(2-(ethyl(methyl)amino)pyridin-5-yl)-2,4-dimethyl-N-((6-methyl-4-(methylthio)-2-oxo-1,2-dihydropyridin-3-yl)methyl)-2-(1-(2,2,2-trifluoroethyl)piperidin-4-yl)benzo[d][1,3]dioxole-5-carboxamide
80) Stereoisomer A of 7-(2-(ethyl(methyl)amino)pyridin-5-yl)-2,4-dimethyl-N-((6-methyl-4-(methylthio)-2-oxo-1,2-dihydropyridin-3-yl)methyl)-2-(1-(2,2,2-trifluoroethyl)piperidin-4-yl)benzo[d][1,3]dioxole-5-carboxamide
81) Stereoisomer B of 2-(1-(2,2-difluoroethyl)piperidin-4-yl)-7-(2-(ethyl(methyl)amino)pyrimidin-5-yl)-2,4-dimethyl-N-((6-methyl-4-(methylthio)-2-oxo-1,2-dihydropyridin-3-yl)methyl)benzo[d][1,3]dioxole-5-carboxamide
82) Stereoisomer A of 2-(1-(2,2-difluoroethyl)piperidin-4-yl)-7-(2-(ethyl(methyl)amino)pyrimidin-5-yl)-2,4-dimethyl-N-((6-methyl-4-(methylthio)-2-oxo-1,2-dihydropyridin-3-yl)methyl)benzo[d][1,3]dioxole-5-carboxamide
83) Stereoisomer B of 7-(2-(ethyl(methyl)amino)pyrimidin-5-yl)-2-(1-ethylpiperidin-4-yl)-2,4-dimethyl-N-((6-methyl-4-(methylthio)-2-oxo-1,2-dihydropyridin-3-yl)methyl)benzo[d][1,3]dioxole-5-carboxamide

84) Stereoisomer A of 7-(2-(ethyl(methyl)amino)pyrimidin-5-yl)-2-(1-ethylpiperidin-4-yl)-2,4-dimethyl-N-((6-methyl-4-(methylthio)-2-oxo-1,2-dihydropyridin-3-yl)methyl)benzo[d][1,3]dioxole-5-carboxamide

85) 7-(6-(ethyl(methyl)amino)pyridin-3-yl)-2,4-dimethyl-N-((6-methyl-4-(methylthio)-2-oxo-1,2-dihydropyridin-3-yl)methyl)-2-(1-(2,2,2-trifluoro)piperidin-4-yl)benzo[d][1,3]dioxole-5-carboxamide

86) Stereoisomer B of 7-(6-(ethyl(methyl)amino)pyridin-3-yl)-2,4-dimethyl-N-((6-methyl-4-(methylthio)-2-oxo-1,2-dihydropyridin-3-yl)methyl)-2-(1-(2,2,2-trifluoroethyl)piperidin-4-yl)benzo[d][1,3]dioxole-5-carboxamide

87) Stereoisomer A of 7-(6-(ethyl(methyl)amino)pyridin-3-yl)-2,4-dimethyl-N-((6-methyl-4-(methylthio)-2-oxo-1,2-dihydropyridin-3-yl)methyl)-2-(1-(2,2,2-trifluoroethyl)piperidin-4-yl)benzo[d][1,3]dioxole-5-carboxamide

88) Stereoisomer B of 2-(1-(2,2-difluoroethyl)piperidin-4-yl)-7-(6-(ethyl(methyl)amino)pyridin-3-yl)-2,4-dimethyl-N-((6-methyl-4-(methylthio)-2-oxo-1,2-dihydropyridin-3-yl)methyl)benzo[d][1,3]dioxole-5-carboxamide

89) Stereoisomer B of 7-(6-(ethyl(methyl)amino)pyridin-3-yl)-2-(1-ethylpiperidin-4-yl)-2,4-dimethyl-N-((6-methyl-4-(methylthio)-2-oxo-1,2-dihydropyridin-3-yl)methyl)benzo[d][1,3]dioxole-5-carboxamide

90) 2,4-dimethyl-N-((6-methyl-4-(methylthio)-2-oxo-1,2-dihydropyridin-3-yl)methyl)-7-(4-(piperidin-1-yl)phenyl)-2-(1-(2,2,2-trifluoroethyl)piperidin-4-yl)benzo[d][1,3]dioxole-5-carboxamide

91) 2,4-dimethyl-N-((6-methyl-4-(methylthio)-2-oxo-1,2-dihydropyridin-3-yl)methyl)-7-(6-(piperidin-1-yl)pyridin-3-yl)-2-(1-(2,2,2-trifluoroethyl)piperidin-4-yl)benzo[d][1,3]dioxole-5-carboxamide

92) 2,4-dimethyl-N-((6-methyl-4-(methylthio)-2-oxo-1,2-dihydropyridin-3-yl)methyl)-7-(2-thiomorpholinopyrimidin-5-yl)-2-(1-(2,2,2-trifluoroethyl)piperidin-4-yl)benzo[d][1,3]dioxole-5-carboxamide

93) 2',2'-difluoro-2,7-dimethyl-N-((6-methyl-4-(methylthio)-2-oxo-1,2-dihydropyridin-3-yl)methyl)-2-(1-(2,2,2-trifluoroethyl)piperidin-4-yl)-[4,5'-bibenzo[d][1,3]dioxole]-6-carboxamide

94) 2,7-dimethyl-N-((6-methyl-4-(methylthio)-2-oxo-1,2-dihydropyridin-3-yl)methyl)-2-(1-(2,2,2-trifluoroethyl)piperidin-4-yl)-[4,5'-bibenzo[d][1,3]dioxole]-6-carboxamide

95) 2-(1-(2,2-difluoroethyl)piperidin-4-yl)-2,7-dimethyl-N-((6-methyl-4-(methylthio)-2-oxo-1,2-dihydropyridin-3-yl)methyl)-[4,5'-bibenzo[d][1,3]dioxole]-6-carboxamide

96) 7-(benzofuran-5-yl)-2,4-dimethyl-N-((6-methyl-4-(methylthio)-2-oxo-1,2-dihydropyridin-3-yl)methyl)-2-(1-(2,2,2-trifluoroethyl)piperidin-4-yl)benzo[d][1,3]dioxole-5-carboxamide

97) 7-(benzofuran-5-yl)-2-(1-(2,2-difluoroethyl)piperidin-4-yl)-2,4-dimethyl-N-((6-methyl-4-(methylthio)-2-oxo-1,2-dihydropyridin-3-yl)methyl)benzo[d][1,3]dioxole-5-carboxamide

98) 7-(6-(butyl(methyl)amino)pyridin-3-yl)-2,4-dimethyl-N-((6-methyl-4-(methylthio)-2-oxo-1,2-dihydropyridin-3-yl)methyl)-2-(1-(2,2,2-trifluoroethyl)piperidin-4-yl)benzo[d][1,3]dioxole-5-carboxamide

99) 7-(6-(butyl(methyl)amino)pyridin-3-yl)-2,4-dimethyl-N-((6-methyl-4-(methylthio)-2-oxo-1,2-dihydropyridin-3-yl)methyl)-2-(1-(2,2,2-trifluoroethyl)piperidin-4-yl)benzo[d][1,3]dioxole-5-carboxamide

100) 2-(trans-4-aminocyclohexyl)-7-(6-((2S,6R)-2,6-dimethylmorpholino)pyridin-3-yl)-2,4-dimethyl-N-((6-methyl-4-(methylthio)-2-oxo-1,2-dihydropyridin-3-yl)methyl)benzo[d][1,3]dioxole-5-carboxamide hydrochloride

101) 7-(6-((2S,6R)-2,6-dimethylmorpholino)pyridin-3-yl)-2-(trans-4-(3-hydroxyazetidin-1-yl)cyclohexyl)-2,4-dimethyl-N-((6-methyl-4-(methylthio)-2-oxo-1,2-dihydropyridin-3-yl)methyl)benzo[d][1,3]dioxole-5-carboxamide

102) 7-(6-((2S,6R)-2,6-dimethylmorpholino)pyridin-3-yl)-2,4-dimethyl-N-((6-methyl-4-(methylthio)-2-oxo-1,2-dihydropyridin-3-yl)methyl)-2-(trans-4-(3-(2,2,2-trifluoroethyl)azetidin-1-yl)cyclohexyl)benzo[d][1,3]dioxole-5-carboxamide

103) 2-(trans-4-(3-(2,2-difluoroethoxy)azetidin-1-yl)cyclohexyl)-7-(6-((2S,6R)-2,6-dimethylmorpholino)pyridin-3-yl)-2,4-dimethyl-N-((6-methyl-4-(methylthio)-2-oxo-1,2-dihydropyridin-3-yl)methyl)benzo[d][1,3]dioxole-5-carboxamide

104) 2-(trans-4-(3-(2,2-difluoroethoxy)azetidin-1-yl)cyclohexyl)-7-(6-((2S,6R)-2,6-dimethylmorpholino)pyridin-3-yl)-2,4-dimethyl-N-((6-methyl-4-(methylthio)-2-oxo-1,2-dihydropyridin-3-yl)methyl)benzo[d][1,3]dioxole-5-carboxamide

105) 7-(6-((2S,6R)-2,6-dimethylmorpholino)pyridin-3-yl)-2-(trans-4-(3-isopropoxyazetidin-1-yl)cyclohexyl)-2,4-dimethyl-N-((6-methyl-4-(methylthio)-2-oxo-1,2-dihydropyridin-3-yl)methyl)benzo[d][1,3]dioxole-5-carboxamide

106) 7-(6-((2S,6R)-2,6-dimethylmorpholino)pyridin-3-yl)-2-(trans-4-(3-methoxyazetidin-1-yl)cyclohexyl)-2,4-dimethyl-N-((6-methyl-4-(methylthio)-2-oxo-1,2-dihydropyridin-3-yl)methyl)benzo[d][1,3]dioxole-5-carboxamide

107) Stereoisomer B of 7-(6-((2S,6R)-2,6-dimethylmorpholino)pyridin-3-yl)-2,4-dimethyl-2-(trans-4-(methyl(2,2,2-trifluoroethyl)amino)cyclohexyl)-N-((6-methyl-4-(methylthio)-2-oxo-1,2-dihydropyridin-3-yl)methyl)benzo[d][1,3]dioxole-5-carboxamide

108) Stereoisomer A of 7-(6-((2S,6R)-2,6-dimethylmorpholino)pyridin-3-yl)-2,4-dimethyl-2-(trans-4-(methyl(2,2,2-trifluoroethyl)amino)cyclohexyl)-N-((6-methyl-4-(methylthio)-2-oxo-1,2-dihydropyridin-3-yl)methyl)benzo[d][1,3]dioxole-5-carboxamide

109) 2-(trans-4-((2,2-difluoroethyl(methyl)amino)cyclohexyl)-7-(6-((2S,6R)-2,6-dimethylmorpholino)pyridin-3-yl)-2,4-dimethyl-N-((6-methyl-4-(methylthio)-2-oxo-1,2-dihydropyridin-3-yl)methyl)benzo[d][1,3]dioxole-5-carboxamide

110) 7-(6-((2S,6R)-2,6-dimethylmorpholino)pyridin-3-yl)-2-(trans-4-(isopropyl(methyl)amino)cyclohexyl)-2,4-dimethyl-N-((6-methyl-4-(methylthio)-2-oxo-1,2-dihydropyridin-3-yl)methyl)benzo[d][1,3]dioxole-5-carboxamide

111) 7-(6-((2S,6R)-2,6-dimethylmorpholino)pyridin-3-yl)-2-(trans-4-(ethyl(methyl)amino)cyclohexyl)-2,4-dimethyl-N-((6-methyl-4-(methylthio)-2-oxo-1,2-dihydropyridin-3-yl)methyl)benzo[d][1,3]dioxole-5-carboxamide

112) 7-(6-((2S,6R)-2,6-dimethylmorpholino)pyridin-3-yl)-2,4-dimethyl-2-(trans-4-(methyl(phenethyl)amino)cyclohexyl)-N-((6-methyl-4-(methylthio)-2-oxo-1,2-dihydropyridin-3-yl)methyl)benzo[d][1,3]dioxole-5-carboxamide

113) 7-(6-((2S,6R)-2,6-dimethylmorpholino)pyridin-3-yl)-2,4-dimethyl-2-(trans-4-(methyl(phenethyl)amino)cyclohexyl)-N-((6-methyl-4-(methylthio)-2-oxo-1,2-dihydropyridin-3-yl)methyl)benzo[d][1,3]dioxole-5-carboxamide

114) 2-(trans-4-(cyclohexyl(methyl)amino)cyclohexyl)-7-(6-((2S,6R)-2,6-dimethylmorpholino)pyridin-3-yl)-2,4-dimethyl-N-((6-methyl-4-(methylthio)-2-oxo-1,2-dihydropyridin-3-yl)methyl)benzo[d][1,3]dioxole-5-carboxamide

115) 7-(6-((2S,6R)-2,6-dimethylmorpholino)pyridin-3-yl)-2,4-dimethyl-N-((6-methyl-4-(methylthio)-2-oxo-1,2-dihydropyridin-3-yl)methyl)-2-(trans-4-(2,2,2-trifluoro-N-methylacetamido)cyclohexyl)benzo[d][1,3]dioxole-5-carboxamide.

**6.** The pharmaceutical composition of claim 1 or 5, wherein the solid cancer is at least one selected from rhabdoid tumor, melanoma, pancreatic cancer, colorectal cancer, adrenal cancer, prostate cancer, bladder cancer, lung cancer, breast cancer, ovarian cancer, liver cancer, brain cancer, stomach cancer, renal cancer, thyroid cancer, epithelioid sarcoma, and synovial sarcoma.

**7.** A method for preventing or treating EZH1 or EZH2 activity-associated diseases, the method comprising administering the compound according to any one of claims 1 to 5, the stereoisomer thereof, the pharmaceutically acceptable salt thereof, the hydrate or solvate thereof into an individual, wherein the EZH1 or EZH2 activity-associated disease is a solid cancer.

**8.** A use of the compound according to any one of claims 1 to 5, the stereoisomer thereof, the pharmaceutically acceptable salt thereof, the hydrate or solvate thereof for preventing or treating EZH1 or EZH2 activity-associated diseases, wherein the EZH1 or EZH2 activity-associated disease is a solid cancer.

**9.** A use of the compound according to any one of claims 1 to 5, the stereoisomer thereof, the pharmaceutically acceptable salt thereof, the hydrate or solvate thereof in the preparation of a medicament for preventing or treating EZH1 or EZH2 activity-associated diseases, wherein the EZH1 or EZH2 activity-associated disease is a solid cancer.

**INTERNATIONAL SEARCH REPORT**

International application No.
**PCT/IB2024/056539**

**A. CLASSIFICATION OF SUBJECT MATTER**

**A61K 31/5377**(2006.01)i; **A61K 31/444**(2006.01)i; **A61K 31/506**(2006.01)i; **A61P 35/00**(2006.01)i

According to International Patent Classification (IPC) or to both national classification and IPC

**B. FIELDS SEARCHED**

Minimum documentation searched (classification system followed by classification symbols)

A61K 31/5377(2006.01); A61K 31/443(2006.01); A61K 31/4545(2006.01); A61K 33/243(2019.01); C07D 405/12(2006.01); C07D 405/14(2006.01); C07D 413/14(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal), STN (Registry, Caplus), Google & keywords: EZH 1 (Enhancer of zeste homolog 1), EZH 2 (Enhancer of zeste homolog 2), 고형암 (solid cancer)

**C. DOCUMENTS CONSIDERED TO BE RELEVANT**

| Category* | Citation of document, with indication, where appropriate, of the relevant passages | Relevant to claim No. |
|---|---|---|
| X | WO 2021-180235 A1 (SICHUAN HAISCO PHARMACEUTICAL CO., LTD.) 16 September 2021. See abstract; claims 1-34; and page 2. | 1-6,8,9 |
| X | KR 10-2021-0003160 A (CONSTELLATION PHARMACEUTICALS, INC.) 11 January 2021 (2021-01-11) See claims 1-23; paragraph [0008]; and table 5. | 1,6,8,9 |
| X | KR 10-2022-0041130 A (CONSTELLATION PHARMACEUTICALS, INC.) 31 March 2022 (2022-03-31) See claims 1-21. | 1,6,8,9 |
| X | CN 116082320 A (HAISCO PHARMACEUTICAL GROUP CO., LTD.) 09 May 2023 (2023-05-09) See claims 1-21; and paragraphs [0003]-[0005]. | 1-6,8,9 |
| PX | KR 10-2023-0110210 A (DONG WHA PHARM. CO., LTD.) 21 July 2023 (2023-07-21) See claims 1-8. | 1-6,8,9 |

☐ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **07 October 2024** | **07 October 2024** |

| Name and mailing address of the ISA/KR | Authorized officer |
|---|---|
| **Korean Intellectual Property Office**<br>**Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2022)

**INTERNATIONAL SEARCH REPORT**

International application No.
**PCT/IB2024/056539**

**Box No. II Observations where certain claims were found unsearchable (Continuation of item 2 of first sheet)**

This international search report has not been established in respect of certain claims under Article 17(2)(a) for the following reasons:

1. ☑ Claims Nos.: **7**
because they relate to subject matter not required to be searched by this Authority, namely:
Claim 7 pertains to a method for treatment of the human body by surgery or therapy (PCT Article 17(2)(a)(i) and PCT Rule 39.1(iv)).

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

Form PCT/ISA/210 (continuation of first sheet) (July 2022)

**INTERNATIONAL SEARCH REPORT**
**Information on patent family members**

International application No.
**PCT/IB2024/056539**

| Patent document cited in search report | Publication date (day/month/year) | Patent family member(s) | Publication date (day/month/year) |
|---|---|---|---|
| WO 2021-180235 A1 | 16 September 2021 | CN 115175905 A | 11 October 2022 |
| | | TW 202144341 A | 01 December 2021 |
| | | US 2023-0365541 A1 | 16 November 2023 |
| KR 10-2021-0003160 A | 11 January 2021 | AR 114793 A1 | 14 October 2020 |
| | | AU 2019-255310 A1 | 15 October 2020 |
| | | AU 2019-255310 B2 | 24 November 2022 |
| | | BR 112020021194 A2 | 23 March 2021 |
| | | CA 3098428 A1 | 24 October 2019 |
| | | CL 2020002698 A1 | 15 January 2021 |
| | | CN 111989325 A | 24 November 2020 |
| | | CO 2020014217 A2 | 08 March 2021 |
| | | CR 20200553 A | 08 April 2021 |
| | | DK 3781561 T3 | 17 June 2024 |
| | | EA 202092490 A1 | 23 December 2020 |
| | | EP 3781561 A1 | 24 February 2021 |
| | | EP 3781561 B1 | 13 March 2024 |
| | | EP 4421072 A1 | 28 August 2024 |
| | | FI 3781561 T3 | 13 June 2024 |
| | | IL 278013 A | 30 November 2020 |
| | | JP 2021-522166 A | 30 August 2021 |
| | | JP 2023-082132 A | 13 June 2023 |
| | | JP 7256823 B2 | 12 April 2023 |
| | | MA 52288 A | 07 April 2021 |
| | | MX 2020010999 A | 20 January 2021 |
| | | PE 20201448 A1 | 10 December 2020 |
| | | PH 12020551578 A1 | 13 September 2021 |
| | | SG 11202009438 A | 27 November 2020 |
| | | TW 202003505 A | 16 January 2020 |
| | | TW I828677 B | 11 January 2024 |
| | | UA 127357 C2 | 26 July 2023 |
| | | US 10689371 B2 | 23 June 2020 |
| | | US 11274095 B2 | 15 March 2022 |
| | | US 2019-0322651 A1 | 24 October 2019 |
| | | US 2020-0317651 A1 | 08 October 2020 |
| | | US 2024-0116905 A1 | 11 April 2024 |
| | | WO 2019-204490 A1 | 24 October 2019 |
| KR 10-2022-0041130 A | 31 March 2022 | AU 2020-316072 A1 | 24 February 2022 |
| | | BR 112022001154 A2 | 07 June 2022 |
| | | CA 3148444 A1 | 28 January 2021 |
| | | CL 2022000176 A1 | 20 September 2022 |
| | | CN 114423426 A | 29 April 2022 |
| | | CN 114423426 B | 05 April 2024 |
| | | CN 118236503 A | 25 June 2024 |
| | | CO 2022001480 A2 | 18 March 2022 |
| | | EP 4003343 A1 | 01 June 2022 |
| | | IL 290011 A | 01 March 2022 |
| | | JP 2022-541633 A | 26 September 2022 |
| | | JP 7530420 B2 | 07 August 2024 |
| | | MX 2022000933 A | 06 May 2022 |
| | | PE 20230253 A1 | 07 February 2023 |

Form PCT/ISA/210 (patent family annex) (July 2022)

**INTERNATIONAL SEARCH REPORT**
**Information on patent family members**

International application No.
**PCT/IB2024/056539**

| Patent document cited in search report | Publication date (day/month/year) | Patent family member(s) | Publication date (day/month/year) |
|---|---|---|---|
| | | US 2022-0257577 A1 | 18 August 2022 |
| | | WO 2021-016409 A1 | 28 January 2021 |
| CN 116082320 A | 09 May 2023 | None | |
| KR 10-2023-0110210 A | 21 July 2023 | WO 2023-135564 A1 | 20 July 2023 |

Form PCT/ISA/210 (patent family annex) (July 2022)